# EUROPEAN PATENT SPECIFICATION

(11) **EP 3 921 419 B1**
(45) Date of publication and mention of the grant of the patent: **08.04.2026**
(21) Application number: 20752090.9
(22) Date of filing: 06.02.2020
(51) Int. Cl.: A61K 35/17, C12N 15/113, A61P 35/00, C12N 5/0783

(54) **SIRT2-ABLATED CHIMERIC T CELLS**
SIRT2-ABLATIERTE CHIMÄRE T-ZELLEN
LYMPHOCYTES T CHIMÉRIQUES AYANT SUBI UNE ABLATION DE SIRT2

(30) Priority: 08.02.2019 US 201962803101 P; 05.06.2019 US 201962857586 P
(43) Date of publication of application: 15.12.2021
(73) Proprietor: H. Lee Moffitt Cancer Center And Research Institute, Inc., Tampa, FL 33612-9497 (US)
(72) Inventor: KIM, Sungjune, Tampa, Florida 33647 (US)
(74) Representative: Bryers Intellectual Property Ltd
(86) International application number: PCT/US2020/016963
(87) International publication number: WO 2020/163569

(56) References cited:
- US-A1- 2009 259 044
- US-A1- 2011 268 754
- GOMES P ET AL: "Emerging Role of Sirtuin 2 in the Regulation of Mammalian Metabolism", TRENDS IN PHARMACOLOGICAL SCIENCES, vol. 36, no. 11, November 2015 (2015-11-01), pages 756 - 768, XP093029652, ISSN: 0165-6147, DOI: 10.1016/j.tips.2015.08.001
- GEUKES FOPPEN M H ET AL: "Tumor-infiltrating lymphocytes for the treatment of metastatic cancer", MOLECULAR ONCOLOGY, vol. 9, no. 10, 30 October 2015 (2015-10-30), pages 1918 - 1935, XP055539330, ISSN: 1574-7891, DOI: 10.1016/j.molonc.2015.10.018
- HAMAIDI I ET AL: "Sirt2 Inhibition Enhances Metabolic Fitness and Effector Functions of Tumor-Reactive T Cells", CELL METABOLISM, vol. 32, no. 3, 1 September 2020 (2020-09-01), pages 420 - 436.e12, XP093029650, ISSN: 1550-4131, DOI: 10.1016/j.cmet.2020.07.008
- MAYURI GOGOI, CHANDRA KASTURI, SARIKHANI MOHSEN, RAMANI RAMYA, SUNDARESAN NAGALINGAM RAVI, CHAKRAVORTTY DIPSHIKHA: "Salmonella escapes adaptive immune response via SIRT2 mediated modulation of innate immune response in dendritic cells", PLOS PATHOGENS, vol. 14, no. 11, 19 November 2018 (2018-11-19), pages 1 - 22, XP055730712
- MACLEOD ET AL.: "Integration of a CD 19 CAR into the TCR Alpha Chain Locus Streamlines Production of Allogeneic Gene -Edited CAR T Cells", MOLECULAR THERAPY, vol. 25, no. 4, 5 April 2017 (2017-04-05), pages 949 - 961, XP055397282, DOI: 10.1016/j.ymthe.2017.02.005

## Description

### BACKGROUND

Immunotherapy targeting immune checkpoints within the immune synapse has become a paradigm-shifting approach showing unprecedented success (Pardoll, D.M., Nat Rev Cancer, 2012. 12(4):252-64). Despite such breakthrough, many cancers remain incurable and alternative strategies to enhance tumor immunity are of pivotal importance (Wei, S.C., et al. Cancer Discov, 2018. 8(9):1069-1086; Gide, T.N., et al. Clin Cancer Res, 2018. 24(6):1260-1270).

Autologous T cell therapy is described, e.g., in US2011/268754A1. There is growing consensus that metabolic fitness of immune cells is crucial for theirs effector functions and an altered metabolic framework contributes to immune escape particularly within the metabolically challenging tumor microenvironment (Chang, C.H., et al. Cell, 2015. 162(6):1229-41; O'Neill, L.A., et al. Nat Rev Immunol, 2016. 16(9):553-65; Shevchenko, I., et al. Front Immunol, 2018. 9:1816). Thereby, metabolic manipulation of T cells to optimally compete with cancer cells is an attractive strategy to stimulate a productive anti-tumor response.

### SUMMARY

According to the present invention, there is provided a method comprising treating lymphocytes collected from a subject with cancer with a Sirt2 inhibitor or genetically modifying the lymphocytes to inhibit or ablate Sirt2 expression.

In some embodiments, the Sirt2 inhibitor is AGK2, AK-1, SirReal2, Tenovin-6, Thiomyristoyl, AEM1, or AEM2.

In some embodiments, the Sirt2 inhibitor is an siRNA, antisense, or gRNA oligonucleotide.

In some embodiments, the lymphocytes are genetically modified by inserting a chimeric receptor into the genome of the cell at a location that disrupts expression or activity of an endogenous Sirt2 protein. Optionally, the chimeric receptor is a chimeric antigen receptor (CAR) polypeptide.

In some embodiments, the lymphocytes are tumor infiltrating lymphocytes (TILs) (Geukes Foppen, M. H., et al., Molecular Oncology, 2015, 9(10), 1918-35, 30 Oct. 2015).

Naive T cells are metabolically quiescent and rely on oxidative phosphorylation (OXPHOS), a highly efficient pathway, for generating ATP from glucose (Chang, C.H., et al. Cell, 2013. 153(6):1239-51). Upon activation, a switch to aerobic glycolysis takes place despite oxygen availability; a phenomenon referred as Warburg effect (Warburg, O., Science, 1956. 124(3215):269-70). Activated T cells undergo a dramatic metabolic reprogramming to sustain their increased energetic demands of proliferation and effector functions. Glycolysis appears very inefficient, due to the low ATP/glucose ratio; however it allows rapid production of ATP and yields a higher number of building blocks for anabolism (Lunt, S.Y., et al. Annu Rev Cell Dev Biol, 2011. 27:441-64). Consistent with the metabolism of non-proliferative cells, T memory cells (T_{M}) return to quiescent OXPHOS metabolism (Pearce, E.L., et al. Science, 2013. 342(6155):1242454).

Histone deacetylases (HDAC) and histone acetyl transferase (HAT) are key epigenetic regulators of multiple biological processes (Seto, E., et al. Cold Spring Harb Perspect Biol, 2014. 6(4):a018713). Prior studies have demonstrated a direct effect of HDAC in immune response and a potential immunomodulatory activity of HDAC inhibitors (Dubovsky, J.A., et al. Curr Pharm Des, 2010. 16(3):268-76; Ellmeier, W., et al. Nat Rev Immunol, 2018. 18(10):617-634). More recently, protein acetylation has emerged as a key post-translational modification in cell metabolism with virtually every enzyme of glycolysis and tricarboxylic acid (TCA) cycle is acetylated (Zhao, S., et al. Science, 2010. 327(5968):1000-4), but its relevance in the immune system metabolism has not been previously explored.

HDAC Class III, also termed Sirtuins, are nicotinamide adenine dinucleotide (NAD⁺)-dependent protein deacetylases. Sirtuin family consists of seven members, which vary in subcellular localizations and substrates (Guarente, L., et al. N Engl J Med, 2011. 364(23):2235-44; Houtkooper, R.H., et al. Nat Rev Mol Cell Biol, 2012. 13(4):225-238). Sirt2 is the predominantly cytosolic member, but can transiently migrate to the nuclei during mitosis to deacetylate histone H4 (Vaquero, A., et al. Genes Dev, 2006. 20(10):1256-61). Sirt2 is involved in the anti-aging process and is induced in response to caloric restriction and oxidative stress (Gomes, P., et al. Trends Pharmacol Sci, 2015. 36(11):756-768). Sirt2 stabilizes the gluconeogenic enzyme phosphoenolpyruvate carboxykinase (PEPCK) and promotes gluconeogenesis (Jiang, W., et al. Mol Cell, 2011. 43(1):33-44).

Immunologically, Sirt2 has been associated with anti-inflammatory phenotype through its negative regulation of p65, NF-κB subunit (Rothgiesser, K.M., et al. J Cell Sci, 2010. 123(Pt 24):4251-8). In fact, Sirt2 knockout mice were more susceptible to TNF-α-induced systemic inflammatory response and developed severe forms of dextran sodium sulfate (DSS)-induced colitis via pro-inflammatory macrophage polarization (Lo Sasso, G., et al. PLoS One, 2014. 9(7):e103573; Newton, K., et al. Nature, 2014. 506(7489):E4-6). However, the role of Sirt2 in T cell homeostasis and tumor immunity remains elusive. Given that Sirt2 is associated with an anti-inflammatory effect; it was speculated that Sirt2 inhibition may have a protective effect against cancer challenge by promoting tumor-specific immune response.

As disclosed herein, Sirt2 deficiency in mice leads to improved tumor rejection by T cells. Surprisingly, glycolysis is a key target of Sirt2 in T cells. Mechanistically, Sirt2 deficiency was found to metabolically reprogram T cells to amplify aerobic glycolysis during activation, which ultimately led to hyper-reactive T cell effector functions against tumor.

Therefore, disclosed herein are lymphocytes, such as tumor infiltrating lymphocyts (TILs), for use in adoptive cell transfer that have chemically- or genetically-inhibited Sirt2 expression. Also disclosed are methods of inhibiting or ablating Sirt2 expression in lymphocytes, such asTILs, ex vivo and methods of using these cells to treat subjects with cancer.

In some cases, the lymphocytes are treated ex vivo with an effective amount of a Sirt2 inhibitor to reduce or ablate Sirt2 expression or activity. Chemical Sirt2 inhibitors are known in the art and include AGK2, AK-1, SirReal2, Tenovin-6, and Thiomyristoyl (TM), AEM1, and AEM2. In some embodiments, the Sirt2 inhibitor is an oligonucleotide, such as an antisense oligonucleotide, siRNA, or gRNA. In some cases the Sirt2 inhibitor is an aptamer or an antibody specific to the *Sirt2* gene.

In some cases, the lymphocytes are genetically engineered ex vivo to inhibit or ablate Sirt2 expression. Methods for genetically manipulating gene expression and activity are known in the art and include gene editing and DNA recombination.

In some cases, the lymphocytes also expresses a chimeric receptor. In some embodiments, the chimeric receptor comprises a chimeric antigen receptor (CAR) polypeptide. CARs generally combine an antigen recognition domain with transmembrane signaling motifs involved in lymphocyte activation. The antigen recognition domain can be, for example, the single-chain variable fragments (scFv) of a monoclonal antibody (mAb) or a fragment of a natural ligand that binds a target receptor. CARs are generally made up of three domains: an ectodomain, a transmembrane domain, and an endodomain. The ectodomain comprises the antigen recognition domain. It also optionally contains a signal peptide (SP) so that the CAR can be glycosylated and anchored in the cell membrane of the immune effector cell. The transmembrane domain (TD), is as its name suggests, connects the ectodomain to the endodomain and resides within the cell membrane when expressed by a cell. The endodomain is the business end of the CAR that transmits an activation signal to the immune effector cell after antigen recognition. For example, the endodomain can contain an intracellular signaling domain (ISD) and optionally a co-stimulatory signaling region (CSR).

In some cases, the *Sirt2* gene is disrupted by insertion of the gene encoding the chimeric receptor into the *Sirt2* gene loci of the cell. Therefore, disclosed herein is a chimeric cell expressing a chimeric receptor, wherein the chimeric receptor is encoded by a transgene, and wherein the transgene is inserted in the genome of the cell at a location that disrupts expression or activity of an endogenous Sirt2 protein. Site-specific insertion of the transgene can be done, for example, by gene editing techniques, such as CRISPR or TALEN. In some cases, two different gene editing systems are used: one for integration of the transgene, and another one for effective ablation of all *Sirt2* variants (for instance, with a target at exon 10, common to all of them).

Also disclosed are isolated nucleic acid sequences encoding the disclosed polypeptides, vectors comprising these isolated nucleic acids, and cells containing these vectors. The lymphocytes disclosed herein can be an immune effector cell selected from the group consisting of an alpha-beta T cells, a gamma-delta T cell, a Natural Killer (NK) cells, a Natural Killer T (NKT) cell, a B cell, an innate lymphoid cell (ILC), a cytokine induced killer (CIK) cell, a cytotoxic T lymphocyte (CTL), a lymphokine activated killer (LAK) cell, and a regulatory T cell. In some embodiments, the lymphocytes are TILs.

Also disclosed is a method of providing an anti-cancer immunity in a subject, comprising administering to the subject an effective amount of a chimeric cell disclosed herein, thereby providing an anti-tumor immunity in the subject. Therefore, disclosed are methods of treating cancer in a subject that involves collecting lymphocytes, such as tumor infiltrating lymphocytes (TILs), from the subject, treating the lymphocytes ex vivo to inhibit Sirt2 expression, and transferring the modified lymphocytes back to the subject.

The details of one or more embodiments of the invention are set forth in the accompanying drawings and the description below. Other features, objects, and advantages of the invention will be apparent from the description and drawings, and from the claims.

### DESCRIPTION OF DRAWINGS

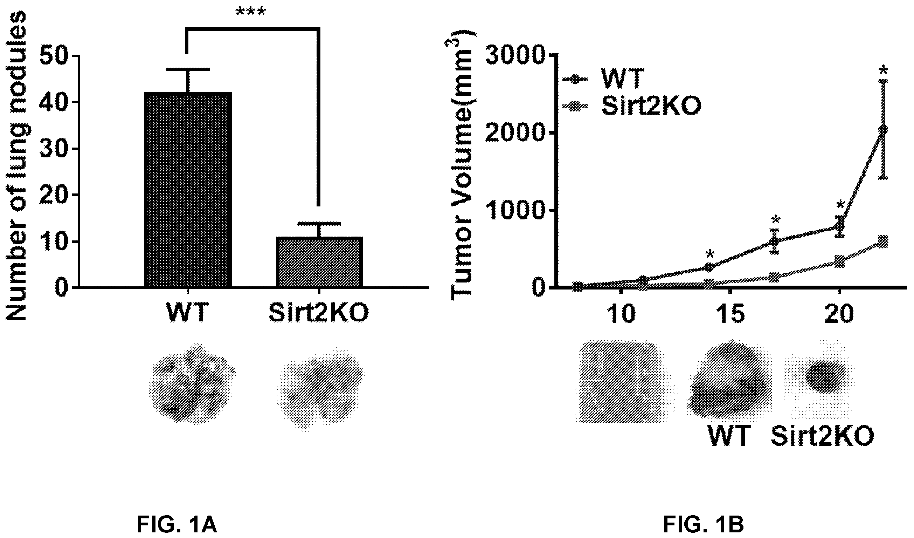

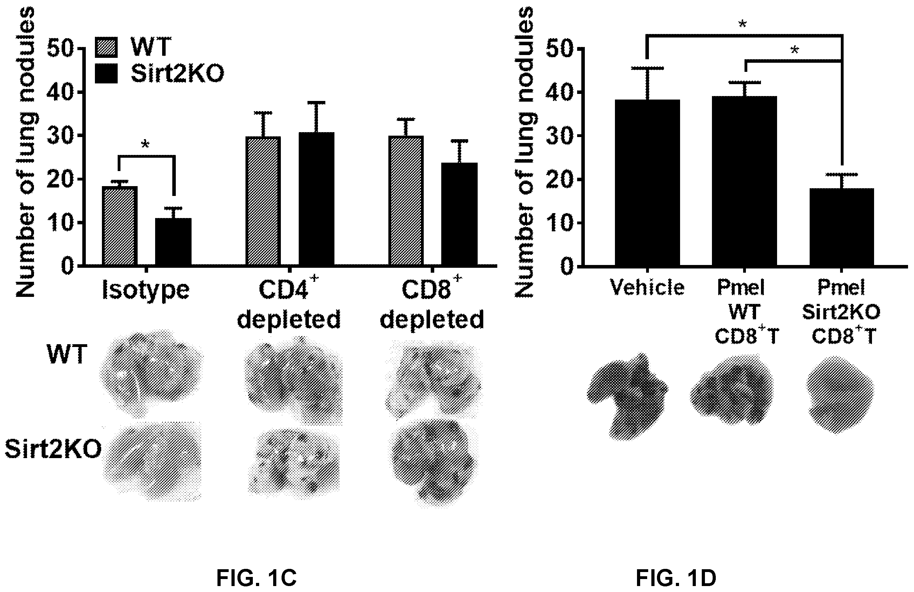

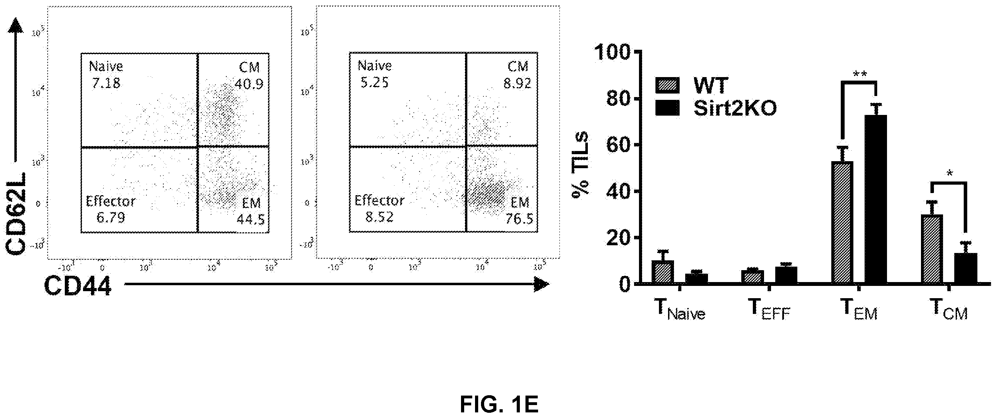

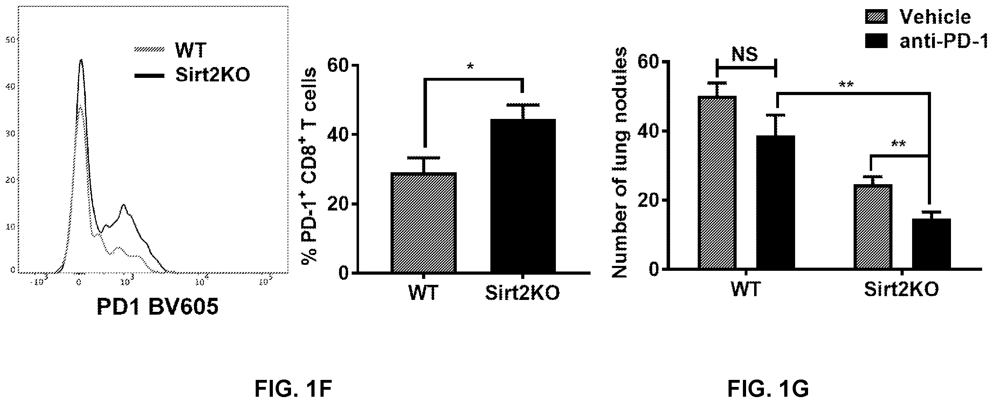

FIGs. 1A to 1G show Sirt2 deficiency enhances T cell mediated tumor rejection in vivo. FIG. 1A shows the number of lung metastatic nodules in WT and Sirt2KO mice challenged i.v. with B16F10 cells (n = 5). FIG. 1B shows subcutaneous B16F10 tumor growth curves of WT and Sirt2KO mice (n = 5). FIG. 1C shows number of lung metastatic nodules in WT and Sirt2KO mice treated with isotype control, anti-CD4+, or anti-CD8+ monoclonal antibodies before B16F10 i.v. challenge (n = 5). FIG. 1D shows CD8+ T cells isolated from spleens of WT Pmel and Sirt2KO Pmel mice were adoptively transferred into NSG mice and challenged i.v. with B16F10 cells. The number of metastatic nodules in the lungs was determined (n = 8). FIGs. 1E to 1F show TILs were isolated from subcutaneous tumors from WT and Sirt2KO mice. FIG. 1E show naive, effector (TEFF), effector memory (TEM) and central memory (TCM) CD8+ T cell subsets were analyzed by CD44 and CD62L expression. Numbers in quadrants indicate percentage of cells. Right, frequency of each subset among TILs (n = 3-4). FIG. 1F shows characterization of PD-1 expression in WT and Sirt2KO TILs. Right, Frequency of PD1+ CD8+ TILs is shown (n = 3-4). FIG. 1G shows number of lung metastatic nodules in WT and Sirt2KO mice challenged i.v. with B16F10 cells, and treated with either vehicle or anti-PD-1 mAb (n = 6). Data are representative of one (FIG. 1F), two (FIG. 1C-1E), three (FIG. 1B) and four (FIG. 1A) independent experiments. Data are mean ± s.e.m. P values are determined by two-tailed Student's t-test (FIGs. 1A-1C, 1F), one-way ANOVA (FIG. 1D) or two-way ANOVA (FIG. 1E). NS, not significant,*P < 0.05, **P < 0.01, ***P < 0.001.

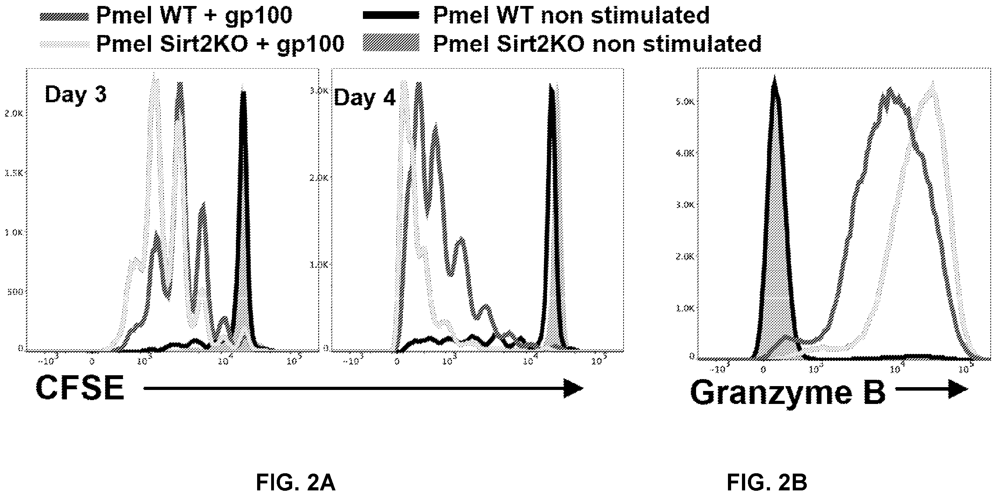

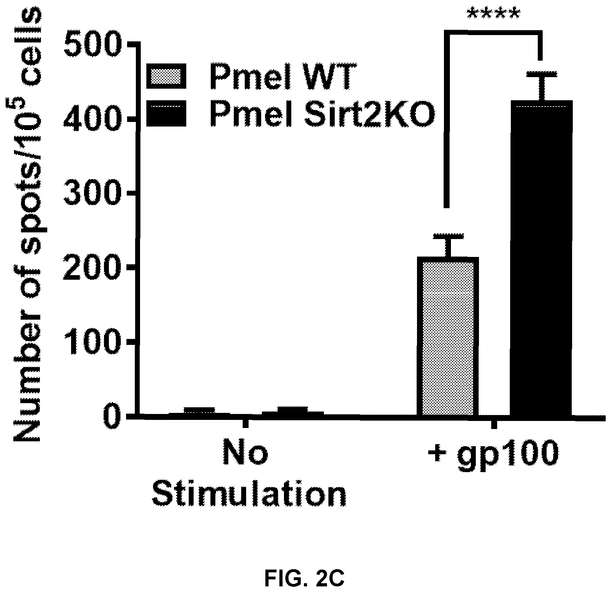

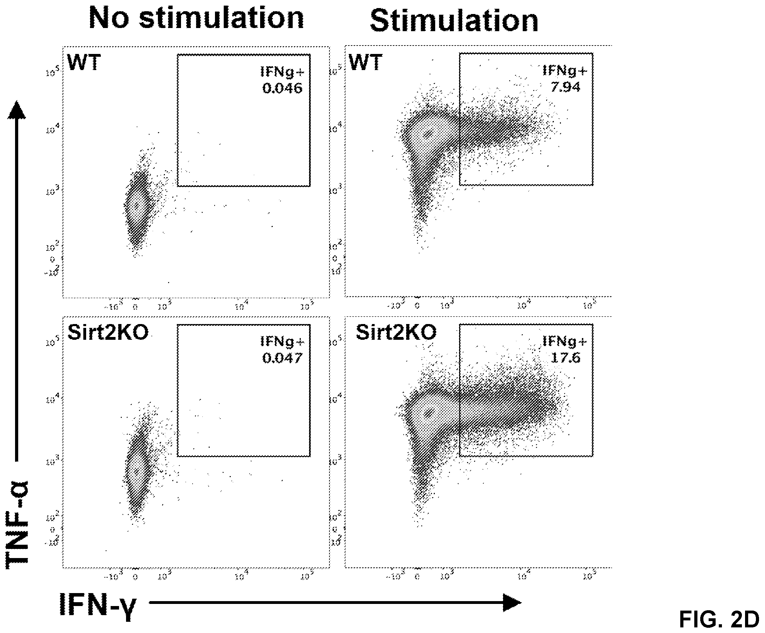

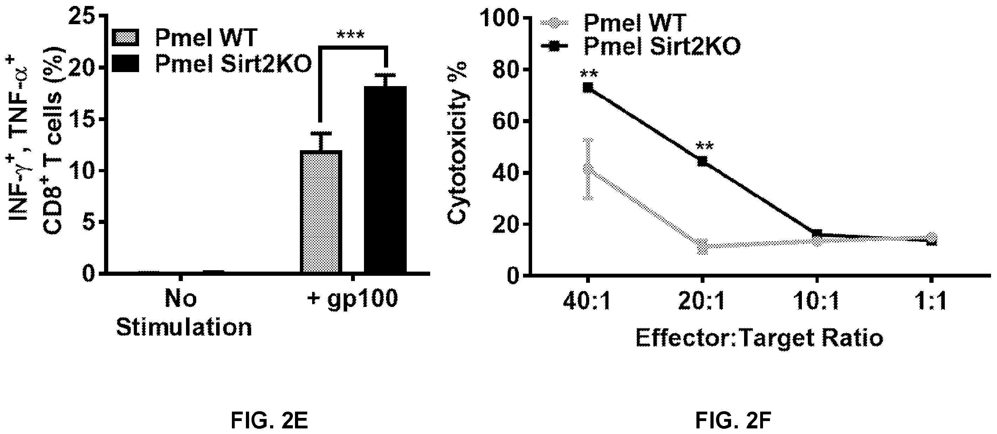

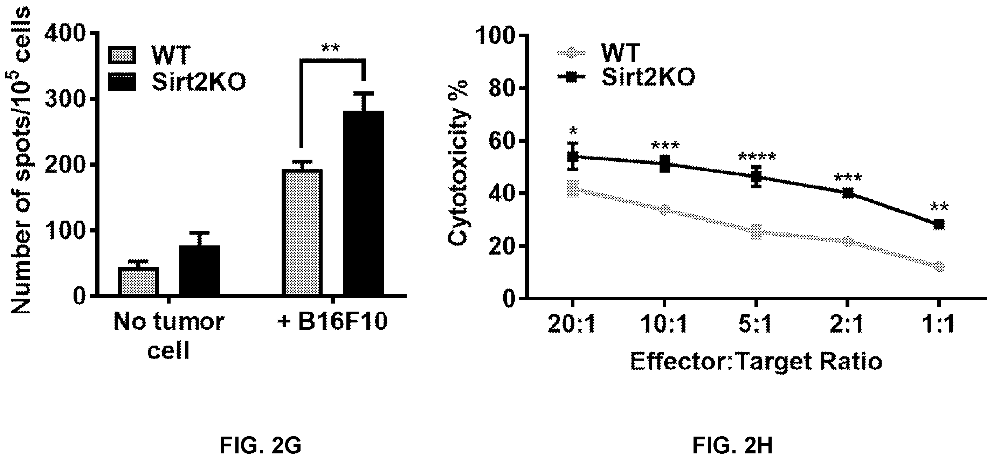

FIGs. 2A to 2H show Sirt2KO T cells are hyper-reactive to antigenic stimulation ex vivo. FIG. 2A shows CFSE-labeled WT and Sirt2KO Pmel splenocytes were stimulated or not stimulated with gp100 and the proliferation was measured by CFSE dilution 3 and 4 days post-activation. (n = 7). FIGs. 2B to 2F show functional characterization on WT and Sirt2KO Pmel splenocytes stimulated or not stimulated with gp100 for 48 hours. FIG. 2B shows flow cytometric analysis of intracellular granzyme B on WT and Sirt2KO Pmel CD8+ T cells (n = 7). FIG. 2C shows IFN-γ ELISPOT assay for 48 hours (n = 4). FIG. 2D shows flow cytometric analysis of cytokines TNF-α and INF-γ expression in WT and Sirt2KO Pmel CD8+ T cells. Numbers in quadrants indicate percentage of cells. FIG. 2E shows frequencies of TNF-α+ IFN-γ+ CD8+ Pmel T cells are shown (n = 7). FIG. 2F shows cytotoxic activities were determined by LDH release assay on gp100-stimulated WT and Sirt2KO CD8+ T cells after co-cultured on B16F10 targets at the indicated Effector:Target ratios (n = 3). FIGs. 2G to 2H show functional characterization on CD3+ TILs isolated from B16F10 tumor nodules from WT or Sirt2KO mice. FIG. 2G shows IFN-γ Elispot assay was performed on CD3+ TILs after re-challenge with vehicle or irradiated B16F10 cells ex vivo (n = 3). FIG. 2H shows cytotoxic activities were determined by LDH release assay on CD3+ TILs after co-culture with B16F10 at the indicated Effector:Target ratios (n = 3). Data are representative of two (FIGs. 2G-2H), three (FIGs. 2A-2E) and five (FIG. 2F) independent experiments. Data are mean ± s.e.m. P values are determined by two-way ANOVA (FIG. 2C, 2E-2H). *P < 0.05, **P < 0.01, ***P < 0.001, ****P < 0.0001.

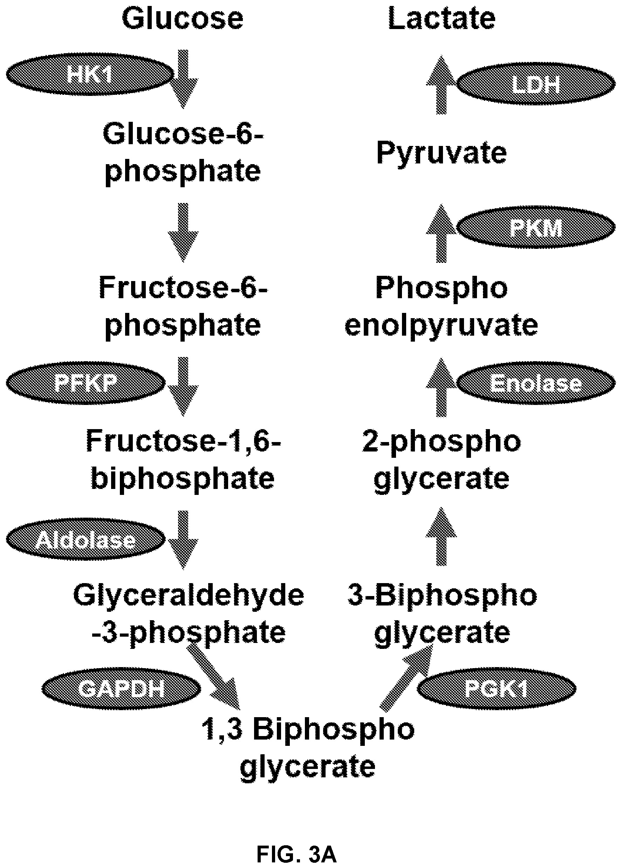

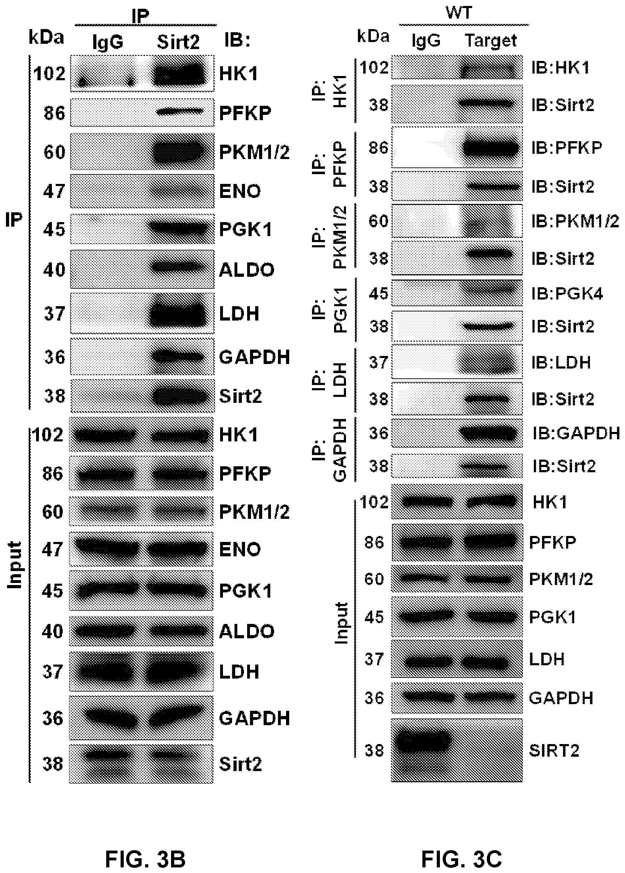

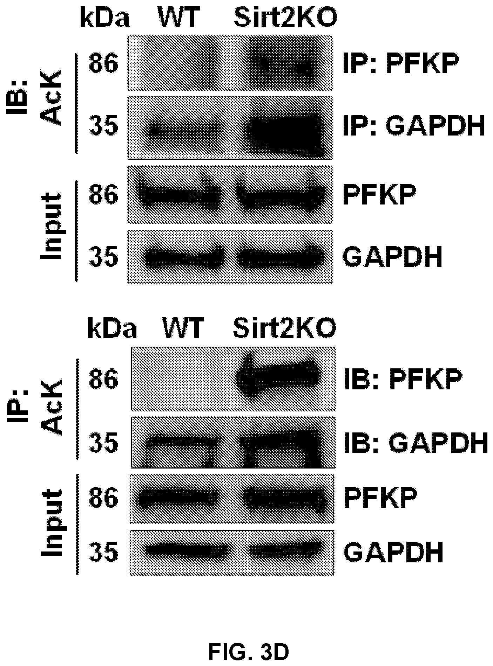

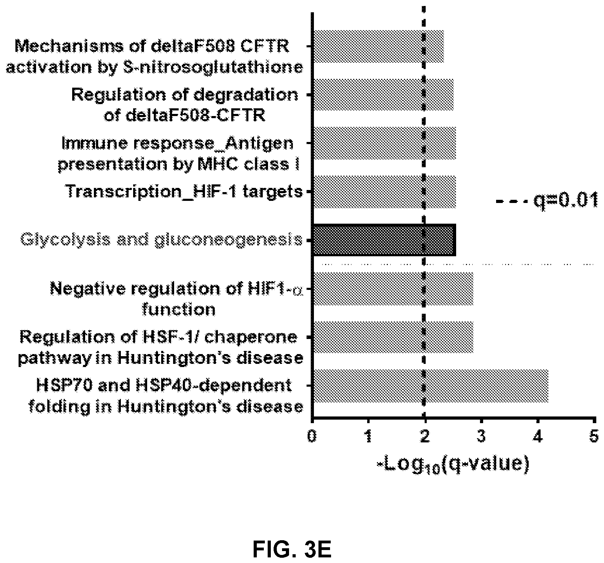

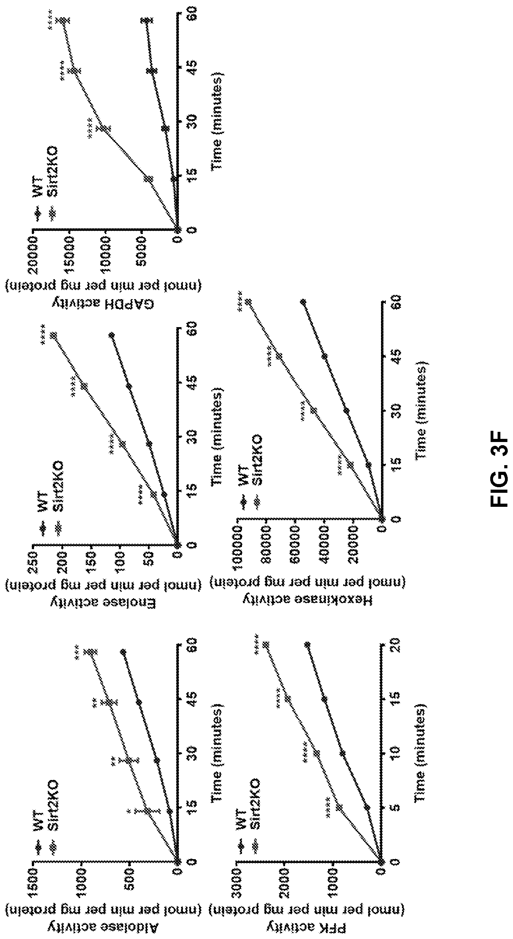

FIGs. 3A to 3F show Sirt2 directly interacts with glycolytic enzymes and regulates their enzymatic activity. FIG. 3A shows schematic of glycolytic enzymes identified to interact with Sirt2 via IP-LC-MS/MS analyses. FIG. 3B shows co-immunoprecipitation of glycolytic enzymes by anti-Sirt2 on activated WT CD3+ T cells protein extracts followed by immunoblotting with anti-HK1, anti-PFKP anti-PKM1/2, anti-ENO, anti-PGK1 anti-ALDO, anti-LDH, anti-GAPDH, and anti-Sirt2 antibodies. FIG. 3C shows co-immunoprecipitation of Sirt2 by anti-HK1, anti-PKM1/2, anti-PGK1, anti-LDH or anti-GAPDH on activated WT CD3+ T cells protein extracts followed by immunoblotting with anti-Sirt2 or the corresponding specific antibody. FIG. 3D shows determination of acetylation levels of GAPDH and PFKP by Immunoprecipitation of PFKP, GAPDH or acetylated lysine on activated WT and Sirt2KO CD3+ T cells protein extracts followed by immunoblotting with anti-acetyl-Lysine (anti-AcK) or the corresponding specific antibody. FIGs. 3B-3D show Western blotting of each target using equal amounts of total protein extracts is shown as control (Input) of equal concentrations for the IP. FIG. 3E shows enrichment analysis of Sirt2 downstream deacetylation targets from IP-LC-MS/MS analyses. A false discovery rate q-value < 0.01 was used as a cut-off. FIG. 3F shows glycolytic enzyme activities of Aldolase (n = 5), Enolase (n = 4), GAPDH (n = 5), PFK (n = 5), Hexokinase (n = 6) were assessed from activated WT and Sirt2KO CD3+ T cell extracts. Data are mean ± s.e.m. P values are determined by two-way ANOVA. * P<0.05; ** P<0.01; *** P< 0.001. Data are representative of at least one (FIGs. 3B-3C), two (FIGs. 3E-3F) and four (FIG. 3D) independent experiments.

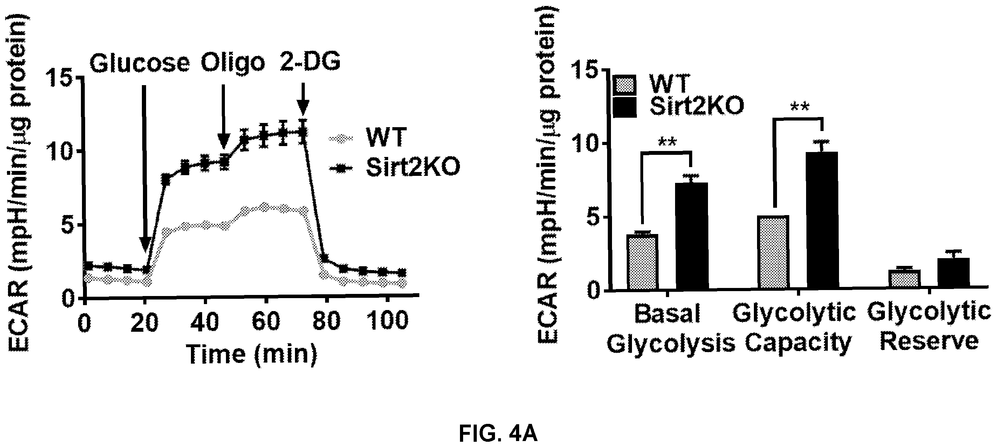

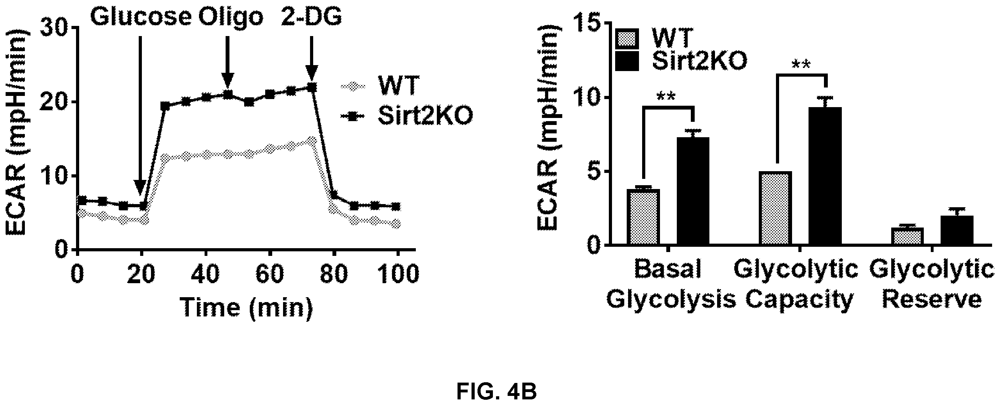

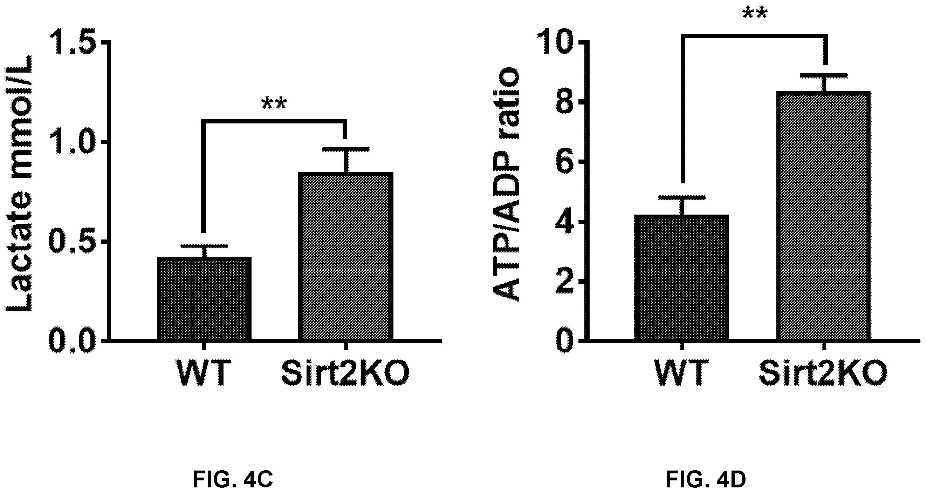

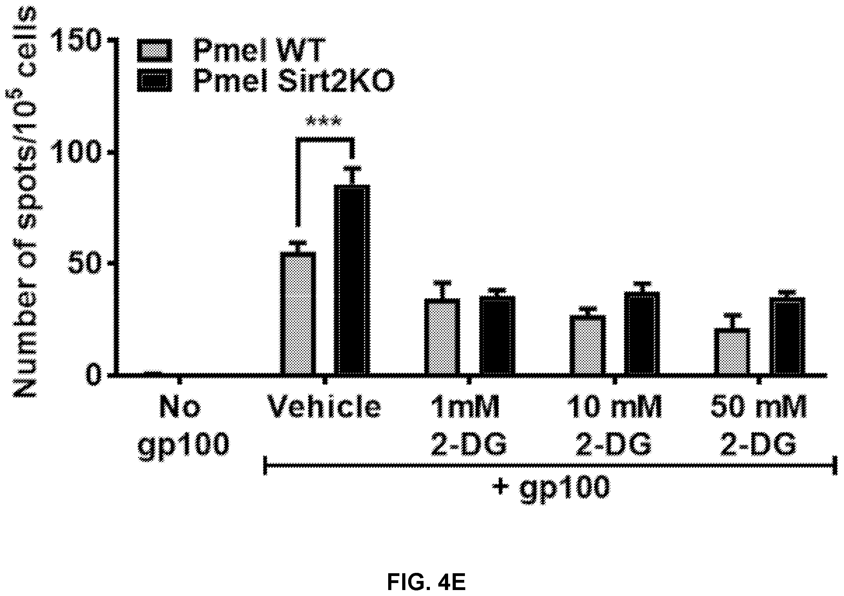

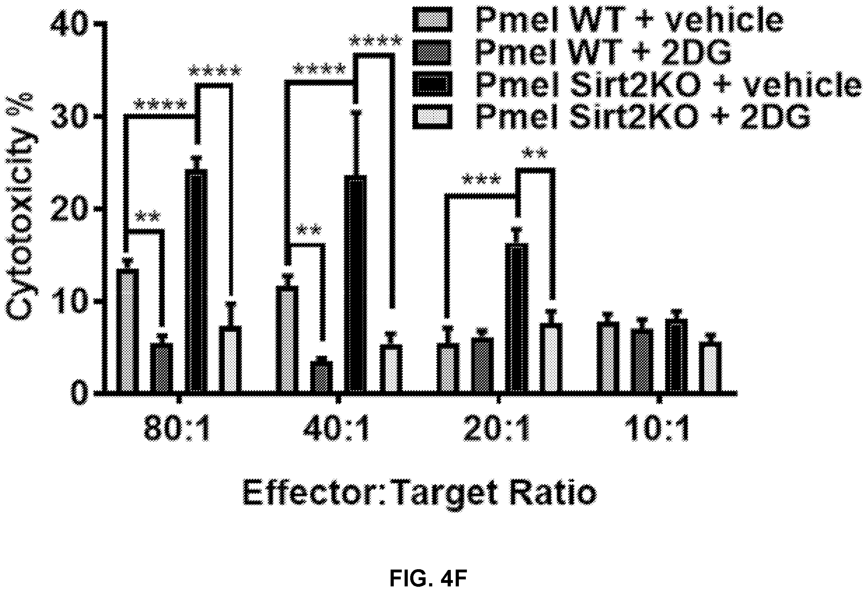

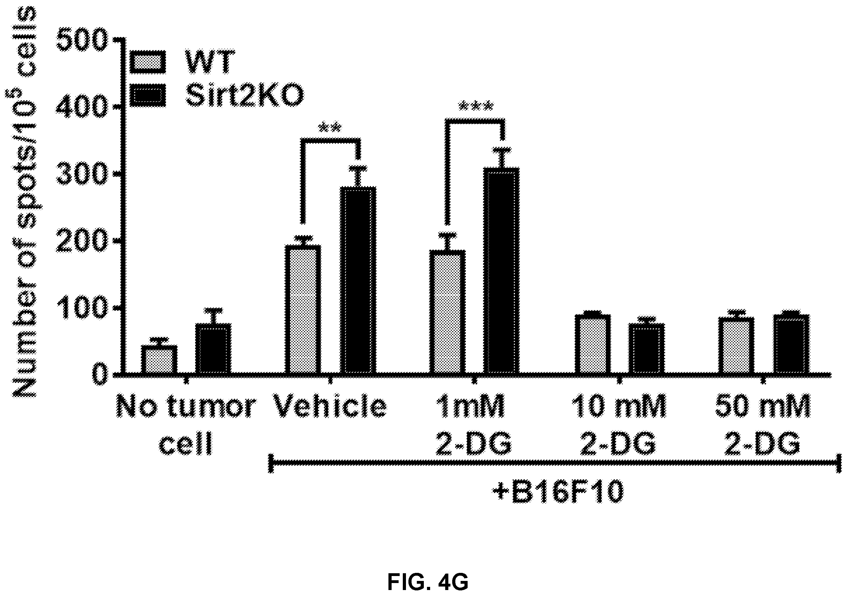

FIGs. 4A to 4G show increased glycolysis in Sirt2KO T cells and blocking glycolysis reverse their hyper-reactivity. FIG. 4A shows glycolytic flux of CD3-stimulated WT and Sirt2KO CD3+ T cells was measured as extracellular acidification rate (ECAR) using the Seahorse XF analyzer. Glucose, Oligomycin (Oligo) and 2-deoxyglucose (2-DG) were injected at the points indicated. Right: Basal glycolytic rate, glycolytic capacity and glycolytic reserve were calculated (n = 3). FIG. 4B shows glycolytic flux of CD3+ TILs isolated from B16F10 tumor nodules from WT or Sirt2KO mice, measured as ECAR. Right: Basal glycolytic rate, glycolytic capacity and glycolytic reserve were calculated (WT; n = 13, Sirt2KO; n = 7). FIG. 4C shows extracellular lactate production by CD3-stimulated WT and Sirt2KO CD3+ T cells was measured using YSI 2900 Biochemistry Analyzer (n = 3). FIG. 4D shows ATP/ADP ratio was measured using a bioluminescent assay from CD3-stimulated WT and Sirt2KO CD3+ T cell extracts (n = 3). FIG. 4E shows IFN-γ ELISPOT assay was performed on non-stimulated or gp100-stimulated WT and Sirt2KO Pmel T cells with the indicated concentrations of 2-DG (n = 3). FIG. 4F shows cytotoxic activities were determined by LDH release assay on gp100-stimulated WT and Sirt2KO CD8+ Pmel T cells after co-culture on B16F10 targets at the indicated Effector: Target ratios with with vehicle or 10mM 2-DG (n = 3). FIG. 4G shows IFN-γ Elispot assay was performed on WT and Sirt2KO CD3+ TILs after re-challenge with vehicle or irradiated B16F10 cells at the indicated concentration of 2-DG (n = 3). Data are representative of two (FIG. 4G), three (FIGs. 4A-4B, 4E-4F) and four (FIGs. 4C-4D) independent experiments. Data are mean ± s.e.m. P values are determined by two-tailed Student's t-test (a-d) or two-way ANOVA (FIGs. 4E-4F) *P < 0.05, **P < 0.01, ***P < 0.001, ****P < 0.0001.

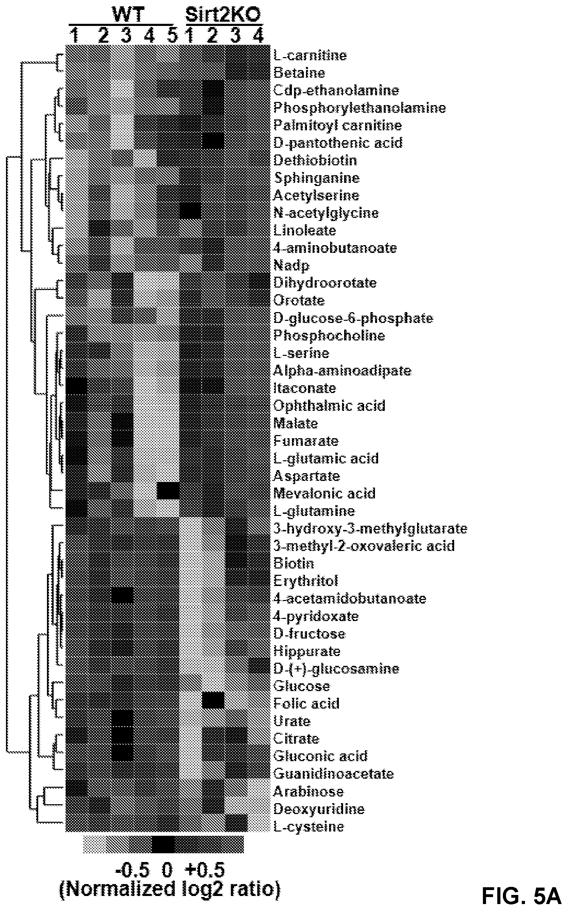

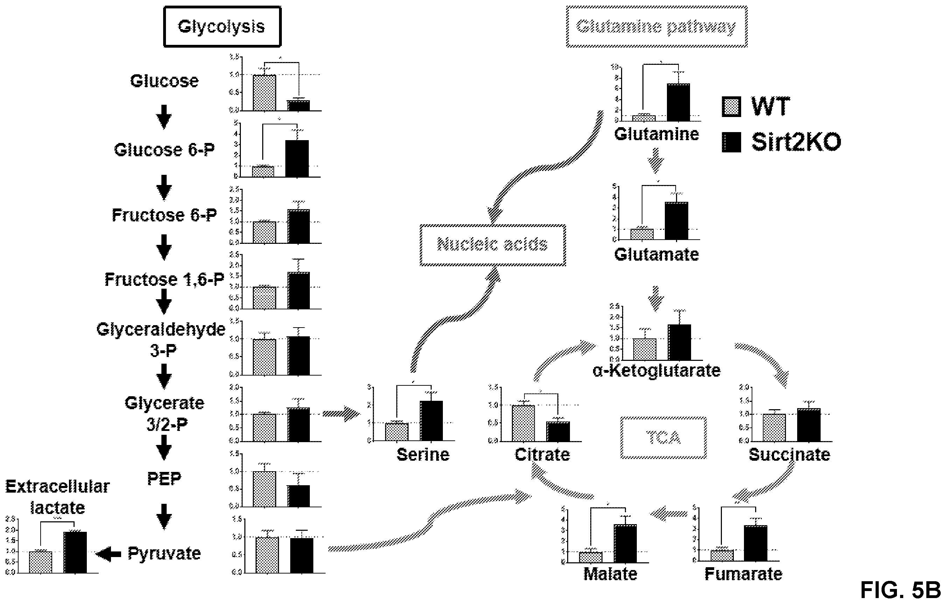

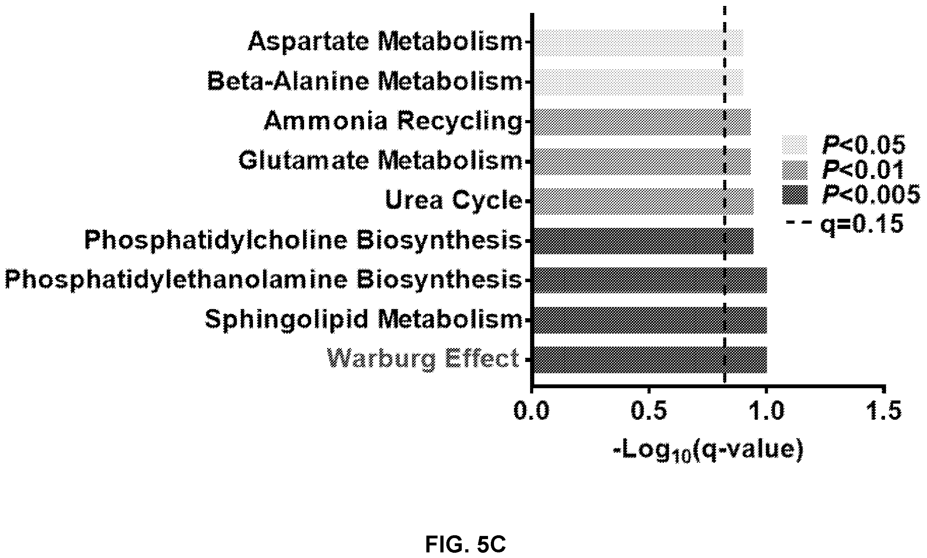

FIGs. 5A to 5C show metabolic reprogramming in activated Sirt2KO T cells. WT (n = 5) and Sirt2KO (n = 4) CD8+ T cells were stimulated ex vivo with anti-CD3 for 72 hours. Intracellular metabolites were extracted form T cell lysates samples and analyzed using high-resolution LC-MS/MS. FIG. 5A is a heat map showing relative abundance of metabolites differentially expressed and unsupervised hierarchical clustering. FIG. 5B shows relative levels of metabolites implicated in glycolysis, TCA-cycle and glutamate pathway are shown. Samples are normalized to WT T cells as indicated by dashed lines. Data are mean ± s.e.m. P values are determined by two-tailed Student's t-test, *P < 0.05; **P<0.01. FIG. 5C shows metabolite set enrichment of the upregulated metabolic pathways in Sirt2KO compared to WT T cell. A false discovery rate q value < 0.15 was used as a cut-off. Data are representative of two independent experiments.

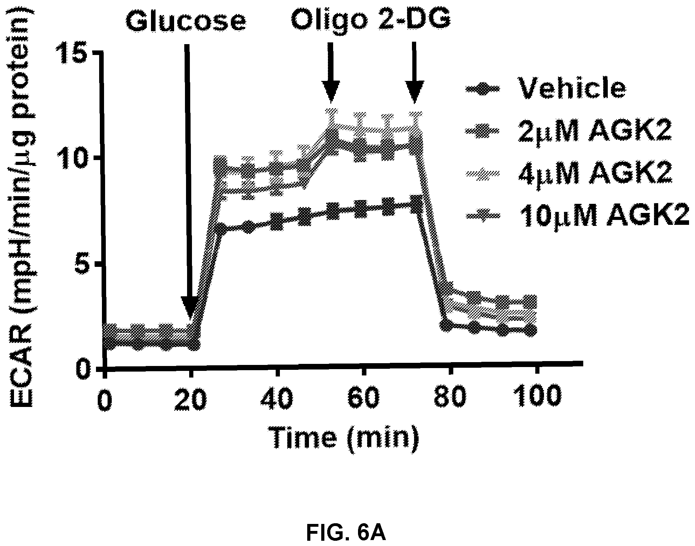

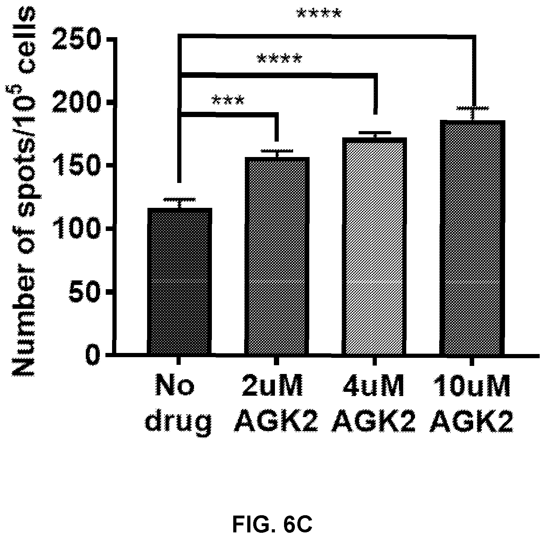

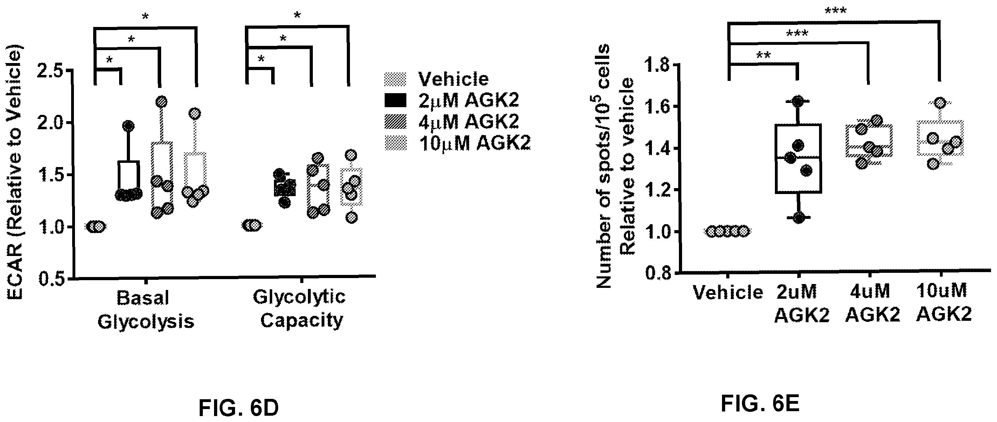

FIGs. 6A to 6E shows pharmacologic inhibition of Sirt2 enhances glycolysis and effector functions in human NSCLC TILs. Human TILs samples isolated from tumor biopsies of NSCLC patients were stimulated with anti-CD3 in combination with Sirt2 inhibitor AGK2 vs. vehicle for 48 hours. FIG. 6A shows glycolytic flux measured as ECAR using the Seahorse XF analyzer. Glucose, Oligomycin and 2-DG were injected at the points indicated b, Basal glycolytic rate, glycolytic capacity and glycolytic reserve were calculated from FIG. 6A, n = 4-7. FIG. 6C shows IFN-γ ELISPOT assay was performed on CD3-stimulated TILs with increasing concentrations of AGK2 versus vehicle for an additional 48 hours (n = 5). FIGs. 6A to 6C show one human TILs sample is representative of 5 samples tested. FIG. 6D shows basal glycolytic rate and glycolytic capacity of five human TILs samples were normalized to vehicle and plotted as shown. FIG. 6E shows number of IFN-γ spots of five human TILs samples were normalized to vehicle and plotted as shown. Data are mean ± s.e.m. P values are determined by one-way ANOVA (b-c, e) or Kruskal-Wallis test (d), *P < 0.05, **P < 0.01, ***P < 0.001, ****P < 0.0001.

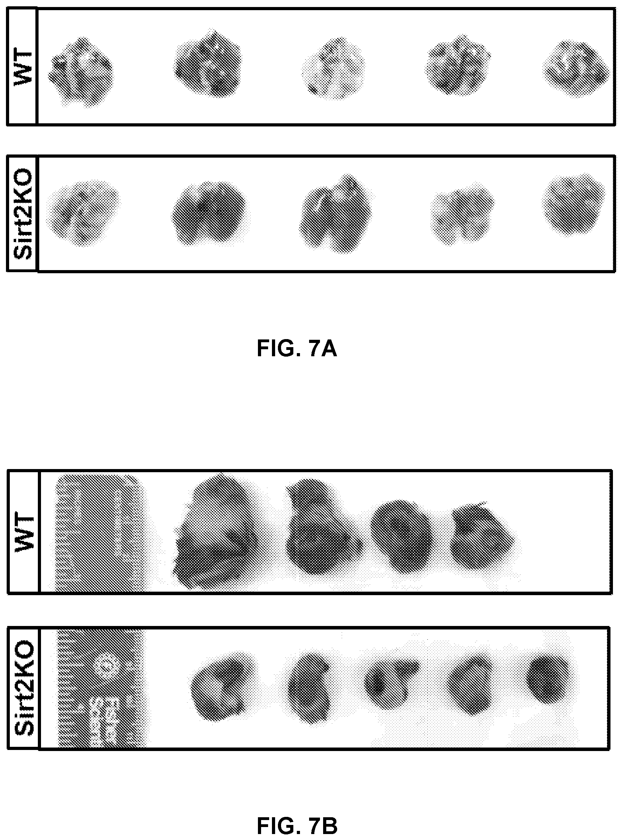

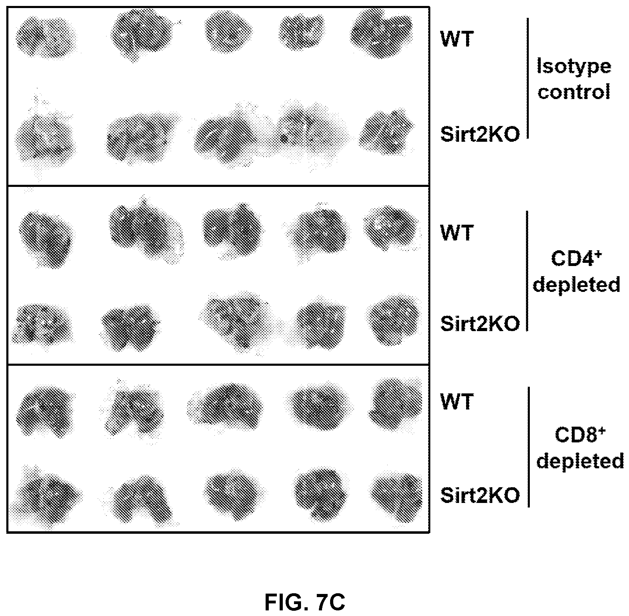

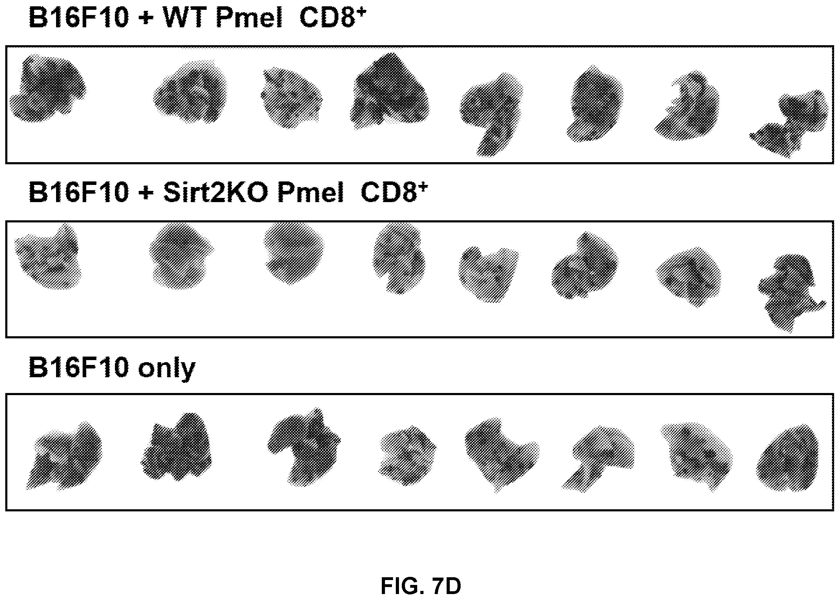

FIGs. 7A to 7D show Sirt2 Deficiency protects mice from B16F10 cell challenge. FIG. 7A shows lungs from WT and Sirt2KO mice challenged i.v. with B16F10 cells, collected 18 days post-injection. FIG. 7B shows subcutaneous tumors dissected from WT and Sirt2KO mice challenged s.c. with B16F10 cells 21 days post-injection. One WT mouse reached tumor size endpoint prior to the Day 21 timepoint and thus not depicted. FIG. 7C shows lungs from WT and Sirt2KO mice treated with isotype control, anti-CD4+, or anti-CD8+ monoclonal antibodies before B16F10 i.v. challenge, collected 18 days post-injection. FIG. 7D shows lungs from NSG mice challenged i.v. with B16F10 and treated with vehicle, WT Pmel CD8+ T cells or Sirt2KO Pmel CD8+ T cells, collected 18 days post-injection. Data are representative of two (FIGs. 7C-7D), three (FIG. 7B) and four (FIG. 7A) independent experiments.

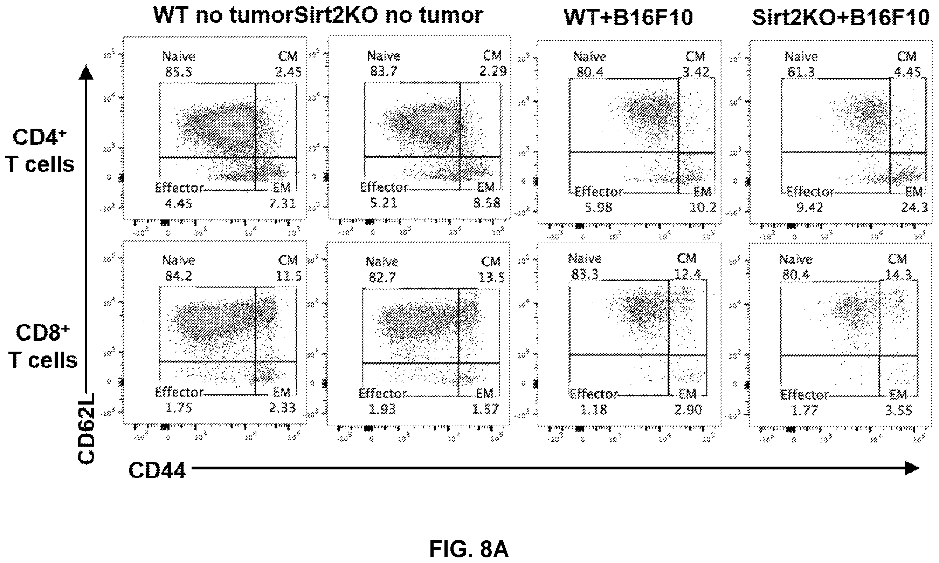

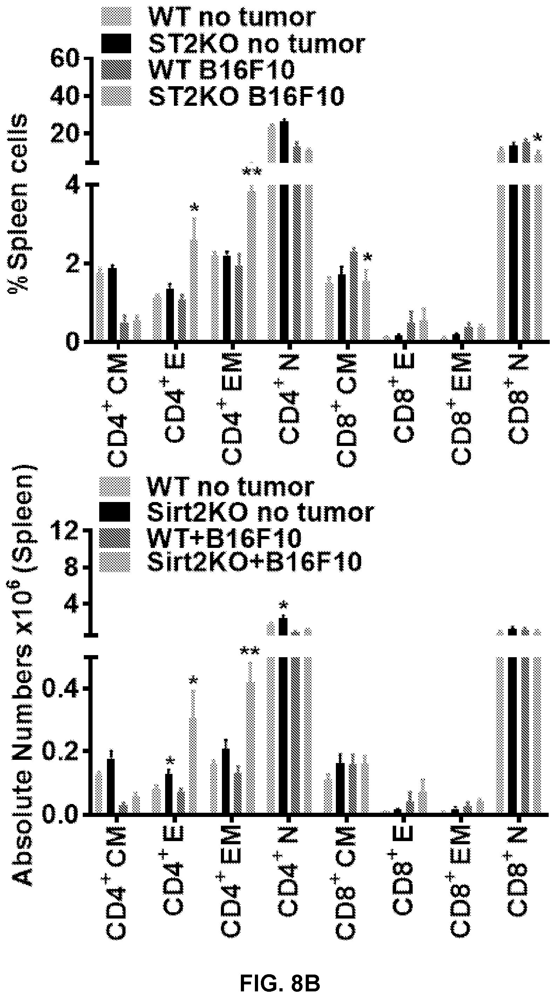

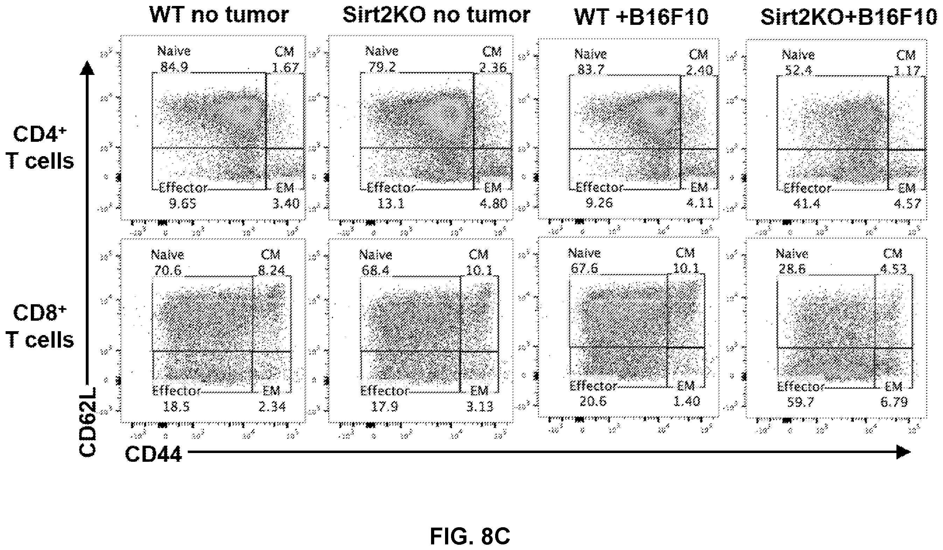

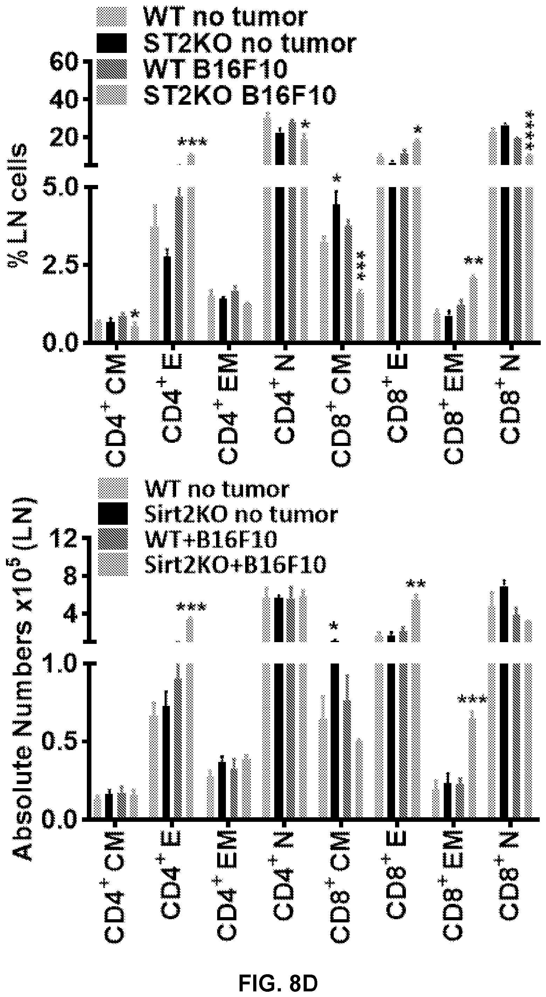

FIGs. 8A to 8D shows T cell compartment in the spleen and lymph node of B16F10 challenged mice. FIG. 8A and 8C show representative flow cytometric analysis of splenocytes (FIG. 8A) and Lymph node T cells (FIG. 8C) from WT and Sirt2KO mice i.v. challenged with B16F10 cells versus control for naive, effector, effector memory (EM) and central memory (CM) CD8+ and CD4+ T cell subsets. Numbers in quadrants indicate percentage of cells. FIGs. 8B and 8D show frequency (upper) and absolute number (Lower) of each subset from spleens (FIG. 8B) and Lymph nodes (FIG. 8D) are indicated. n = 5 (FIG. 8B) and n = 4 (FIG. 8D) in each group. Data are mean ± s.e.m. P values are determined by two-tailed Student's t-test. *P < 0.05, **P < 0.01, ***P < 0.001, ****P < 0.0001. Data are representative of two? independent experiments.

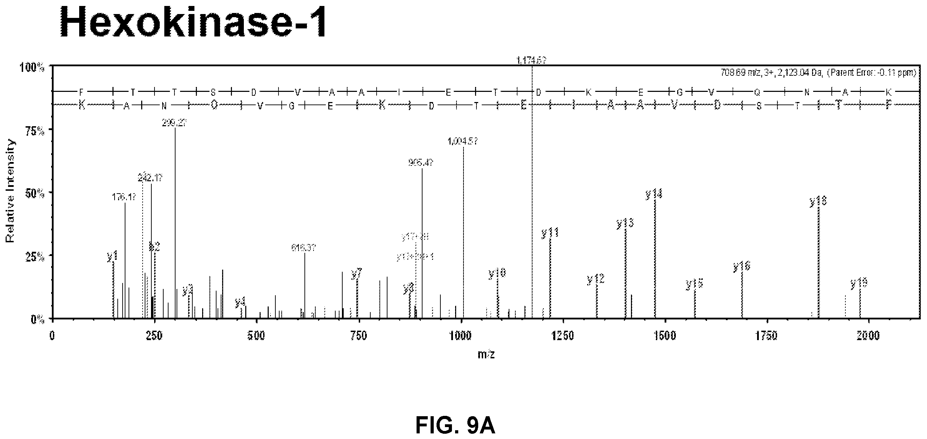

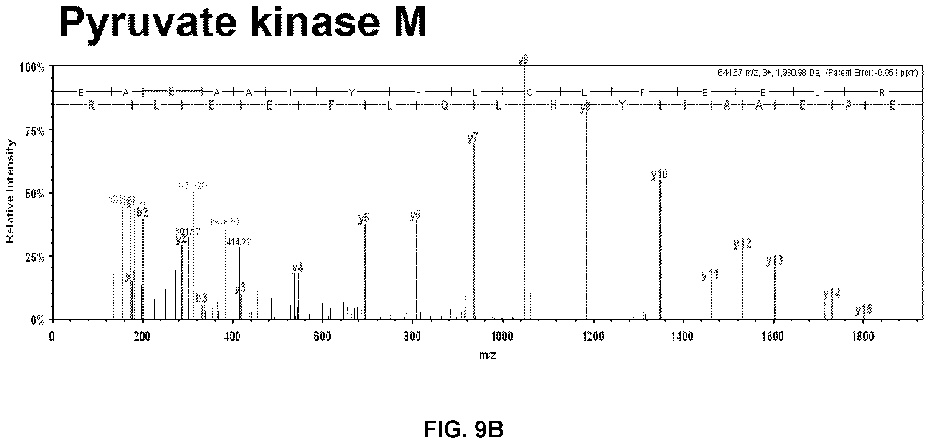

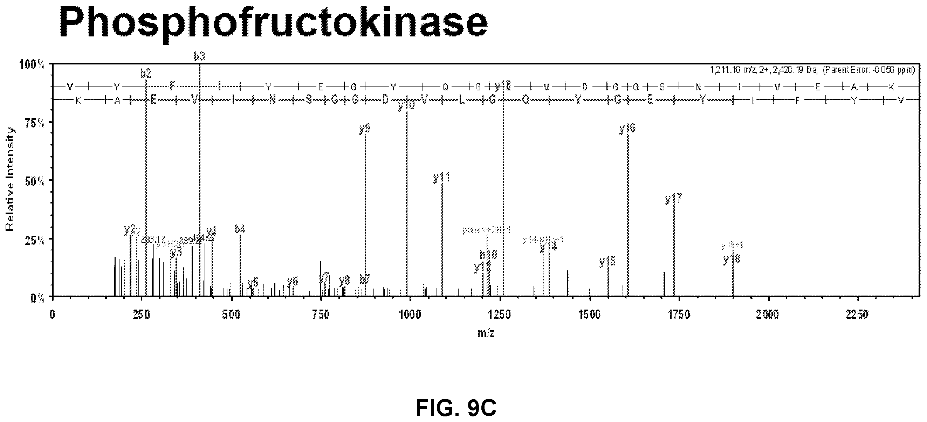

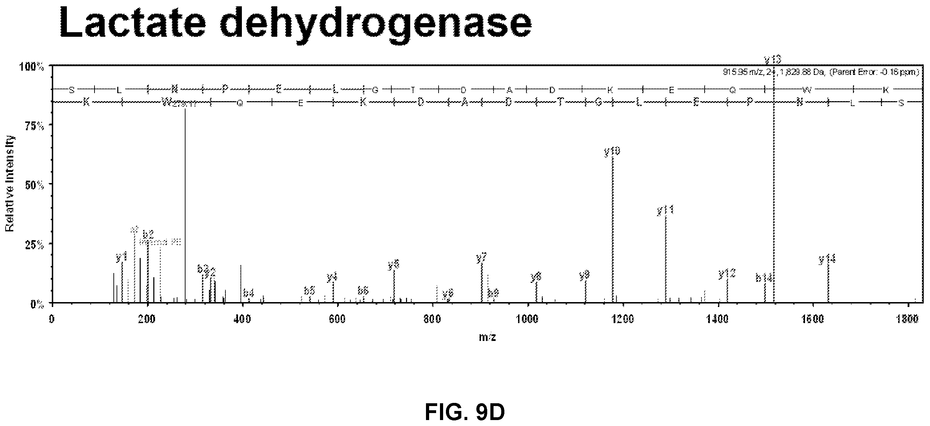

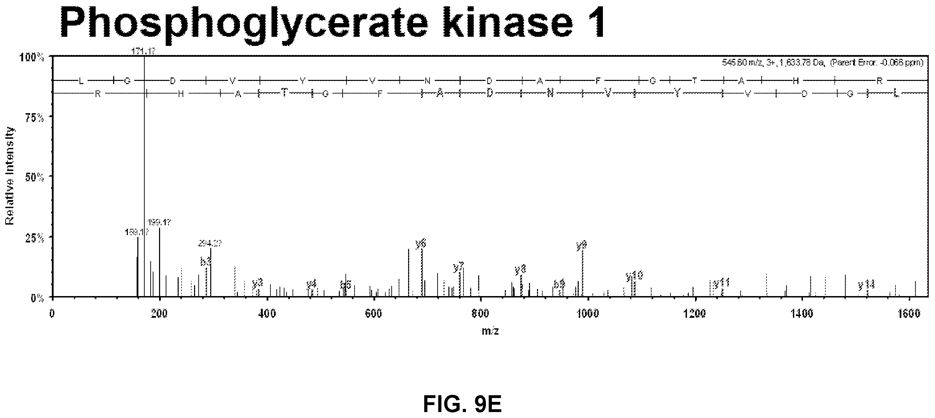

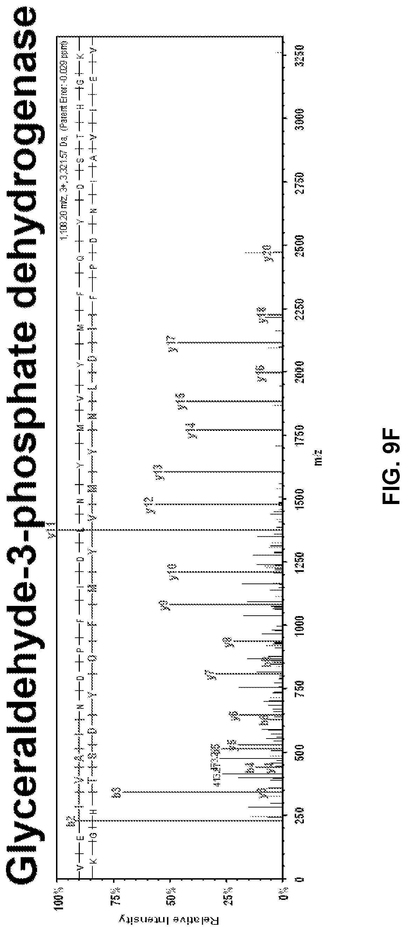

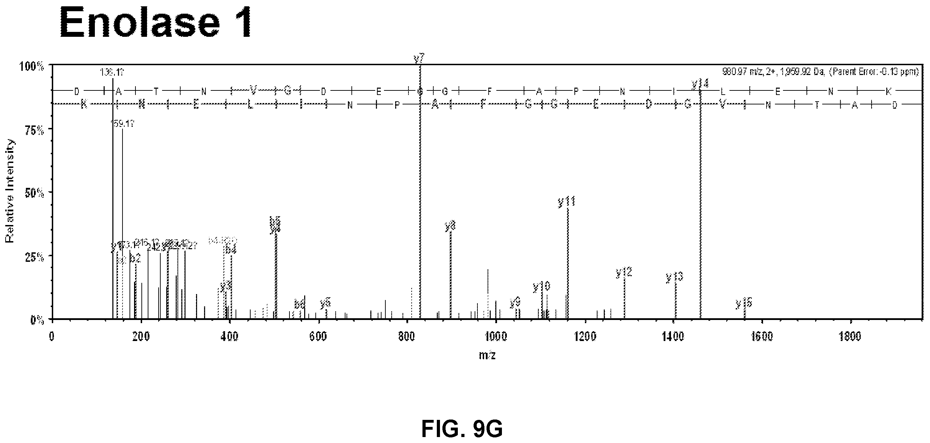

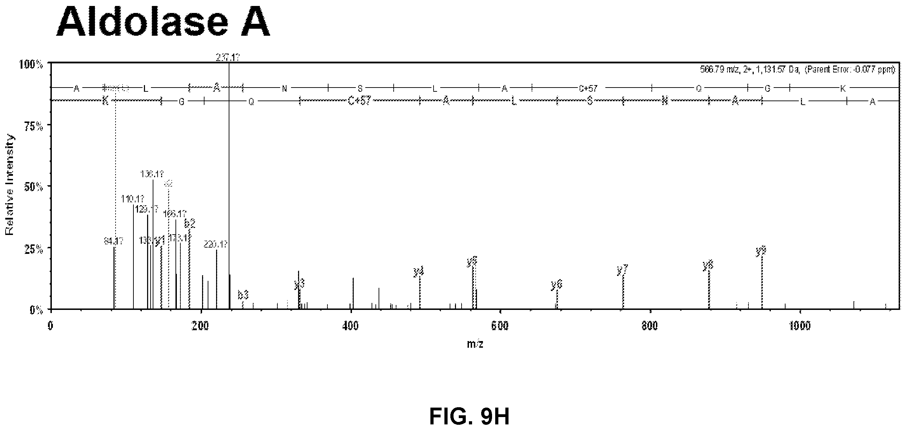

FIGs. 9A to 9H show IP-MS pulls down key glycolytic pathway enzymes Collision-induced dissociation (CID) spectra of the peptides derived from the proteins: Hexokinase-1 (FIG. 9A), phosphofructokinase (FIG. 9C), phosphoglycerate kinase 1 (FIG. 9E), enolase 1 (FIG. 9G), pyruvate kinase isozymes M1/M2 (FIG. 9B), lactate dehydrogenase (FIG. 9D), glyceraldehyde-3-phosphate dehydrogenase (FIG. 9F) and aldolase (FIG. 9H) were detected via immunoprecipitation of Sirt2 on activated WT CD3+ T cell protein extracts followed by LC-MS/MS analyses. Data are representative of two independent experiments.

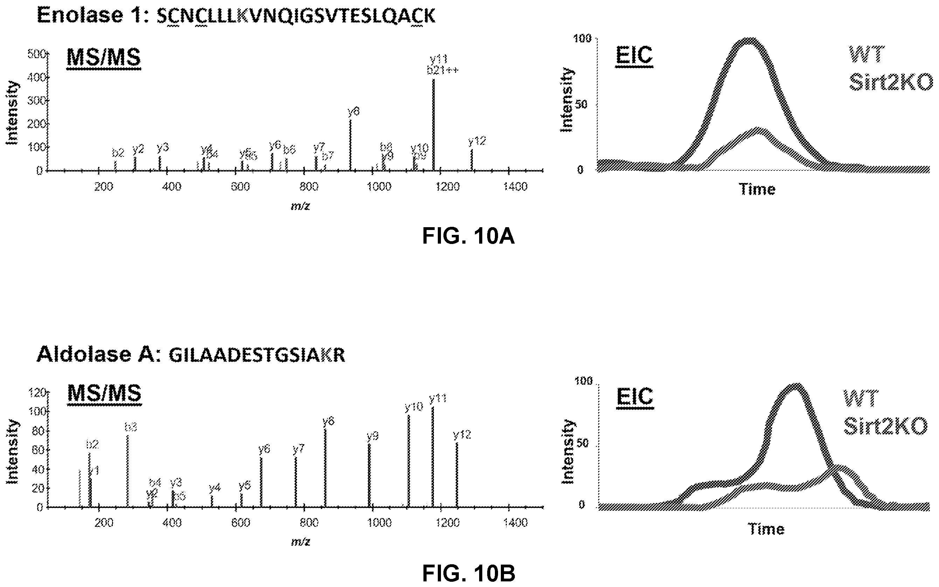

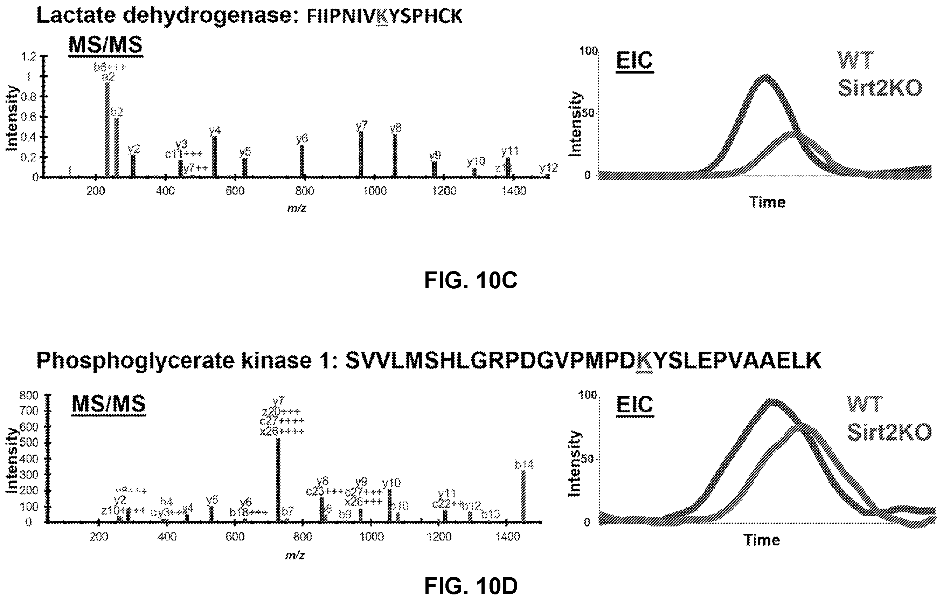

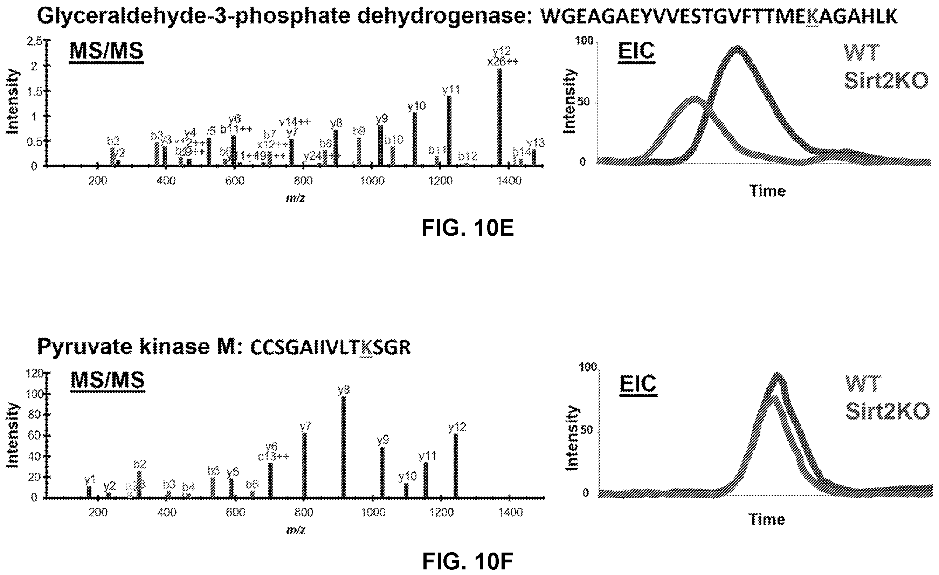

FIGs. 10A to 10F show glycolytic enzymes are highly acetylated in Sirt2KO T cell. Immunoprecipitation of acetylated proteins was performed using anti-acetyl-lysine antibodies on WT and Sirt2 CD3+ T cell protein extracts followed by LC-MS/MS analyses. FIGs. 10A to 10F, Left: The MS/MS spectrum was matched to: (FIG. 10A) peptide GILAADESTGSIAKR (SEQ ID NO:1) from Aldolase 1 and y1, y2 fragments indicate the acetylation site; (FIG. 10B) peptide SCNCLLLKVNQIGSVTESLQACK (SEQ ID NO:2) from Enolase and b7, b8 fragments indicates the acetylation site; (FIG. 10C) peptide FIIPNIVKYSPHCK (SEQ ID NO:3) from Lactate dehydrogenase and y6, y7 fragments indicates the acetylation site; (FIG. 10D) peptide SVVLMSHLGRPDGVPMPDKYSLEPVAAELK (SEQ ID NO:4) from Phosphoglycerate kinase 1 and y11 fragment indicates the acetylation site; (FIG. 10E) peptide WGEAGAEYVVESTGVFTTMEKAGAHLK (SEQ ID NO:5) from Glyceraldehyde-3-phosphate dehydrogenase and y6, y7 fragments indicate the acetylation site; and (FIG. 10F) peptide CCSGAIIVLTKSGR (SEQ ID NO:6) from Pyruvate kinase M and y3, y5 fragments indicate the acetylation site. FIGs. 10A to 10F, Right: Extracted ion chromatogram (EIC) plot shows the increased intensity in Sirt2KO sample versus WT of each target. Data are representative of two independent experiments.

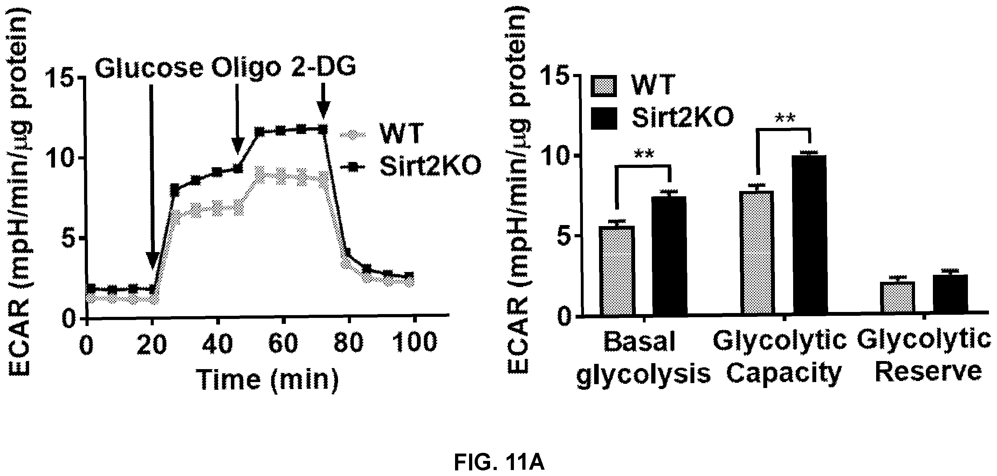

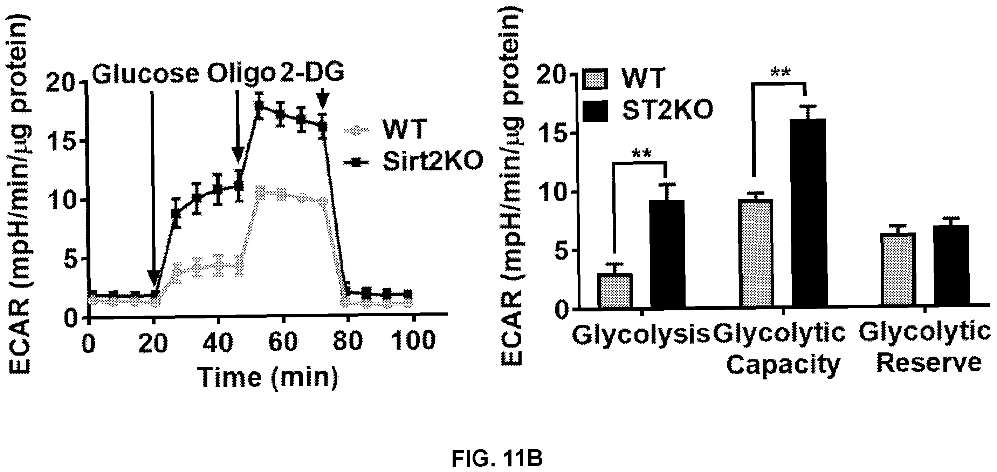

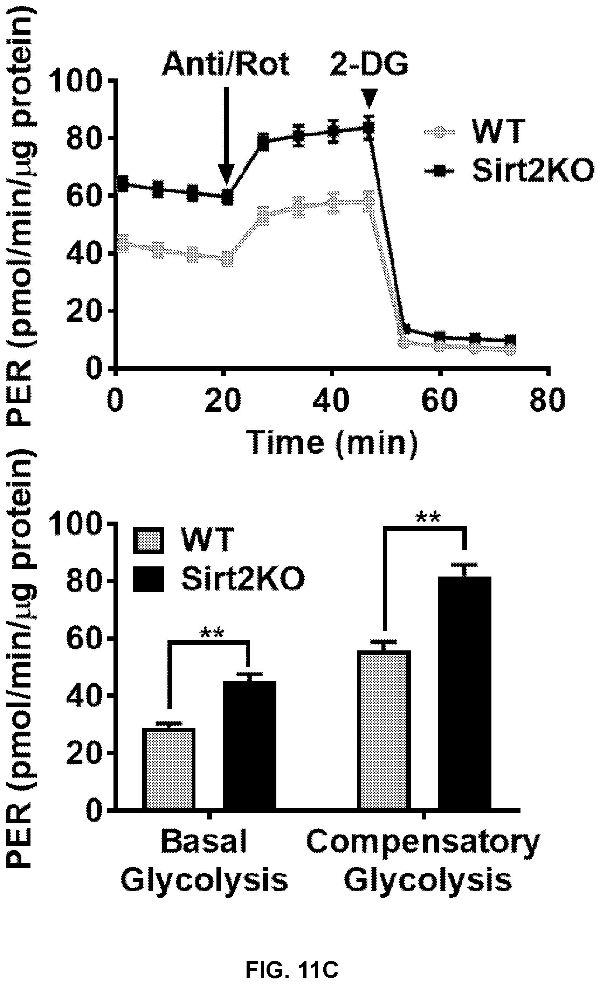

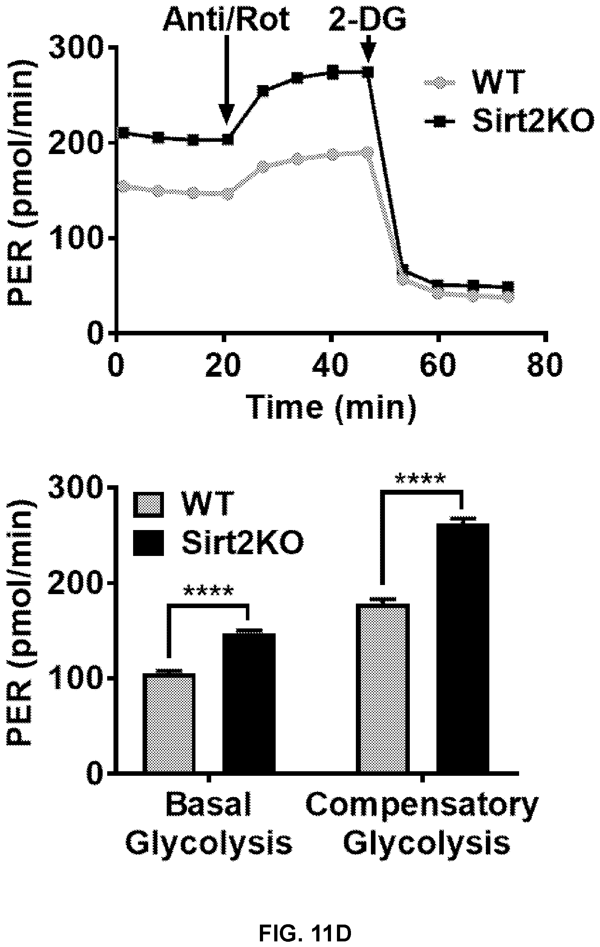

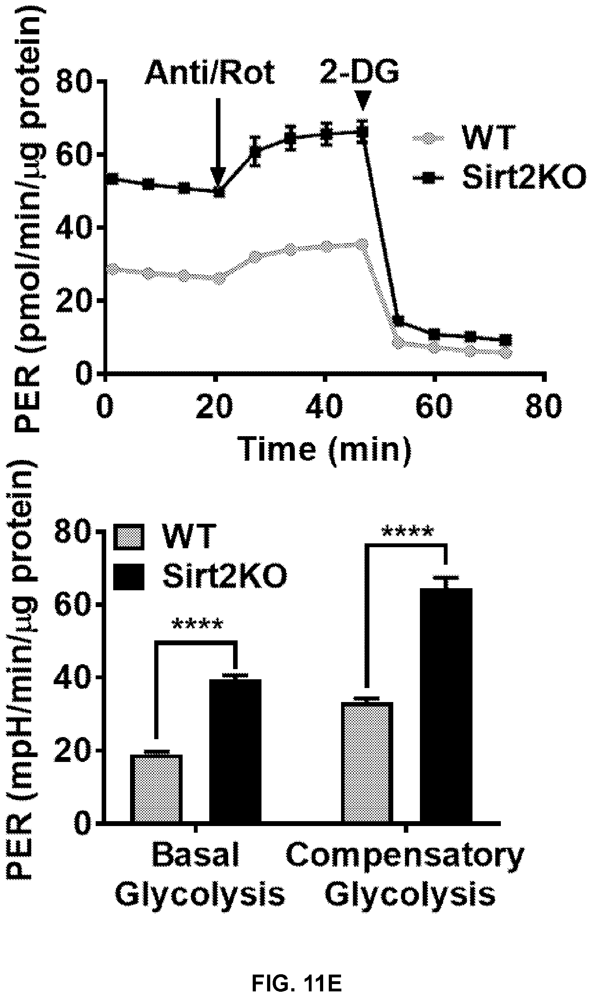

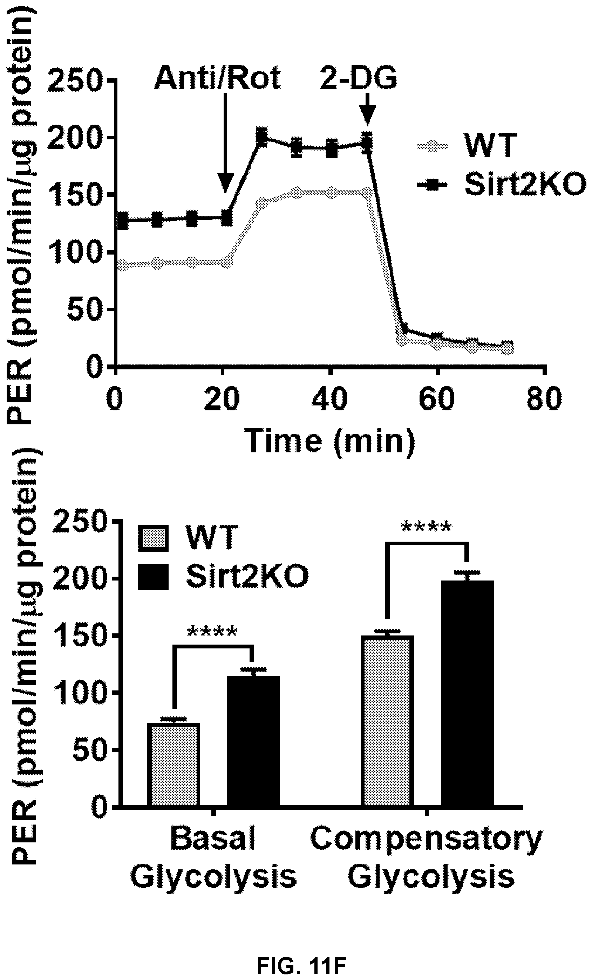

FIGs. 11A to 11F show increased glycolysis in Sirt2KO vs. WT T cells. FIGs. 11A to 11B, Left: Glycolytic flux was measured as ECAR using the Seahorse XF analyzer on: (FIG. 11A) CD3-stimulated WT (n = 4) and Sirt2KO (n = 4) CD8+ T cells and (FIG. 11B) gp100-stimulated WT (n = 4) and Sirt2KO (n = 5) Pmel CD8+ T cells. Glucose, Oligomycin and 2-DG were injected at the points indicated. Right: Basal glycolytic rate, glycolytic capacity and glycolytic reserve were calculated from the corresponding curves. FIGs. 11C to 11F, Upper: Glycolytic rate was measured as proton efflux rate (PER) using the Seahorse XF analyzer on: (FIG. 11C) CD3-stimulated WT (n = 4) and Sirt2KO (n = 4) CD3+ T cells, (FIG. 11D) CD3+ TILs freshly isolated from B16F10 tumor nodules from WT (n =11) or Sirt2KO mice (n = 6) (FIG. 11E) CD3-stimulated WT (n = 5) and Sirt2KO (n = 5) CD8+ T cells and (FIG. 11F) gp100-stimulated WT (n = 8) and Sirt2KO (n = 5) Pmel CD8+ T cells. Antimycin/Rotenone (Anti/Rot) and 2-DG were injected at the points indicated. FIGs. 11C to 11F, Lower: Basal glycolytic rate and Compensatory glycolysis were calculated from the corresponding curves. Data are representative of two (FIG. 11A, 11F), three (FIG. 11B, 11D) and four (FIG. 11C, 11E) independent experiments. Data are mean ± s.e.m. P values are determined by two-tailed Student's t-test. *P < 0.05, **P < 0.01, ***P < 0.001 , ****P < 0.0001.

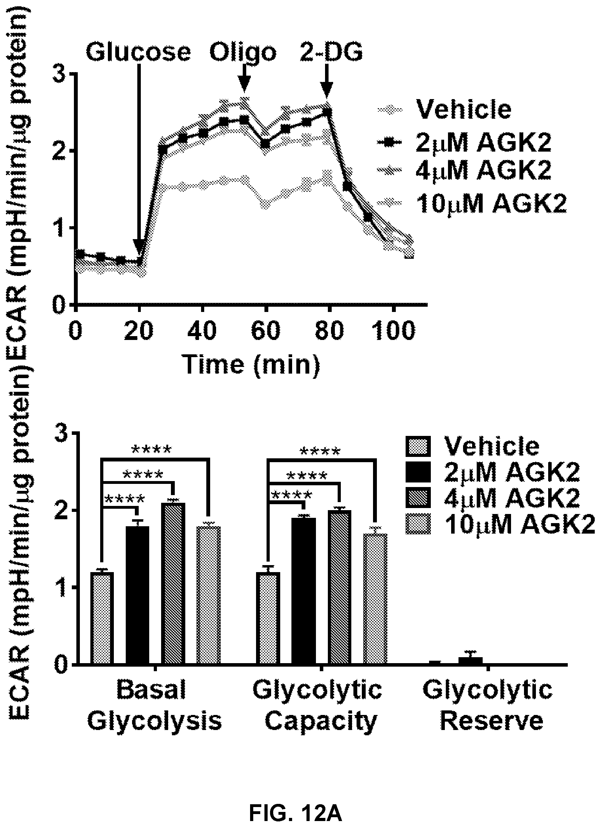

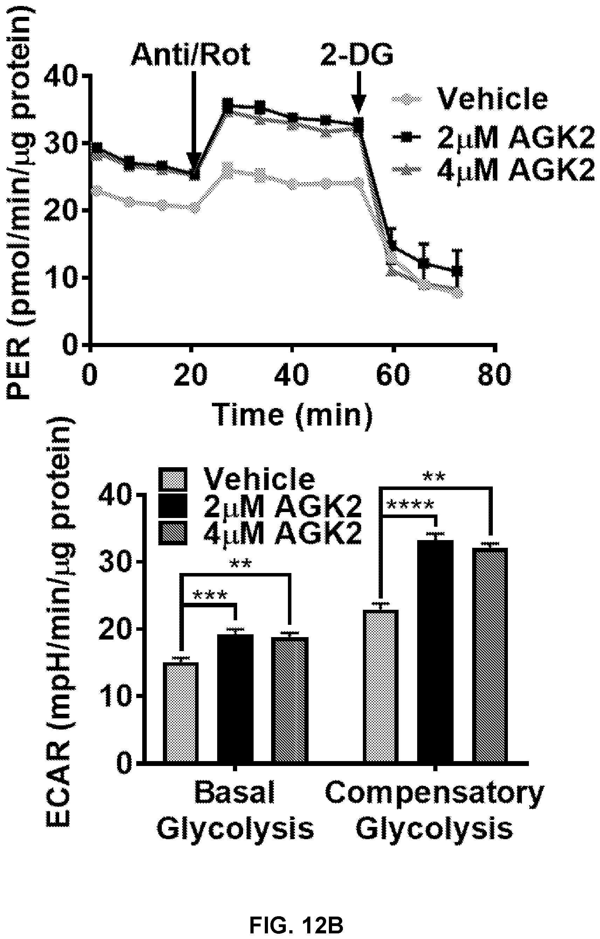

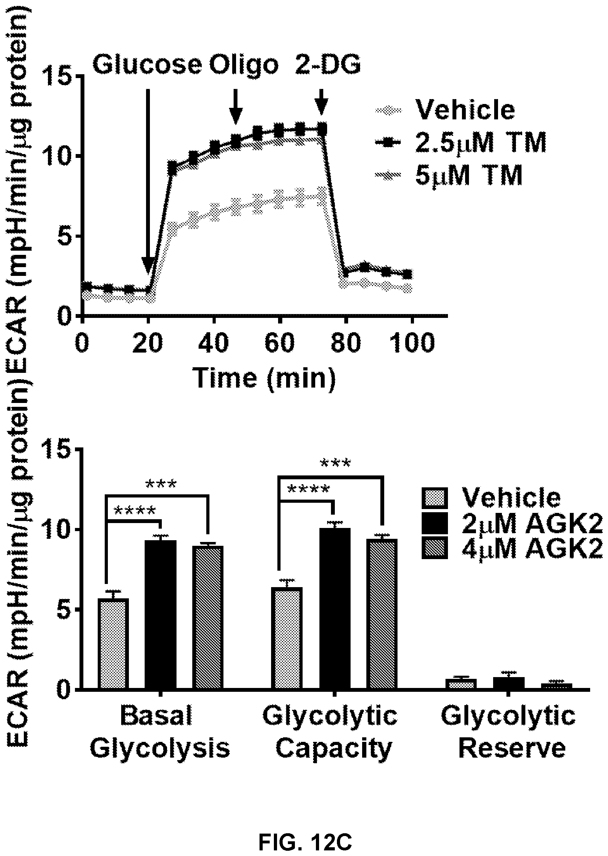

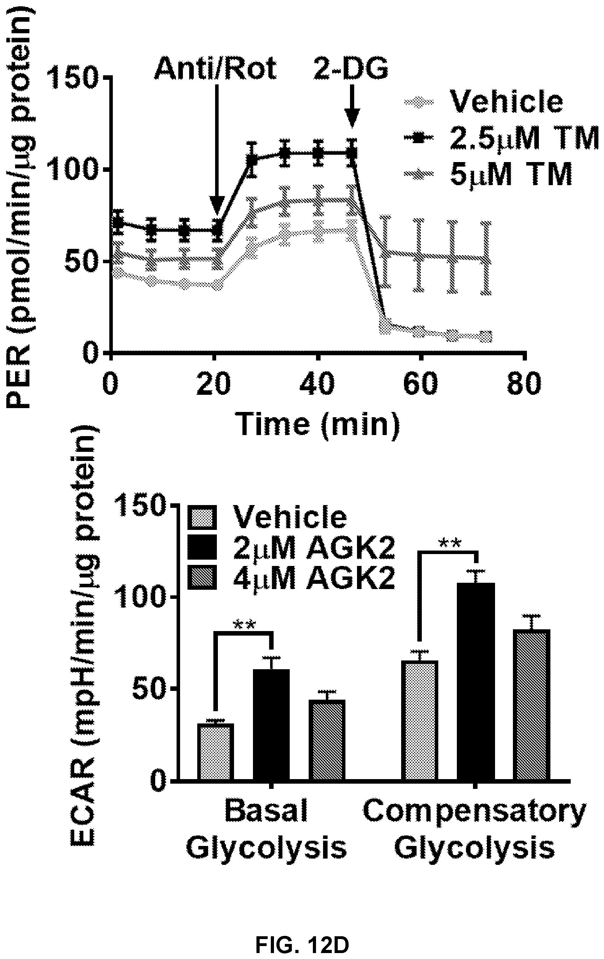

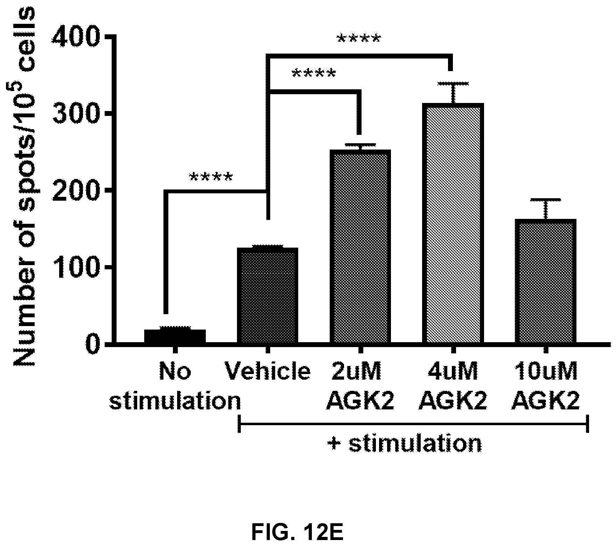

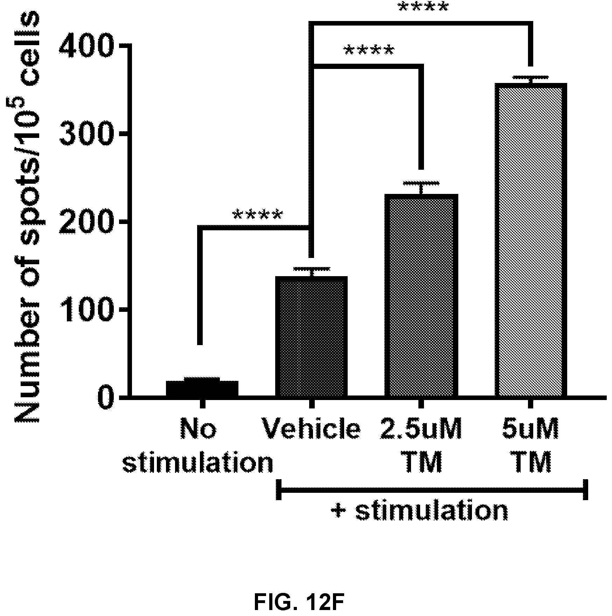

FIGs. 12A to 12F show blocking Sirt2 with AGK2 increases glycolysis and effector functions of human T cells. Human CD3+ T cells isolated from peripheral blood of self-reporting healthy donors were stimulated with anti-CD3 in combination with Sirt2 inhibitor AGK2 (FIGs. 12A-12B, 12E) or thiomyristoyl (TM) (FIGs. 12C-12D, 12F) vs. vehicle for 48 hours. FIGs. 12A and 12C, Upper: Glycolytic flux measured as ECAR using the Seahorse XF analyzer on: (FIG. 12A) AGK2-Treated CD3+ T cells (n = 7-8) and (FIG. 12C) TM-treated CD3+ T cells (n = 4-8). Glucose, Oligo and 2-DG were injected at the points indicated. FIGs. 12A and 12C, Lower: Basal glycolytic rate, glycolytic capacity and glycolytic reserve were calculated from the corresponding curves. FIGs. 12B and 12B, Upper: Glycolytic rate measured as PER using the Seahorse XF analyzer on: (FIG. 12B) AGK2-Treated CD3+ T cells (n = 2-8) and (FIG. 12D) TM-treated CD3+ T cells (n = 3-5). Antimycin/Rotenone and 2-DG were injected at the points indicated. Lower: Basal glycolytic rate and Compensatory glycolysis were calculated from the corresponding curves. FIGs. 12E to 12F show IFN-γ ELISPOT assay was performed on: (FIG. 12E) AGK2-treated human CD3+ T cells (n = 6) and (FIG. 12F) TM-treated human CD3+ T cells (n = 3-6) versus vehicle for 48 hours. Data are mean ± s.e.m. P values are determined by one-way ANOVA *P < 0.05, **P < 0.01, ***P < 0.001, ****P < 0.0001.

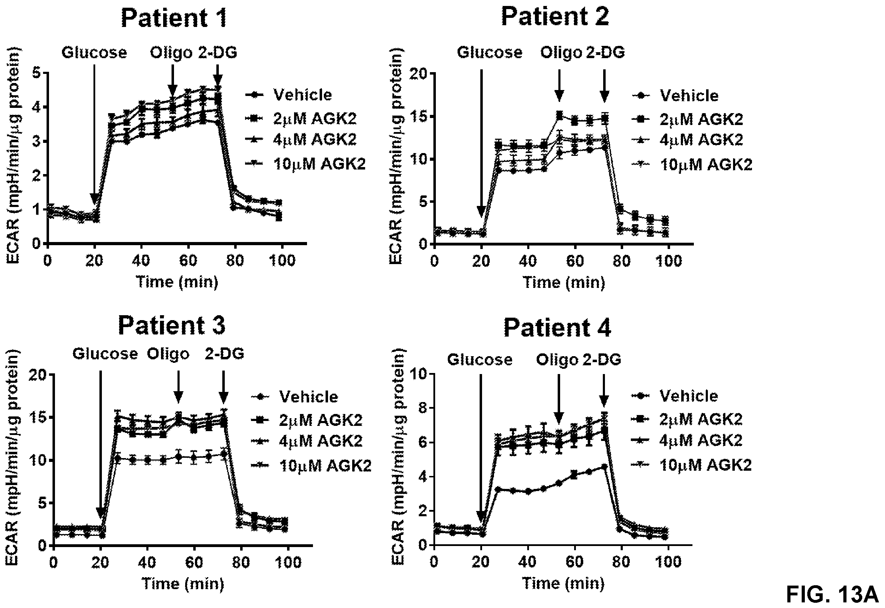

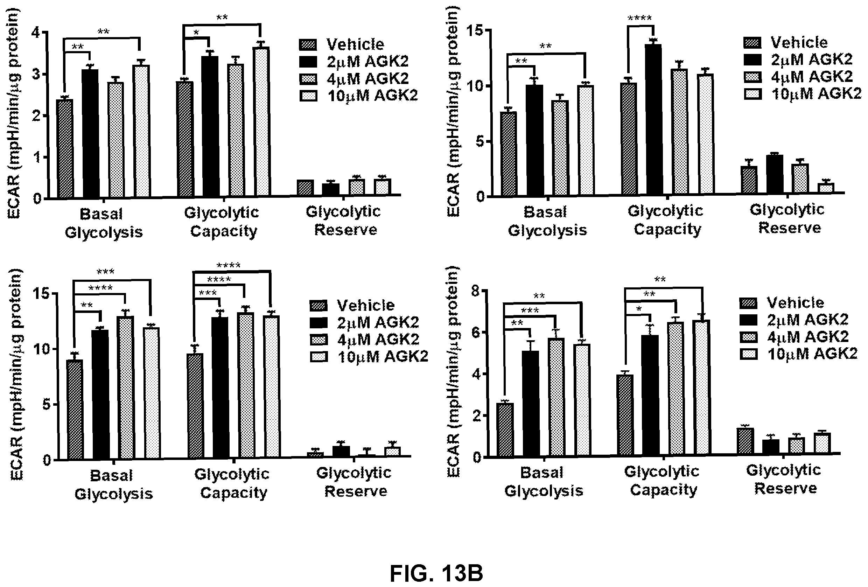

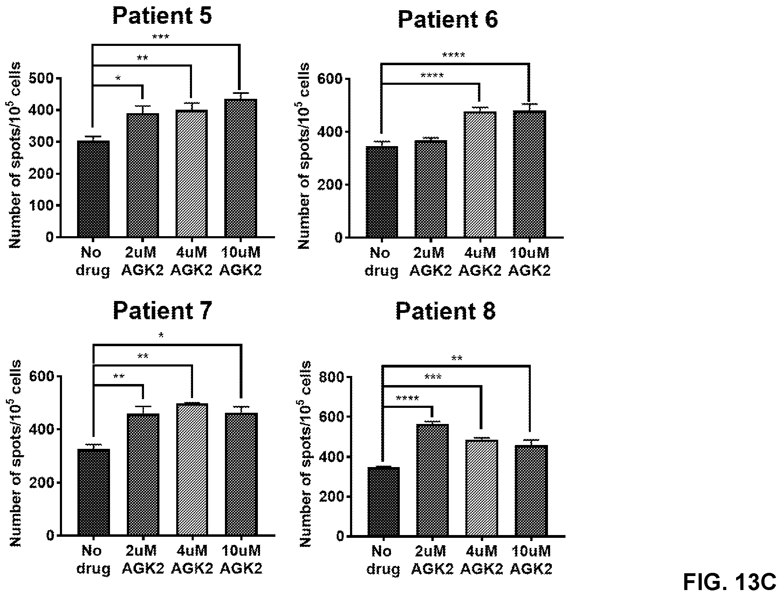

FIGs. 13A to 13C show blocking Sirt2 with AGK2 increases glycolysis and effector functions of human TILs from NSCLC patients. Human TILs samples isolated from tumor biopsies of NSCLC patients were stimulated with anti-CD3 in combination with Sirt2 inhibitor AGK2 vs. vehicle for 48 hours. FIG. 13A shows glycolytic flux measured as ECAR using the Seahorse XF analyzer. FIG. 13B shows basal glycolytic rate, glycolytic capacity and glycolytic reserve were calculated from FIG. 13A. Patient 1: n = 3, Patient 2: n = 3, Patient 3: n = 4-6, Patient 4: n = 3. FIG. 13C shows IFN-γ ELISPOT assay was performed on CD3-stimulated TILs with increasing concentrations of AGK2 versus vehicle for an additional 48 hours. Patient 5: n = 3, Patient 6: n = 6, Patient 7: n = 6, Patient 8: n = 3-6. Data are mean ± s.e.m. P values are determined by one-way ANOVA, *P < 0.05, **P < 0.01, ***P < 0.001, ****P < 0.0001.

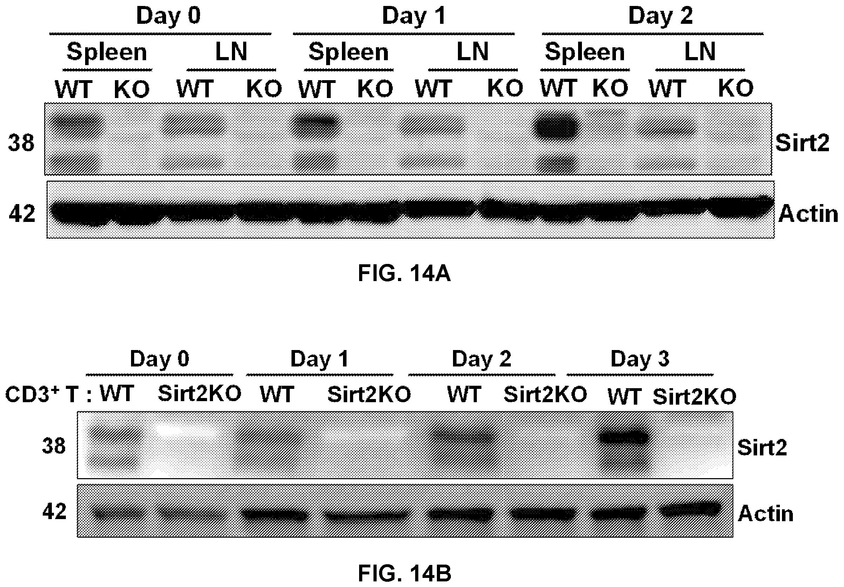

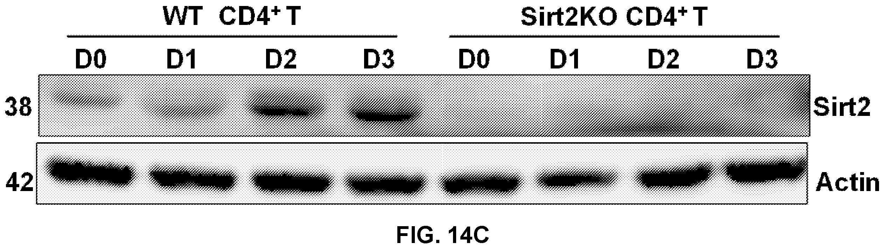

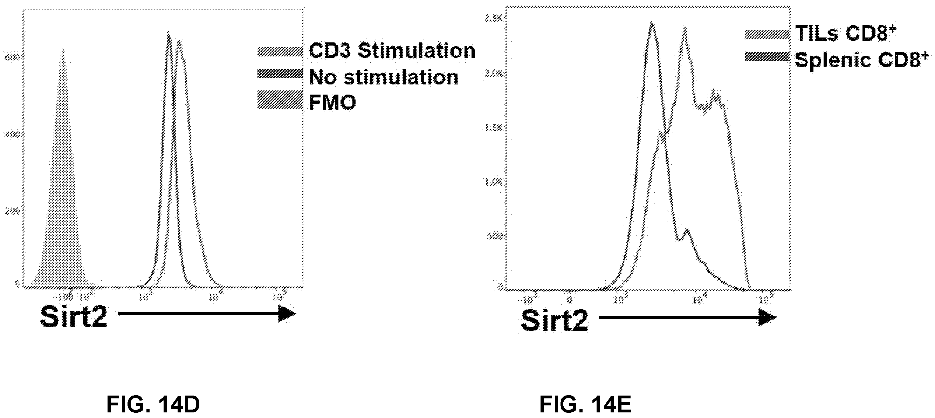

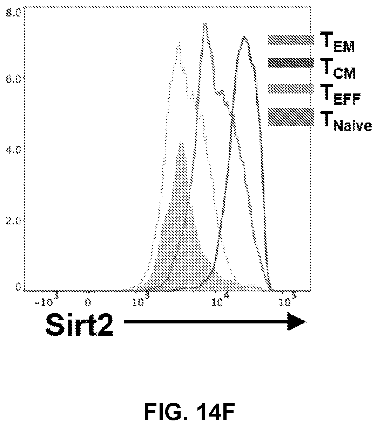

FIGs. 14A to 14F show Sirt2 expression is induced upon activation in T cells. FIGs. 14A to 14C show Sirt2 expression levels were determined by Western blot on WT and Sirt2KO (FIG. 14A) splenocytes and lymph node (LN) T cells, (FIG. 14B) CD3+ enriched T cells and (FIG. 14C) CD4+ enriched T cells, naïve (Day 0) and CD3-stimulated for 1-3 days. Actin levels were used as control loading. FIG. 14D shows Sirt2 expression levels were determined by flow cytometry on non-stimulated and CD3-stimulated WT CD8+ T cells, (n = 2). FMO, Fluorescence Minus One control. FIG. 14E shows Sirt2 expression levels of CD8+ TILs isolated from subcutaneous B16F10 tumor nodules from WT mice were compared to splenic CD8+ T cells by flow cytometry (n = 3). FIG. 14F shows Sirt2 expression levels in naïve, effector (TEFF), effector memory (TEM), and central memory (TCM) subsets of CD8+ T cells from TILs were determined by flow cytometry (n = 3). Data are representative of at least one (FIG. 14A), two (FIGs. 14C-14F), and three (FIG. 14B) independent experiments.

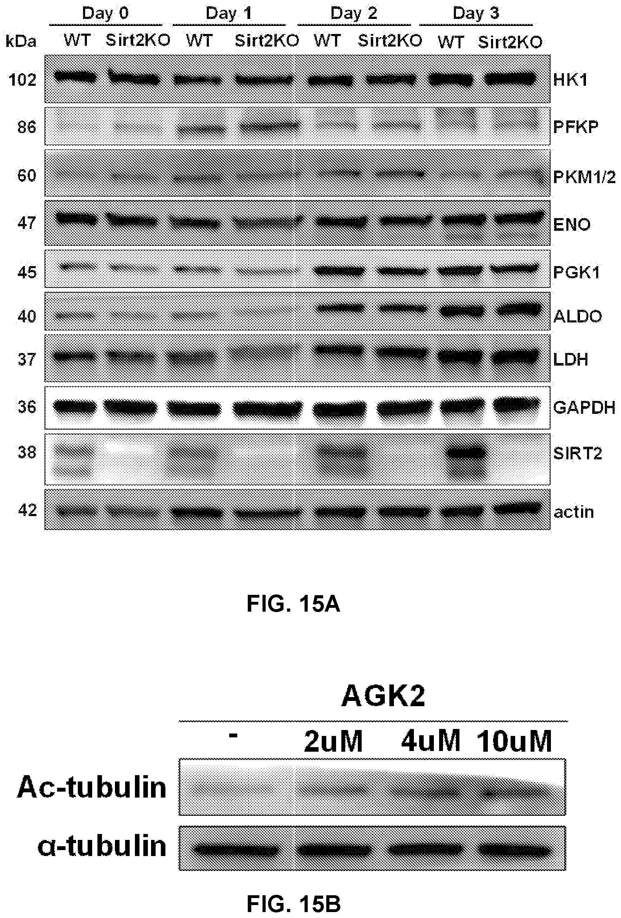

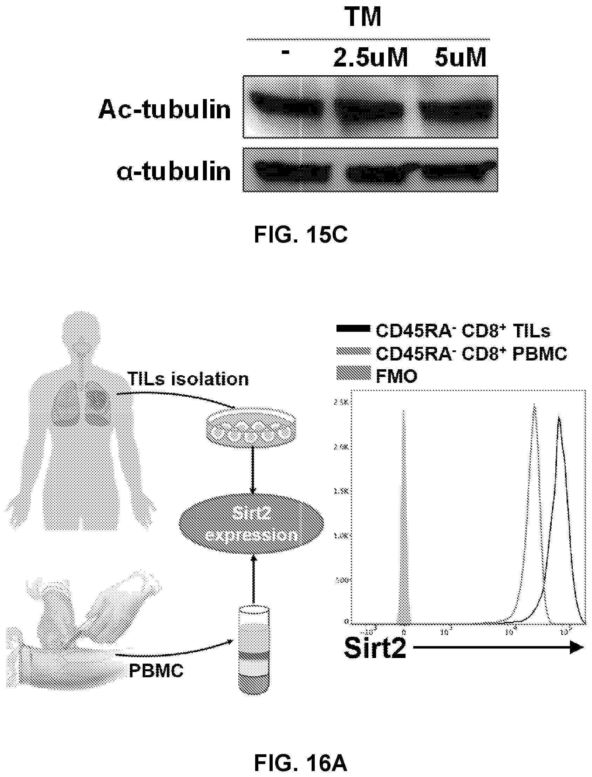

FIGs. 15A to 15C show glycolytic enzymes expression levels of: HK1, PFKP, PKM1/2, Enolase, PGK1, Aldolase, GAPDH and LDH were determined by Western blot on WT and Sirt2KO CD3+ enriched T cells naïve (Day 0) and CD3-stimulated for 1-3 days. Actin levels were used as control loading. FIGs. 15B to 15C shows human CD3+ enriched T cells were cultured with the indicated concentrations of AGK2 (FIG. 15B) or thiomyristoyl (TM) (FIG. 15C) for 48 hours. Acetylation levels of tubulin were determined by Western blot on protein extracts. Total α-tubulin levels were used as control loading. Data are representative of at least two (FIG. 15A), three (FIG. 15C), and six (FIG. 15B) independent experiments.

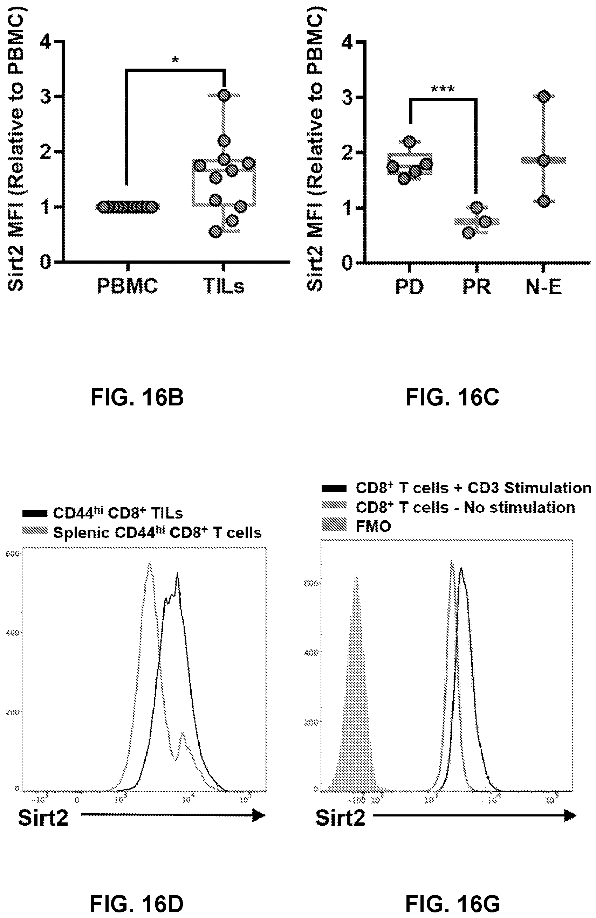

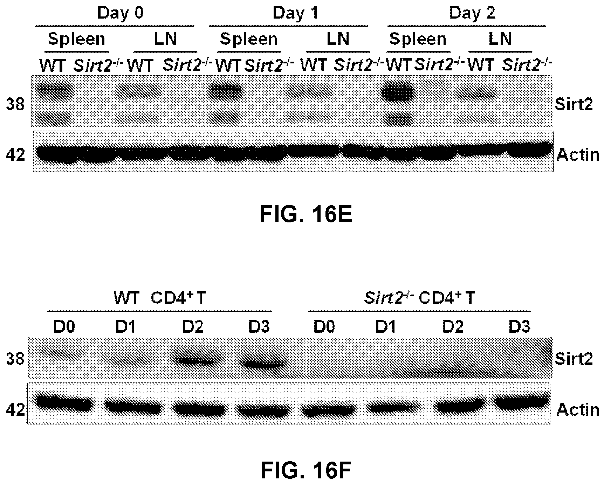

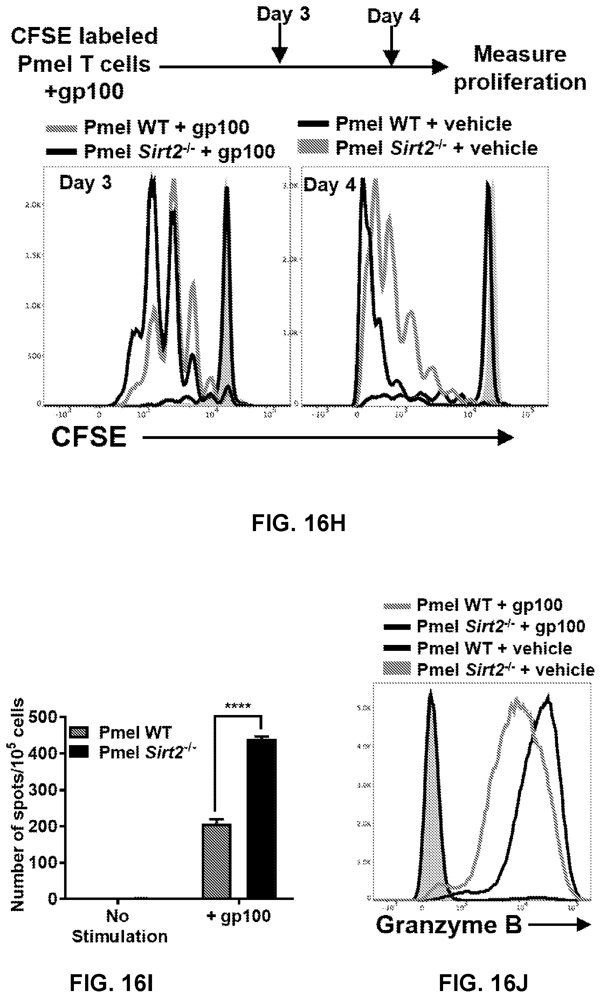

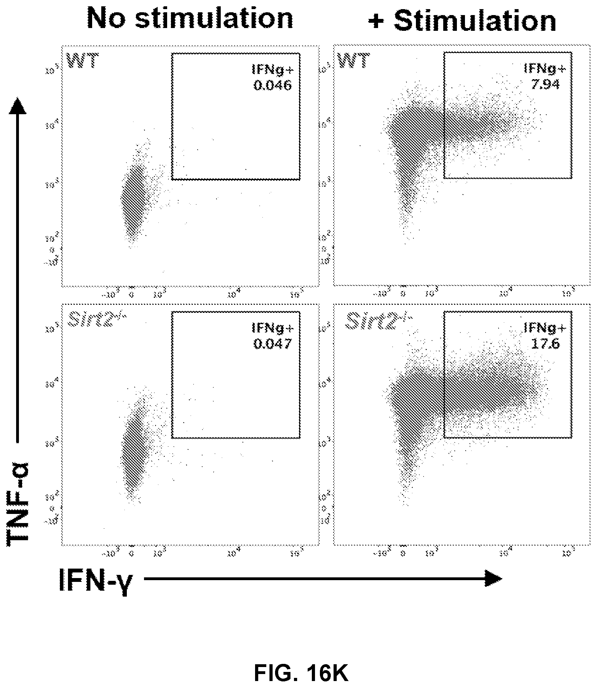

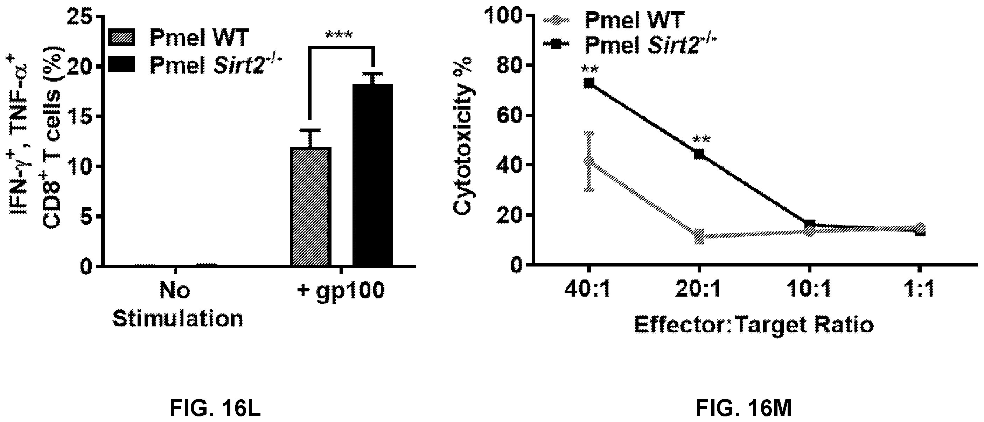

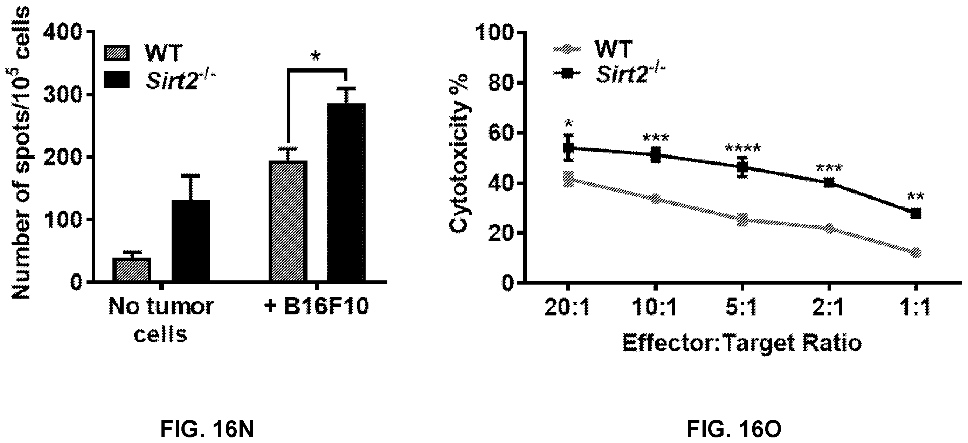

FIGs. 16A to 16O show Sirt2 Expression is induced within the TME and *Sirt2*^{*-*/*-*}T Cells are Hyper-Reactive to Antigenic Stimulation *Ex Vivo.* FIG. 16A shows Sirt2 expression levels in human CD45RA⁻ CD8⁺ TILs were compared to the corresponding PBMC CD45RA⁻ CD8⁺ T cells by flow cytometry. FMO, Fluorescence Minus One control. One human sample is shown and is representative of 11 patient samples. FIG. 16B shows Sirt2 expression levels in CD45RA⁻ CD8⁺ TILs analyzed by flow cytometry were normalized to the corresponding PBMC CD45RA⁻ CD8⁺ T cells and plotted as shown. FIG. 16C shows Sirt2 expression levels in human TILs were correlated to the clinical response to immunotherapy. PD, progressive disease; PR, partial response; N-E, non-evaluable response. FIG. 16D shows Sirt2 expression levels in CD44^{hi} CD8⁺ TILs isolated from subcutaneous B16F10 tumor nodules from WT mice were compared to splenic CD44^{hi} CD8⁺ T cells by flow cytometry (n = 3). FIGs. 16E and 16F show Sirt2 expression levels were determined by Western blot on WT and *Sirt2*^{*-*/*-*} T cells from spleen and lymph node (LN) (FIG. 16E) and CD4⁺ enriched T cells (Fig. 16F), naïve (Day 0) and CD3-stimulated for 1-3 days. Actin levels were used as loading control. FIG. 16G shows Sirt2 expression levels were determined by flow cytometry on non-stimulated and CD3-stimulated WT CD8⁺ T cells (n = 2). FMO, Fluorescence Minus One control. Fig. 16H shows CFSE-labeled WT and *Sirt2* ^{-/-} Pmel splenocytes were stimulated with gp100 peptide or remained unstimulated and CD8⁺ T cell proliferation was measured by flow cytometry 3 and 4 days post-activation (n = 7). FIGs. 16I to 16M show functional characterization of WT and *Sirt2*^{*-*/*-*} Pmel splenocytes stimulated with gp100 peptide or remained unstimulated for 48 hr. FIG. 16I shows IFN-γ ELISPOT assay was performed (n = 4). FIG. 16J shows flow cytometric analysis of intracellular granzyme B on WT and *Sirt2*^{*-*/*-*} Pmel CD8⁺ T cells (n = 7). FIG. 16K shows flow cytometric analysis of cytokines TNF-α and INF-γ expression in WT and *Sirt2*^{*-*/*-*} Pmel CD8⁺ T cells. Numbers in quadrants indicate percentage of cells. FIG. 16L shows frequencies of TNF-α⁺ IFN-γ⁺ CD8⁺ Pmel T cells are shown (n = 7). FIG. 16M shows cytotoxic activities were determined by LDH release assay on gp100-stimulated WT and *Sirt2*^{*-*/*-*} Pmel CD8⁺ T cells after co-culture with B16F10 target cells at the indicated Effector: Target ratios (n = 3). FIGs. 16N and 16O show functional characterization of CD3⁺ TILs isolated from B16F10 tumor nodules from WT or *Sirt2*^{*-*/*-*} mice. Fig. 16N shows IFN-γ Elispot assay was performed on CD3⁺ TILs after re-challenge with vehicle or irradiated B16F10 cells *ex vivo* (n = 3). FIG. 16O shows cytotoxic activities were determined by LDH release assay on CD3⁺ TILs after co-culture with B16F10 target cells at the indicated Effector:Target ratios (n = 3). Data are representative of at least one (FIGs. 16A-16C and 16E), two (FIGs. 16G, 16D, 16N and 16O), and three (FIGs. 16F, 16H-16L) and five (FIG. 16M) independent experiments. Data are mean ± SEM. *P* values are determined by paired two-tailed Student's t-test (FIG. 16B), unpaired two-tailed Student's t-test (FIG. 16C) and two-way ANOVA (FIGs. 16I, 16L-16O). **P* < 0.05, ***P* < 0.01, ****P* < 0.001, *****P* < 0.0001.

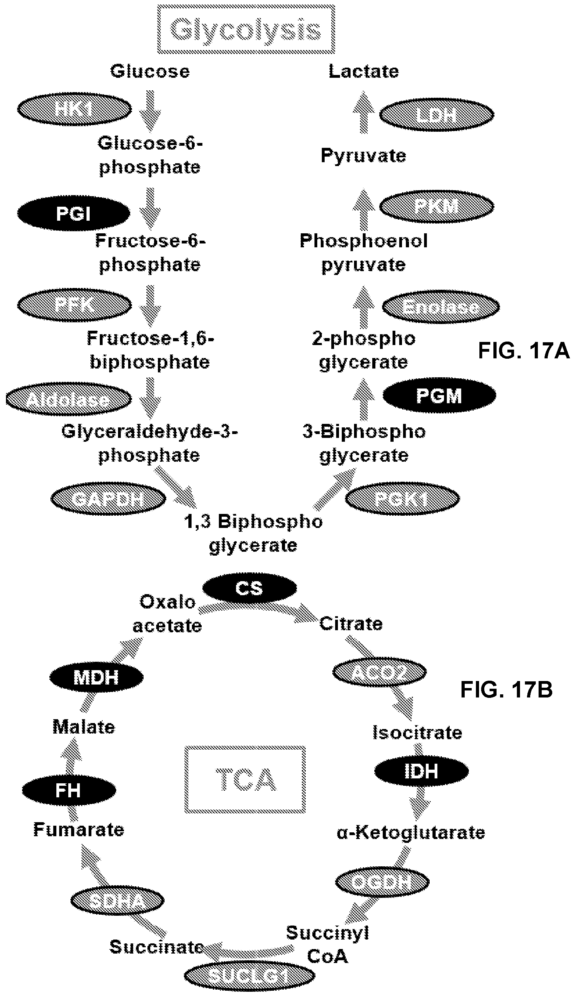

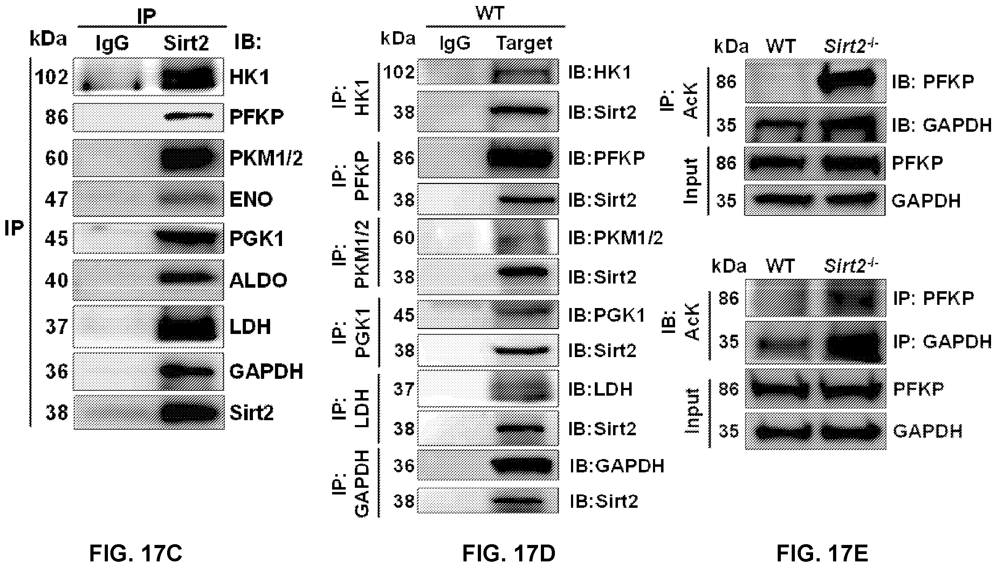

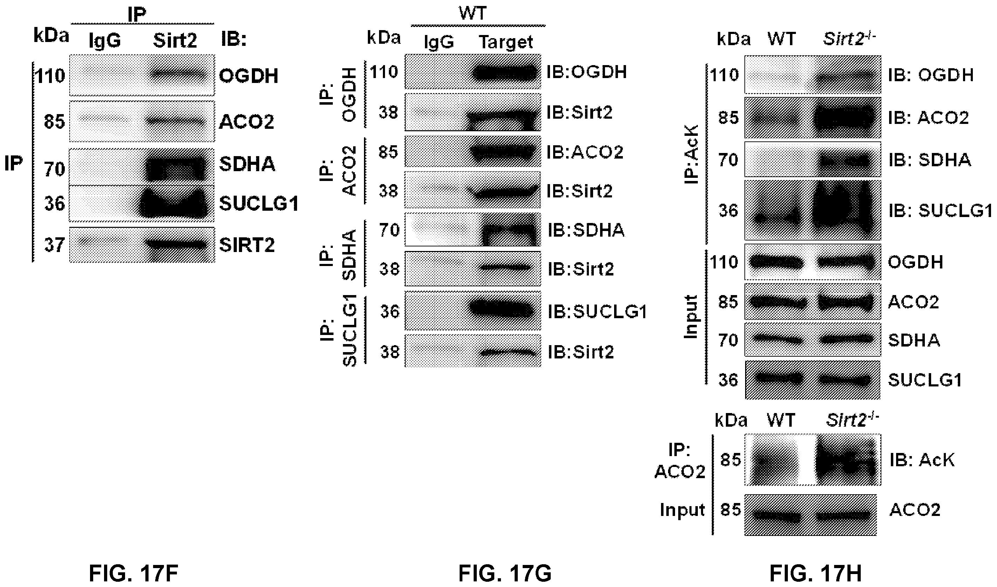

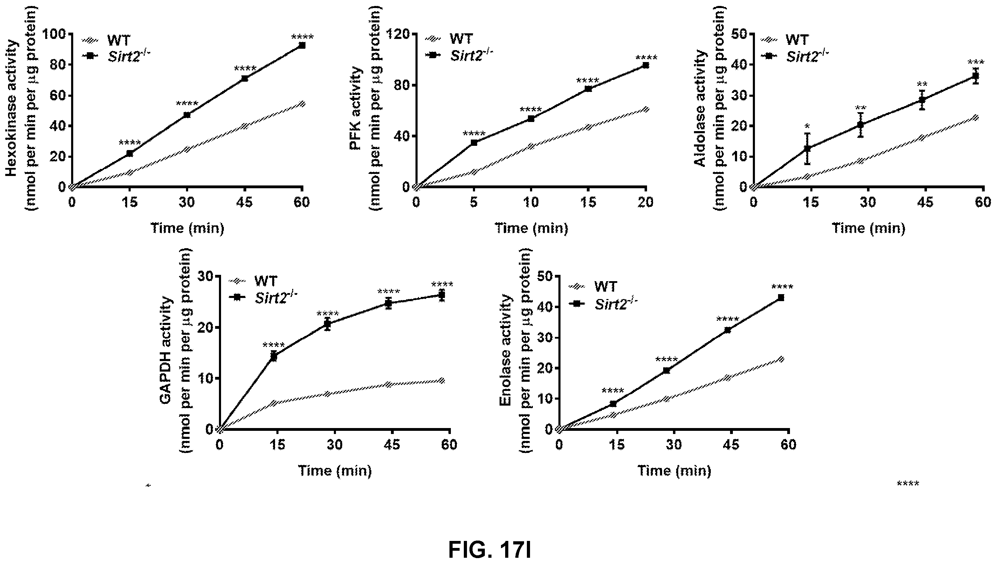

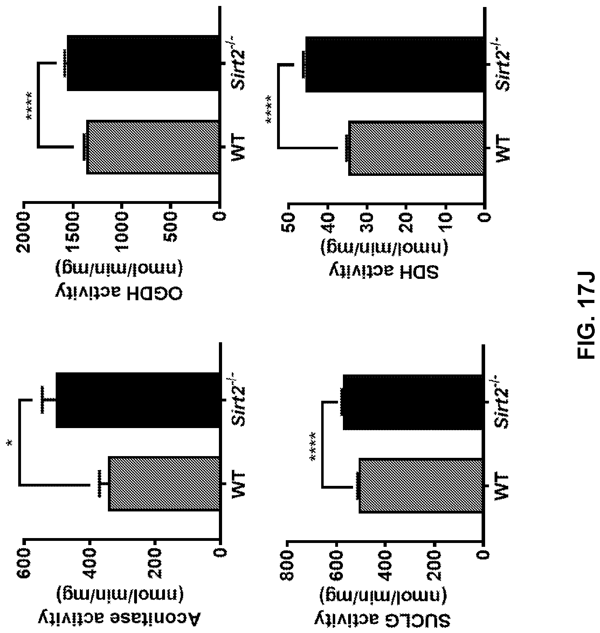

FIGs. 17A to 17J show Sirt2 Directly Interacts with Glycolytic and TCA-Cycle Enzymes and Impairs their Enzymatic Activity. FIGs. 17A and 17B are schematic diagrams of the glycolytic pathway (FIG. 17A) and the TCA-cycle (FIG. 17B). Enzymes identified to interact with Sirt2 via IP-MS/MS analyses are indicated in red. FIGs. 17C and 17D show co-immunoprecipitation of HK1, PFKP, PKM1/2, ENO, PGK1, ALDO, LDH, GAPDH from the glycolytic pathway (FIG. 17C) and OGDH, ACO2, SDHA, SUCLG1 from the TCA-cycle (FIG. 17D) by anti-Sirt2 antibody on protein extracts from activated WT CD3⁺ T cells followed by immunoblotting with anti-Sirt2 and the corresponding specific antibodies. FIGs 17E and 17F show co-immunoprecipitation of Sirt2 by anti-HK1, anti-PKM1/2, anti-PGK1, anti-LDH, anti-GAPDH from the glycolytic pathway (FIG. 17E) and anti-OGDH, anti-ACO2, anti-SDHA, anti-SUCLG1 from the TCA-cycle (FIG. 17F) on protein extracts from activated WT CD3⁺ T cells followed by immunoblotting with anti-Sirt2 or the corresponding specific antibodies. FIGs 17G and 17H show determination of acetylation levels of GAPDH, PFKP from the glycolytic pathway (FIG. 17G) and OGDH, ACO2 SDHA, SUCLG1 from the TCA-cycle (FIG. 17H) by immunoprecipitation of acetylated lysine (AcK), PFKP, GAPDH or ACO2 on activated WT and *Sirt2*^{*-*/*-*} CD3⁺ T cells protein extracts followed by immunoblotting with anti-AcK or the corresponding specific antibodies. Western blotting of each target using equal amounts of total protein extracts is shown as control (Input) of equal concentrations for the IP. FIGs. 17I and 17J show enzymatic activities of aldolase (n = 5), enolase (n = 4), GAPDH (n = 5), PFK (n = 5), hexokinase (n = 6) from the glycolytic pathway (FIG. 17I), and aconitase (n = 3), SUCLG (n = 6), SDH (n = 8), OGDH (n = 6) from the TCA-cycle (FIG. 17J) were assessed from activated WT and *Sirt2*^{*-*/*-*} CD3⁺ T cell extracts. Data are mean ± SEM. *P* values are determined by two-tailed Student's t-test. **P* <0.05, ****P* < 0.001, *****P* < 0.0001.

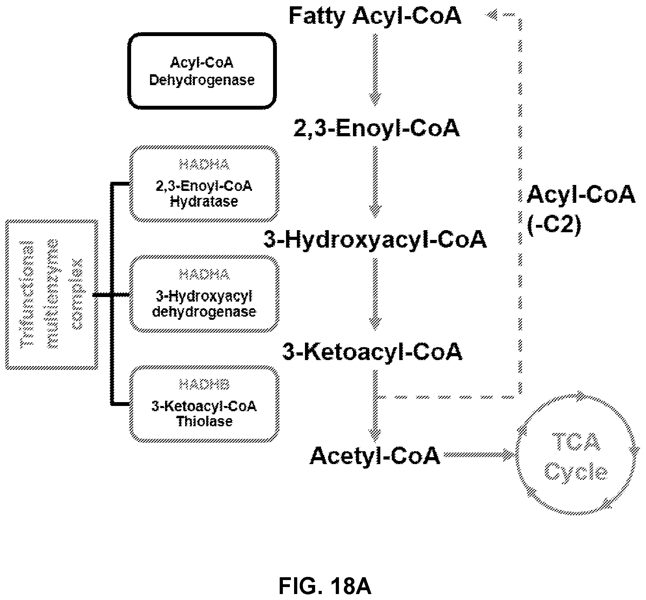

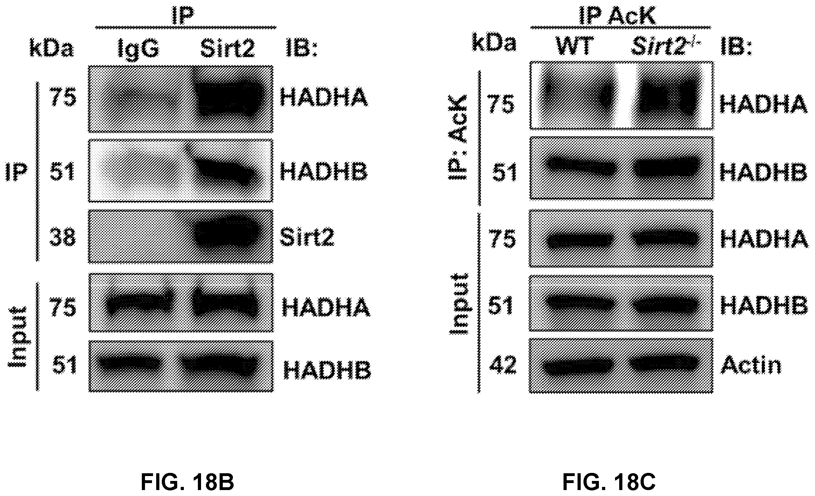

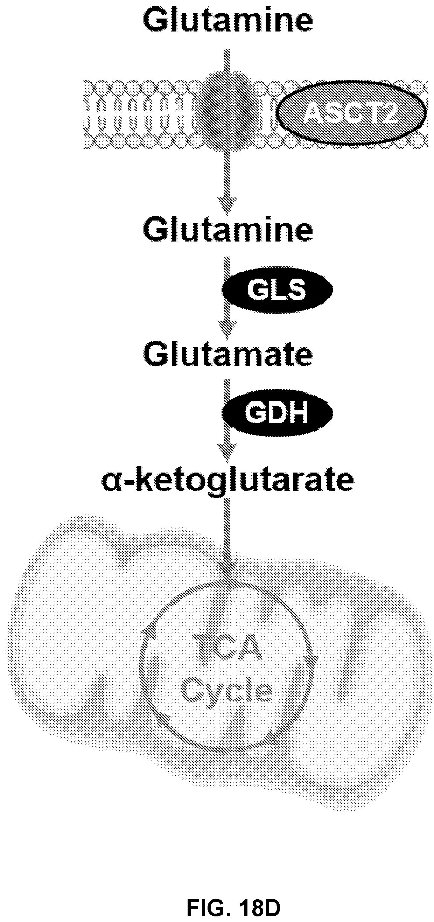

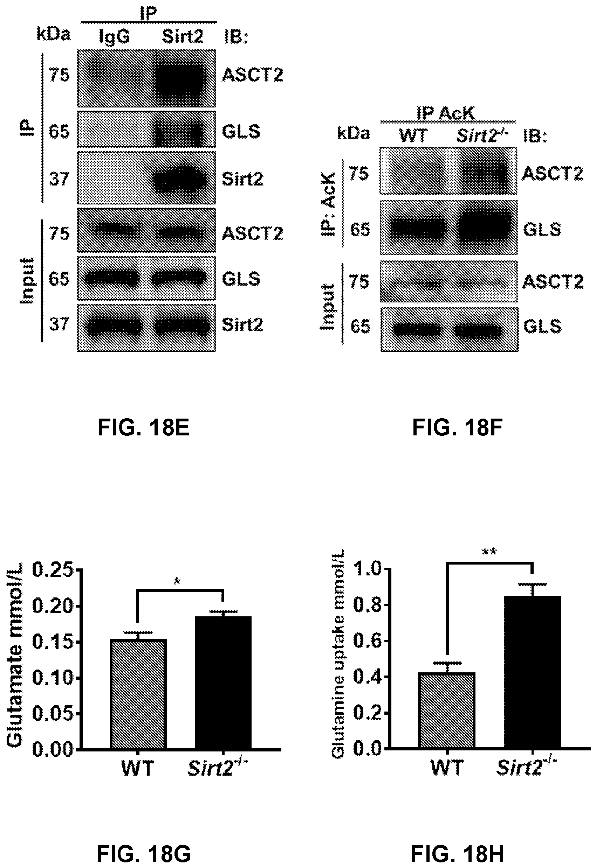

FIGs. 18A to 18H show Sirt2 Directly Interacts with FAO and Glutaminolysis Enzymes. FIGs. 18A and 18D are schematic diagrams of FAO (FIG. 18A) and Glutaminolysis (FIG. 18D) pathways. Enzymes identified to interact with Sirt2 via IP-MS/MS analyses are indicated in red. FIGs. 18B and 18E show co-immunoprecipitation of HADHA, HADHA from the FAO pathway (FIG. 18B) and ASCT2, GLS from the glutaminolysis pathway (FIG. 18E) by anti-Sirt2 antibody on protein extracts from activated WT CD3⁺ T cells followed by immunoblotting with anti-Sirt2 and the corresponding specific antibodies. FIG. 18C and 18F show determination of acetylation levels of HADHA and HADHA from the FAO pathway (FIG. 18C), and ASCT2 and GLS from the glutaminolysis pathway (FIG. 18F) by immunoprecipitation of acetylated lysine (AcK) on activated WT and *Sirt2*^{*-*/*-*} CD3⁺ T cell protein extracts followed by immunoblotting with the corresponding specific antibodies. FIGs. 18B-18C and 18E-18F are Western blots of each target using equal amounts of total protein extracts is shown as the input control for IP. FIG. 18G and 18H show glutamine consumption (FIG. 18G) and Glutamate production (FIG. 18H) were measured using YSI 2900 Biochemistry Analyzer from activated WT and *Sirt2*^{*-*/*-*}CD3⁺ T cell culture media (n=3). Data are mean ± SEM. P values are determined by two-tailed Student's t-test. **P* <0.05, ***P* < 0.01.

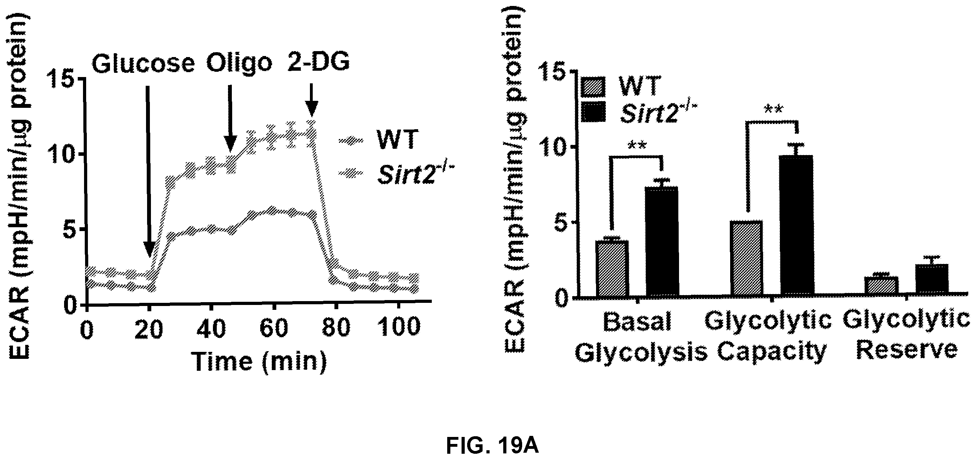

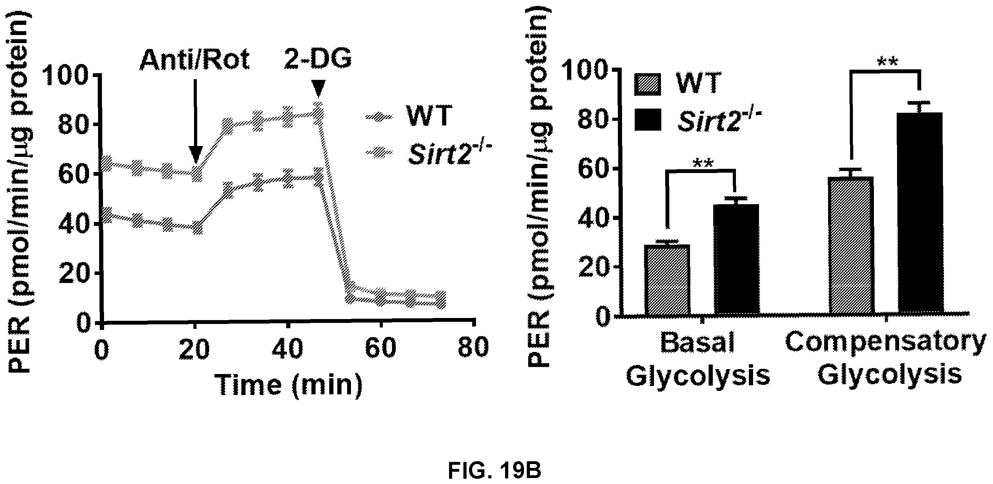

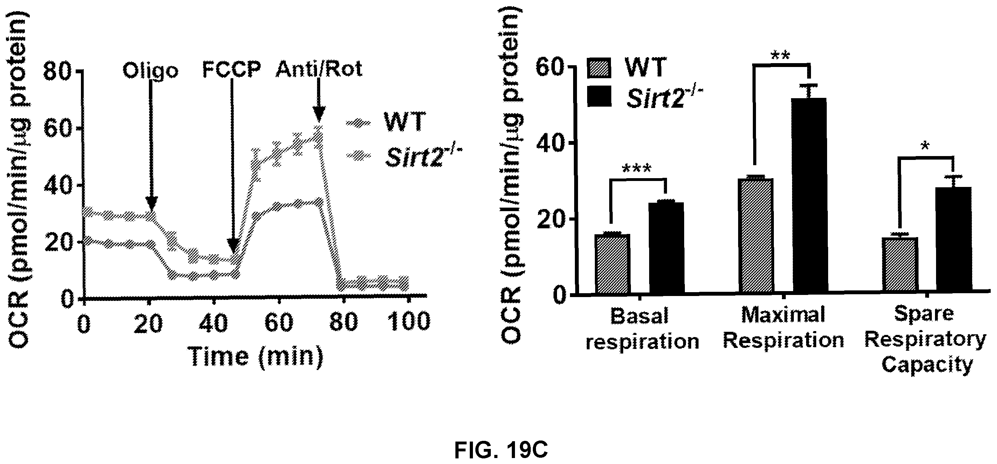

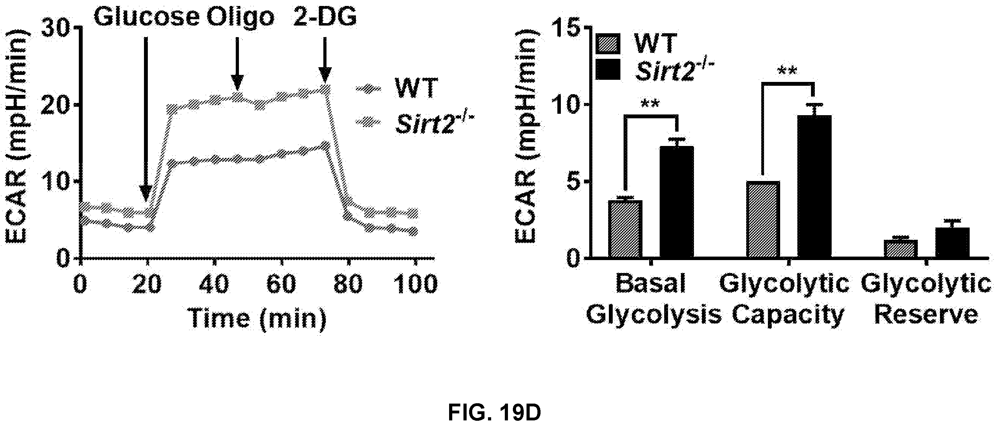

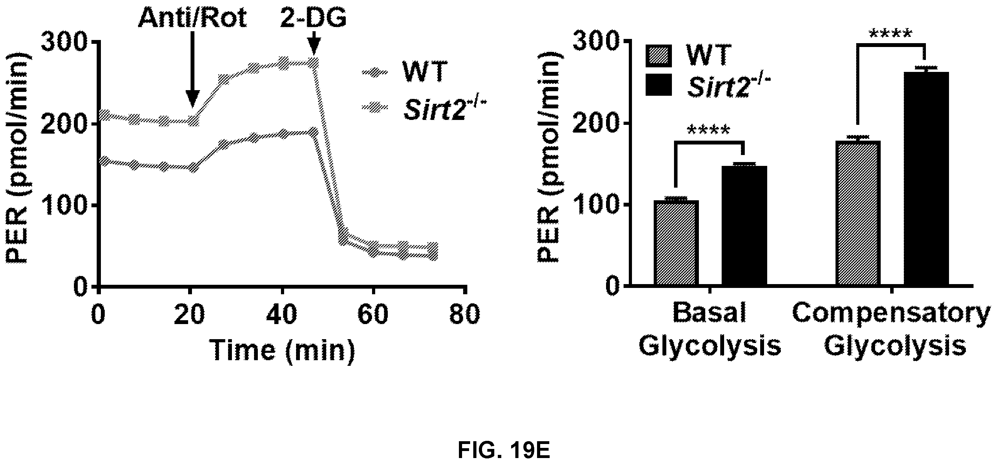

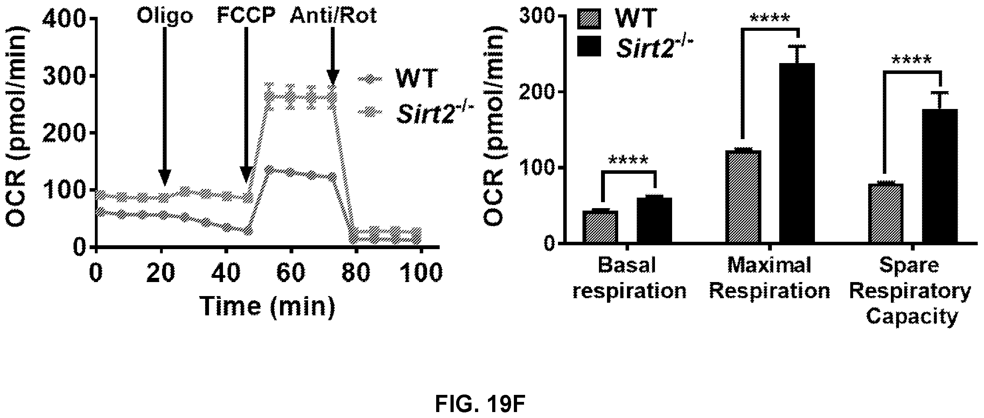

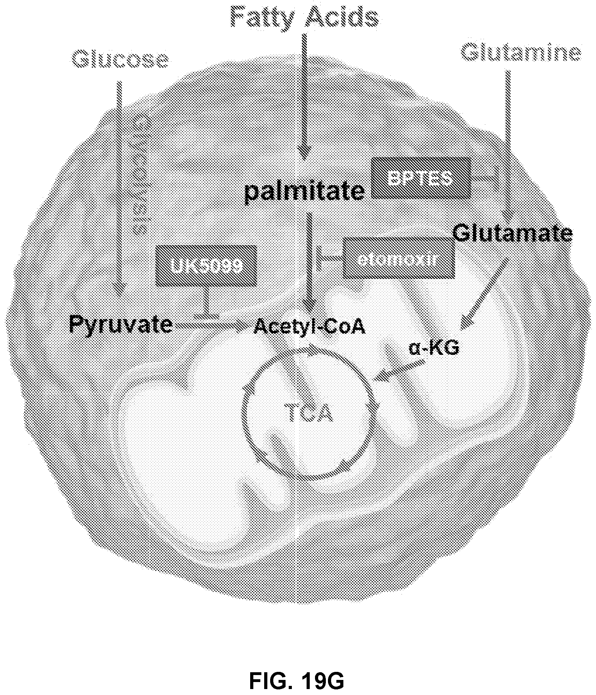

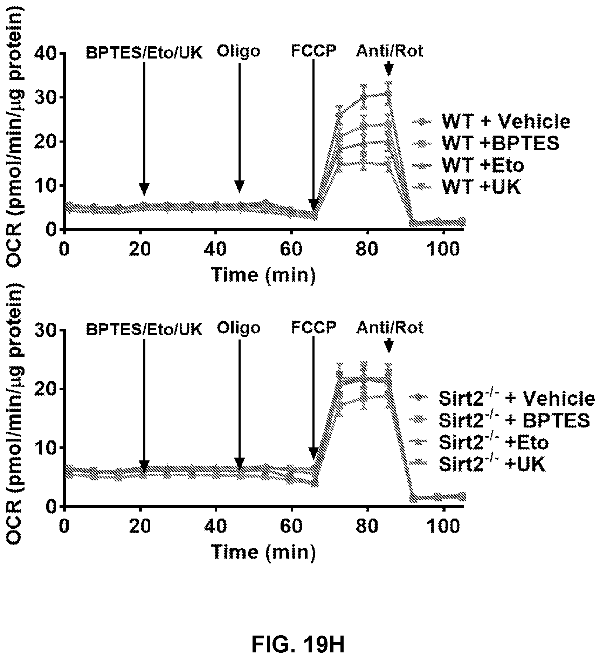

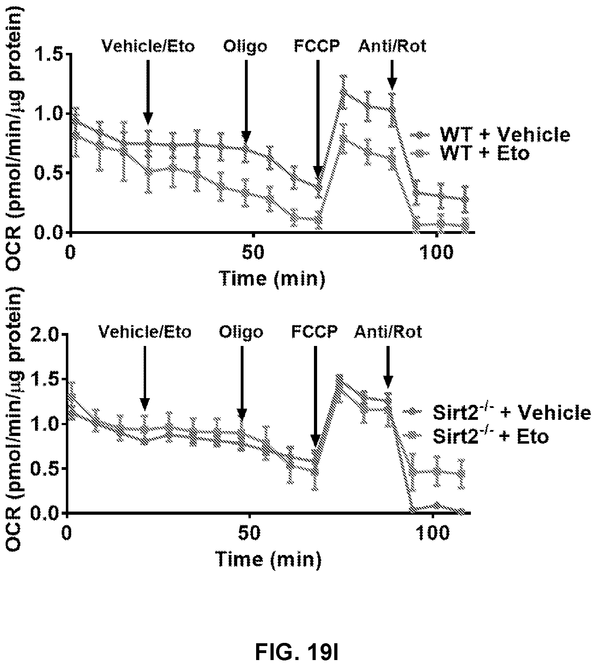

FIGs. 19A to 19K show increased glycolysis and OxPhos in *Sirt2*^{*-*/*-*} T Cells. FIGs. 19A and 19D show (*Left*) glycolytic flux measured as extracellular acidification (ECAR) using the Seahorse XF analyzer on: CD3-stimulated WT (n = 3) and *Sirt2*^{*-*/}*⁻ (n* = 3) CD3⁺ T cells (FIG. 19A); and CD3⁺ TILs freshly isolated from B16F10 tumor nodules from WT (n =13) or *Sirt2*^{*-*/*-*} mice (n = 7) (FIG. 19D). Glucose, Oligomycin (Oligo) and 2-deoxyglucose (2-DG) were injected at the points indicated. *Right,* basal glycolytic rate, glycolytic capacity and glycolytic reserve were calculated from the corresponding curves. FIGs. 19B and 19E (*Left*) show glycolytic rate measured as proton efflux rate (PER) using the Seahorse XF analyzer on: CD3-stimulated WT (n = 4) and *Sirt2*^{*-*/}*⁻ (n* = 4) CD3⁺ T cells (FIG. 19B); and CD3⁺ TILs freshly isolated from B16F10 tumor nodules from WT (n =11) or *Sirt2*^{*-*/*-*} mice (n = 6) (FIG. 19E). Antimycin A/Rotenone (Anti/Rot) and 2-DG were injected at the points indicated. FIGs. 19B and 19E (*Right*) show basal glycolytic rate and compensatory glycolysis calculated from the corresponding curves. FIGs. 19C and 19F (*Left*) show mitochondrial activity measured as oxygen consumption rate (OCR) using the Seahorse XF analyzer on CD3-stimulated WT and *Sirt2*^{*-*/*-*} CD3⁺ splenic T cells (FIG. 19C, n = 3 each group); and CD3⁺ TILs freshly isolated from B16F10 tumor nodules from WT (FIG. 19F, n = 11) and *Sirt2^{-,-} (n* = 8) mice. Oligomycin (Oligo), FCCP and Antimycin A/Rotenone (Anti/Rot) were injected at the points indicated. FIGs. 19C and 19F (*Right*) show basal respiration, maximal respiration and spare respiratory capacity were calculated from the corresponding curves. FIG. 19G shows measurement of oxygen consumption in stimulated T cells treated with mitochondrial pyruvate carrier (UK5099) or GLS (BPTES) or CPT1A (Etomoxir) inhibitors to determine the relative contribution of glycolysis, glutaminolysis and FAO as fuels for mitochondrial respiration. FIG. 19H shows mitochondrial activity measured as oxygen consumption rate (OCR) using the Seahorse XF analyzer on CD3-stimulated WT (*upper panel*) and *Sirt2*^{*-*/*-*} (*lower panel*) CD3⁺ splenic T cells (n = 10-12). BPTES/Etomoxir/UK5099, Oligomycin (Oligo), FCCP and Antimycin A/Rotenone (Anti/Rot) were injected at the time points indicated. FIG. 19I shows mitochondrial activity measured as oxygen consumption rate (OCR) using the Seahorse XF analyzer on TILs freshly isolated from B16F10 tumor nodules from WT (*upper panel*) and *Sirt2*^{*-*/*-*} (*lower panel*) mice (n = 4-7). Vehicle/Etomoxir, Oligomycin (Oligo), FCCP and Antimycin A/Rotenone (Anti/Rot) were injected at the time points indicated. FIG. 19J shows WT *versus Sirt2*^{*-*/*-*} CD3⁺ T cells activated for 3 days in completed media and transferred to substrate-limited media overnight. Palmitate or BSA substrates were added immediately before the assay. OCR was measured under basal conditions and in response to sequential injections of vehicle, or Etomoxir and then oligomycin, FCCP and rotenone/antimycin A. Consumption of exogenous (brown) and endogenous (yellow) FA by WT *versus Sirt2*^{*-*/*-*} CD3⁺ T cells were calculated as described in the method section (n=4-6). FIG. 19K shows WT *versus Sirt2*^{*-*/*-*} OT-II splenocytes were activated with OVA-peptide for 3 days and subsequently cultured in the presence of IL-15 for 4 additional days to generate T_{M} cells. *Left,* OT-II CD4⁺ T cells were analyzed by CD3 and CD4 expression. Numbers in quadrants indicate the percentage of OT-II CD4⁺ T cells after exclusion of dead cells. *Right,* Graphic representation of the frequency of live *Sirt2*^{*-*/*-*} OT-II T_{M} cells relative to WT OT-II T_{M} cells (n = 4). Data are representative of two (FIGs. 19E, 19F, 19J, 19K), three (FIGs. 19A, 19C, 19D, 19I) and four (FIGs. 19B, 19H) independent experiments. Data are mean ± SEM. *P* values are determined by two-tailed Student's t-test (FIGs. 19A-19F, 19J, 19K). **P* < 0.05, ***P* < 0.01, ****P* < 0.001, *****P* < 0.0001.

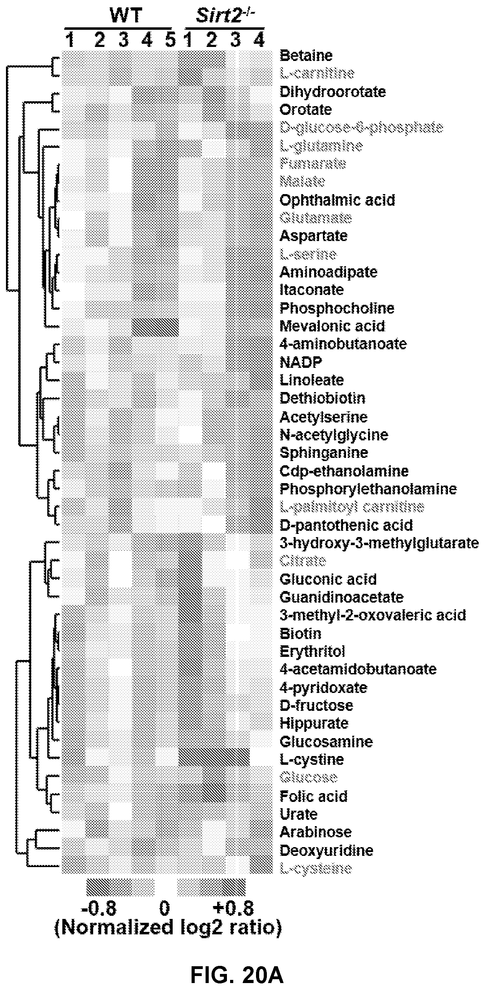

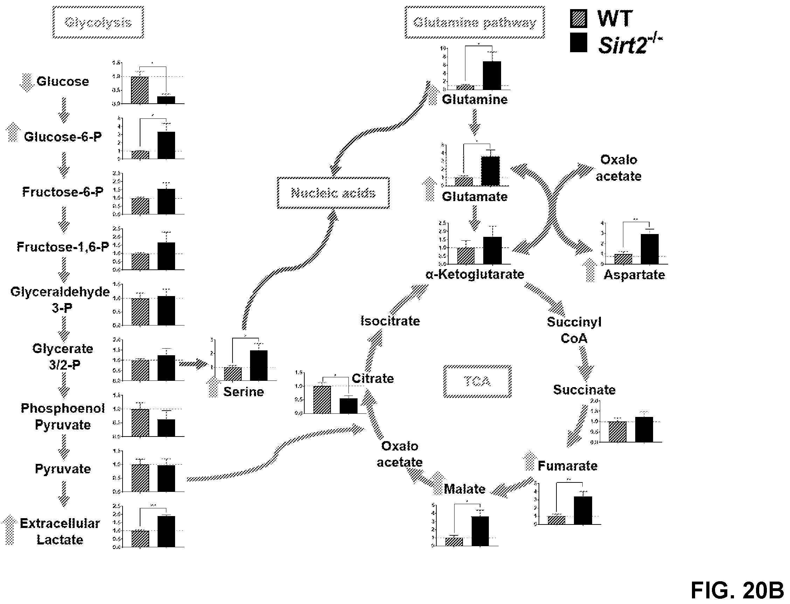

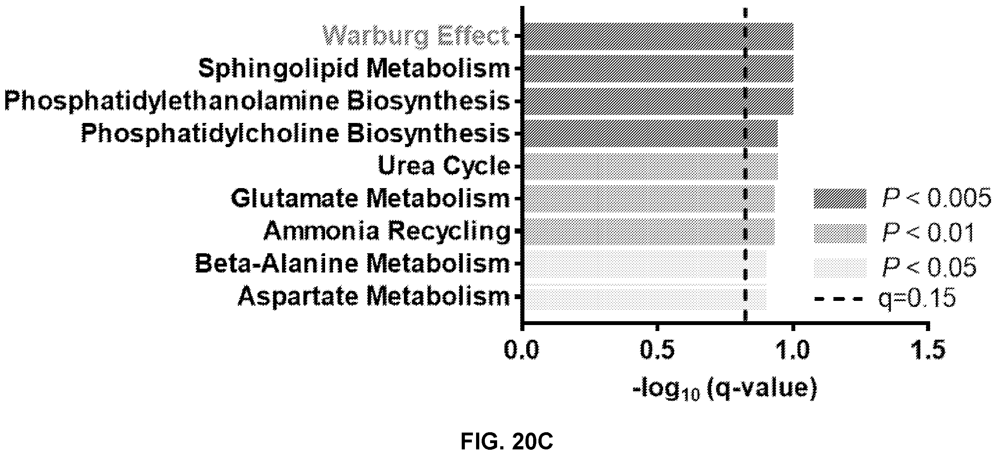

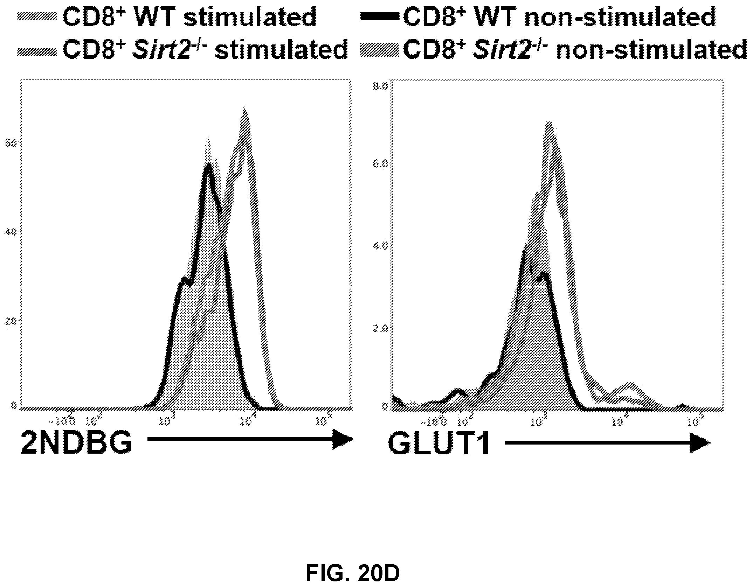

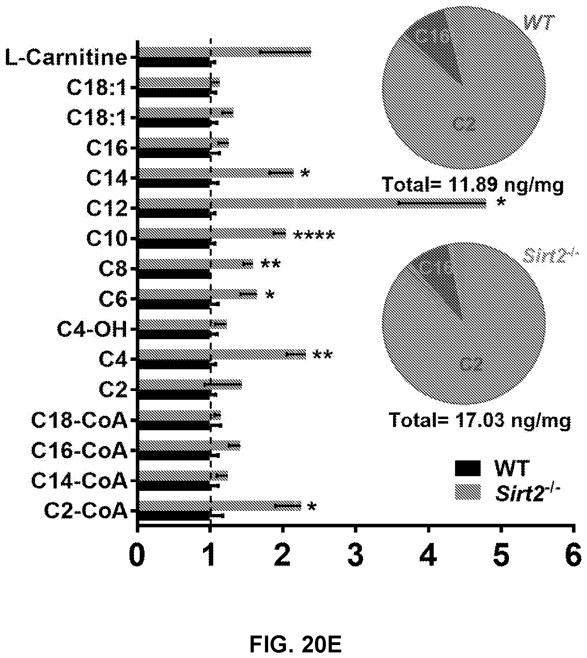

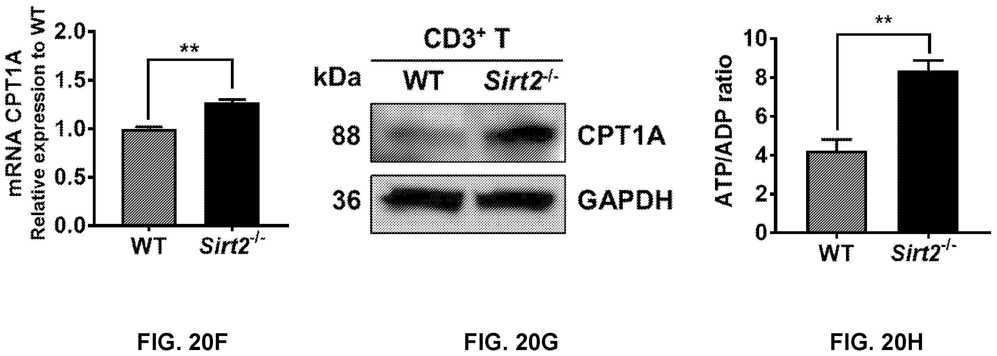

FIGs. 20A to 20H show metabolic reprogramming in activated *Sirt2*^{*-*/*-*}T Cells. FIGs. 20A and 20B show CD8⁺ enriched T cells from WT (n = 5) and *Sirt2*^{*-*/*-*}(n = 4) mice were stimulated *ex vivo* with anti-CD3 for 72 hr. Intracellular metabolites were extracted from T cell lysates and analyzed using high-resolution LC-MS/MS. FIG. 20A contains heat maps with unsupervised hierarchical clustering showing relative abundance of differentially expressed metabolites. FIGs. 20B show relative levels of metabolites implicated in glycolysis, TCA-cycle and glutaminolysis are shown. Extracellular lactate production was measured using YSI 2900 Biochemistry Analyzer. Samples are normalized to WT T cells as indicated by dashed lines. FIG. 20C shows metabolite set enrichment analysis of the upregulated metabolic pathways in *Sirt2*^{*-*/*-*} CD8⁺ T cells compared to WT was performed from FIG. 20A. A false discovery rate q-value < 0.15 was used as a cut-off. FIG. 20D show splenocytes from WT (n = 3) and *Sirt2*^{*-*/}*⁻ (n* = 3) mice were stimulated with anti-CD3 or remained unstimulated. At Day 3, 2-NBDG uptake (*left panel*) and GLUT1 expression (*right panel*) were assessed by flow cytometry on CD8⁺ T cells. FIG. 20E shows CD8⁺ enriched T cells from WT (n = 5) and *Sirt2*^{*-*/}*⁻ (n* = 6) mice were stimulated ex *vivo* with anti-CD3 for 72 hr. Intracellular metabolites were extracted from T cell lysates and analyzed using targeted high-resolution LC-MS/MS. FIG. 20A shows relative levels of Free Carnitine, Acyl-Carnitines, Acyl-CoA and Acetyl-CoA are shown. Samples are normalized to WT T cells and the dashed line represents the ratio of 1. Pie charts indicate the relative abundance of different Acyl-Carnitines (C2-C18). Total numbers indicate the amount of combined Acyl-Carnitines. FIG. 20F shows relative CPT1A mRNA expression levels were determined by RNA-sequencing analysis on activated WT and *Sirt2*^{*-*/*-*} CD4⁺ T cells RNA extracts. Samples are normalized to WT T cells (n = 3). FIG. 20G shows CPT1A protein expression levels were determined by Western blot on activated WT and *Sirt2*^{*-*/*-*} CD3⁺ T cells protein extracts. GAPDH levels were used as the loading control. FIG. 20H shows ATP/ADP ratio in CD3-stimulated WT and *Sirt2*^{*-*/*-*} CD3⁺ T cell extracts was measured using a bioluminescent assay (n = 3). Data are representative of at least one (FIG. 20E and 20F), two (FIG. 20A-20C), three (FIG. 20D) and four (FIG. 20G, 20H) independent experiments. Data are mean ± SEM. *P* values are determined by two-tailed Student's t-test, (FIGs. 20B, 20E, 20F, 20H). **P* < 0.05, ***P* < 0.01.

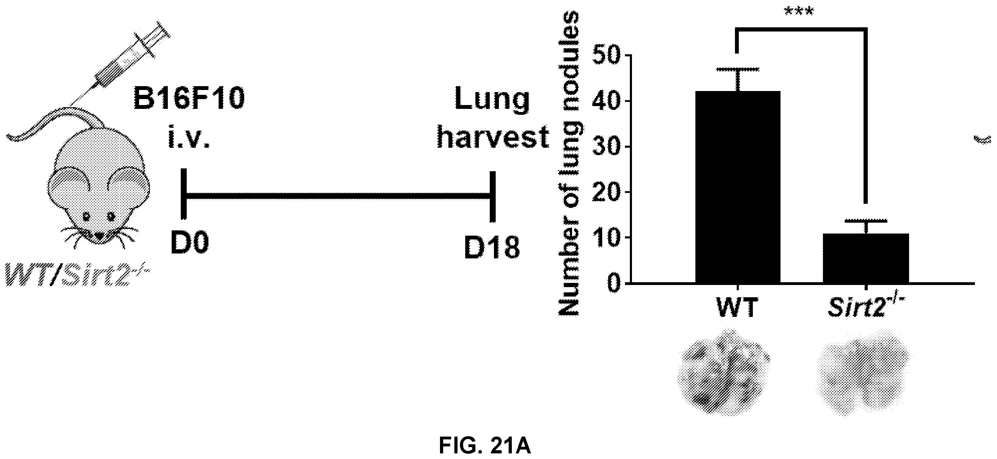

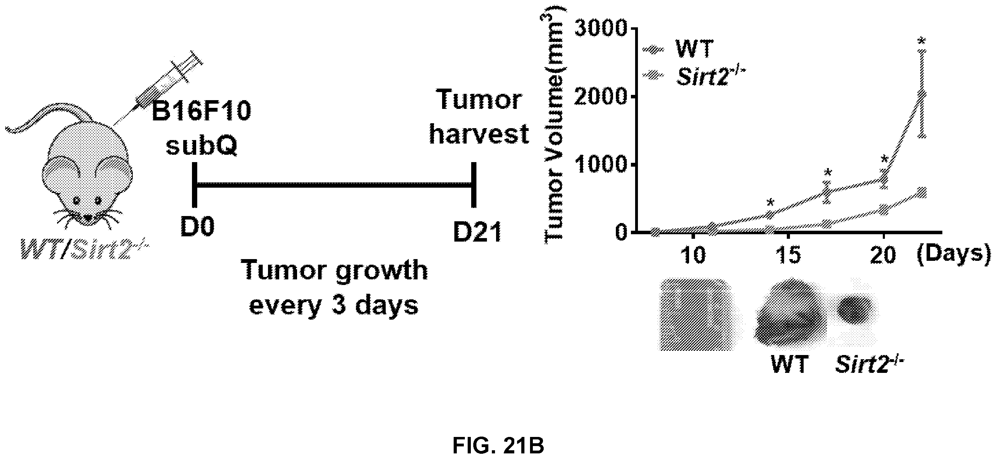

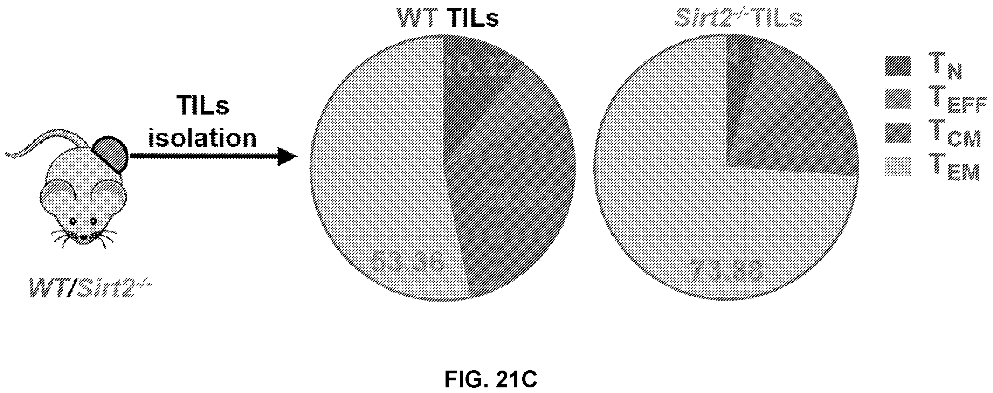

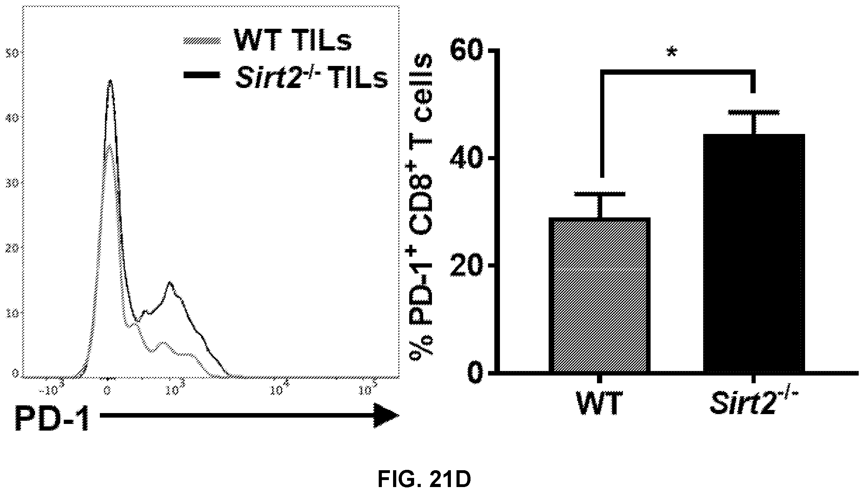

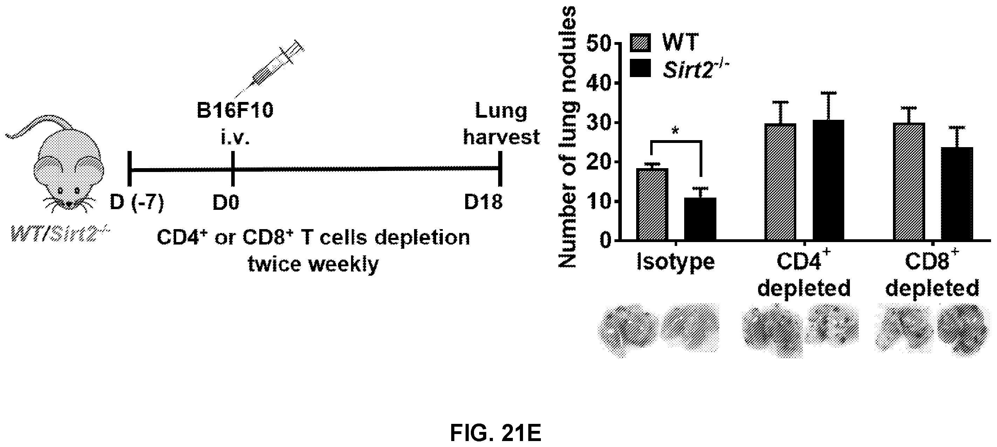

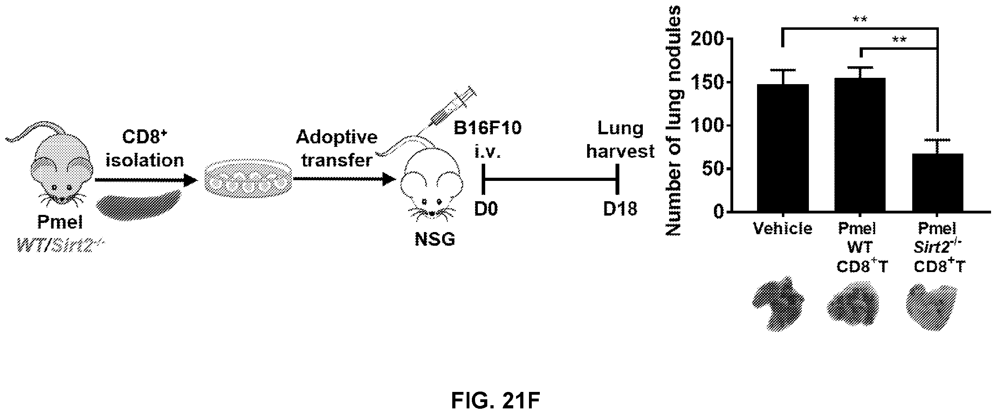

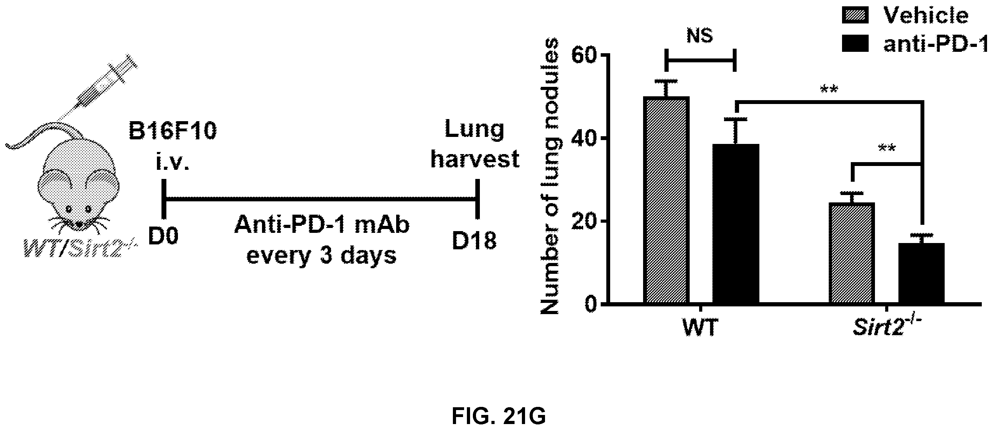

FIGs. 21A to 21G show Sirt2 Deficiency Augments T Cell Mediated Tumor Rejection *In Vivo.* FIG. 21A shows number of lung metastatic nodules in WT and *Sirt2*^{-/-} mice challenged i.v. with B16F10 cells (n = 5). FIG. 21B shows subcutaneous B16F10 tumor growth curves of WT and *Sirt2*^{-/-} mice (n = 5). FIGs. 21C and 21D show TILs isolated from subcutaneous tumors from WT and *Sirt2*^{*-*/*-*} mice. FIG. 21C shows pie charts show mean percentages of naïve (T_{N}), effector (T_{EFF}), effector memory (T_{EM}) and central memory (T_{CM}) CD8⁺ T cell subsets among TILs (n = 3-4). FIG. 21D (Left) shows characterization of PD-1 expression in WT and *Sirt2*^{*-*/*-*}TILs by flow cytometry. FIG. 21D (Right) shows frequency of PD1⁺, CD8⁺ TILs is shown (n = 3-4). FIG. 21E shows number of lung metastatic nodules in WT and *Sirt2*^{*-*/*-*} mice treated with isotype control, anti-CD4, or anti-CD8 monoclonal antibodies before B16F10 i.v. challenge (n = 5). FIG. 21F shows CD8⁺ T cells isolated from spleens of WT Pmel and *Sirt2*^{*-*/*-*} Pmel mice were adoptively transferred into NSG mice and challenged i.v. with B16F10 cells. The number of metastatic nodules in the lungs was determined (n = 8). FIG. 21G shows number of lung metastatic nodules in WT and *Sirt2*^{*-*/*-*} mice challenged i.v. with B16F10 cells, and treated with either vehicle or anti-PD-1 mAb (n = 6). Data are representative of one (FIG. 21G), two (FIGs. 21C-21F), three (FIG. 21B) and four (FIG. 21A) independent experiments. Data are mean ± SEM. *P* values are determined by two-tailed Student's t-test (FIGs. 21A-21B, 21D-21E, 21G) or one-way ANOVA (FIG. 21F). NS, not significant, **P* < 0.05, ***P* < 0.01, ****P* < 0.001.

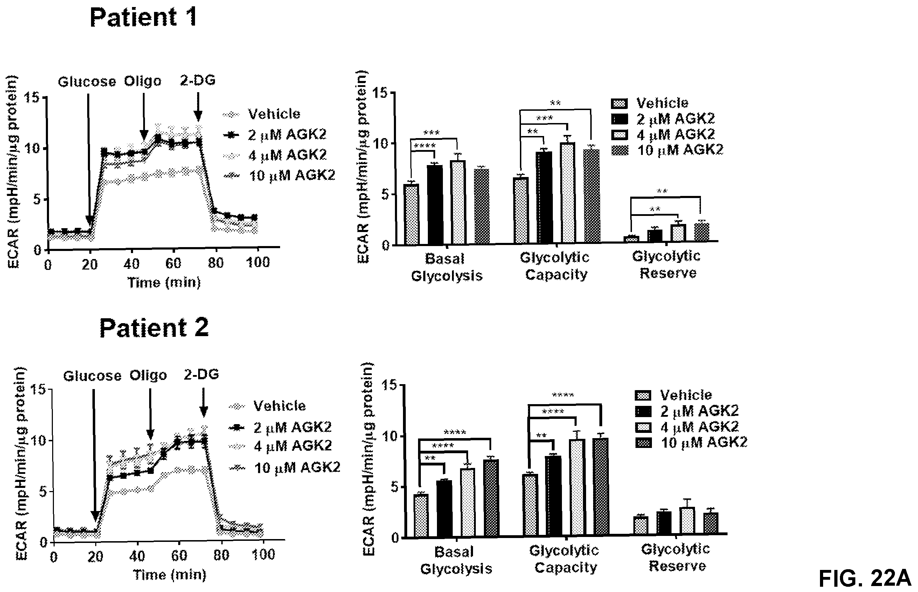

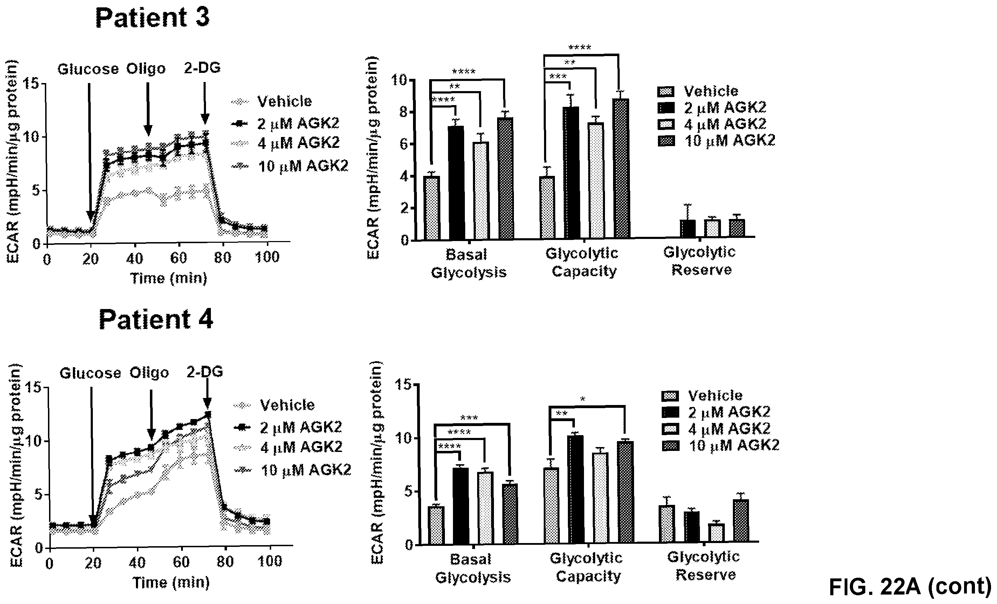

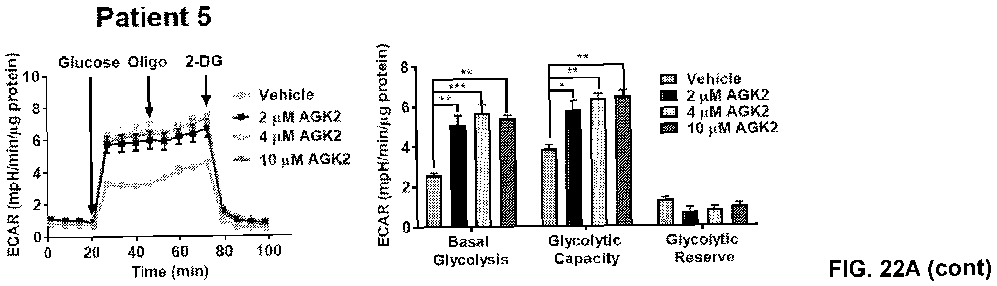

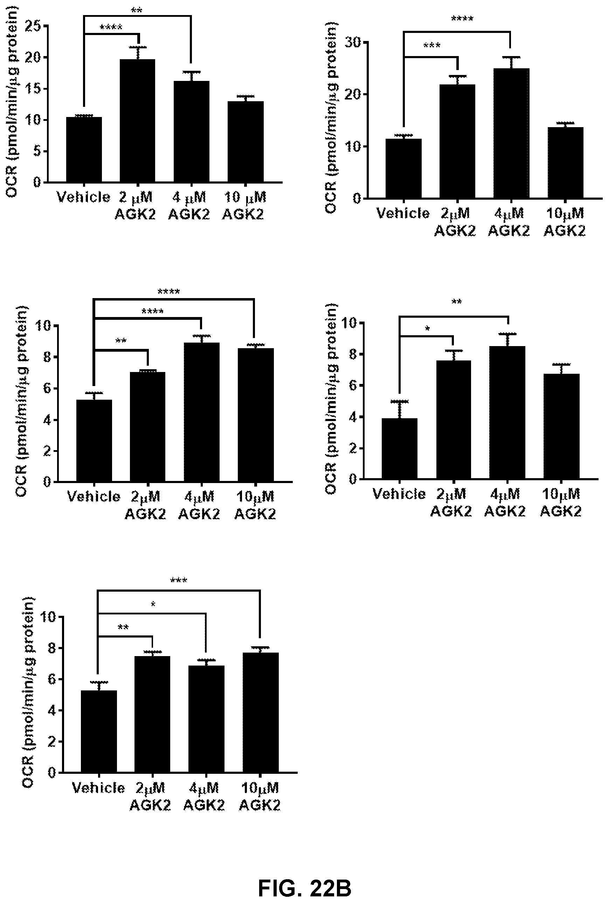

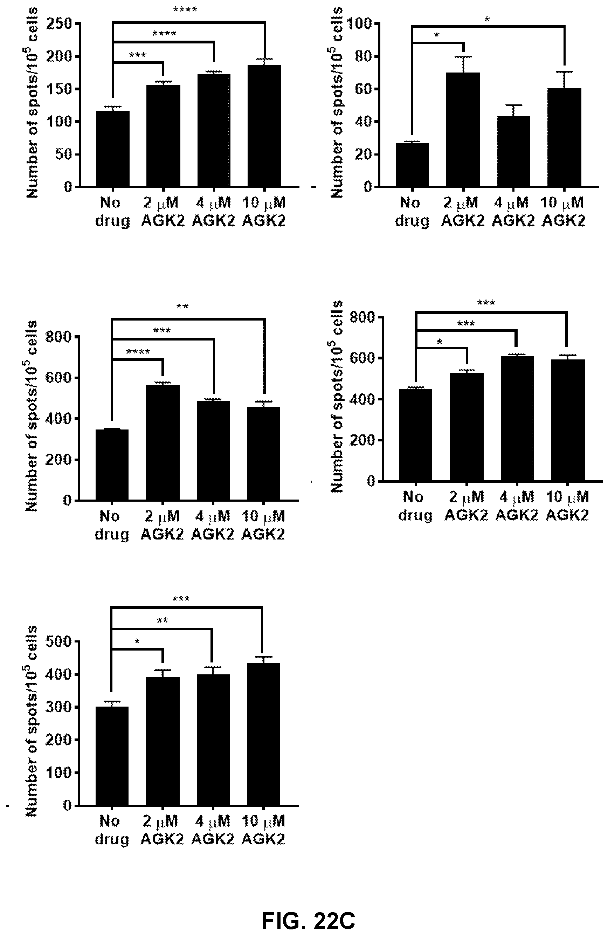

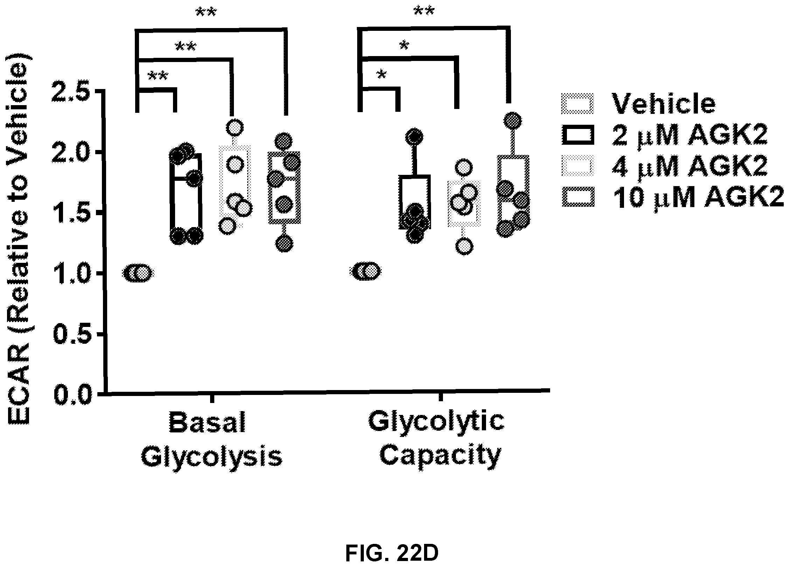

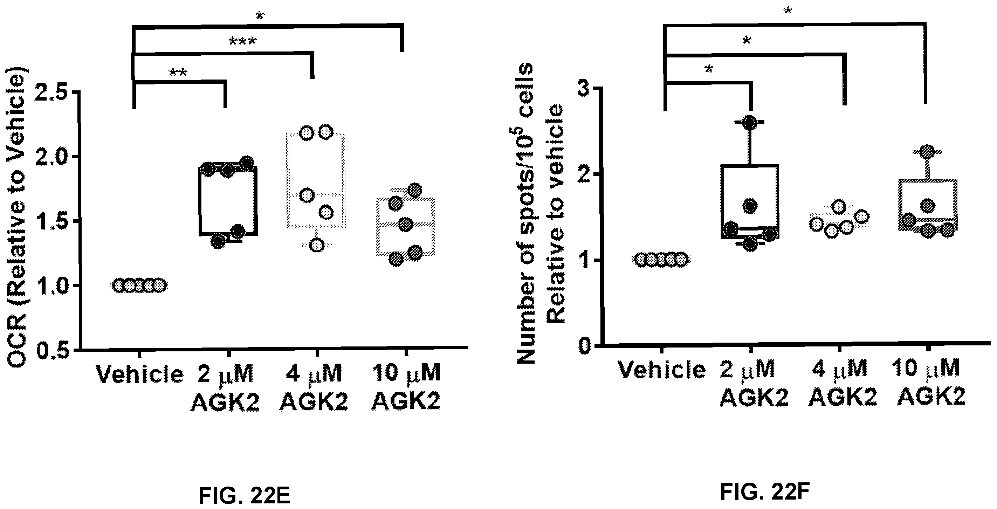

FIGs. 22A to 22F show pharmacologic inhibition of Sirt2 enhances metabolic fitness and effector functions in human NSCLC TILs. Human TILs isolated from tumor biopsies of NSCLC patients were stimulated with anti-CD3 in combination with Sirt2 inhibitor AGK2 *versus* vehicle for 48 hr. FIG. 22A (Left) shows glycolytic flux measured as ECAR using the Seahorse XF analyzer. Glucose, Oligomycin (Oligo) and 2-DG were injected at the indicated time points. FIG. 22A (Right) shows basal glycolytic rate, glycolytic capacity and glycolytic reserve were calculated from the corresponding curves. Fig. 22B shows oxygen consumption rate (OCR) was measured using the Seahorse XF analyzer. FIGs. 22A and 22B: patient 1: n = 4-7. Patient 2: n = 4-8; Patient 3: n = 4-7; Patient 4: n = 3-6; Patient 5: n = 3. FIG. 22C show IFN-γ ELISPOT assay performed on CD3-stimulated TILs with increasing concentrations of AGK2 *versus* vehicle for additional 48 hr. Patient 1: n = 5; Patient 2: n = 3; Patient 3: n = 6; Patient 4: n = 3; Patient 5: n = 3. FIGs. 22D to 22F show basal glycolytic rate, glycolytic capacity (FIG. 22D), OCR (FIG. 22E), and number of IFN-γ spots (FIG. 22F) of five human TILs samples were normalized to vehicle and plotted as shown. Data are mean ± SEM. *P* values are determined by one-way ANOVA (FIG. 22A-22F). **P* < 0.05, **P < 0.01, ****P* < 0.001, *****P* < 0.0001.

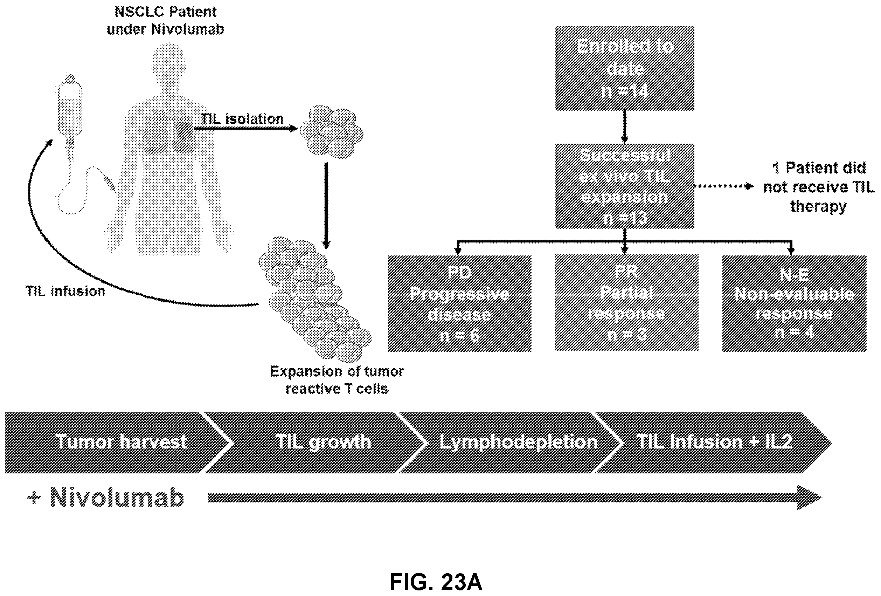

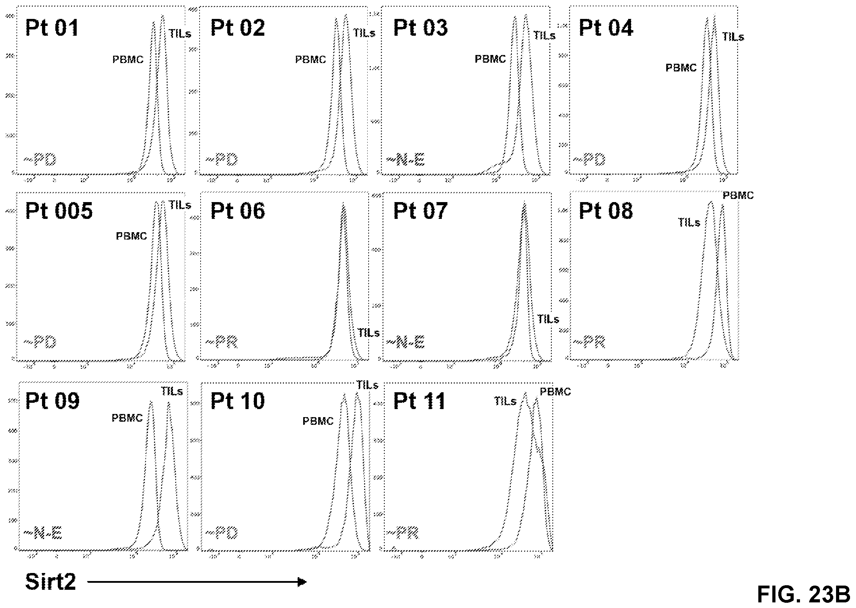

FIGs. 23A and 23B show Sirt2 expression induced within the TME. FIG. 23A is a flow chart of the phase I clinical trial combining Nivolumab and TIL therapy for patients with advanced NSCLC. Paired PBMC and TIL Samples from 11 patients were tested. 3 patients achieved partial response (PR), 5 patients failed the TIL therapy with progressive disease (PD), and 3 patients were non-evaluable (N-E). FIG. 23B shows Sirt2 expression levels in human CD45RA- CD8+ TILs compared to the corresponding PBMC CD45RA- CD8+ T cells by flow cytometry in 11 patients. FMO, Fluorescence Minus One control.

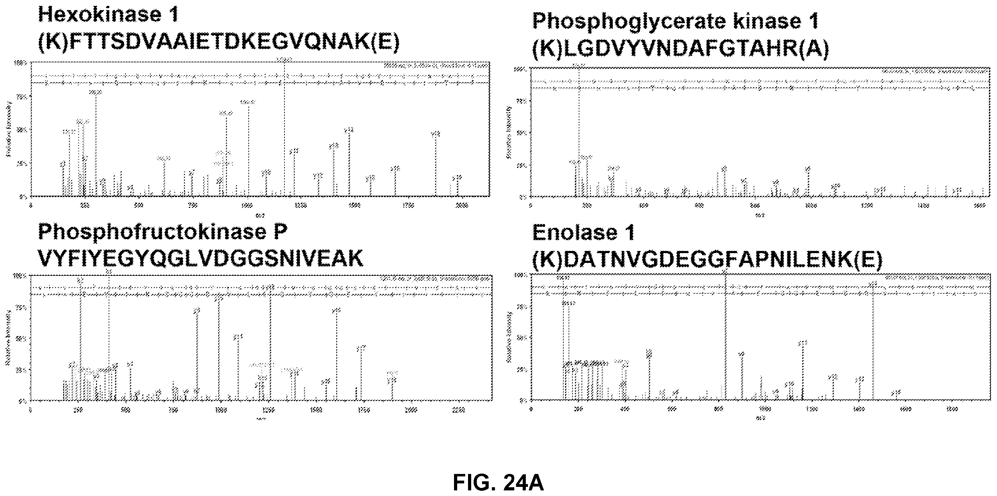

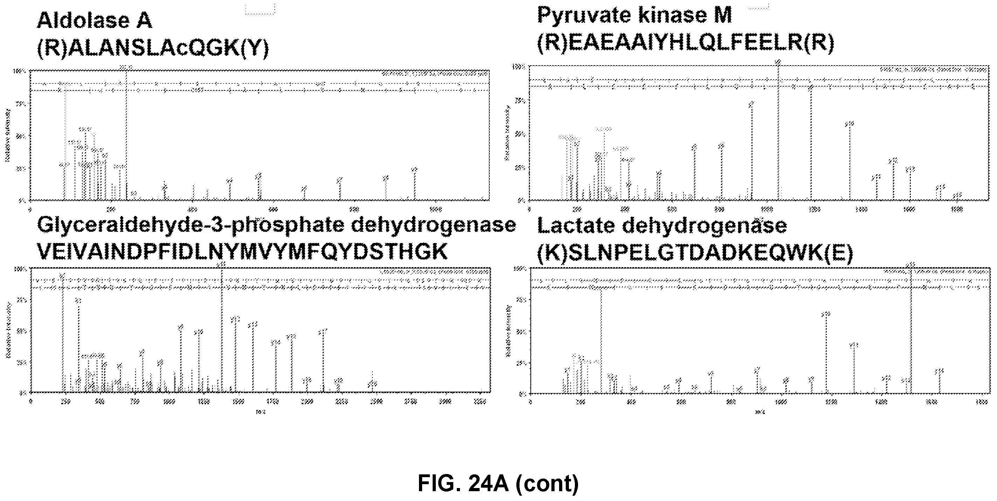

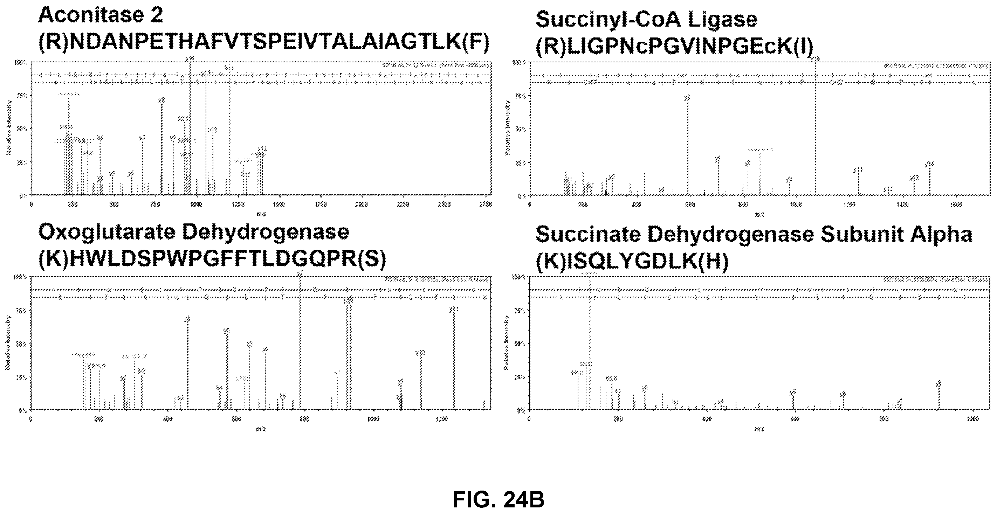

FIGs. 24A and 24B show IP-MS/MS analysis identifies key glycolytic and TCA-Cycle enzymes as Sirt2 binding partners. Immunoprecipitation of Sirt2 on activated WT CD3+ T cell protein extracts followed by LC-MS/MS analysis. FIG. 24A shows collision-induced dissociation (CID) spectra of selected peptides derived from the glycolytic enzymes: hexokinase 1, phosphofructokinase P, aldolase A, glyceraldehyde-3-phosphate dehydrogenase, phosphoglycerate kinase 1, pyruvate kinase M, enolase 1 and lactate dehydrogenase were detected. FIG. 24B shows CID spectra of selected peptides derived from the TCA-cycle enzymes: aconitase 2, oxoglutarate dehydrogenase, succinyl-CoA ligase alpha subunit and succinate dehydrogenase subunit alpha were detected. Data are representative of two independent experiments.

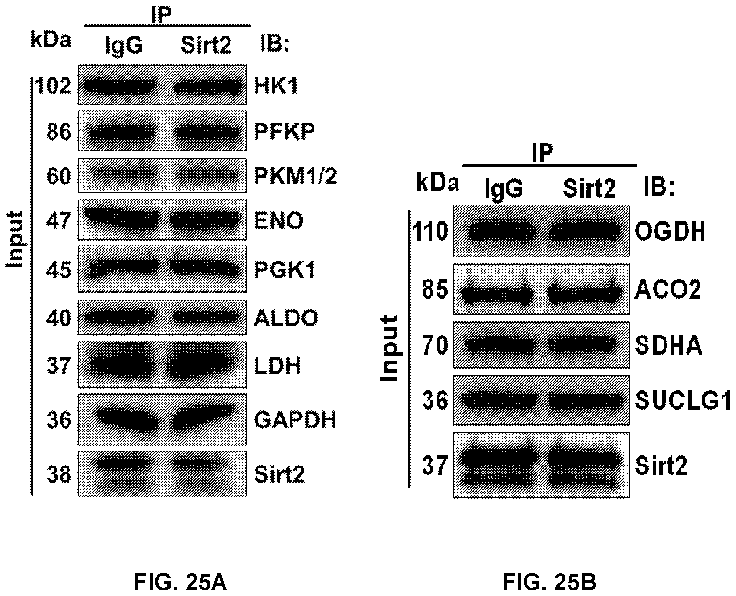

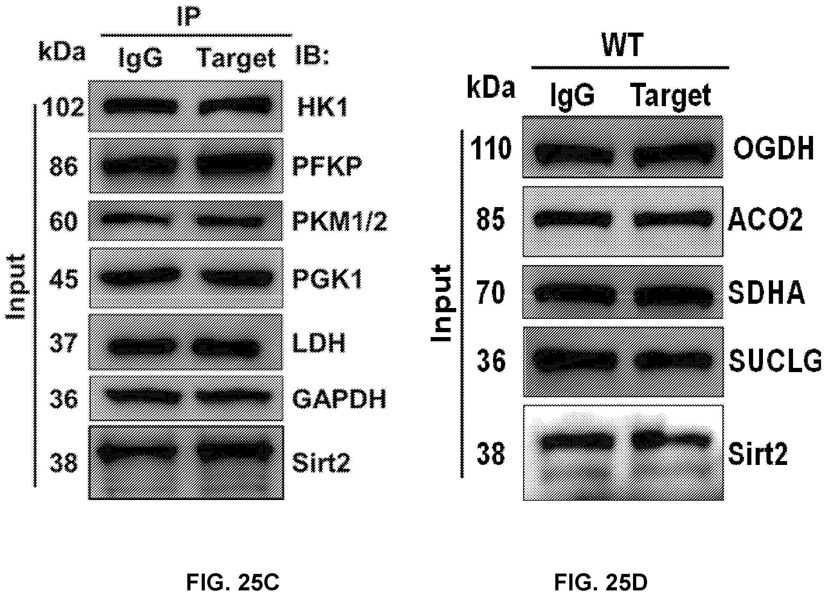

FIGs. 25A to 25D show Sirt2 directly interacts with glycolytic and TCA-cycle enzymes. FIGs. 25A-25D show Western blotting of each target using equal amounts of total protein extracts is shown as control (input) of equal concentrations for the co-IP Sirt2 (FIG. 25A and 25B) and reverse co-IP Sirt2 (FIG. 25C and 25D).

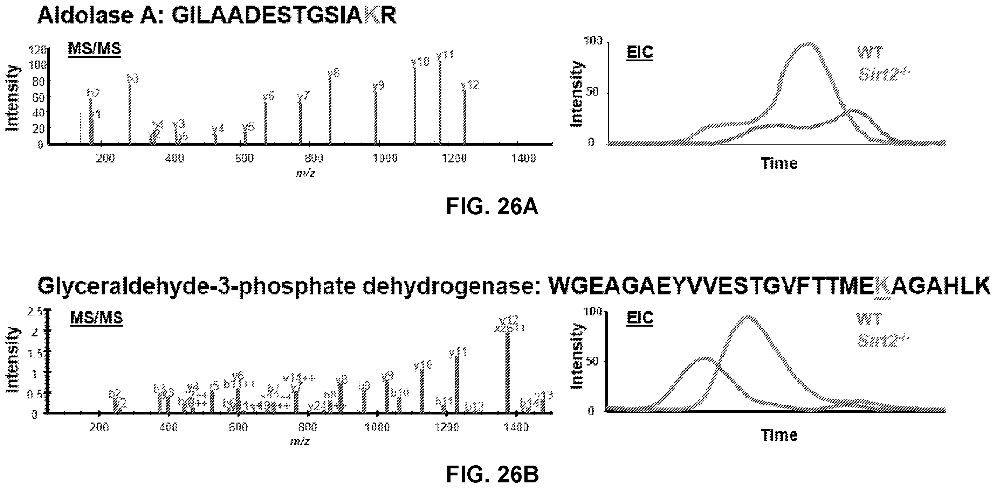

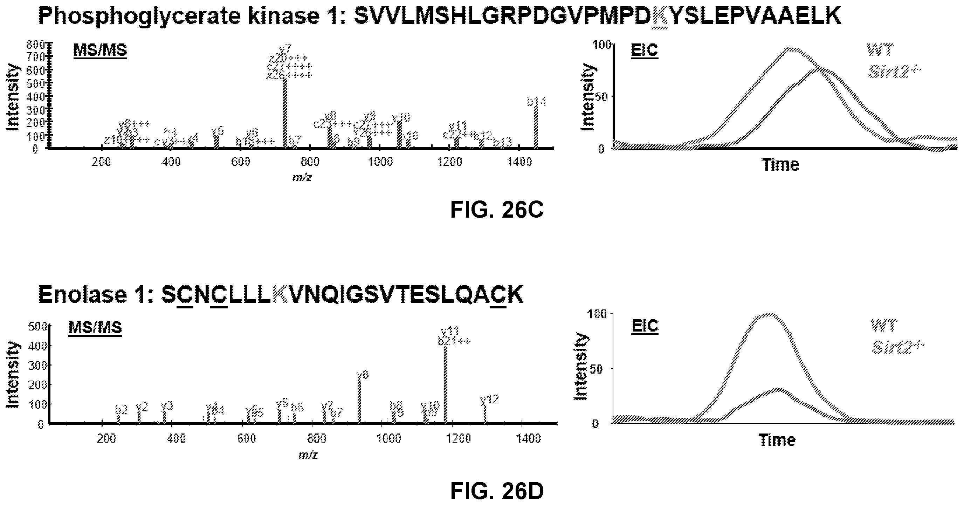

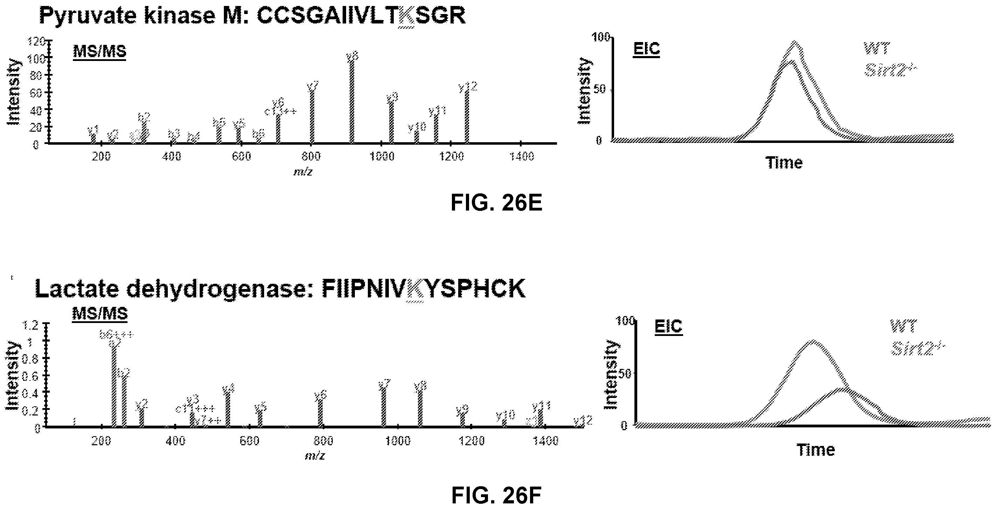

FIGs. 26A to 26F show glycolytic enzymes are highly acetylated in Sirt2^{-/-} T cells. Immunoprecipitation of acetylated proteins was performed using anti-acetyl-lysine antibodies on WT and Sirt2^{-/-} CD3+ T cell protein extracts followed by LC-MS/MS analysis. FIGs. 26A to 26F (Left) show the MS/MS spectrum was matched to: peptide GILAADESTGSIAKR (SEQ ID NO: 1) from aldolase A and y1, y2 fragments indicate the acetylation site K42 (FIG. 26A); peptide WGEAGAEYVVESTGVFTTMEKAGAHLK (SEQ ID NO:5) from glyceraldehyde-3-phosphate dehydrogenase and y6, y7 fragments indicate the acetylation site K105 (FIG. 26B); peptide SVVLMSHLGRPDGVPMPDKYSLEPVAAELK (SEQ ID NO:4) from phosphoglycerate kinase 1 and y11 fragment indicates the acetylation site K75 (FIG. 26C); peptide SCNCLLLKVNQIGSVTESLQACK (SEQ ID NO:2) from enolase 1 and b7, b8 fragments indicate the acetylation site K343 (FIG. 26D); peptide CCSGAIIVLTKSGR (SEQ ID NO:6) from pyruvate kinase M and y3, y5 fragments indicate the acetylation site K433 (FIG. 26E); and peptide FIIPNIVKYSPHCK (SEQ ID NO:3) from lactate dehydrogenase and y6, y7 fragments indicate the acetylation site K126 (FIG. 26F). FIGs. 26A to 26F (Right) show extracted ion chromatogram (EIC) plot shows the increased acetylation intensity in Sirt2^{-/-} sample versus WT of each target. Data are representative of two independent experiments.

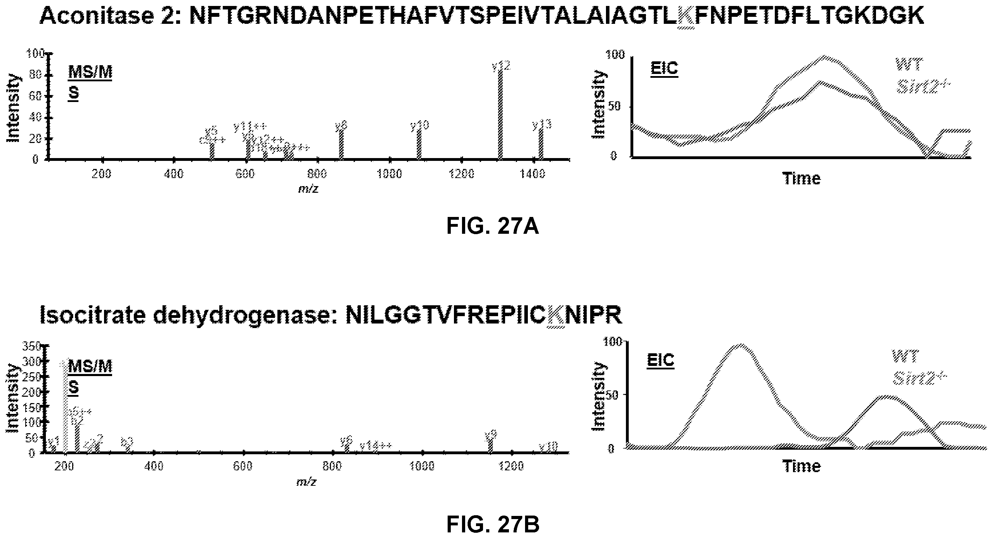

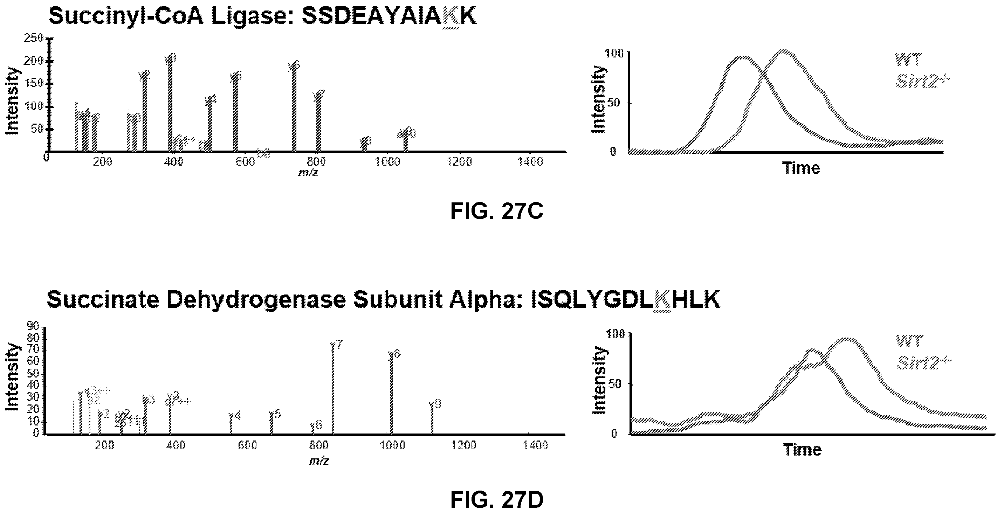

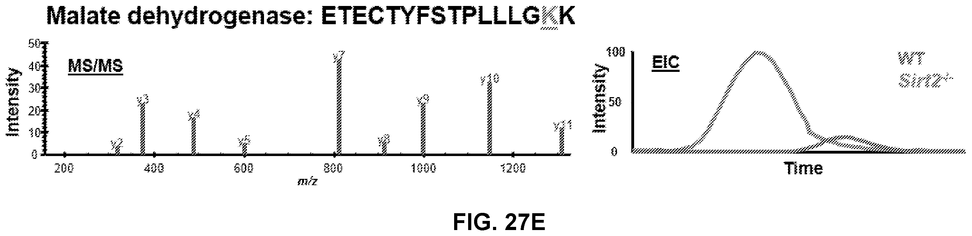

FIGs. 27A to 27E show TCA-Cycle enzymes are highly acetylated in Sirt2^{-/-} T Cells. Immunoprecipitation of acetylated proteins was performed using anti-acetyl-lysine antibodies on WT and Sirt2^{-/-} CD3+ T cell protein extracts followed by LC-MS/MS analysis. FIGs. 27A to 27E (Left) show the MS/MS spectrum was matched to: peptide NFTGRNDANPETHAFVTSPEIVTALAIAGTLKFNPETDFLTGKDGK (SEQ ID NO:7) from aconitase 2 and y13 fragment indicates the acetylation site K506 (FIG. 27A); peptide NILGGTVFREPIICKNIPR (SEQ ID NO:8) from isocitrate dehydrogenase and y6 fragment indicates the acetylation site K155 (FIG. 27B); peptide SSDEAYAIAKK (SEQ ID NO:9) from Succinyl-CoA Ligase and y1 and y2 fragments indicate the acetylation site K125 (FIG. 27C); peptide ISQLYGDLKHLK (SEQ ID NO:10) from Succinate Dehydrogenase Subunit Alpha and y3 and y4 fragments indicate the acetylation site K544 (FIG. 27D); and peptide ETECTYFSTPLLLGKK (SEQ ID NO:11) from malate dehydrogenase and y2 fragment indicates the acetylation site K296 (FIG. 27E). FIGs. 27A to 27E (Right) show extracted ion chromatogram (EIC) plot shows the increased acetylation intensity in Sirt2^{-/-} sample versus WT of each target. Data are representative of two independent experiments.

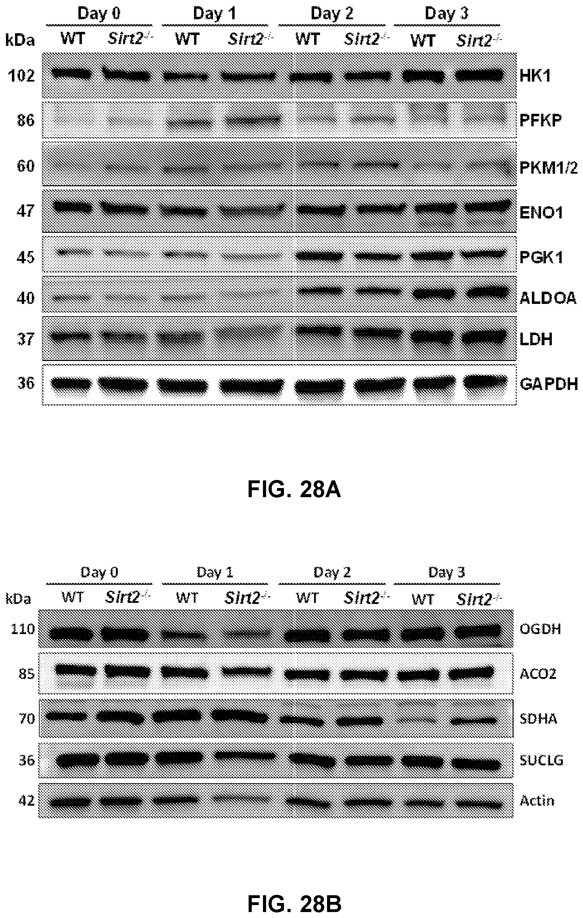

FIGs. 28A and 28B show expression levels of glycolytic and TCA-cycle enzymes in T Cells. FIGs. 28A and 28B show expression levels of glycolytic enzymes (FIG. 28A): HK1, PFKP, PKM1/2, ENO1, PGK1, ALDOA, LDH, GAPDH and TCA-cycle enzymes (FIG. 28B): OGDH, ACO2, SDHA, SUCLG1 were determined by Western blot on WT and Sirt2^{-/-} CD3+ enriched T cells naïve (Day 0) and CD3-stimulated for 1-3 days. GAPDH (FIG. 28A) and actin (FIG. 28B) levels were used as loading control. Data are representative of at least two independent experiments.

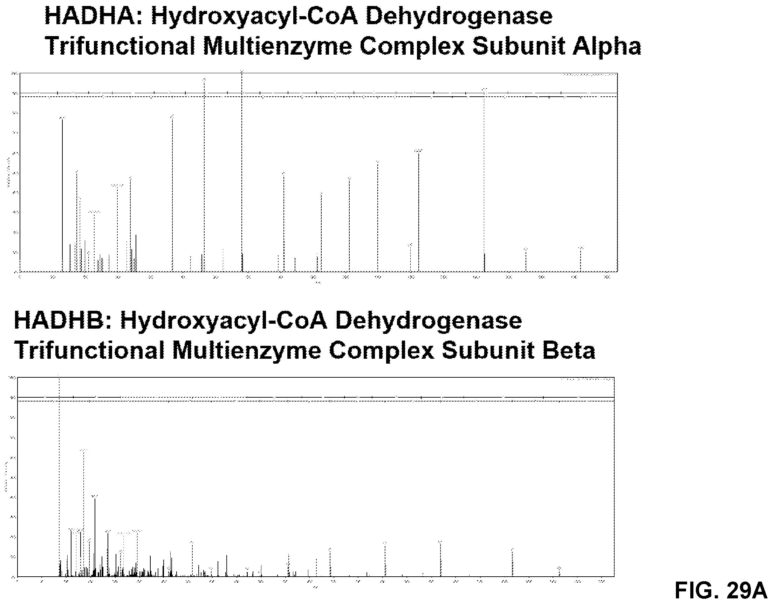

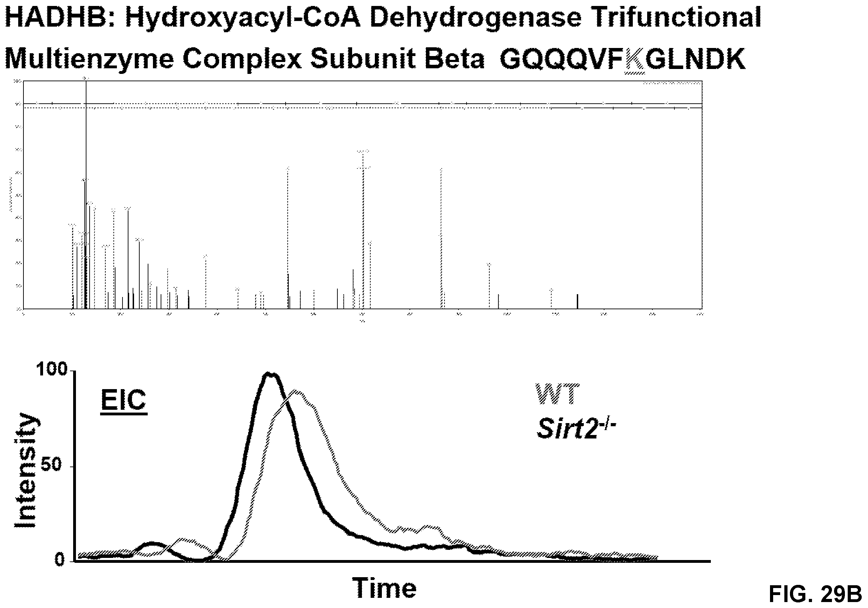

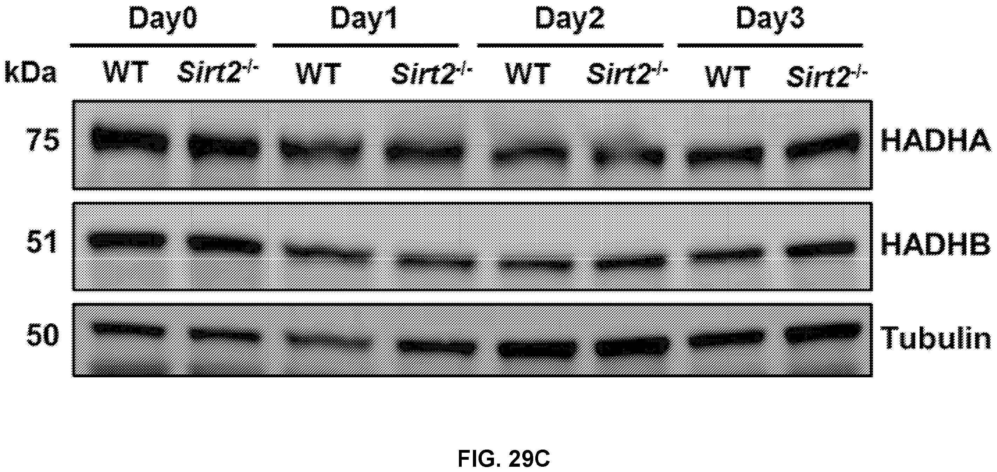

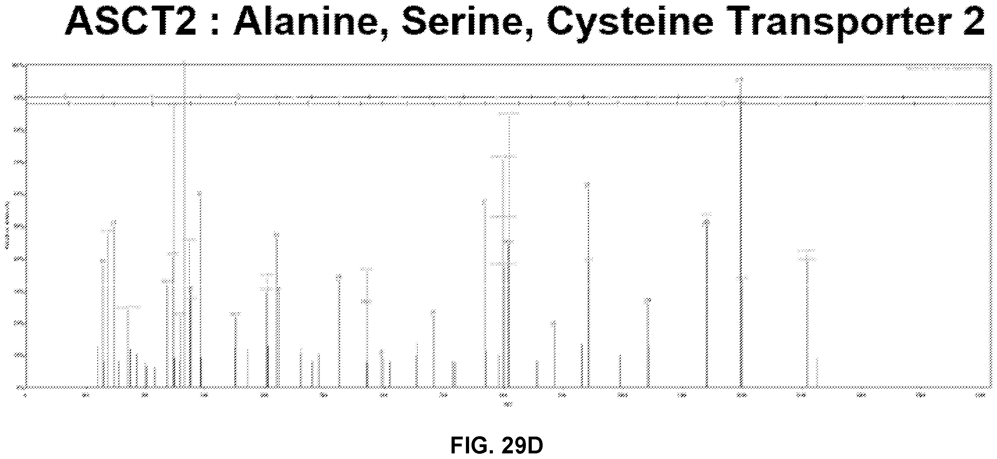

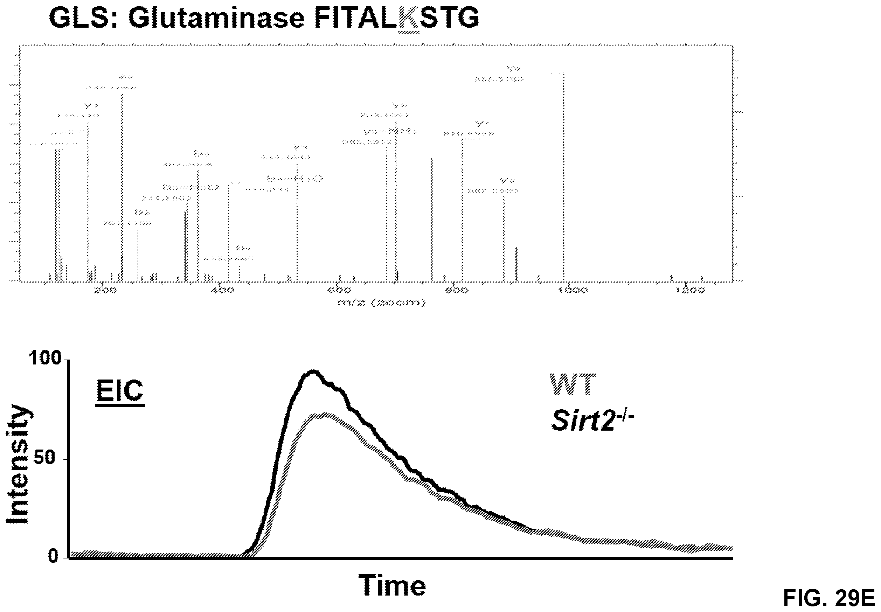

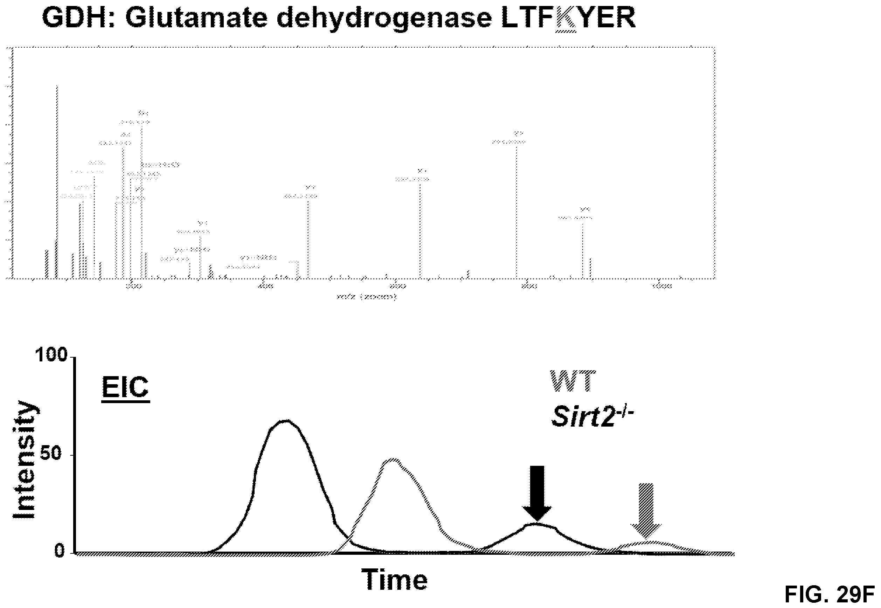

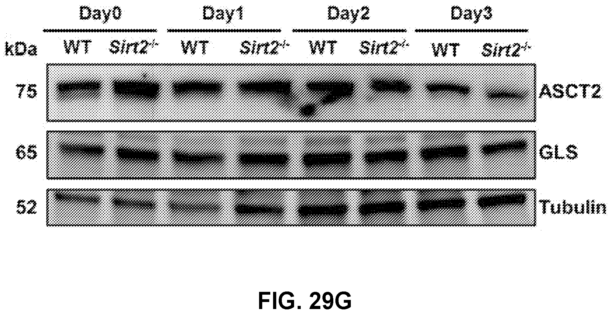

FIGs. 29A to 29G show Sirt2 Interacts with FAO and glutaminolysis enzymes. Fig. A and D, Immunoprecipitation of Sirt2 on activated WT CD3+ T cell protein extracts followed by LC-MS/MS analysis. CID spectra of selected peptides derived from the mitochondrial trifunctional protein subunits, HADHA and HADHB (FIG. 29A), and the Glutamine transporter ASCT2 were detected (FIG. 29D). FIGs. 29B and 29E-29F show immunoprecipitation of acetylated proteins on WT and Sirt2^{-/-} CD3+ T cell protein extracts followed by LC-MS/MS analysis. FIGs. 29B and 29E-29F (Left) show the MS/MS spectrum matched to: peptide GQQQVFKGLNDK (SEQ ID NO:12) from HADHB and y5 and y6 fragments indicate the acetylation site K406 (FIG. 29B); peptide FITALKSTG (SEQ ID NO:13) from GLS and y5 and y6 fragments indicate the acetylation site K169 (FIG. 29E); and peptide LTFKYER (SEQ ID NO:14) from GDH and y3 and y4 fragments indicate the acetylation site K457 (FIG. 29F). FIGs. 29B and 29E-29F (Right) are EIC plots showing the increased acetylation intensity in Sirt2^{-/-} sample versus WT of each target. FIG. 29C and 29G show expression levels of trifunctional protein subunits (FIG. 29C), HADHA and HADHB, and Glutaminolysis pathway targets (FIG. 29G), ASCT2 and GLS, were determined by Western blot on WT and Sirt2-/- CD3+ enriched T cells naïve (Day 0) and CD3-stimulated for 1-3 days. Tubulin levels were used as loading control. Data are representative of at least two independent experiments.

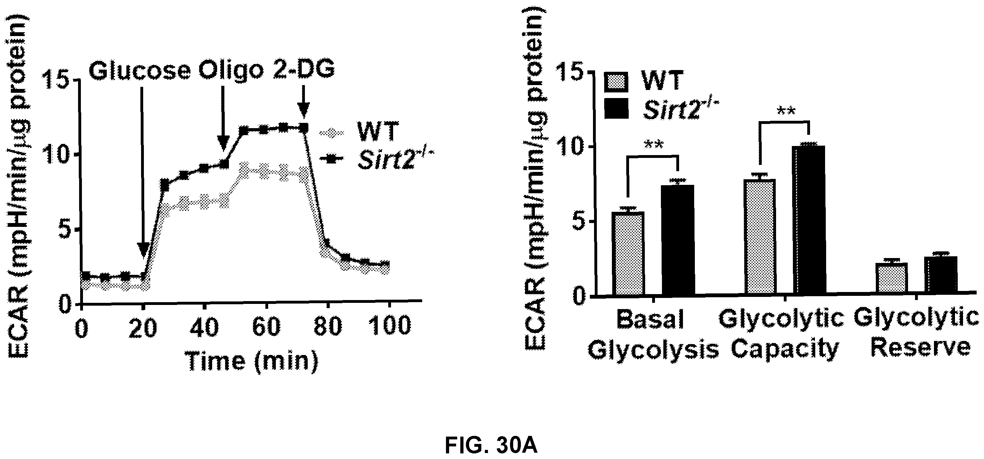

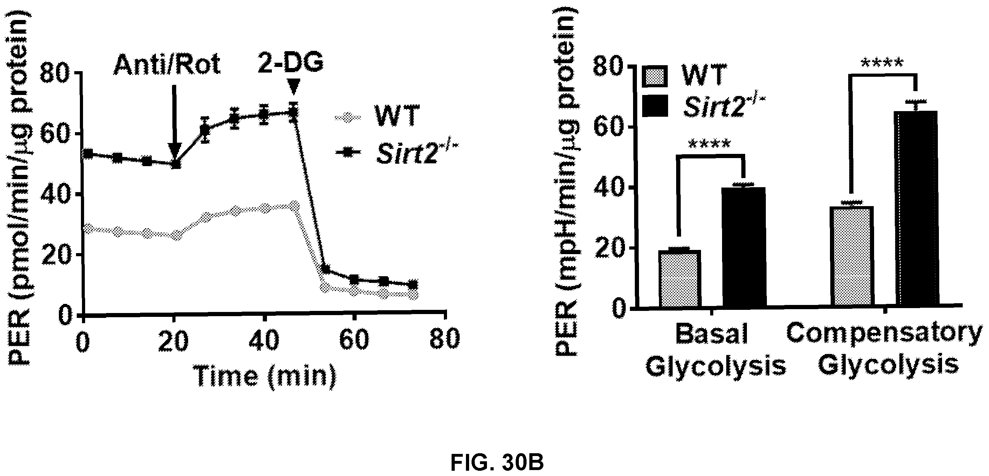

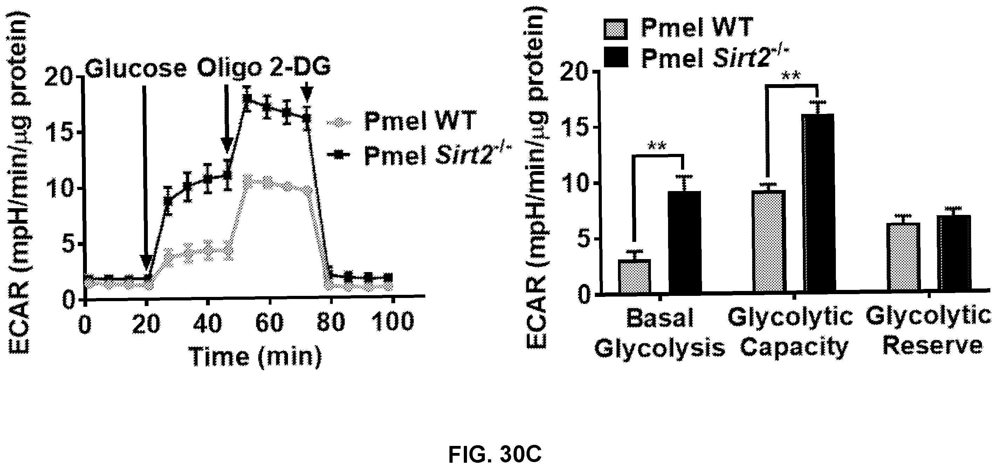

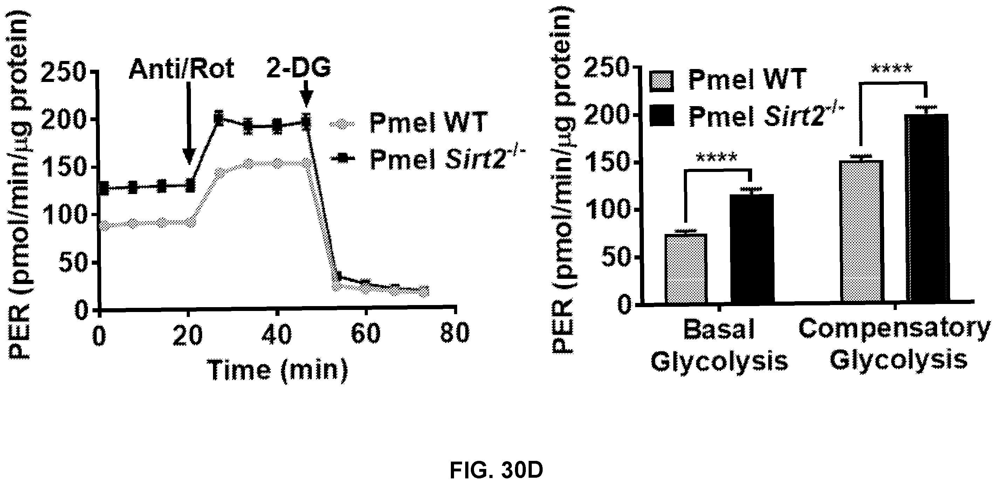

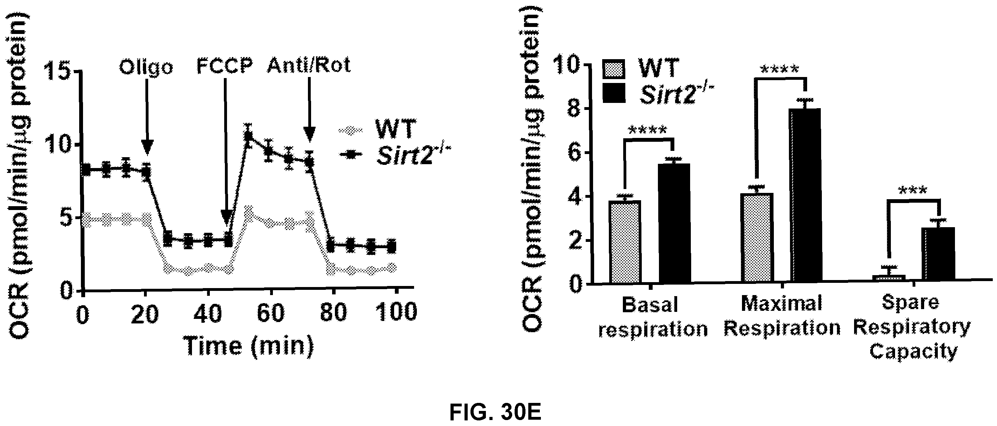

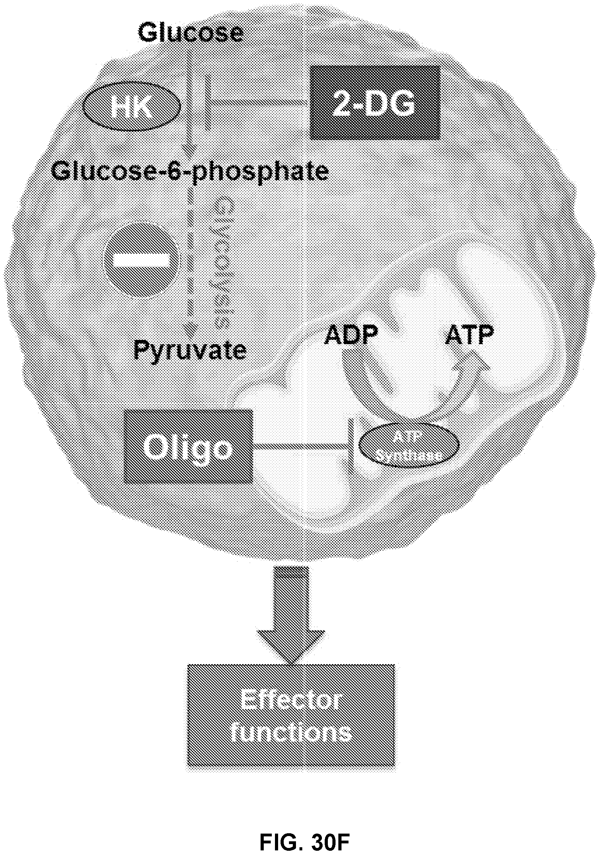

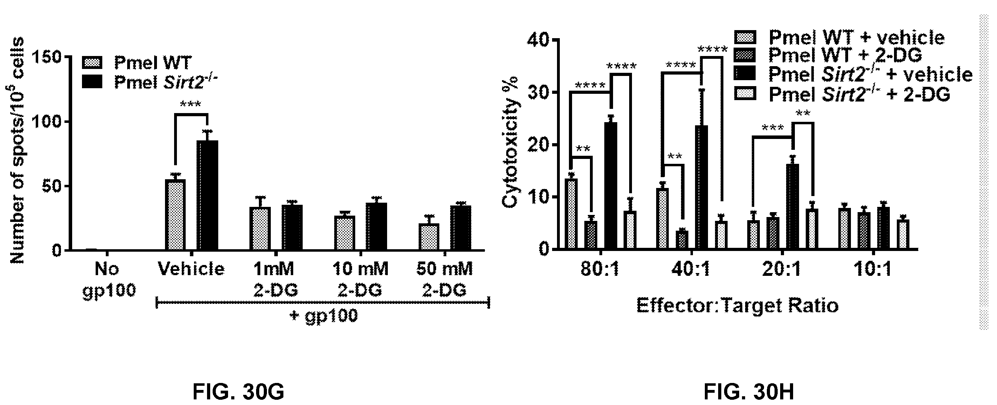

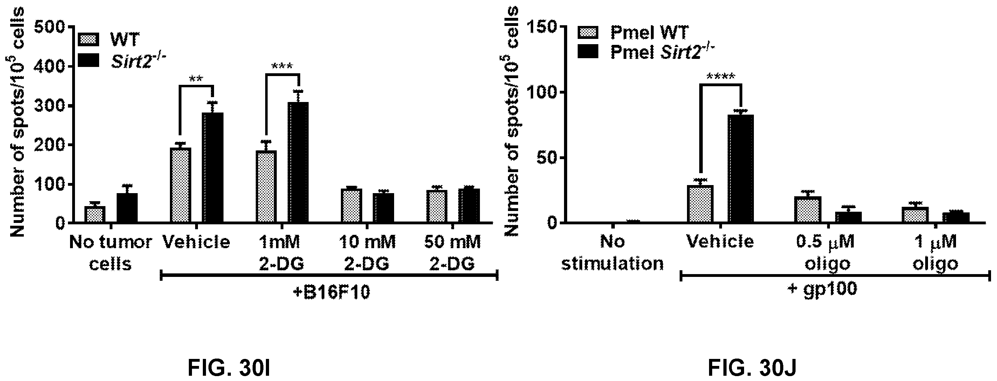

FIGs. 30A to 30J show increased glycolysis and OxPhos in Sirt2^{-/-} T cells. FIGs. 30A and 30C (Left) show glycolytic flux measured as extracellular acidification (ECAR) using the Seahorse XF analyzer on: CD3-stimulated WT (n = 4) and Sirt2^{-/-} (n = 4) CD8+ T cells (FIG. 30A); and gp100-stimulated WT (n = 4) and Sirt2^{-/-} (n = 5) Pmel CD8+ T cells (FIG. 30C). Glucose, Oligomycin (Oligo) and 2-deoxyglucose (2-DG) were injected at the points indicated. FIGs. 30A and 30C (Right) show basal glycolytic rate, glycolytic capacity and glycolytic reserve calculated from the corresponding curves. FIGs. 30B and 30D (Left) show glycolytic rate measured as proton efflux rate (PER) using the Seahorse XF analyzer on: CD3-stimulated WT (n = 5) and Sirt2^{-/-} (n = 5) CD8+ T cells (FIG. 30B); and gp100-stimulated WT (n = 8) and Sirt2^{-/-} (n = 5) Pmel CD8+ T cells (FIG. 30D). Antimycin A/Rotenone (Anti/Rot) and 2-DG were injected at the points indicated. FIGs. 30B and 30D (Right) show basal glycolytic rate and compensatory glycolysis calculated from the corresponding curves. FIG. 30E (Left) shows mitochondrial activity measured as oxygen consumption rate (OCR) using the Seahorse XF analyzer on CD3-stimulated WT (n = 4) and Sirt2-/- (n = 4) CD8+ T cells. Oligomycin (Oligo), FCCP and Antimycin A/Rotenone (Anti/Rot) were injected at the points indicated. FIG. 30E (Right) shows basal respiration, maximal respiration and spare respiratory capacity were calculated from the corresponding curves. FIG. 30F shows T cells effector functions, IFN-γ release and/or cytotoxic activity measured on activated T cells treated with the glycolytic inhibitor 2-deoxyglucose (2-DG) or the mitochondrial ATP synthase inhibitor oligomycin. FIG. 30G and 30J show IFN-γ ELISPOT assay performed on non-stimulated or gp100-stimulated WT and Sirt2^{-/-} Pmel T cells with the indicated concentrations of 2- DG (FIG. 30G) or oligomycin (FIG. 30J) (n = 3). FIG. 30H shows cytotoxic activities determined by LDH release assay on gp100-stimulated WT and Sirt2^{-/-} CD8+ Pmel T cells after co-culture with B16F10 target cells at the indicated Effector:Target ratios with vehicle or 10mM 2-DG (n = 3). I, IFN-γ Elispot assay was performed on WT and Sirt2^{-/-} CD3+ TILs after re-challenge with vehicle or irradiated B16F10 cells at the indicated concentration of 2-DG (n = 3). Data are representative of two (FIG. 30A, 30D, 30I), three (FIG. 30C, 30E, 30G, 30H, 30J) and four (FIG. 30B) independent experiments. Data are mean ± SEM. P values are determined by two-tailed Student's t-test (FIGs. 30A-30E) or two-way ANOVA (FIGs. 30G-30J). *P < 0.05, **P < 0.01, ***P < 0.001, ****P < 0.0001.

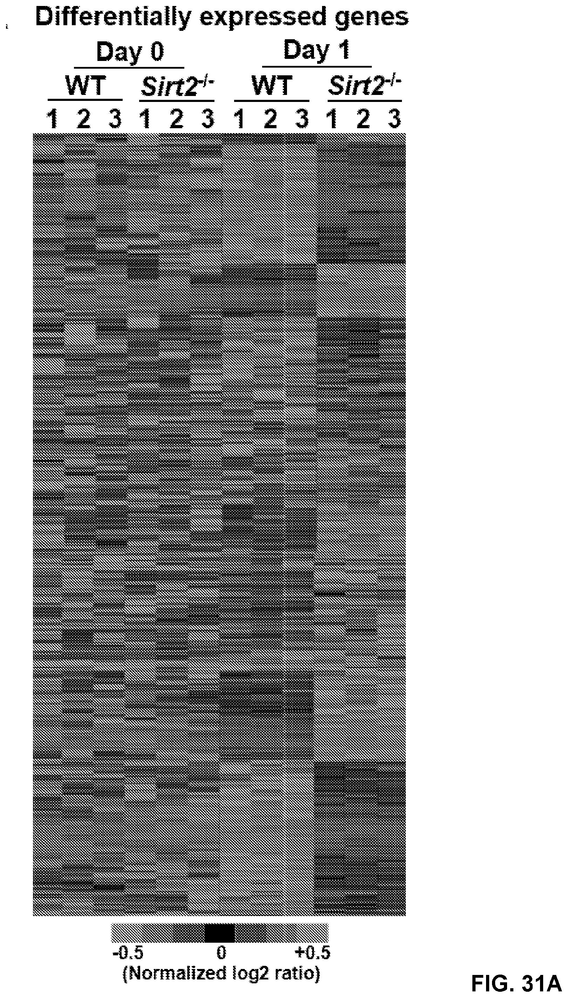

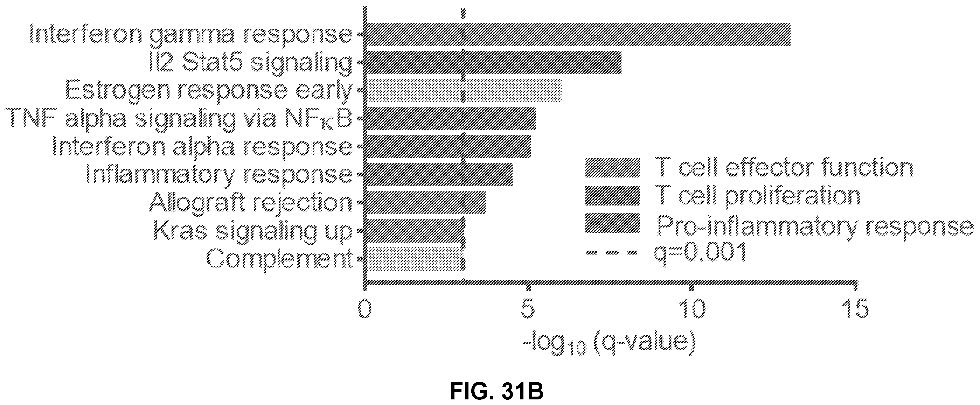

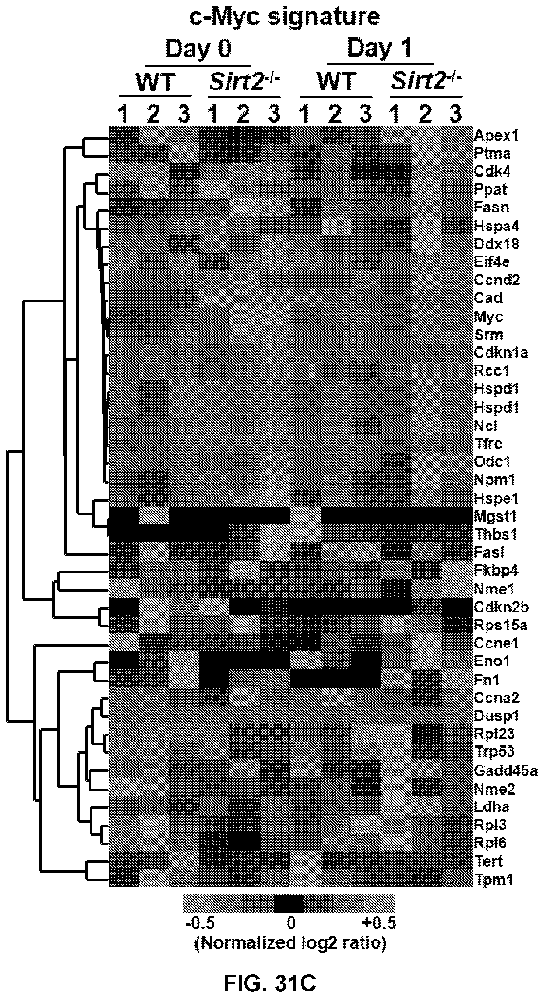

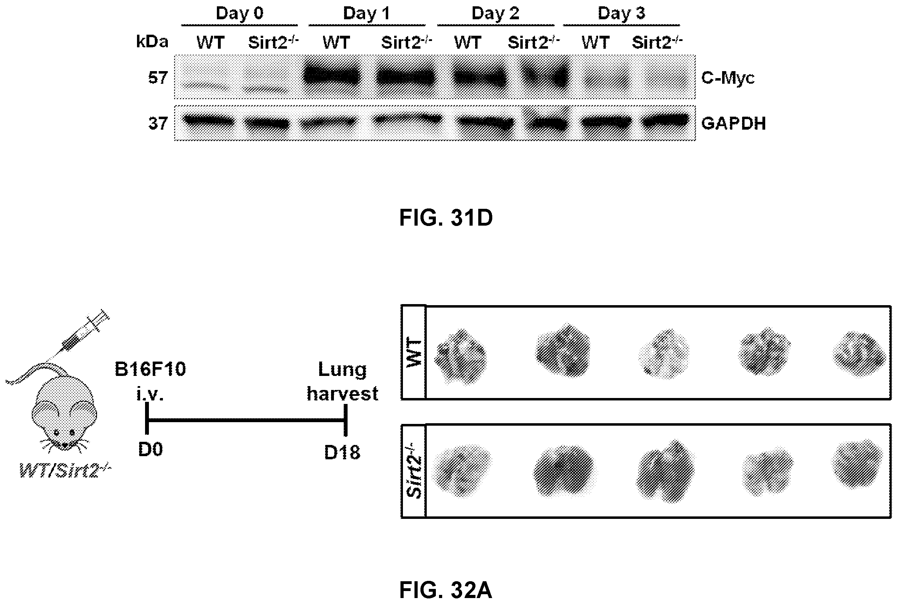

FIGs. 31A to 31D show differentially expressed genes and c-Myc Signature in Sirt2^{-/-} versus WT T Cells. FIG. 31A is a heatmap of differentially expressed genes in naïve (Day 0) and activated (Day 1) Sirt2^{-/-} CD4+ T cells versus WT CD4+ T cells. Fig. 31B shows gene set enrichment analysis (GSEA) of upregulated differentially expressed genes in activated Sirt2-/- CD4+ T cells compared to activated WT CD4+ T cells. A false discovery rate q-value <0.001 was used as a cut-off. FIG. 31C is a heatmap representation of c-Myc target genes expression in Sirt2^{-/-} CD4+ T cells compared to WT CD4+ T cells. FIG. 31D shows c-Myc expression levels determined by Western blot on WT and Sirt2^{-/-} CD3+ enriched T cells naïve (Day 0) and CD3-stimulated for 1-3 days. GAPDH levels were used as loading control.

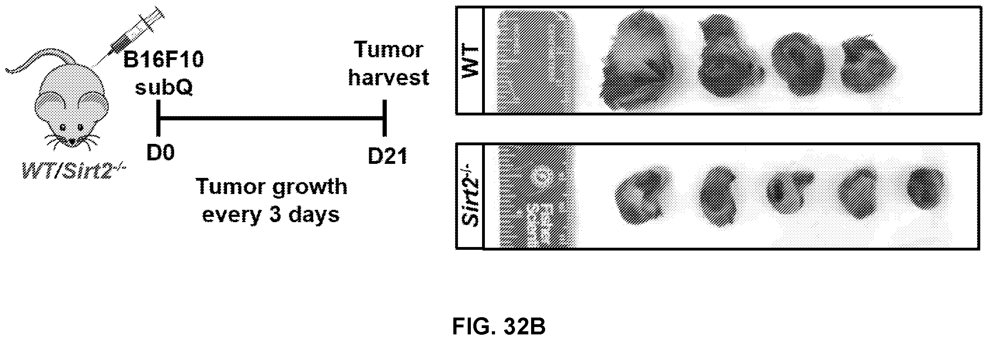

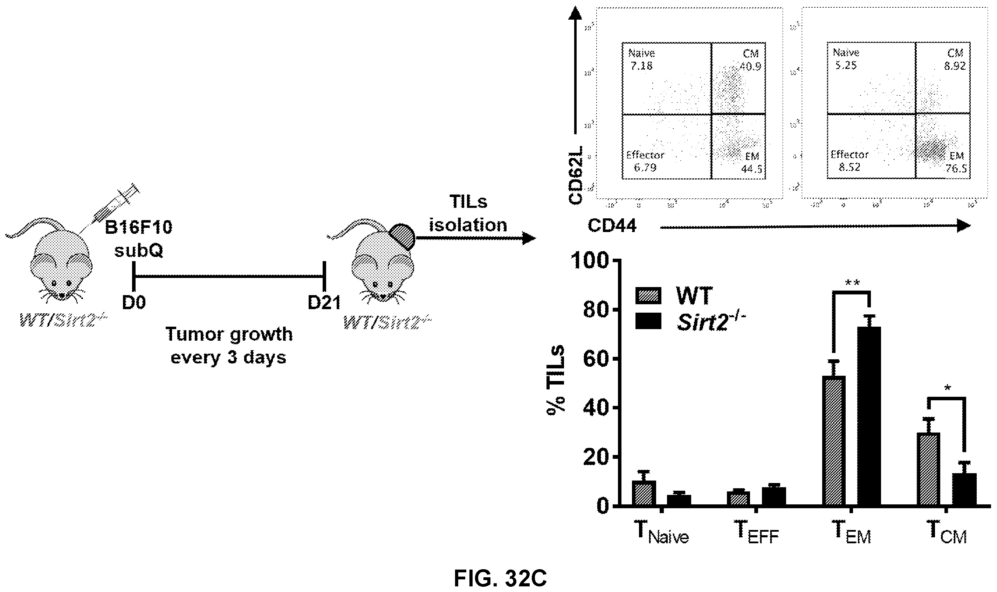

FIGs. 32A to 32C show Sirt2 deficiency protects mice from B16F10 tumor challenge. FIG. 32A shows lungs from WT and Sirt2^{-/-} mice challenged i.v. with B16F10 cells, collected 18 days post-injection. FIG. 32B shows subcutaneous tumors dissected from WT and Sirt2^{-/-} mice challenged s.c. with B16F10 cells 21 days post-injection. One WT mouse reached tumor size endpoint prior to the Day 21 time point and is thus not depicted. FIG. 32C shows TILs isolated from B16F10 subcutaneous tumors from WT and Sirt2^{-/-} mice. FIG. 32C shows naïve, effector (TEFF), effector memory (TEM) and central memory (TCM) CD8+ T cell subsets were analyzed by CD44 and CD62L expression. Numbers in quadrants indicate the percentage of cells. FIG. 32 shows frequency of each subset among TILs is indicated (n = 3-4). Data are representative of two (FIG. 32C), three (FIG. 32B) and four (FIG. 32A) independent experiments. Data are mean ± SEM. P values are determined by two-way ANOVA (FIG. 32C). *P < 0.05, **P < 0.01, ***P < 0.001.

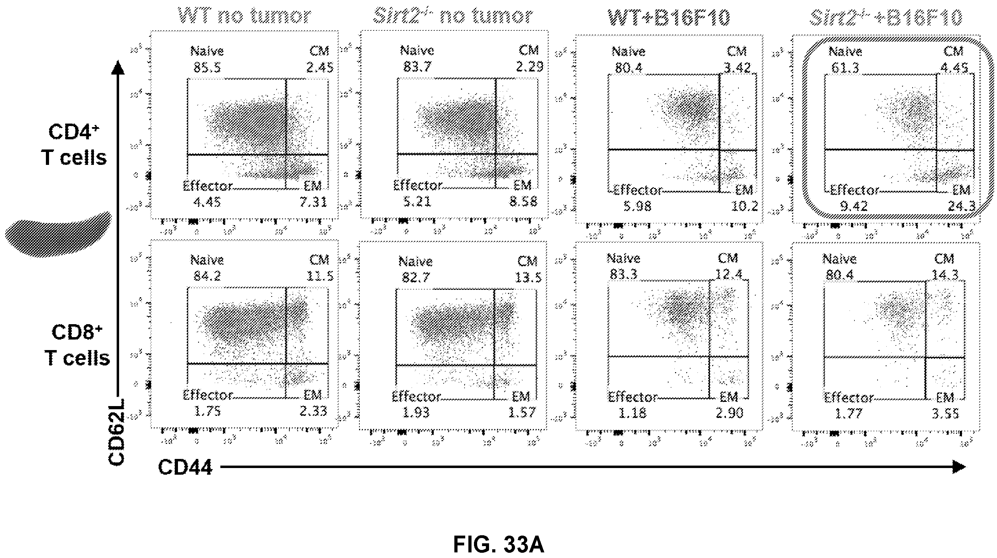

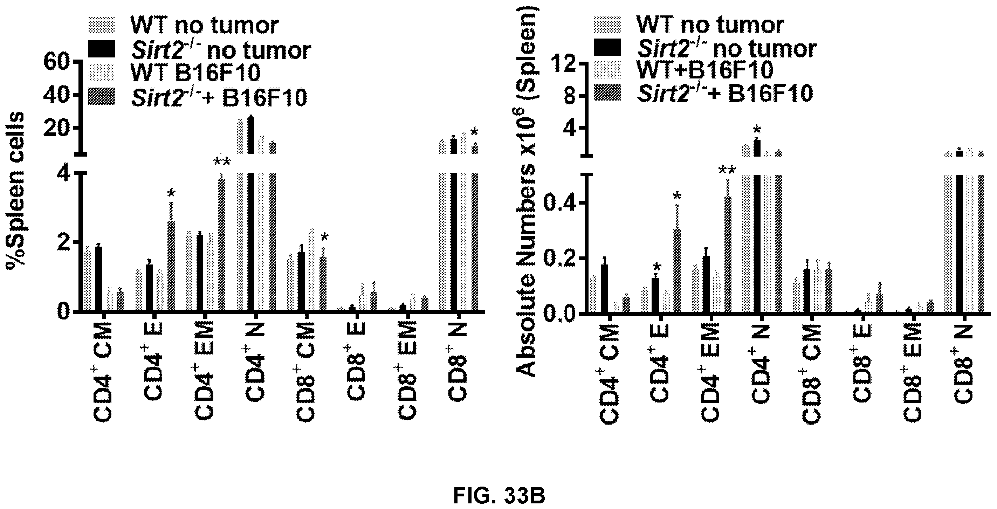

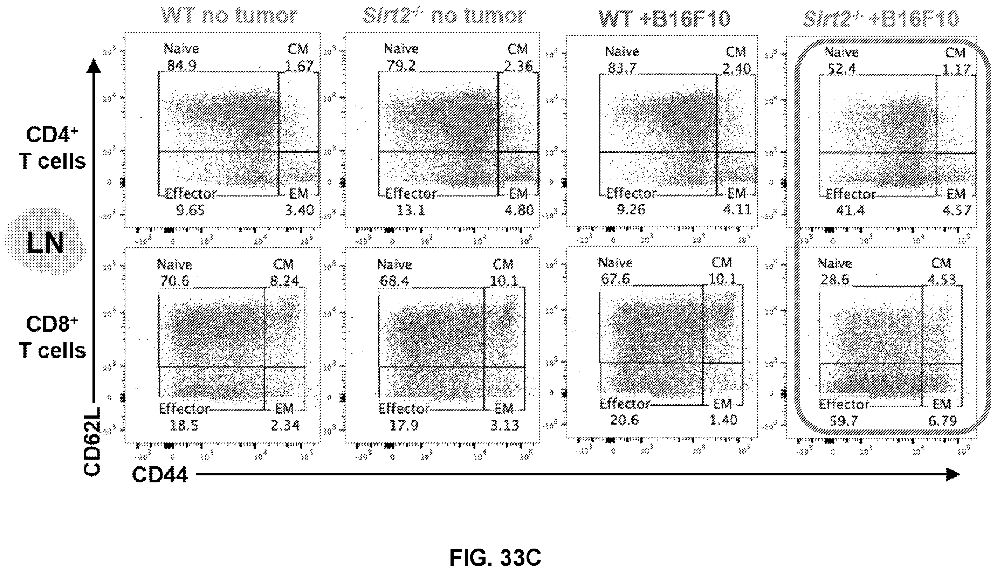

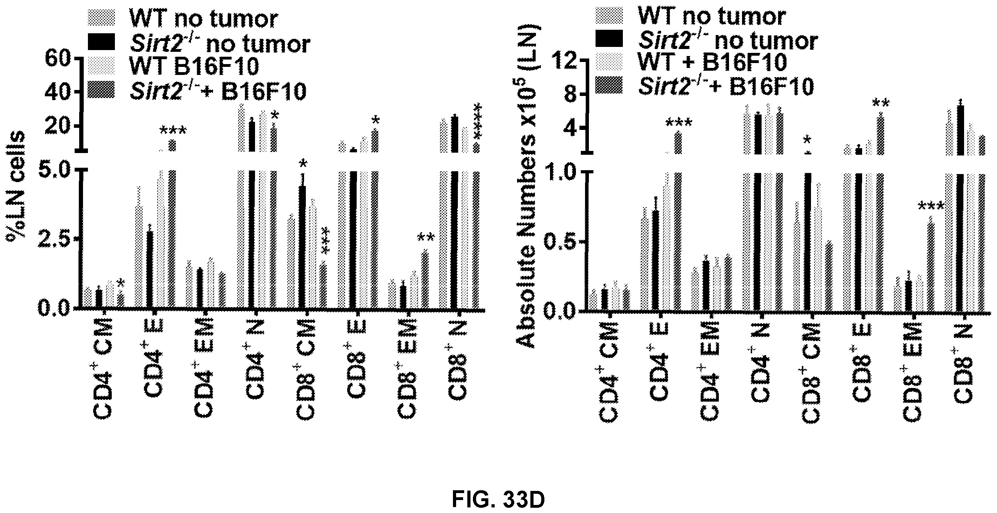

FIG. 33A and 33B show T cell compartments in the spleen and lymph node of B16F10 Challenged WT and Sirt2^{-/-} Mice. FIGs. 33A and 33C show representative flow cytometric analysis of splenocytes (FIG. 33A) and lymph node T cells (FIG. 33C) from WT and Sirt2^{-/-} mice i.v. challenged with B16F10 cells versus control for naïve, effector, effector memory (EM) and central memory (CM) CD8+ and CD4+ T cell subsets as defined by CD44 and CD62L. Numbers in quadrants indicate percentage of cells. FIGs. 33B and 33D show frequency (left) and absolute number (right) of each subset from spleens (FIG. 33B) and lymph nodes (FIG. 33D) are indicated. n = 5 (FIG. 33B) and n = 4 (FIG. 33D) in each group. Data are representative of two independent experiments. Data are mean ± SEM. P values are determined by two-tailed Student's t-test. *P < 0.05, **P < 0.01, ***P < 0.001, ****P < 0.0001.

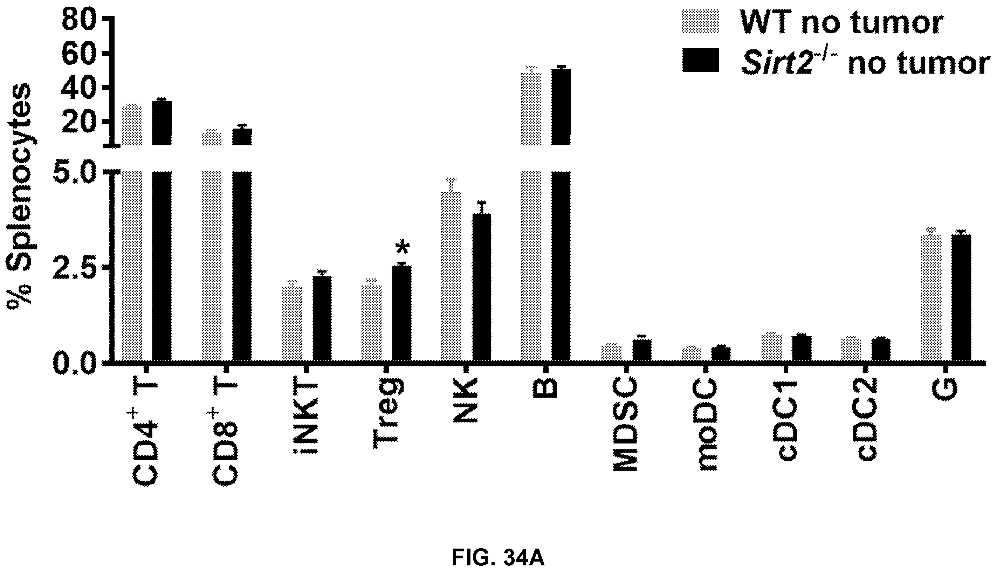

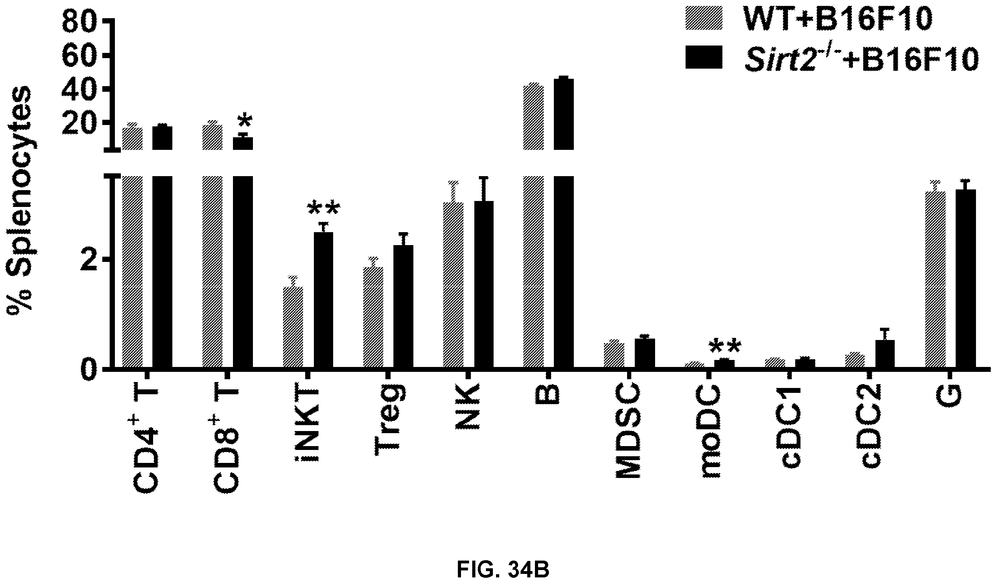

FIG. 34A and 34B show immune cell compartments in the spleen of B16F10 challenged WT and Sirt2^{-/-} Mice. FIGs. 33A and 33B show frequencies of CD4+, CD8+, invariant natural killer T (iNKT), regulatory T cells (Treg), natural killer T cells (NK), B cells (B), myeloid derived suppressor cells (MDSC), monocytic dendritic cell (moDC), conventional DC 1, 2 (cDC1, cDC2) and granulocyte cells (G) were analyzed by flow cytometry in spleens of WT and Sirt2^{-/-} unchallenged mice (FIG. 34A) and intravenously B16F10 challenged mice (FIG. 34B); (n = 4 - 5). Data are representative of two independent experiments. Data are mean ± SEM. P values are determined by two-tailed Student's t-test. *P < 0.05, **P < 0.01.

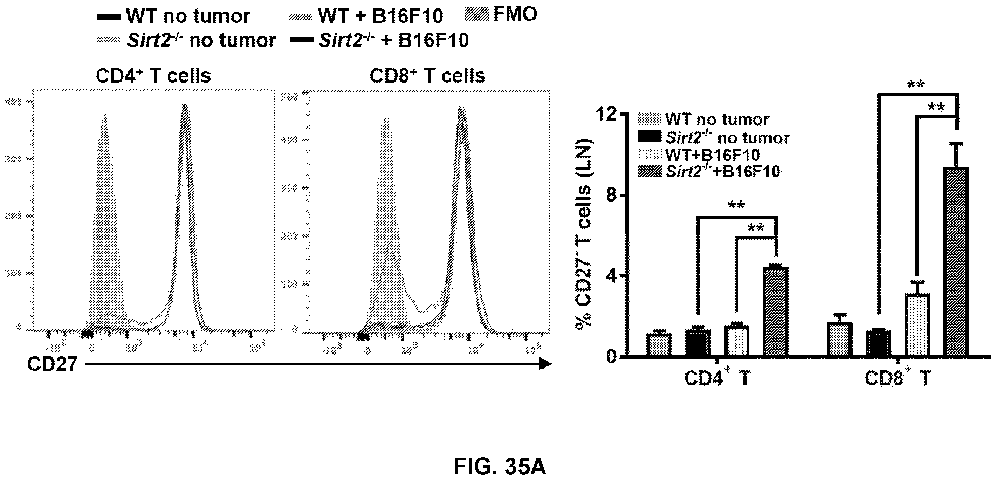

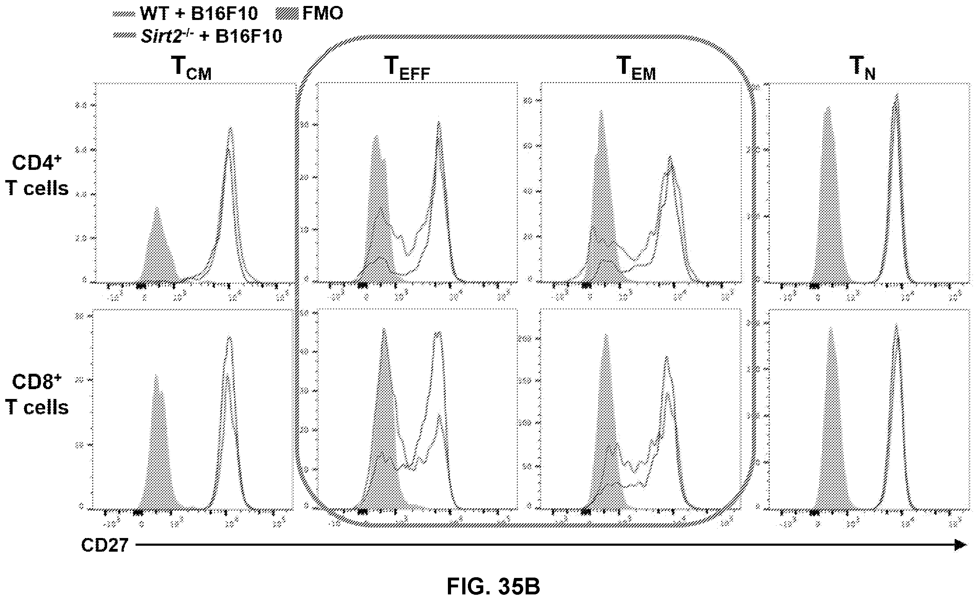

FIGs. 35A and 35B show characterization of CD27 Expression in T cells from lymph nodes of B16F10 challenged mice. WT and Sirt2^{-/-} mice were intravenously challenged or not with B16F10 cells for 18 days. FIG. 35A, Left, contains histograms display surface expression of CD27, in CD4+ and CD8+ T cell subsets from lymph nodes. FMO, fluorescence minus one. One mouse representative of n = 4-5 mice tested in each group. FIG. 35A, Right, shows frequency of CD27- T cells in each subset is represented. FIG. 35B shows characterization of CD27 expression in naïve (TN), effector (TEFF), effector memory (TEM) and central memory (TCM) CD8+ and CD4+ T cell subsets from lymph nodes. FMO, fluorescence minus one. One mouse representative of n = 4 mice tested in each group. Data are representative of two independent experiments. Data are mean ± SEM. P values are determined by two-tailed Student's t-test. *P < 0.05, **P < 0.01.

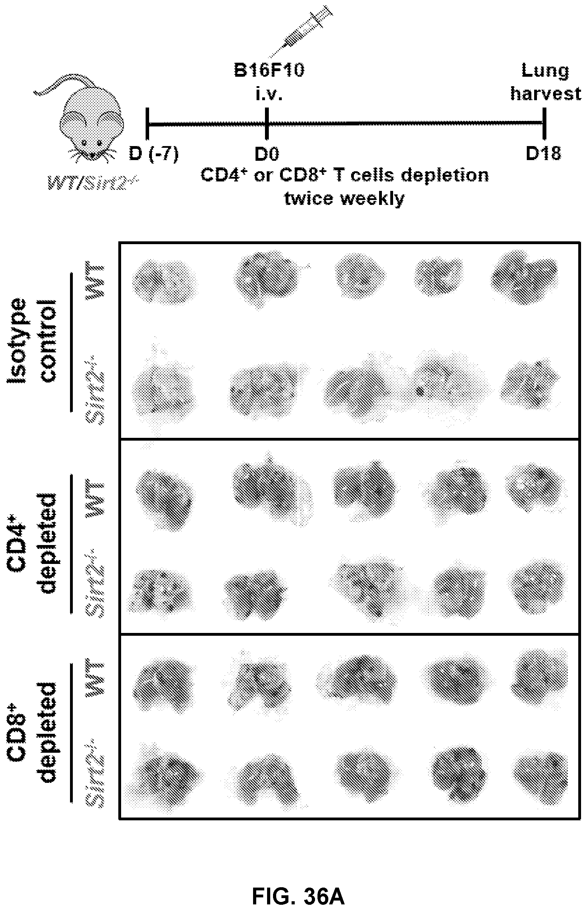

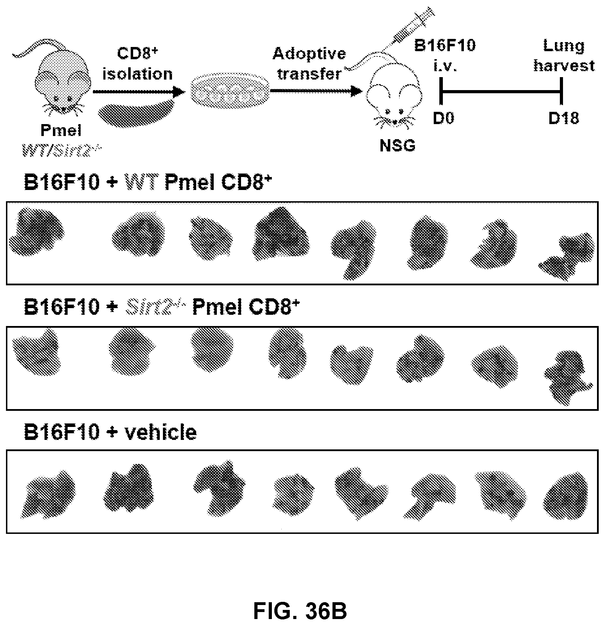

FIGs. 36A and 36B show T cells play a major role in B16F10 tumor rejection in Sirt2^{-/-} mice. FIG. 36A shows lungs from WT and Sirt2-/- mice treated with isotype control, anti-CD4, or anti-CD8 monoclonal antibodies before B16F10 i.v. challenge, collected 18 days post-injection. FIG. 36B shows lungs from NSG mice challenged i.v. with B16F10 and treated with vehicle, WT Pmel CD8+ T cells or Sirt2^{-/-} Pmel CD8+ T cells, collected 18 days post-injection. The photograph for this experiment was taken after lung fixation in paraformaldehyde. Data are representative of two independent experiments.

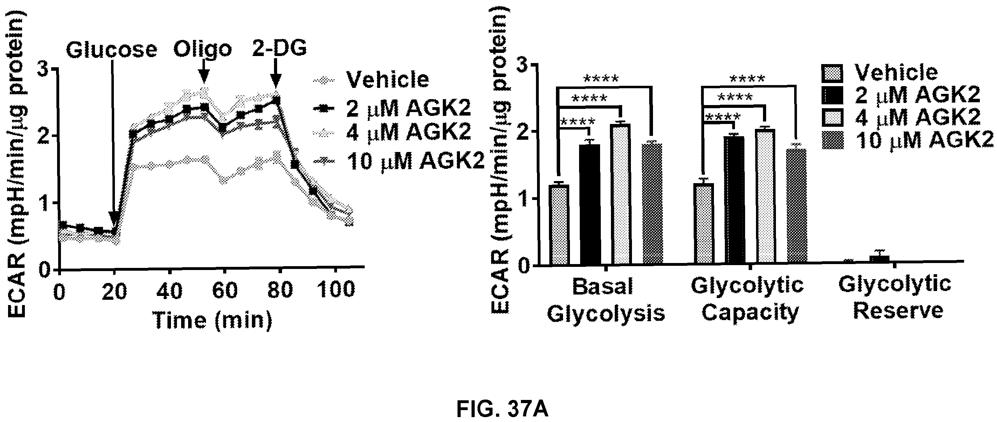

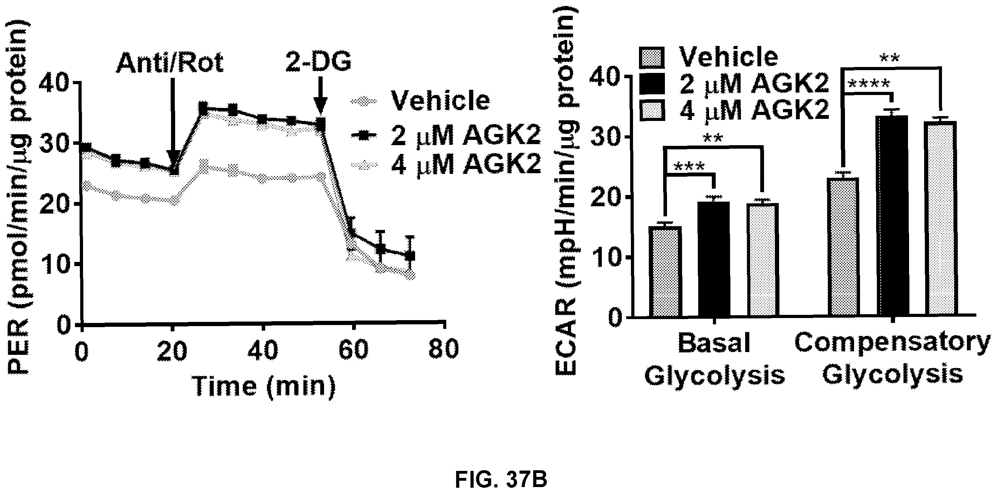

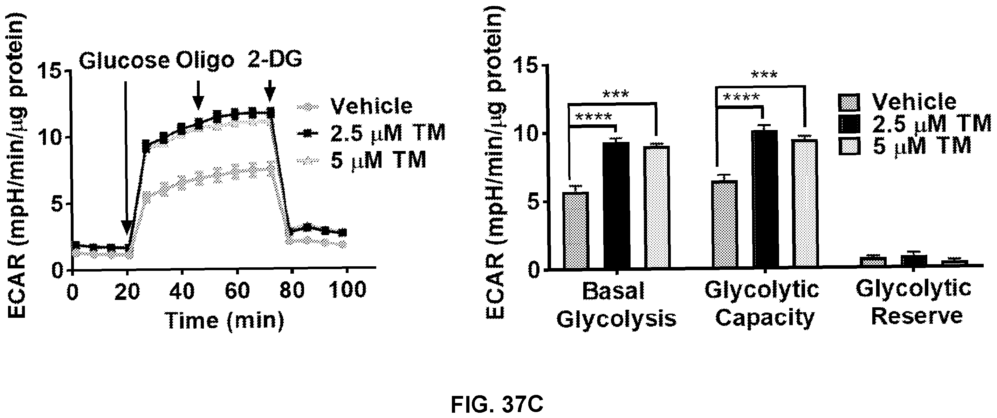

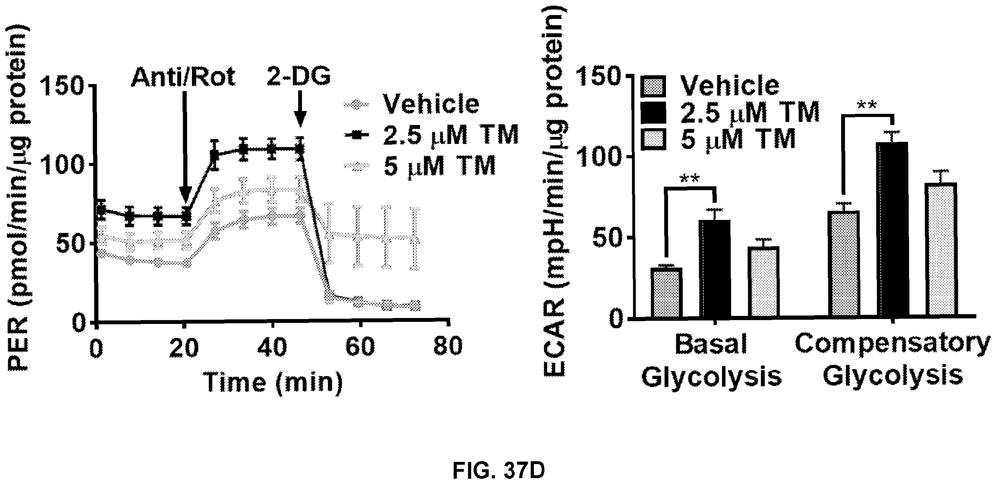

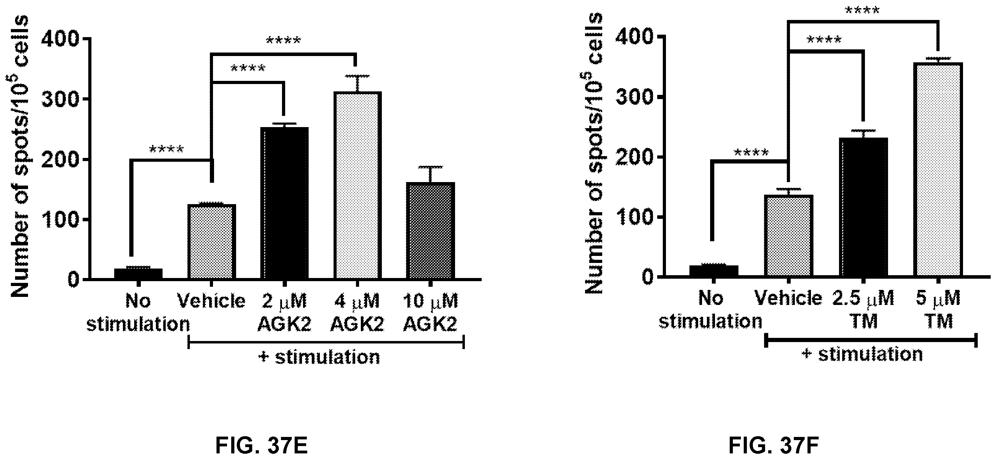

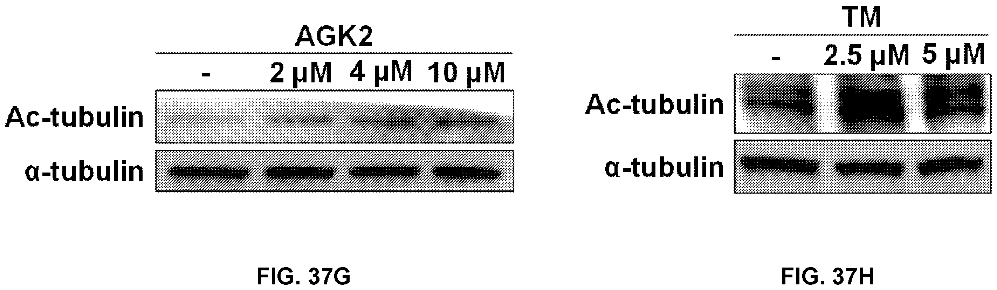

FIGs. 37A to 37H show treatment with Sirt2 Inhibitors, AGK2 and TM, augments the metabolic fitness and effector functions of human T cells from healthy donors. Human CD3+ T cells isolated from peripheral blood of healthy donors were stimulated with anti-CD3 in combination with AGK2 (FIGs. 37A, 37B, 37E, and 37G) or thiomyristoyl (TM) (FIGs. 37C, 37D, 37F, and 37H) versus vehicle for 48 hr. FIGs. 37A and 37C, Left, show extracellular acidification rate (ECAR) measured using the Seahorse XF analyzer on: AGK2-Treated CD3+ T cells (FIG. 37A, n = 7-8) and TM-treated CD3+ T cells (FIG. 37C, n = 4-8). Glucose, Oligomycin and 2-DG were injected at the points indicated. Right, basal glycolytic rate, glycolytic capacity and glycolytic reserve were calculated from the corresponding curves. FIGs. 37B and 37D, Left, show proton efflux rate (PER) was measured using the Seahorse XF analyzer on: AGK2-Treated CD3+ T cells (FIG. 37B, n = 2-8) and TM-treated CD3+ T cells (FIG. 37D, n = 3-5). Antimycin A/Rotenone and 2-DG were injected at the points indicated. Right, basal glycolytic rate and compensatory glycolysis were calculated from the corresponding curves. E and F, IFN-γ ELISPOT assay was performed on: AGK2-treated human CD3+ T cells (FIG, 37E, n = 6) and TM-treated human CD3+ T cells (FIG. 37F, n = 3-6) versus vehicle for 48 hr. FIGs. 37G and 37H show human CD3+ enriched T cells cultured with the indicated concentrations of AGK2 (FIG. 37G) or TM (FIG. 37H) for 48 hr. Acetylation levels of tubulin were determined by Western blot on protein extracts. Total α-tubulin levels were used as loading control. Data are representative of two independent experiments. Data are mean ± SEM. P values are determined by one-way ANOVA. *P < 0.05, **P < 0.01, ***P < 0.001, ****P < 0.0001.

### DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope of the present disclosure. Any recited method can be carried out in the order of events recited or in any other order that is logically possible.

Embodiments of the present disclosure will employ, unless otherwise indicated, techniques of chemistry, biology, and the like, which are within the skill of the art.

The following examples are put forth so as to provide those of ordinary skill in the art with a complete disclosure and description of how to perform the methods and use the probes disclosed and claimed herein. Efforts have been made to ensure accuracy with respect to numbers (*e.g*., amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise.

The term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

The term "therapeutically effective" refers to the amount of the composition used is of sufficient quantity to ameliorate one or more causes or symptoms of a disease or disorder. Such amelioration only requires a reduction or alteration, not necessarily elimination.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

### Sirt2 Inhibition

In some cases, the T cells are treated ex vivo with an effective amount of a Sirt2 inhibitor to reduce or ablate Sirt2 expression or activity. Sirt2 inhibitors are disclosed, for example, in US20170128459A1, to which further reference should be made for the teaching of these inhibitors. Sirt2 inhibitors include AGK2, AK-1, SirReal2, Tenovin-6, and Thiomyristoyl (TM), AEM1, and AEM2.

AGK2 shown below is a potent, and selective SIRT2 inhibitor with IC50 of 3.5 µM that minimally affects either SIRT1 or SIRT3 at 10-fold higher levels.

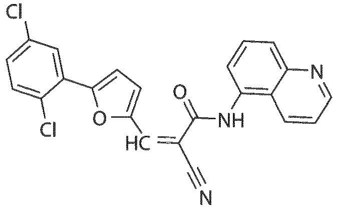

AK-1 shown below is an inhibitor of SIRT with good selectivity for SIRT2 over SIRT1 and SIRT3 (IC50 values >50 µM, 12.5 µM, and >50 µM for SIRT1, SIRT2, and SIRT3 respectively.

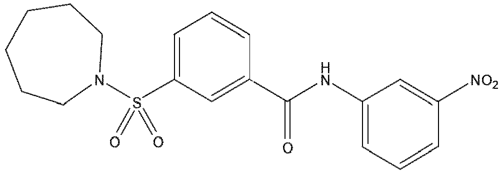

SirReal2 shown below is a potent and selective Sirt2 inhibitor with IC50 of 140 nM.

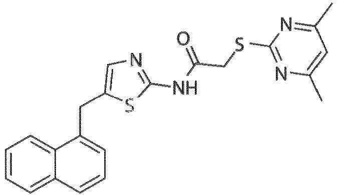

Tenovin-6 shown below inhibits the protein deacetylase activities of purified human SIRT1, SIRT2, and SIRT3 in vitro with IC50 values of 21, 10, and 67 µM, respectively.

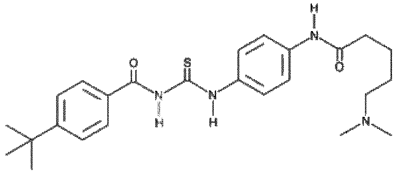

Thiomyristoyl (TM) shown below inhibits SIRT2 with an IC50 value of 0.028 µM

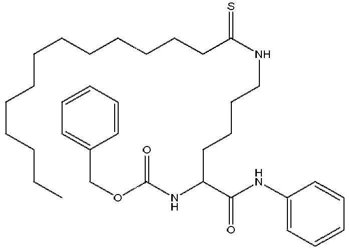

AEM1 and AEM2 (shown below) are selective inhibitors of SIRT2 with IC50 values of 18.5 and 3.8 µm, respectively.

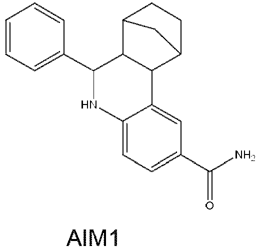

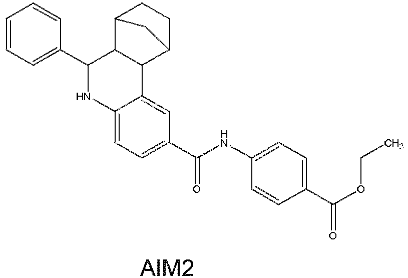

In some embodiments, the Sirt2 inhibitor is an antisense oligonucleotide, siRNA, an aptamer, or an antibody specific to the SIRT2 gene.

### Sirt2 Disruption

In some embodiments, the lymphocytes are genetically engineered to inhibitor or ablate Sirt2 expression.

As used herein the terms "inhibit" and "ablate" connote a partial or complete reduction in the expression and/or function of the *Sirt2* polypeptide encoded by the endogenous gene. Thus, the expression or function of the *Sirt2* gene product can be completely or partially disrupted or reduced (e.g., by 50%, 75%, 80%, 90%, 95% or more, e.g., 100%) in a selected group of cells (e.g., a tissue or organ) or in the entire animal.

Also disclosed are methods of disrupting Sirt2 expression in T cells ex vivo while effectively expressing chimeric receptors. Therefore, disclosed herein is a chimeric cell expressing a chimeric receptor, wherein the chimeric receptor is encoded by a transgene, and wherein the transgene is inserted in the genome of the cell at a location that disrupts expression or activity of an endogenous Sirt2 protein.

In some embodiments, the transgene is inserted into the *Sirt2* gene loci, thereby disrupting gene transcription. The transgene can be inserted at any loci within the *Sirt2* gene that would disrupt gene transcription.

Site-specific insertion of the transgene can be done, for example, by gene editing techniques, such as CRISPR.

### Chimeric antigen receptors (CAR)

In some cases, the lymphocytes also expresses a chimeric receptor. In some embodiments, the chimeric receptor comprises a chimeric antigen receptor (CAR) polypeptide.

CARs generally incorporate an antigen recognition domain from the single-chain variable fragments (scFv) of a monoclonal antibody (mAb) with transmembrane signaling motifs involved in lymphocyte activation (Sadelain M, et al. Nat Rev Cancer 2003 3:35-45). The disclosed CAR is generally made up of three domains: an ectodomain, a transmembrane domain, and an endodomain. The ectodomain comprises the recognition domain. It also optionally contains a signal peptide (SP) so that the CAR can be glycosylated and anchored in the cell membrane of the immune effector cell. The transmembrane domain (TD), is as its name suggests, connects the ectodomain to the endodomain and resides within the cell membrane when expressed by a cell. The endodomain is the business end of the CAR that transmits an activation signal to the immune effector cell after antigen recognition. For example, the endodomain can contain an intracellular signaling domain (ISD) and optionally a co-stimulatory signaling region (CSR).

A "signaling domain (SD)" generally contains immunoreceptor tyrosine-based activation motifs (ITAMs) that activate a signaling cascade when the ITAM is phosphorylated. The term "co-stimulatory signaling region (CSR)" refers to intracellular signaling domains from costimulatory protein receptors, such as CD28, 41BB, and ICOS, that are able to enhance T-cell activation by T-cell receptors.

In some embodiments, the endodomain contains an SD or a CSR, but not both. In these embodiments, an immune effector cell containing the disclosed CAR is only activated if another CAR (or a T-cell receptor) containing the missing domain also binds its respective antigen.

Additional CAR constructs are described, for example, in Fresnak AD, et al. Engineered T cells: the promise and challenges of cancer immunotherapy. Nat Rev Cancer. 2016 Aug 23;16(9):566-81, to which further reference should be made for the teaching of these CAR models.

For example, the CAR can be a TRUCK, Universal CAR, Self-driving CAR, Armored CAR, Self-destruct CAR, Conditional CAR, Marked CAR, TenCAR, Dual CAR, or sCAR.

TRUCKs (T cells redirected for universal cytokine killing) co-express a chimeric antigen receptor (CAR) and an antitumor cytokine. Cytokine expression may be constitutive or induced by T cell activation. Targeted by CAR specificity, localized production of pro-inflammatory cytokines recruits endogenous immune cells to tumor sites and may potentiate an antitumor response.

Universal, allogeneic CAR T cells are engineered to no longer express endogenous T cell receptor (TCR) and/or major histocompatibility complex (MHC) molecules, thereby preventing graft-versus-host disease (GVHD) or rejection, respectively.

Self-driving CARs co-express a CAR and a chemokine receptor, which binds to a tumor ligand, thereby enhancing tumor homing.

CAR T cells engineered to be resistant to immunosuppression (Armored CARs) may be genetically modified to no longer express various immune checkpoint molecules (for example, cytotoxic T lymphocyte-associated antigen 4 (CTLA4) or programmed cell death protein 1 (PD1)), with an immune checkpoint switch receptor, or may be administered with a monoclonal antibody that blocks immune checkpoint signaling.

A self-destruct CAR may be designed using RNA delivered by electroporation to encode the CAR. Alternatively, inducible apoptosis of the T cell may be achieved based on ganciclovir binding to thymidine kinase in gene-modified lymphocytes or the more recently described system of activation of human caspase 9 by a small-molecule dimerizer.

A conditional CAR T cell is by default unresponsive, or switched 'off', until the addition of a small molecule to complete the circuit, enabling full transduction of both signal 1 and signal 2, thereby activating the CAR T cell. Alternatively, T cells may be engineered to express an adaptor-specific receptor with affinity for subsequently administered secondary antibodies directed at target antigen.

Marked CAR T cells express a CAR plus a tumor epitope to which an existing monoclonal antibody agent binds. In the setting of intolerable adverse effects, administration of the monoclonal antibody clears the CAR T cells and alleviates symptoms with no additional off-tumor effects.

A tandem CAR (TanCAR) T cell expresses a single CAR consisting of two linked single-chain variable fragments (scFvs) that have different affinities fused to intracellular co-stimulatory domain(s) and a CD3ζ domain. TanCAR T cell activation is achieved only when target cells co-express both targets.

A dual CAR T cell expresses two separate CARs with different ligand binding targets; one CAR includes only the CD3ζ domain and the other CAR includes only the co-stimulatory domain(s). Dual CAR T cell activation requires co-expression of both targets on the tumor.

A safety CAR (sCAR) consists of an extracellular scFv fused to an intracellular inhibitory domain. sCAR T cells co-expressing a standard CAR become activated only when encountering target cells that possess the standard CAR target but lack the sCAR target.

The antigen recognition domain of the disclosed CAR is usually an scFv. There are however many alternatives. An antigen recognition domain from native T-cell receptor (TCR) alpha and beta single chains have been described, as have simple ectodomains (e.g. CD4 ectodomain to recognize HIV infected cells) and more exotic recognition components such as a linked cytokine (which leads to recognition of cells bearing the cytokine receptor). In fact almost anything that binds a given target with high affinity can be used as an antigen recognition region.

The endodomain is the business end of the CAR that after antigen recognition transmits a signal to the immune effector cell, activating at least one of the normal effector functions of the immune effector cell. Effector function of a T cell, for example, may be cytolytic activity or helper activity including the secretion of cytokines. Therefore, the endodomain may comprise the "intracellular signaling domain" of a T cell receptor (TCR) and optional co-receptors. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal.

Cytoplasmic signaling sequences that regulate primary activation of the TCR complex that act in a stimulatory manner may contain signaling motifs which are known as immunoreceptor tyrosine-based activation motifs (ITAMs). Examples of ITAM containing cytoplasmic signaling sequences include those derived from CD8, CD3ζ, CD3δ, CD3γ, CD3ε, CD32 (Fc gamma RIIa), DAP10, DAP12, CD79a, CD79b, FcγRlγ, FcγRIIIγ, FcεRIβ (FCERIB), and FcεRIγ (FCERIG).

In particular embodiments, the intracellular signaling domain is derived from CD3 zeta (CD3ζ) (TCR zeta, GenBank accno. BAG36664.1). T-cell surface glycoprotein CD3 zeta (CD3ζ) chain, also known as T-cell receptor T3 zeta chain or CD247 (Cluster of Differentiation 247), is a protein that in humans is encoded by the CD247 gene.

First-generation CARs typically had the intracellular domain from the CD3ζ chain, which is the primary transmitter of signals from endogenous TCRs. Second-generation CARs add intracellular signaling domains from various costimulatory protein receptors (e.g., CD28, 41BB, ICOS) to the endodomain of the CAR to provide additional signals to the T cell. Preclinical studies have indicated that the second generation of CAR designs improves the antitumor activity of T cells. More recent, third-generation CARs combine multiple signaling domains to further augment potency. T cells grafted with these CARs have demonstrated improved expansion, activation, persistence, and tumor-eradicating efficiency independent of costimulatory receptor/ligand interaction (Imai C, et al. Leukemia 2004 18:676-84; Maher J, et al. Nat Biotechnol 2002 20:70-5).

For example, the endodomain of the CAR can be designed to comprise the CD3ζ signaling domain by itself or combined with any other desired cytoplasmic domain(s) useful in the context of the CAR of the invention. For example, the cytoplasmic domain of the CAR can comprise a CD3ζ chain portion and a costimulatory signaling region. The costimulatory signaling region refers to a portion of the CAR comprising the intracellular domain of a costimulatory molecule. A costimulatory molecule is a cell surface molecule other than an antigen receptor or their ligands that is required for an efficient response of lymphocytes to an antigen. Examples of such molecules include CD27, CD28, 4-1BB (CD137), OX40, CD30, CD40, ICOS, lymphocyte function-associated antigen-1 (LFA-1), CD2, CD7, LIGHT, NKG2C, B7-H3, and a ligand that specifically binds with CD83, CD8, CD4, b2c, CD80, CD86, DAP10, DAP12, MyD88, BTNL3, and NKG2D. Thus, while the CAR is exemplified primarily with CD28 as the co-stimulatory signaling element, other costimulatory elements can be used alone or in combination with other co-stimulatory signaling elements.

In some embodiments, the CAR comprises a hinge sequence. A hinge sequence is a short sequence of amino acids that facilitates antibody flexibility (see, e.g., Woof et al., Nat. Rev. Immunol., 4(2): 89-99 (2004)). The hinge sequence may be positioned between the antigen recognition moiety (e.g., anti-CD123 scFv) and the transmembrane domain. The hinge sequence can be any suitable sequence derived or obtained from any suitable molecule. In some embodiments, for example, the hinge sequence is derived from a CD8a molecule or a CD28 molecule.

The transmembrane domain may be derived either from a natural or from a synthetic source. Where the source is natural, the domain may be derived from any membrane-bound or transmembrane protein. For example, the transmembrane region may be derived from (i.e. comprise at least the transmembrane region(s) of) the alpha, beta or zeta chain of the T-cell receptor, CD28, CD3 epsilon, CD45, CD4, CD5, CD8 (e.g., CD8 alpha, CD8 beta), CD9, CD16, CD22, CD33, CD37, CD64, CD80, CD86, CD134, CD137, or CD154, KIRDS2, OX40, CD2, CD27, LFA-1 (CD11a, CD18) , ICOS (CD278) , 4-1BB (CD137), GITR, CD40, BAFFR, HVEM (LIGHTR) , SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226) , SLAMF4 (CD244, 2B4) , CD84, CD96 (Tactile) , CEACAM1, CRTAM, Ly9 (CD229) , CD160 (BY55) , PSGL1, CD100 (SEMA4D) , SLAMF6 (NTB-A, Ly108) , SLAM (SLAMF1, CD150, IPO-3) , BLAME (SLAMF8) , SELPLG (CD162) , LTBR, and PAG/Cbp. Alternatively the transmembrane domain may be synthetic, in which case it will comprise predominantly hydrophobic residues such as leucine and valine. In some cases, a triplet of phenylalanine, tryptophan and valine will be found at each end of a synthetic transmembrane domain. A short oligo- or polypeptide linker, such as between 2 and 10 amino acids in length, may form the linkage between the transmembrane domain and the endoplasmic domain of the CAR.

In some embodiments, the CAR has more than one transmembrane domain, which can be a repeat of the same transmembrane domain, or can be different transmembrane domains.

In some embodiments, the CAR is a multi-chain CAR, as described in WO2015/039523, to which further reference should be made. A multi-chain CAR can comprise separate extracellular ligand binding and signaling domains in different transmembrane polypeptides. The signaling domains can be designed to assemble in juxtamembrane position, which forms flexible architecture closer to natural receptors, that confers optimal signal transduction. For example, the multi-chain CAR can comprise a part of an FCERI alpha chain and a part of an FCERI beta chain such that the FCERI chains spontaneously dimerize together to form a CAR.

In some embodiments, the recognition domain is a single chain variable fragment (scFv) antibody. The affinity/specificity of an scFv is driven in large part by specific sequences within complementarity determining regions (CDRs) in the heavy (V_{H}) and light (V_{L}) chain. Each V_{H} and V_{L} sequence will have three CDRs (CDR1, CDR2, CDR3).

In some embodiments, the recognition domain is derived from natural antibodies, such as monoclonal antibodies. In some cases, the antibody is human. In some cases, the antibody has undergone an alteration to render it less immunogenic when administered to humans. For example, the alteration comprises one or more techniques selected from the group consisting of chimerization, humanization, CDR-grafting, deimmunization, and mutation of framework amino acids to correspond to the closest human germline sequence.

Also disclosed are bi-specific CARs that target two different antigens. Also disclosed are CARs designed to work only in conjunction with another CAR that binds a different antigen, such as a tumor antigen. For example, in these embodiments, the endodomain of the disclosed CAR can contain only a signaling domain (SD) or a co-stimulatory signaling region (CSR), but not both. The second CAR (or endogenous T-cell) provides the missing signal if it is activated. For example, if the disclosed CAR contains an SD but not a CSR, then the immune effector cell containing this CAR is only activated if another CAR (or T-cell) containing a CSR binds its respective antigen. Likewise, if the disclosed CAR contains a CSR but not a SD, then the immune effector cell containing this CAR is only activated if another CAR (or T-cell) containing an SD binds its respective antigen.

Tumor antigens are proteins that are produced by tumor cells that elicit an immune response, particularly T-cell mediated immune responses. The additional antigen binding domain can be an antibody or a natural ligand of the tumor antigen. The selection of the additional antigen binding domain will depend on the particular type of cancer to be treated. Tumor antigens are well known in the art and include, for example, a glioma-associated antigen, carcinoembryonic antigen (CEA), EGFRvIII, IL-IIRa, IL-13Ra, EGFR, FAP, B7H3, Kit, CA LX, CS-1, MUC1, BCMA, bcr-abl, HER2, β-human chorionic gonadotropin, alphafetoprotein (AFP), ALK, CD19, TIM3, cyclin BI, lectin-reactive AFP, Fos-related antigen 1, ADRB3, thyroglobulin, EphA2, RAGE-1, RUI, RU2, SSX2, AKAP-4, LCK, OY-TESI, PAX5, SART3, CLL-1, fucosyl GM1, GloboH, MN-CA IX, EPCAM, EVT6-AML, TGS5, human telomerase reverse transcriptase, plysialic acid, PLAC1, RUI, RU2 (AS), intestinal carboxyl esterase, lewisY, sLe, LY6K, mut hsp70-2, M-CSF, MYCN, RhoC, TRP-2, CYPIBI, BORIS, prostase, prostate-specific antigen (PSA), PAX3, PAP, NY-ESO-1, LAGE-la, LMP2, NCAM, p53, p53 mutant, Ras mutant, gplOO, prostein, OR51E2, PANX3, PSMA, PSCA, Her2/neu, hTERT, HMWMAA, HAVCR1, VEGFR2, PDGFR-beta, survivin and telomerase, legumain, HPV E6,E7, sperm protein 17, SSEA-4, tyrosinase, TARP, WT1, prostate-carcinoma tumor antigen- 1 (PCTA-1), ML-IAP, MAGE, MAGE-A1,MAD-CT-1, MAD-CT-2, MelanA/MART 1, XAGE1 , ELF2M, ERG (TMPRSS2 ETS fusion gene), NA17, neutrophil elastase, sarcoma translocation breakpoints, NY-BR-1, ephnnB2, CD20, CD22, CD24, CD30, TIM3, CD38, CD44v6, CD97, CD171, CD179a, androgen receptor, FAP, insulin growth factor (IGF)-I, IGFII, IGF-I receptor, GD2, o-acetyl-GD2, GD3, GM3, GPRC5D, GPR20, CXORF61, folate receptor (FRa), folate receptor beta, ROR1, Flt3, TAG72, TN Ag, Tie 2, TEM1, TEM7R, CLDN6, TSHR, UPK2, and mesothelin. In a preferred embodiment, the tumor antigen is selected from the group consisting of folate receptor (FRa), mesothelin, EGFRvIII, IL-13Ra, CD123, CD19, TIM3, BCMA, GD2, CLL-1, CA-IX, MUCI, HER2, and any combination thereof.

Non-limiting examples of tumor antigens include the following: Differentiation antigens such as tyrosinase, TRP-1, TRP-2 and tumor-specific multilineage antigens such as MAGE-1, MAGE-3, BAGE, GAGE-1, GAGE-2, pi 5; overexpressed embryonic antigens such as CEA; overexpressed oncogenes and mutated tumor-suppressor genes such as p53, Ras, HER-2/neu; unique tumor antigens resulting from chromosomal translocations; such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include TSP- 180, MAGE-4, MAGE-5, MAGE-6, RAGE, NY-ESO, pl85erbB2, pl80erbB-3, c-met, nm- 23H1, PSA, CA 19-9, CA 72-4, CAM 17.1, NuMa, K-ras, beta-Catenin, CDK4, Mum-1, p 15, p 16, 43-9F, 5T4, 791Tgp72, alpha-fetoprotein, beta-HCG, BCA225, BTAA, CA 125, CA 15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCASI, SDCCAG1 6, TA-90\Mac-2 binding protein\cyclophilm C-associated protein, TAAL6, TAG72, TLP, TPS, GPC3, MUC16, LMP1, EBMA-1, BARF-1, CS1, CD319, HER1, B7H6, L1CAM, IL6, and MET.

### Nucleic Acids and Vectors

Also disclosed are polynucleotides and polynucleotide vectors encoding the disclosed chimeric receptors. Also disclosed are oligonucleotides for use in inserting the chimeric receptors into the genome of a T cell at a site that will disrupt Sirt2 expression or activity.

Nucleic acid sequences encoding the disclosed chimeric receptors, and regions thereof, can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

### Immune effector cells

Also disclosed are immune effector cells that are engineered to express the disclosed chimeric receptors. These cells are preferably obtained from the subject to be treated (i.e. are autologous). However, in some embodiments, immune effector cell lines or donor effector cells (allogeneic) are used. Immune effector cells can be obtained from a number of sources, including peripheral blood mononuclear cells, bone marrow, lymph node tissue, cord blood, thymus tissue, tissue from a site of infection, ascites, pleural effusion, spleen tissue, and tumors. Immune effector cells can be obtained from blood collected from a subject using any number of techniques known to the skilled artisan, such as Ficoll^{™} separation. For example, cells from the circulating blood of an individual may be obtained by apheresis. In some embodiments, immune effector cells are isolated from peripheral blood lymphocytes by lysing the red blood cells and depleting the monocytes, for example, by centrifugation through a PERCOLL^{™} gradient or by counterflow centrifugal elutriation. A specific subpopulation of immune effector cells can be further isolated by positive or negative selection techniques. For example, immune effector cells can be isolated using a combination of antibodies directed to surface markers unique to the positively selected cells, e.g., by incubation with antibody-conjugated beads for a time period sufficient for positive selection of the desired immune effector cells. Alternatively, enrichment of immune effector cells population can be accomplished by negative selection using a combination of antibodies directed to surface markers unique to the negatively selected cells.

In some embodiments, the immune effector cells comprise any leukocyte involved in defending the body against infectious disease and foreign materials. For example, the immune effector cells can comprise lymphocytes, monocytes, macrophages, dentritic cells, mast cells, neutrophils, basophils, eosinophils, or any combinations thereof. For example, the immune effector cells can comprise T lymphocytes.

T cells or T lymphocytes can be distinguished from other lymphocytes, such as B cells and natural killer cells (NK cells), by the presence of a T-cell receptor (TCR) on the cell surface. They are called T cells because they mature in the thymus (although some also mature in the tonsils). There are several subsets of T cells, each with a distinct function.

T helper cells (T_{H} cells) assist other white blood cells in immunologic processes, including maturation of B cells into plasma cells and memory B cells, and activation of cytotoxic T cells and macrophages. These cells are also known as CD4+ T cells because they express the CD4 glycoprotein on their surface. Helper T cells become activated when they are presented with peptide antigens by MHC class II molecules, which are expressed on the surface of antigen-presenting cells (APCs). Once activated, they divide rapidly and secrete small proteins called cytokines that regulate or assist in the active immune response. These cells can differentiate into one of several subtypes, including T_{H}1, T_{H}2, T_{H}3, T_{H}17, T_{H}9, or T_{FH}, which secrete different cytokines to facilitate a different type of immune response.

Cytotoxic T cells (T_{C} cells, or CTLs) destroy virally infected cells and tumor cells, and are also implicated in transplant rejection. These cells are also known as CD8⁺ T cells since they express the CD8 glycoprotein at their surface. These cells recognize their targets by binding to antigen associated with MHC class I molecules, which are present on the surface of all nucleated cells. Through IL-10, adenosine and other molecules secreted by regulatory T cells, the CD8+ cells can be inactivated to an anergic state, which prevents autoimmune diseases.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory cells may be either CD4⁺ or CD8⁺. Memory T cells typically express the cell surface protein CD45RO.

Regulatory T cells (T_{reg} cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus. Two major classes of CD4⁺ T_{reg} cells have been described - naturally occurring T_{reg} cells and adaptive T_{reg} cells.

Natural killer T (NKT) cells (not to be confused with natural killer (NK) cells) bridge the adaptive immune system with the innate immune system. Unlike conventional T cells that recognize peptide antigens presented by major histocompatibility complex (MHC) molecules, NKT cells recognize glycolipid antigen presented by a molecule called CD1d.

In some embodiments, the T cells comprise a mixture of CD4+ cells. In other embodiments, the T cells are enriched for one or more subsets based on cell surface expression. For example, in some cases, the T comprise are cytotoxic CD8⁺ T lymphocytes. In some embodiments, the T cells comprise γδ T cells, which possess a distinct T-cell receptor (TCR) having one γ chain and one δ chain instead of α and β chains.

Natural-killer (NK) cells are CD56⁺CD3⁻ large granular lymphocytes that can kill virally infected and transformed cells, and constitute a critical cellular subset of the innate immune system (Godfrey J, et al. Leuk Lymphoma 2012 53:1666-1676). Unlike cytotoxic CD8⁺ T lymphocytes, NK cells launch cytotoxicity against tumor cells without the requirement for prior sensitization, and can also eradicate MHC-I-negative cells (Narni-Mancinelli E, et al. Int Immunol 2011 23:427-431). NK cells are safer effector cells, as they may avoid the potentially lethal complications of cytokine storms (Morgan RA, et al. Mol Ther 2010 18:843-851), tumor lysis syndrome (Porter DL, et al. N Engl J Med 2011 365:725-733), and on-target, off-tumor effects. Although NK cells have a well-known role as killers of cancer cells, and NK cell impairment has been extensively documented as crucial for progression of MM (Godfrey J, et al. Leuk Lymphoma 2012 53:1666-1676; Fauriat C, et al. Leukemia 2006 20:732-733), the means by which one might enhance NK cell-mediated anti-MM activity has been largely unexplored prior to the disclosed CARs.

### Therapeutic Methods

Immune effector cells expressing the disclosed chimeric receptors can elicit an anti-tumor immune response against cancer cells. The anti-tumor immune response elicited by the disclosed chimeric cells may be an active or a passive immune response. In addition, the immune response may be part of an adoptive immunotherapy approach in which chimeric cells induce an immune response specific to the target antigen.

Adoptive transfer of immune effector cells expressing chimeric receptors is a promising anti-cancer therapeutic. Following the collection of a patient's immune effector cells, the cells may be genetically engineered to express the disclosed chimeric receptors while ablating Sirt2 according to the disclosed methods, then infused back into the patient.

The disclosed chimeric effector cells may be administered either alone, or as a pharmaceutical composition in combination with diluents and/or with other components such as IL-2, IL-15, or other cytokines or cell populations. Briefly, pharmaceutical compositions may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions for use in the disclosed methods are in some embodiments formulated for intravenous administration. Pharmaceutical compositions may be administered in any manner appropriate treat tumors. The quantity and frequency of administration will be determined by such factors as the condition of the patient, and the severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, such as 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

In certain embodiments, it may be desired to administer activated T cells to a subject and then subsequently re-draw blood (or have an apheresis performed), activate T cells therefrom according to the disclosed methods, and reinfuse the patient with these activated and expanded T cells. This process can be carried out multiple times every few weeks. In certain embodiments, T cells can be activated from blood draws of from 10 cc to 400 cc. In certain embodiments, T cells are activated from blood draws of 20 cc, 30 cc, 40 cc, 50 cc, 60 cc, 70 cc, 80 cc, 90 cc, or 100 cc. Using this multiple blood draw/multiple reinfusion protocol may serve to select out certain populations of T cells.

The administration of the disclosed compositions may be carried out in any convenient manner, including by injection, transfusion, or implantation. The compositions described herein may be administered to a patient subcutaneously, intradermally, intratumorally, intranodally, intramedullary, intramuscularly, by intravenous (i.v.) injection, or intraperitoneally. In some embodiments, the disclosed compositions are administered to a patient by intradermal or subcutaneous injection. In some embodiments, the disclosed compositions are administered by i.v. injection. The compositions may also be injected directly into a tumor, lymph node, or site of infection.

In certain embodiments, the disclosed chimeric cells are administered to a patient in conjunction with (e.g., before, simultaneously or following) any number of relevant treatment modalities, including but not limited to thalidomide, dexamethasone, bortezomib, and lenalidomide. In further embodiments, the chimeric cells may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycophenolic acid, steroids, FR901228, cytokines, and irradiation. In some embodiments, the CAR-modified immune effector cells are administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In another embodiment, the cell compositions of the present invention are administered following B-cell ablative therapy such as agents that react with CD20, e.g., Rituxan. For example, in some embodiments, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The cancer of the disclosed methods can be any cell in a subject undergoing unregulated growth, invasion, or metastasis. In some aspects, the cancer can be any neoplasm or tumor for which radiotherapy is currently used. Alternatively, the cancer can be a neoplasm or tumor that is not sufficiently sensitive to radiotherapy using standard methods. Thus, the cancer can be a sarcoma, lymphoma, leukemia, carcinoma, blastoma, or germ cell tumor. A representative but non-limiting list of cancers that the disclosed compositions can be used to treat include lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, endometrial cancer, cervical cancer, cervical carcinoma, breast cancer, epithelial cancer, renal cancer, genitourinary cancer, pulmonary cancer, esophageal carcinoma, head and neck carcinoma, large bowel cancer, hematopoietic cancers; testicular cancer; colon and rectal cancers, prostatic cancer, and pancreatic cancer.

The disclosed chimeric cells can be used in combination with any compound, moiety or group which has a cytotoxic or cytostatic effect. Drug moieties include chemotherapeutic agents, which may function as microtubulin inhibitors, mitosis inhibitors, topoisomerase inhibitors, or DNA intercalators, and particularly those which are used for cancer therapy.

The disclosed chimeric cells can be used in combination with a checkpoint inhibitor. The two known inhibitory checkpoint pathways involve signaling through the cytotoxic T-lymphocyte antigen-4 (CTLA-4) and programmed-death 1 (PD-1) receptors. These proteins are members of the CD28-B7 family of cosignaling molecules that play important roles throughout all stages of T cell function. The PD-1 receptor (also known as CD279) is expressed on the surface of activated T cells. Its ligands, PD-L1 (B7-H1; CD274) and PD-L2 (B7-DC; CD273), are expressed on the surface of APCs such as dendritic cells or macrophages. PD-L1 is the predominant ligand, while PD-L2 has a much more restricted expression pattern. When the ligands bind to PD-1, an inhibitory signal is transmitted into the T cell, which reduces cytokine production and suppresses T-cell proliferation. Checkpoint inhibitors include, but are not limited to antibodies that block PD-1 (Nivolumab (BMS-936558 or MDX1106), CT-011, MK-3475), PD-L1 (MDX-1105 (BMS-936559), MPDL3280A, MSB0010718C), PD-L2 (rHIgM12B7), CTLA-4 (Ipilimumab (MDX-010), Tremelimumab (CP-675,206)), IDO, B7-H3 (MGA271), B7-H4, TIM3, LAG-3 (BMS-986016).

Human monoclonal antibodies to programmed death 1 (PD-1) and methods for treating cancer using anti-PD-1 antibodies alone or in combination with other immunotherapeutics are described in U.S. Patent No. 8,008,449, to which further reference should be made for these antibodies. Anti-PD-L1 antibodies and uses therefor are described in U.S. Patent No. 8,552,154, to which further reference should be made for these antibodies. Anticancer agent comprising anti-PD-1 antibody or anti-PD-L1 antibody are described in U.S. Patent No. 8,617,546, to which further reference should be made for these antibodies.

In some embodiments, the PDL1 inhibitor comprises an antibody that specifically binds PDL1, such as BMS-936559 (Bristol-Myers Squibb) or MPDL3280A (Roche). In some embodiments, the PD1 inhibitor comprises an antibody that specifically binds PD1, such as lambrolizumab (Merck), nivolumab (Bristol-Myers Squibb), or MEDI4736 (AstraZeneca). Human monoclonal antibodies to PD-1 and methods for treating cancer using anti-PD-1 antibodies alone or in combination with other immunotherapeutics are described in U.S. Patent No. 8,008,449, to which further reference should be made for these antibodies. Anti-PD-L1 antibodies and uses therefor are described in U.S. Patent No. 8,552,154, to which further reference should be made for these antibodies. Anticancer agent comprising anti-PD-1 antibody or anti-PD-L1 antibody are described in U.S. Patent No. 8,617,546, to which further reference should be made for these antibodies.

The disclosed chimeric cells can be used in combination with other cancer immunotherapies. There are two distinct types of immunotherapy: passive immunotherapy uses components of the immune system to direct targeted cytotoxic activity against cancer cells, without necessarily initiating an immune response in the patient, while active immunotherapy actively triggers an endogenous immune response. Passive strategies include the use of the monoclonal antibodies (mAbs) produced by B cells in response to a specific antigen. The development of hybridoma technology in the 1970s and the identification of tumor-specific antigens permitted the pharmaceutical development of mAbs that could specifically target tumor cells for destruction by the immune system. Thus far, mAbs have been the biggest success story for immunotherapy; the top three best-selling anticancer drugs in 2012 were mAbs. Among them is rituximab (Rituxan, Genentech), which binds to the CD20 protein that is highly expressed on the surface of B cell malignancies such as non-Hodgkin's lymphoma (NHL). Rituximab is approved by the FDA for the treatment of NHL and chronic lymphocytic leukemia (CLL) in combination with chemotherapy. Another important mAb is trastuzumab (Herceptin; Genentech), which revolutionized the treatment of HER2 (human epidermal growth factor receptor 2)-positive breast cancer by targeting the expression of HER2.

Generating optimal "killer" CD8 T cell responses also requires T cell receptor activation plus co-stimulation, which can be provided through ligation of tumor necrosis factor receptor family members, including OX40 (CD134) and 4-1BB (CD137). OX40 is of particular interest as treatment with an activating (agonist) anti-OX40 mAb augments T cell differentiation and cytolytic function leading to enhanced anti-tumor immunity against a variety of tumors.

In some embodiments, such an additional therapeutic agent may be selected from an antimetabolite, such as methotrexate, 6-mercaptopurine, 6-thioguanine, cytarabine, fludarabine, 5-fluorouracil, mmune zine, hydroxyurea, asparaginase, gemcitabine or cladribine.

In some embodiments, such an additional therapeutic agent may be selected from an alkylating agent, such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin.

In some embodiments, such an additional therapeutic agent is a targeted agent, such as ibrutinib or idelalisib.

In some embodiments, such an additional therapeutic agent is an epigenetic modifier such as azacitdine or vidaza.

In some embodiments, such an additional therapeutic agent may be selected from an anti-mitotic agent, such as taxanes, for instance docetaxel, and paclitaxel, and vinca alkaloids, for instance vindesine, vincristine, vinblastine, and vinorelbine.

In some embodiments, such an additional therapeutic agent may be selected from a topoisomerase inhibitor, such as topotecan or irinotecan, or a cytostatic drug, such as etoposide and teniposide.

In some embodiments, such an additional therapeutic agent may be selected from a growth factor inhibitor, such as an inhibitor of ErbBl (EGFR) (such as an EGFR antibody, e.g. zalutumumab, cetuximab, panitumumab or nimotuzumab or other EGFR inhibitors, such as gefitinib or erlotinib), another inhibitor of ErbB2 (HER2/neu) (such as a HER2 antibody, e.g. trastuzumab, trastuzumab-DM I or pertuzumab) or an inhibitor of both EGFR and HER2, such as lapatinib).

In some embodiments, such an additional therapeutic agent may be selected from a tyrosine kinase inhibitor, such as imatinib (Glivec, Gleevec STI571) or lapatinib.

Therefore, in some embodiments, a disclosed antibody is used in combination with ofatumumab, zanolimumab, daratumumab, ranibizumab, nimotuzumab, panitumumab, hu806, daclizumab (Zenapax), basiliximab (Simulect), infliximab (Remicade), adalimumab (Humira), natalizumab (Tysabri), omalizumab (Xolair), efalizumab (Raptiva), and/or rituximab.

In some embodiments, a therapeutic agent for use in combination with chimeric cells for treating the disorders as described above may be an anti-cancer cytokine, chemokine, or combination thereof. Examples of suitable cytokines and growth factors include IFNy, IL-2, IL-4, IL-6, IL-7, IL-10, IL-12, IL-13, IL-15, IL-18, IL-23, IL-24, IL-27, IL-28a, IL-28b, IL-29, KGF, IFNa (e.g., INFa2b), IFN , GM-CSF, CD40L, Flt3 ligand, stem cell factor, ancestim, and TNFa. Suitable chemokines may include Glu-Leu-Arg (ELR)- negative chemokines such as IP-10, MCP-3, MIG, and SDF-la from the human CXC and C-C chemokine families. Suitable cytokines include cytokine derivatives, cytokine variants, cytokine fragments, and cytokine fusion proteins.

In some embodiments, a therapeutic agent for use in combination with chimeric cells for treating the disorders as described above may be a cell cycle control/apoptosis regulator (or "regulating agent"). A cell cycle control/apoptosis regulator may include molecules that target and modulate cell cycle control/apoptosis regulators such as (i) cdc-25 (such as NSC 663284), (ii) cyclin-dependent kinases that overstimulate the cell cycle (such as flavopiridol (L868275, HMR1275), 7-hydroxystaurosporine (UCN-01, KW-2401), and roscovitine (R-roscovitine, CYC202)), and (iii) telomerase modulators (such as BIBR1532, SOT-095, GRN163 and compositions described in for instance US 6,440,735 and US 6,713,055) . Non-limiting examples of molecules that interfere with apoptotic pathways include TNF-related apoptosis-inducing ligand (TRAIL)/apoptosis-2 ligand (Apo-2L), antibodies that activate TRAIL receptors, IFNs, and anti-sense Bcl-2.

In some embodiments, a therapeutic agent for use in combination with chimeric cells for treating the disorders as described above may be a hormonal regulating agent, such as agents useful for anti-androgen and anti-estrogen therapy. Examples of such hormonal regulating agents are tamoxifen, idoxifene, fulvestrant, droloxifene, toremifene, raloxifene, diethylstilbestrol, ethinyl estradiol/estinyl, an antiandrogene (such as flutaminde/eulexin), a progestin (such as such as hydroxyprogesterone caproate, medroxy- progesterone/provera, megestrol acepate/megace), an adrenocorticosteroid (such as hydrocortisone, prednisone), luteinizing hormone-releasing hormone (and analogs thereof and other LHRH agonists such as buserelin and goserelin), an aromatase inhibitor (such as anastrazole/arimidex, aminoglutethimide/cytraden, exemestane) or a hormone inhibitor (such as octreotide/sandostatin).

In some embodiments, a therapeutic agent for use in combination with chimeric cells for treating the disorders as described above may be an anti-cancer nucleic acid or an anti-cancer inhibitory RNA molecule.

Combined administration, as described above, may be simultaneous, separate, or sequential. For simultaneous administration the agents may be administered as one composition or as separate compositions, as appropriate.

In some embodiments, the disclosed chimeric cells are administered in combination with radiotherapy. Radiotherapy may comprise radiation or associated administration of radiopharmaceuticals to a patient is provided. The source of radiation may be either external or internal to the patient being treated (radiation treatment may, for example, be in the form of external beam radiation therapy (EBRT) or brachytherapy (BT)). Radioactive elements that may be used in practicing such methods include, e.g., radium, cesium-137, iridium-192, americium-241, gold-198, cobalt-57, copper-67, technetium-99, iodide-123, iodide-131, and indium-111.

In some embodiments, the disclosed chimeric cells are administered in combination with surgery.

A number of embodiments of the invention have been described. Nevertheless, it will be understood that various modifications may be made without departing from the scope of the invention. Accordingly, other embodiments are within the scope of the following claims.

### EXAMPLES

### Example 1: Targeting Sirt2 reprograms T cell metabolism for optimal immune response

There is a growing evidence that metabolism is a key driver of T cell functions. A switch from oxidative phosphorylation to aerobic glycolysis is a hallmark of T cell activation and is required to meet metabolic demands of proliferation and effector functions. However the mechanisms underlying the metabolic switch in T cells remain unclear. Sirt2 is shown herein to be a crucial immune checkpoint coordinating metabolic and functional fitness of T cells. Sirt2 is induced upon T cells activation and increases in late maturation stages. Sirt2 negatively regulates glycolysis by targeting key glycolytic enzymes. Remarkably, Sirt2 knockout T cells exhibit profound upregulation of aerobic glycolysis with enhanced proliferation and effector function and thus effectively reject tumor challenge *in vivo.* Furthermore pharmacologic inhibition of Sirt2 in human tumor infiltrating lymphocytes demonstrated similar phenotype. Taken together, these results demonstrate Sirt2 as an actionable target to reprogram T cell metabolism to augment immunotherapy.

### Methods

### Mice

C57BL/6J, Pmel mice with a gp100-reactive transgenic T cell receptor, Sirt2 knockout Mince (Sirt2KO) B6.129-Sirt2^{tm1.1Fwa}/J and NOD scid gamma (NSG) mice were purchased from Jackson Laboratories. Pmel TCR transgenic mice were crossed with Sirt2KO mice to generate Pmel/Sirt2KO mice. All mice were bred and maintained under specific pathogen-free conditions at the animal facility of Moffitt Cancer Center. All animal protocols were approved by the Institutional Animal Care and Use Committee. Mice were used at 7-8 weeks old with the age- and gender-matched to controls.

### Mouse T cell culture

Spleens and/or Lymph nodes (LN) were collected from WT, Sirt2KO, Pmel/WT, Pmel/Sirt2KO mice and processed into single-cell suspensions. CD3⁺, CD4⁺ and CD8⁺ T cells were negatively enriched using Mouse Pan T cell, CD4⁺ T cell and CD8⁺ T cell isolation kits^{™} respectively according to the manufacturers' instructions. The purity of the isolated cells was confirmed by flow cytometry (>95%). Purified T cells were cultured in completed RPMI 1640 medium (ThermoFisher Scientific) supplemented with 10% (vol/vol) Fetal bovine serum (FBS, Biowest) and 1% (vol/vol) penicillin/streptomycin (P/S, Thomas Scientific Inc). WT and Sirt2KO T cells were activated with plate-bound anti-CD3 antibody (5 µg/ml; 145-2C11, BioXCell) for the indicated time points. Pmel/WT and Pmel/Sirt2KO T cells were stimulated with gp100 (1µg/ml, AnaSpec) for the indicated time points.

### Human PBMC

Human peripheral blood mononuclear cells (PBMC) were obtained from healthy donors by density gradient centrifugation using Ficoll-Paque^{™} PLUS Media (GE Healthcare). CD3⁺ lymphocytes were negatively enriched using human Pan T cell isolation kit^{™} (Miltenyi Biotec). Enriched CD3⁺ T cells were cultured in completed RPMI medium and activated with plate-bound anti-CD3 antibody (5µg/ml; OKT-3, BioXCell) in the presence of DMSO (Vehicle), or one of Sirt2 inhibitors: AGK2 and Thiomyristoyl (Selleck Chemicals) at the indicated concentrations.

### Human TILs

Human tumor infiltrating lymphocytes (hTILs) samples isolated from tumor biopsies of patients with non-small cell lung cancer (NSCLC) were generously provided by Dr. Antonia lab (Moffitt Cancer Center). All human samples remained totally de-identified and no Institutional Review Board (IRB) approval was needed.

hTILs were maintained in RPMI 1640 Medium, supplemented with 10% human AB serum (Valley Biomedical, Inc.), 1 mM Hepes (Sigma-Aldrich), 1% P/S (Thomas Scientific Inc), 50µg/ml Gentamicin (ThermoFisher Scientific), 50µM β-mercaptoethanol (ThermoFisher Scientific) and 6000U/ml of recombinant human IL-2 (Peprotech). For metabolic and functional assays, hTILs were cultured in completed RPMI medium, IL-2 free, on plate-bound anti-CD3 antibody (5µg/ml; OKT-3, BioXCell) in the presence of vehicle or AGK2 (Selleck Chemicals) at the indicated concentrations.

### Cell line for tumor model

B16F10 melanoma cells were obtained from the American Type Culture Collection. Cells were passaged minimally and maintained in completed Dulbecco's Modified Eagle Medium DMEM/F12 (ThermoFisher Scientific) containing 10% FBS (Biowest) and 1% P/S (Thomas Scientific Inc).

### Lung metastasis model

2.5 × 10⁵ B16F10 tumor cells in 100 µl of sterile phosphate buffered saline (PBS, Sigma) were injected intravenously (i.v.) into lateral tail veins of mice. For anti-PD-1 administration, mice were injected intraperitoneally (i.p.) with 200 µg of anti-PD-1 antibody (RMP1-14, BioXCell) every 3 days for a total of three injections. Eighteen days post-tumor cells injection, lungs were removed from euthanized mice. The number of visible black metastatic nodules on the lung surface was counted visually using a magnifying glass.

### Subcutaneous tumor model

Anesthetized mice were injected subcutaneously (s.c.) into the hind limb with 2.5 × 10⁵ B16F10 tumor cells in 200 µl of sterile PBS. Tumor volumes were monitored twice a week during 3 weeks and calculated using the equation for ellipsoidal volume = length x width x height x π/6.

### Mouse TILs isolation

Lymphocytes from subcutaneous tumors were isolated by dicing the tissues and enzymatic digestion in PBS containing collagenase type 4 (2 mg/ml, Worthington Biochemical) and Dnase I (0.25mg/ml, Sigma) for 45 min with occasional shaking at 37 °C. Cell suspensions were then twice filtered through 100 µm and 40 µm cell strainers (ThermoFisher Scientific) to single-cell suspension followed by PBS wash and red blood cell lysis using RBC Lysis Buffer (Biolegend). TILs were finally isolated by density gradient centrifugation using Percoll (GE Healthcare) and further purified using CD3ε MicroBeads kit^{™} (Miltenyi Biotec) according to the manufacturer's instructions. T cell purity was greater than 90% (Data not shown). Fresh TILs were used directly for phenotypic and metabolic analyses or were cultured in completed RPMI Medium for functional assays.

### In vivo T cell depletion

*In vivo* depletion of CD4⁺ or CD8⁺ T lymphocyte subsets was performed by i.p. injection of 100 µg of purified monoclonal anti-CD4 (GK1.5, BioXCell) or anti-CD8 (2.43, BioXCell) twice weekly. Control (non-depleted) mice were treated with i.p. injection of 100 µg of nonimmune IgG isotype control (LTF-2, BioXCell). Depleted and control mice received i.v 2.5 × 10⁵ B16F10 cells (n = 5 per group).

### Adoptive transfer of Pmel T cells

Naive CD8⁺ T cells were purified from spleens of Pmel/WT and Pmel/Sirt2KO donor mice using CD8⁺ T Cell Isolation kit^{™} (Miltenyi Biotec). NSG mice (n = 8 per group) were injected i.v. with 1 × 10⁵ B16F10 melanoma cells. Concurrently 5 × 10⁵ naïve CD8⁺ Pmel/WT or Pmel/Sirt2KO T cells were adoptively transferred into the recipient mice. Control mice received B16F10 tumor cells only.

### Flow cytometry

For analysis of surface markers, cells were stained in PBS containing 5% FBS (vol/vol, FACS buffer) with: CD3 (BUV395), CD4 (BUV805), PD-1 (BV605) from BD Biosciences, CD8 (Alexa Fluor 700), CD44 (Alexa Fluor 488) and CD62L (PE-Cy7) from Biolegend, incubated at 4 °C for 1 hour, then washed twice with FACS buffer, and finally fixed in PBS containing 1% paraformaldehyde. Dead cells were excluded using the Zombie Violet^{™} Fixable Viability Kit (Biolegend) following the manufacturer's protocol.

For Sirt2 intracellular staining, cells were first labeled with surface markers CD4 (BUV 805), CD8 (Alexa Fluor 700), CD44 (Alexa Fluor 488), CD62L (PE-Cy7), and then fixed and permeabilized with Cytofix/Cytoperm^{™} Buffer (BD Biosciences). Intracellular labeling of Sirt2 was then performed using a specific Sirt2 antibody (Sigma, #9441) combined with a secondary Goat anti-rabbit IgG antibody (Alexa647, ThermoFisher) according to manufacturer's instruction.

For intracellular cytokine staining, cells were firstly stimulated with phorbol 12-myristate 13-acetate (PMA, 10 ng/ml, Sigma) and ionomycin (1 µM/ml, Sigma) for 1 hour followed by GolgiPlug^{™} treatment (1‰, BD Biosciences) for 6 h and then stained with TNF-α (FITC, Biolegend), IFN-γ (BV711, BD Biosciences) and Granzyme B (Alexa647, Biolegend). Cells were acquired on a BD FACSymphony^{™} A5 and LSR II (Becton Dickinson), and data were analyzed with FlowJo software Version 10.0 software.

### CFSE Proliferation assay

Naive Pmel T cells were first labelled with 5 µM of Carboxyl fluorescein succinimidyl ester (CFSE, CellTrace^{™}, ThermoFisher Scientific) for 30 min at room temperature and then stimulated or not with gp100 for 3-4 days. Cell proliferation was determined by flow cytometry through CFSE dilution with cell divisions.

### Enzyme-linked immunospot (ELISPOT) assay

The number of IFN-γ producing cells was evaluated in an ELISPOT assay. Briefly, mouse TILs were plated at 1 x 10⁵ cells and co-cultured with 1 x 10³ irradiated B16F10 tumor cells (50 Gray) in the presence of the indicated concentrations of 2-DG (Sigma), in a 96-well nitrocellulose flat-bottom (ELISPOT plate, Millipore) pre-coated with an anti-mouse IFN-γ antibody (AN-18, ThermoFisher Scientific) and incubated for 48 hours at 37°C. For Pmel T cells, splenocytes were first stimulated with gp100 for 48 hours in presence of the indicated concentrations of 2-DG (Sigma) and then transferred to the ELISPOT plate pre-coated with an anti-mouse IFN-γ antibody (AN-18, ThermoFisher Scientific) for additional 48hours. For human CD3⁺ T cells and hTILs, cells were first stimulated with plate-coated anti-CD3 for 48hours in the presence of vehicle or AGK2 or TM at the indicated concentration and then transferred to the ELISPOT plate pre-coated with an anti-human IFNγ antibody (1-D1K, Mabtech, Inc.). PMA (10 ng/ml, Sigma) was used as positive assay control. After washing steps and biotinylated anti-IFN-γ antibody (R4-6A2, ThermoFisher Scientific) + Streptavidin-HRP (BD Bioscience) labeling, spots were detected using AEC Substrate Kit (BD Biosciences) and counted using an AID ELISPOT Reader System (Autoimmun Diagnostika GmbH).

### LDH release assay

Evaluation of functional cytotoxic activity was performed using Pierce^{™} LDH Cytotoxicity Assay Kit (ThermoFisher Scientific) according to the manufacturer's protocol. B16F10 cells were used as targets. Mouse CD3⁺ TILs isolated from subcutaneous B16F10 tumor nodules and CD8⁺ enriched T cells from gp100-stimulated Pmel splenocytes were used as effector cells. Briefly, target B16F10 tumor cells were co-cultured with effector cells in 96-well round-bottom microplates at the indicated effector:target ratios. After 6 hours, the percentage of specific LDH release was calculated according to the following formula: % cell lysis = (experimental value - effector cells spontaneous release - target cells spontaneous release) x 100/(target cells maximum release - target cells spontaneous release), where "experimental" corresponds to the experimental signal value, "effector spontaneous" to spontaneous release of LDH from effector cells alone, "target spontaneous" to spontaneous release of LDH from target cells alone and "maximum release" to the maximum release of LDH from target cells in medium containing 1% Triton X-100.

### Extracellular Flux Analysis

Extracellular acidification rates (ECAR) were measured using the Seahorse XF analyzer (Agilent). Glycolysis Stress Test (GST) and glycolytic rate assay (GRA) were performed according to kit manufacturer's instructions.

The day of the assay, 2 x 10⁵ activated T cells were plated in Cell-Tak (Corning)-coated XF96 plates in XF media (XF RPMI Medium pH 7.4 containing 2 mM L-glutamine only for GST or supplemented with 10 mM glucose, 2 mM L-glutamine and 1 mM sodium pyruvate for GRA test, all from Agilent technologies) via centrifugation to ensure cell attachment. Cells were equilibrated for 1 hour in a non-CO2 incubator before starting the assay.

For GST, ECAR was measured under basal conditions and in response to 10 mM glucose, 1 µM oligomycin and 50 mM 2-DG successively to calculate basal glycolytic rate, glycolytic capacity (in response to oligomycin), and glycolytic reserve (glycolytic capacity - basal rate). For GRA, ECAR was measured under basal conditions and in response to 0.5µM rotenone + 0.5µM antimycin A and 50 mM 2-DG successively to calculate basal glycolysis and compensatory glycolysis (in response to rotenone + antimycin A). Results were normalized to total protein quantified using Pierce^{™} BCA Protein Assay Kit (ThermoFisher Scientific).

### Enzyme activity assay

WT and Sirt2KO CD3⁺ enriched T cells were activated on anti-CD3 coated-plate for 3 days. Enzyme activities of aldolase, enolase, GAPDH, hexokinase and phosphofructokinase were measured from activated T cells lysates using an enzymatic colorimetric assay kit (All from Biovision) according to the manufacturer's instructions.

### Lactate measurement

Extracellular Lactate concentration was determined using the YSI 2900 Biochemistry Analyzer (YSI Incorporated). Briefly, 2 × 10⁶ CD3⁺ enriched T cells were cultured in three separate 2 ml fresh media on anti-CD3 bound wells. Three days post-activation, cell culture supernatants were harvested for lactate quantitation. All assays were performed in triplicate with results normalized to the I-lactate Calibrator Standard (YSI Incorporated).

### ADP/ATP ratio assay

The intracellular ATP/ADP level was measured using ADP/ATP Ratio Assay Kit (Sigma), based on bioluminescence detection reaction according to the manufacturer's instructions. CD3⁺ enriched T cells were plated in anti-CD3 bound 96-well microplate. Three days post activation, cells were lysed in nucleotide-releasing buffer. ATP and ADP levels were determined by measuring bioluminescent intensities in the absence and presence of ADP converting enzyme successively using a multi-mode microplate reader (Synergy HTX, BioTek).

### Immunoprecipitation

For immunoprecipitation assays (IP), activated CD3⁺ T cells were lysed in IP lysis buffer [20 mM Hepes, pH 7.9, 180 mM KCI, 0.2 mM EDTA, 1.5 mM MgCl₂, 20% (vol/vol) glycerol, 0.1% Nonidet P-40, containing a mixture of protease inhibitors]. Cell lysates were incubated at 4 °C overnight with specific antibodies against Sirt2 (Sigma, #9441; 1:50), PKM1/2 (Cell Signaling Technology, #3106; 1:50), Hexokinase (Novus biologicals, #NBP2-67503; 1:50), PGK1 (Novus biologicals, #NBP2-67534; 1:50), LDH (Abcam, #ab52488; 1:30), PFKP (Abcam, #ab204131; 1:80), or GAPDH (Abcam, #ab181602; 1:60). Rabbit mAb IgG (Cell Signaling Technology, #3900) was used as an isotype control. After addition of anti-rabbit lg agarose-beads (TrueBlot^{®}, Rockland), samples were incubated at 4°C for 2 hours. Beads were washed five times with IP lysis buffer and proteins were released from the beads by boiling in 3X SDS sample loading buffer and loaded into 10 % SDS-PAGE gel for Western blot analysis.

### Western blot analysis

Whole cell lysates were prepared using lysis buffer (Pierce RIPA buffer, ThermoFisher Scientific) supplemented with cOmplete^{™} Protease Inhibitor Cocktail and PhosSTOP Phosphatase Inhibitor (Roche Applied Science).

Cell lysates (20ug) or IP samples were loaded onto 10% SDS-PAGE and separated by electrophoresis followed by semi-dry transfer into polyvinylidene fluoride membranes (Immun-Blot PVDF membrane, Bio-Rad) using Trans-Blot Turbo transfer system (Bio-Rad). After transfer, the membranes were blocked at room temperature with Tris-buffered saline (TBS) containing 0.05% Tween-20 (TBST) and 5% (w/v) milk for 1 h and then incubated overnight at 4 °C with a specific primary antibody (indicated below). The membranes were washed three times with TBST and then incubated for 1 h with horseradish peroxidase (HRP)-conjugated goat anti-mouse IgG H&L (Abcam, #ab97051, 1:3,000) for regular western blotting analysis, or HRP-conjugated mouse anti-rabbit IgG light-chain specific (Cell signaling technology, #93702, 1:1,000) for IP samples. After washing 3 times with TBST, bound antibodies were detected by chemiluminescence using the Pierce^{™} ECL Western Blotting Substrate and the Super Signal West Femto Maximum Sensitivity Substrate kits (ThermoFisher Scientific). Image acquisition was performed with the Amersham imager 600 system (GE Healthcare Bio-Sciences)

Immunoblotting was performed using primary antibodies against: Acetyl-Lys (#9441, 1:1,000), Enolase (#3810, 1:1,000), Acetylated-tubulin (#5335, 1:1,000), α-tubulin (#2144, 1:1.000) Aldolase A (#8060, 1:1,000), PKM1/2 (# 3106, 1:1,000) from Cell signaling technology, β-Actin (#ab8227, 1:5,000), Sirt2 (#ab67299, 1:200), GAPDH (#ab181602, 1:10,000), LDH (#ab52488, 1:5,000), PFKP (#ab204131, 1:2,000), Hexokinase (#ab150423, 1:1,000) from Abcam and PGK1 (#NBP2-67534, 1:1,000) from Novus biologicals.

### Liquid chromatography mass spectrometry (LC-MS/MS)

LC-MS/MS analysis was performed at the Proteomics and Metabolomics Core Facility of Moffitt Cancer Center.

For identification of Sirt2 interacting targets, CD3⁺ purified T cells from WT mice spleens were cultured on anti-CD3 coated-plate. One day post-activation, cells were collected, washed with PBS and lysed in the IP lysis buffer. Immunoprecipitation assay was performed as described previously using Anti-Sirt2 (Sigma) versus isotype control (Cell signaling). The immune-precipitated proteins were loaded onto 10% SDS-PAGE and separated by electrophoresis. The gel bands were excised, the proteins were reduced with Tris (2-carboxy-ethyl) phosphine hydrochloride (TCEP), followed by alkylation with iodoacetamide. Trypsin in-gel digestion was carried-on at 37°C overnight. Ziptip C18 was used to desalt the tryptic peptides.

For identification of acetylated proteins, CD3⁺ purified T cells from WT and Sirt2KO mice spleens were cultured on anti-CD3 coated-plates. Three days post-activation, cells were collected, washed with PBS and lysed in denaturing lysis buffer containing 8 M urea, 20 mM Hepes (pH 8), 1 mM sodium orthovanadate, 2.5 mM sodium pyrophosphate and 1 mM β-glycerophosphate. A Bradford assay was carried out to determine the protein concentration. Equal amount of heavy and light proteins were mixed together. The mixed proteins were reduced with 4.5 mM DTT and alkylated with 10 mM iodoacetamide. Trypsin digestion was carried out at room temperature overnight, and tryptic peptides were then acidified with 1% trifluoroacetic acid (TFA) and desalted with C18 Sep-Pak cartridges according to the manufacturer's procedure.

Following lyophilization, the dried peptide pellet was re-dissolved in IAP buffer containing 50 mM MOPS pH 7.2, 10 mM sodium phosphate and 50 mM sodium chloride. Phosphotyrosine-containing peptides were immunoprecipitated with immobilized bead-conjugated anti-Acetyl-Lysine (Cell Signaling Technology, #13416). After 2-hour incubation, the antibody beads were washed 3 times with IAP buffer, followed by 2 washes with H₂O. The phosphotyrosine peptides were eluted twice with 0.15% TFA, and the volume was reduced to 20 µl via vacuum centrifugation. The eluted peptides were fractionated using Pierce High pH Reversed-Phase Peptide Fractionation Kit (ThermoFisher Scientific).

A nanoflow ultra high performance liquid chromatograph (RSLC, Dionex) coupled to an electrospray bench top orbitrap mass spectrometer (Q-Exactive plus, ThermoFisher Scientific) was used for tandem mass spectrometry peptide sequencing experiments. The sample was first loaded onto a pre-column (2 cm x 100 µm ID packed with C18 reversed-phase resin, 5µm, 100Å) and washed for 8 minutes with aqueous 2% acetonitrile and 0.04% trifluoroacetic acid. The trapped peptides were eluted onto the analytical column, (C18, 75 µm ID x 25 cm, 2 µm, 100Å, Dionex). The 90-minute gradient was programmed as: 95% solvent A (2% acetonitrile + 0.1% formic acid) for 8 minutes, solvent B (90% acetonitrile + 0.1% formic acid) from 5% to 38.5% in 60 minutes, then solvent B from 50% to 90% B in 7 minutes and held at 90% for 5 minutes, followed by solvent B from 90% to 5% in 1 minute and re-equilibrate for 10 minutes. The flow rate on analytical column was 300 nl/min. Sixteen tandem mass spectra were collected in a data-dependent manner following each survey scan. Both MS and MS/MS scans were performed in Orbitrap to obtain accurate mass measurement using 15 second exclusion for previously sampled peptide peaks.

For data analysis, both MASCOT (Perkins, D.N., et al. Electrophoresis, 1999. 20(18):3551-67) and SEQUEST (Eng, J.K., et al. J Am Soc Mass Spectrom, 1994. 5(11):976-89) search algorithms were used to identify proteins from IP samples. The results were summarized in Scaffold. MaxQuant (Cox, J., et al. Nat Biotechnol, 2008. 26(12):1367-72) (version 1.2.2.5) was used to identify and quantify relative intensities of acetyl-lysine containing peptides.

### Metabolomics analysis

Metabolomics analysis was performed at the Proteomics and Metabolomics Core Facility of Moffitt Cancer Center.

CD8⁺ purified T cells from WT (n = 5) and Sirt2KO (n = 4) mice spleens were stimulated with plate-coated anti-CD3 for 72h, followed by extensive wash with PBS.

Metabolites were extracted from cells using 80% methanol (pre-cooled at least 1 hour in advance in -80 °C) containing the corresponding internal standards: Glucose (2,3,4,5,6-13C5), D-Glucose-6-phosphate (U-13C6), D-Fructose-1,6-bisphosphate (U-13C6), L-Serine (13C3), Glycine (1,2-13C2), L-Cysteine (3,3-D2), Phosphoenol Pyruvate (2,3-13C2), Lactate (3,3,3-D3), Pyruvate (D3), Acetyl-1,2-13C2 CoA, Citric Acid (2,2,4,4-D4), Alpha-Ketoglutaric Acid (1,2,3,4-13C4), Succinic Acid (D4), Fumaric Acid (D4), DL-Malic Acid (2,3,3-D3), D-Fructose-6-phosphate (U-13C6), which were all purchased from Cambridge Isotope Labs. After 30 min incubation in -80 °C, the samples were centrifuged (10 min, 18,800 g, 4 °C) and the supernatants were collected, dried and re-suspended in 80% methanol. The protein pellets were used for protein quantification.

UHPLC-MS was performed using a Vanquish LC (ThermoFisher Scientific) interfaced with a Q Exactive HF mass spectrometer (Thermo Fisher Scientific). Chromatographic separation was performed on a SeQuant ZIC-pHILIC LC column (150 × 4.6 mm, 5 µm particle size, Millipore). In order to maintain stable column pressure and further filtering solvents, SeQuant ZIC-pHILIC guard column (20 × 4.6 mm, 5 µm particle size, Millipore Sigma) was connected to the LC column. The mobile phase A was 10mM ammonium carbonate (Sigma) and 0.05% ammonium hydroxide (Sigma) in LC-MS grade Water (VWR), and the mobile phase B was 100% acetonitrile (VWR). The total running time was 20min. The column temperature was set to 30 °C and the injection volume was 2 µl. MS analyses were carried out in the positive and negative ion mode separately and the mass scan range was 60 to 900 m/z. In addition to MS1, PRM were acquired for the important intermediates involved in Glycolysis and TCA cycle. mzMine 2.33 coupled with internal retention time library was used to identify and quantify the metabolites. Results were normalized to total protein.

### Enrichment analysis

The genes list to interact with Sirt2 was mapped to GeneGO database by MetaCore^{™} for pathway enrichment analysis (Bessarabova, M., et al. BMC Bioinformatics, 2012. 13 Suppl 16:S13). The statistical significance of P value represents the probability to randomly obtain the intersection of certain size between two datasets following hyper geometric distribution. Pathways with a FDR < 0.01 were considered to be significantly enriched.

The web-based tool MSEA (metabolite set enrichment analysis) which is incorporated into MetaboAnalyst 4.0 platform (Xia, J., et al. Nucleic Acids Res, 2010. 38(Web Server issue):W71-7), was used to perform metabolite enrichment. Data for increased metabolites in Sirt2KO T cells were submitted into MSEA. Overrepresentation analysis was the selected enrichment analysis method. The overlap with Pathway-associated metabolites set library was implemented using the hypergeometric test. One-tailed *P* values were provided after adjusting for multiple testing. A FDR < 0.15 was used as a cutoff.

### Statistical analysis

Statistical parameters can be found in the figure legends. *P* values were calculated by two-tailed unpaired Student's t-test, Kruskal-Wallis test, one-way ANOVA or two-way ANOVA as indicated using GraphPad Prism 7.0, unless otherwise noted. In all cases, statistical significance was considered when *P*<0.05. Graphs show mean values and error bars represent SEM. *P* values were represented as follows: * *P*< 0.05; ** *P*<0.01; *** *P*<0.001, **** *P*<0.0001.

### Results

### Sirt2 knockout T cells are hyper-reactive

Sirt2KO T cell functions were characterized *ex vivo.* Upon antigenic challenge with gp100 peptide, Pmel/Sirt2KO T cells demonstrated increased proliferation by CFSE dilution assay (Fig. 2A) and IFN-γ production by ELISPOT (Fig. 2C). Further, intracellular staining showed an increase of IFN-γ, and granzyme B in Pmel/Sirt2KO compared to Pmel/WT after stimulation (Fig. 2B, 2D-2E). This hyper-reactive phenotype of Pmel/Sirt2KO T cells led to enhanced cytotoxicity against B16F10 cells *ex vivo* (Fig. 2F). More importantly, when CD3⁺ TILs, isolated from subcutaneous B16F10 tumor nodules from WT and Sirt2KO mice, were re-challenged *ex vivo* with B16F10 cells, Sirt2KO TILs demonstrated superior effector functions compared to WT TILs as measured by IFN-γ release (Fig. 2G) and cytotoxicity (Fig. 2H).

Next, the expression pattern of Sirt2 upon activation and maturation was addressed. Interestingly, Sirt2 expression was upregulated upon *in vitro* stimulation of splenocytes, LN, enriched CD3⁺ and enriched CD4⁺ T cells (Fig. 15A-15D). Sirt2 was also highly expressed in CD8⁺ TILs *versus* splenic CD8⁺ T cells (Fig. 15E). Interestingly, we found an increase of Sirt2 expression in TILs subsets in the following order of intensity T_{CM} (Central memory)>T_{EM}>T_{EFF}>T naïve (Fig. 15F).

Taken together, these results demonstrate that Sirt2 deletion has protective effects against cancer challenge due to enhanced T cell proliferation and effector functions.

### Sirt2 targets the glycolytic pathway

To understand the molecular mechanism underlying the hyper-reactivity of Sirt2KO T cells, there was an effort to identify potential Sirt2 interaction partners regulating T cell functions. To this end, 2 different co-immunoprecipitation (co-IP) experiments were conducted followed by Liquid chromatography mass spectrometry (LC-MS/MS) analysis.

First, to identify direct Sirt2 binding partners, Co-IP was performed using Sirt2 antibodies *versus* isotype control Ab followed by LC-MS/MS analysis on activated WT CD3⁺ T cells. More than 800 interacting proteins were identified. To exclude nonspecific targets, proteins identified with Sirt2 antibody with higher intensity than isotype control Ab wweree selected. Interestingly 8 out of 10 glycolytic enzymes were identified and indicated in Fig. 3A: Hexokinase 1 (HK1), Phosphofructokinase (PFK), aldolase A (ALDOA), glyceraldehyde-3-phosphate dehydrogenase (GAPDH), phosphoglycerate kinase 1 (PGK1), enolase 1 (ENO1), Pyruvate kinase (PKM) and Lactate dehydrogenase (LDH). Collision-induced dissociation (CID) spectra of the identified peptides derived from these glycolytic proteins are shown in Fig. 8. GAPDH and Enolase were excluded from the analysis because of nonspecific isotype control Ab binding.

Second, to identify hyper-acetylated proteins in the absence of Sirt2, IP was performed using acetyl-Lysine antibodies followed by LC-MS/MS analysis on naïve and activated WT *versus* Sirt2KO CD3⁺T cells. More than 250 proteins were found hyper-acetylated in naïve and/or activated Sirt2KO T cells compared to WT. Importantly, this analysis revealed 6 out of 10 glycolytic enzymes hyper-acetylated in Sirt2KO T cells including aldolase, GAPDH, PGK1, enolase, PKM and LDH. CID spectra of the identified peptides derived from these glycolytic proteins as well as the extracted ion chromatogram (EIC) plot showing the increased intensity in Sirt2KO compared to WT of the corresponding acetylated peptides are represented in Fig. 9.

Finally, to identify the potential pathways interacting with Sirt2, pathway enrichment analysis was performed using GeneGO MetaCore^{™} software on the intersection between the two IP-MS/MS lists. Forty three proteins were selected as binding Sirt2 and hyper-acetylated with Sirt2 deficiency. The top 10 enriched pathways based on GeneGO analysis reveal glycolysis in the fourth position (Fig. 3E).

Sirt2 binding to HK1, PFKP, PKM, enolase, PGK1, aldolase, GAPDH and LDH was therefore validated by co-IP/immunoblot (Fig. 3B). Reverse co-IP/immunoblot for HK1, PFKP, PKM, PGK1, GAPDH and LDH confirmed the interaction (Fig. 3C). The hyper acetylation of PFK and GAPDH in Sirt2KO *versus* WT T cells was also confirmed by IP and reverse IP/immunoblot (Fig. 3D), while their total proteins were unchanged (see Input; Fig. 3D).

To address if acetylation level of glycolytic enzymes affects their activity, enzymatic activity assay of HK, PFK, Aldolase, GAPDH and enolase was performed on WT *versus* Sirt2KO T cells. As expected, all enzymes activities tested were increased with Sirt2 deficiency (Fig. 3F), while the total proteins of the corresponding enzymes were unchanged between WT and Sirt2KO T cells (Fig. 15A).

Because Sirt2 interacts with glycolytic enzymes to modify their acetylation status and enzymatic activity, it was investigated whether Sirt2KO T cells exhibit altered glycolytic flux during activation by measuring extracellular acidification rate (ECAR) using 2 different assays on the Seahorse Analyzer: glycolysis stress test and glycolytic rate assay.

Indeed, Sirt2 deficiency increased basal, maximal and compensatory glycolysis in activated Sirt2KO CD3⁺ T cells (Fig. 4A, 11C) as well as activated Sirt2KO CD8⁺ T cells (Fig. 11A, 11E) and Pmel/Sirt2KO CD8⁺ T cells (Fig. 11B, 11F) when compared to Pmel/WT. Overall glycolytic reserves were not affected.

Next explored was whether murine TILs challenged to the harsh tumor microenvironment display the same metabolic profile as *in vitro* stimulated T cells. Consistent with the previous results, CD3⁺ TILs from subcutaneous nodules of Sirt2KO mice displayed an increase of glycolytic flux compared to WT (Fig. 4B, 11D). Consistent with these findings, extracellular lactate, a key metabolite of glycolysis and the one responsible of the extracellular acidification, was found elevated in Sirt2KO (Fig. 4C). Furthermore, in agreement with high energetic status Sirt2KO T cells displayed higher ATP/ADP ratio (Fig. 4D).

### Sirt2KO hyper-activity is glycolysis-dependent

It remained to be addressed, whether enhanced Sirt2KO T cell effector functions are specific to increased glycolysis. Next investigated therefore was whether direct inhibition of glycolysis would reverse Sirt2KO T cell hyper-reactivity. The glucose analog 2-deoxyglucose (2-DG) profoundly suppressed INF-γ release (Fig. 4E) and cytotoxicity (Fig. 4F) of Pmel/Sirt2KO T cells to Pmel/WT T cells levels upon antigenic challenge with gp100. Similarly, treatment with 2-DG suppressed INF-γ release by murine CD3⁺ TILs isolated from subcutaneous B16F10 tumor nodules from Sirt2KO *versus* WT mice (Fig. 4G). These results provide a direct link between Sirt2's regulation of glycolysis and T cell effector functions.

### Sirt2 orchestrates T cell metabolism

Also examined was intracellular metabolites levels on activated WT *versus* Sirt2KO CD8⁺ T cells to investigate whether Sirt2 regulates other metabolic pathways. Unbiased metabolomic profiling by Ultra-high performance LC-MS demonstrated a significant alteration in key metabolites relevant to T cell functions (Fig. 5). Most prominently, there was a drop of glucose level in Sirt2KO T cells which is consistent with the increase of its utilization by glycolysis as Sirt2KO T cells did not demonstrate any change in glucose uptake or GLUT1 expression in comparison to WT. In addition, Sirt2KO T cells showed a trend toward higher levels of early glycolytic intermediates up to 3-phosphoglycerate which appeared to fuel the generation of serine. Sirt2KO T cells also demonstrated higher levels of extracellular lactate, confirming preferential pyruvate conversion to lactate. Despite pyruvate conversion to lactate, Sirt2KO T cells had higher levels of late TCA-cycle intermediates from α-ketoglutarate which indicates preferential glutamine oxidation by Sirt2KO T cells to fuel TCA cycle (Fig. 5B).

Metabolites set enrichment analysis (MSEA) using *Metaboanalyst* tool indicated upregulation of multiple metabolic pathways in Sirt2KO T cells, most notably the Warburg effect, lipid, glutamate, and amino acid metabolisms. Acyl-carnitines, glutamate and amino acids, are intermediates reflecting available mitochondrial fuels (Fig. 5E). No clear patterns of decreased metabolites were observed in Sirt2KO T cells except a decrease of cysteine level which indicates its utilization for nucleic acids metabolism in response to increased proliferation. (Fig. 5A) Taken together, these data indicate that Sirt2 is a master metabolic regulator of glycolysis and other key metabolic pathways in activated T cells.

### Sirt2 an actionable target in human T cells

Finally, to extend the observations beyond mouse models, the effects of Sirt2 inhibition on human T cells was analyzed using 2 different Sirt2 inhibitors: AGK2 and thiomyristoyl I. Their capacity to block Sirt2 deacetylase activity was confirmed by monitoring acetylated α-tubulin levels, a well-established Sirt2 substrate (North, B.J., et al. Mol Cell, 2003. 11(2):437-44) (Figs. 15B-15C). Consistent with these findings using murine knockout model, Sirt2 inhibitors, AGK2 and TM, increased aerobic glycolysis and INF-γ production by human CD3⁺ T cells from normal donor blood (Fig. 12).

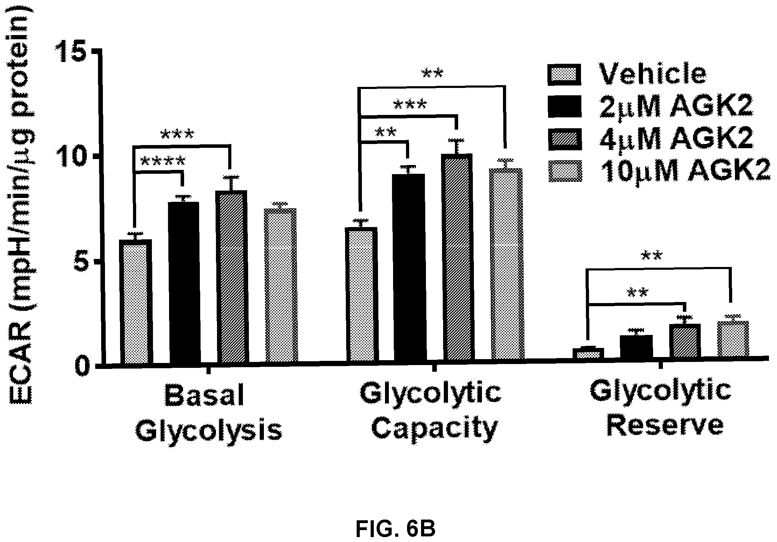

For further translational applications of Sirt2 blockade, the effect of AGK2 on human TILs was investigated from patients with non-small cell lung cancer (NSCLC). The data show consistent results within 5 patient TILs samples tested with increased aerobic glycolysis and INF-γ production (Figs. 6, 13).

### Discussion

Novel immunotherapies targeting immune checkpoints have redefined the treatment of cancer, however several cancers remain resistant and new strategies are urgently needed. An alluring alternative strategy to reinvigorate antitumor immune responses comes from the emerging field mmunemuno-metabolism (Chang, C.H., et al. Cell, 2015. 162(6):1229-4'; O'Neill, L.A., et al. Nat Rev Immunol, 2016. 16(9):553-65; Shevchenko, I., et al. Front Immunol, 2018. 9:1816). Sirt2 is identified herein as a crucial immune checkpoint coordinating metabolic and functional fitness of T cells.

Importantly, Sirt2KO T cells display hyper-reactive phenotype with increased proliferation and effector functions and thus reject efficiently tumor challenge *in vivo.* Interestingly, there was an increase in T_{EM} in Sirt2KO TILs as well as in periphery, which appear to play an essential role in tumor-specific immune responses (Mami-Chouaib, F., et al. J Immunother Cancer, 2018. 6(1):87). Indeed, a high frequency of T_{EM} in tumors correlates with favorable disease progression in patients with cancer (Pages, F., et al. N Engl J Med, 2005. 353(25):2654-66). Consistent with this *in vivo* phenotype, Sirt2KO TILs were more cytotoxic against tumor cells *ex vivo.* Moreover, PD-1 expression is increased in Sirt2KO TILs and its blockade further augmented the protective effect of Sirt2 deficiency against tumor. In fact, PD-1, a major inhibitory checkpoint, is paradoxically a marker of activated tumor-reactive CD8⁺ T cells in melanoma, and tumor regression with PD-1 blockade requires pre-existing PD-1⁺/CD8⁺ T cells (Simon, S., et al. Oncoimmunology, 2017. 7(1):e1364828; Inozume, T., et al. J Immunother, 2010. 33(9):956-64; Tumeh, P.C., et al. ature, 2014. 515(7528):568-71). These observations on Sirt2KO T cells hyper-reactivity with enhanced tumor-specific immune response, although not previously reported, are consistent with prior reports on the anti-inflammatory role of Sirt2 (Rothgiesser, K.M., et al. J Cell Sci, 2010. 123(Pt 24):4251-8; Lo Sasso, G., et al. PLoS One, 2014. 9(7):e103573; Newton, K., et al. Nature, 2014. 506(7489):E4-6; Pais, T.F., et al. EMBO J, 2013. 32(19):2603-16; Lin, J., et al. Biochem Biophys Res Commun, 2013. 441(4):897-903).

The disclosed investigation into the underlying mechanism of Sirt2KO T cell hyper-reactivity revealed a surprising role for Sirt2 as a master regulator of T cell metabolism. The unbiased screening for the molecular pathways impacted by Sirt2 using IP-MS and acetylomics analyses suggested the glycolytic pathway as the major target of Sirt2. Although surprising, the profound increase in glycolytic enzymatic activities and glycolytic flux with Sirt2 deletion was also observed in a recent report on the role of Sirt2 in metabolic reprogramming of induced human pluripotent stem cells (ihPSC) (Cha, Y., et al. Nat Cell Biol, 2017. 19(5):445-456). Indeed, 5 glycolytic enzymes identified as Sirt2 substrates in ihPSC model, and 6 glycolytic enzymes identified as Sirt2 targets in HeLa cells, are included in the 8 glycolytic enzymes we identified as Sirt2 targets in T cells (Cha, Y., et al. Nat Cell Biol, 2017. 19(5):445-456; Park, S.H., et al. Cancer Res, 2016. 76(13):3802-12).

A switch to glycolytic phenotype is required in activated T cells for their proliferation and effector functions (Pearce, E.L., et al. Science, 2013. 342(6155):1242454). It is well established that blocking glycolysis using 2-DG, a structural analog of glucose, inhibits T cells proliferation and effector function (Cham, C.M., et al. Eur J Immunol, 2008. 38(9):2438-50). 2-DG treatment reversed enhanced effector functions of Sirt2KO T cells confirming that Sirt2KO T cells hyper-reactivity was linked to increased glycolysis.

The subsequent return to reliance on OXPHOS and disengagement of glycolysis are essential for T_{M} differentiation especially T_{CM} (Pearce, E.L., et al. Science, 2013. 342(6155):1242454; Zhang, L., et al. Trends Mol Med, 2018. 24(1):30-48). Interestingly, Sirt2 expression gradually increases from T cells activation to late maturation stages while glycolysis is disengaged. In fact, Sirt2 expression is higher in T_{M} compared to T_{EFF} and higher in T_{CM} compared to T_{EM}. Surprisingly, Sirt2KO mice were able to generate more T_{EM} than T_{CM} among TILs. The fact that Sirt2KO TILs display higher glycolytic flux may explain the persistence of T_{EM} instead of T_{CM}. Consistent with these result, a recent study on constitutive activation of glycolysis in VHL deficient mice also favored the formation of T_{EM} instead of T_{CM} with enhanced anti-viral immune response (Phan, A.T., et al. Immunity, 2016. 45(5):1024-1037).

The metabolomics data further corroborated the findings on aerobic glycolysis. Decreased glucose levels in Sirt2KO T cells together with increased end-products of glycolysis including lactate and serine confirm increased glycolytic flux observed with Seahorse experiments. In addition, metabolomics profiling demonstrated up-regulation of other key metabolites supporting metabolic fitness of Sirt2KO T cells. Notably, glutamine and serine metabolism were markedly increased in Sirt2KO T cells. Glutamine is critical for T cells functions and mainly supports their metabolism under glucose limitation conditions (Carr, E.L., et al. J Immunol, 2010. 185(2):1037-44; Wang, R., et al. Immunity, 2011. 35(6):871-82; Johnson, M.O., et al. Cell, 2018. 175(7):1780-1795 e19). Upon progression, cancer cells become addicted to glutamine which limits T cell fuels and impacts theirs functions within the tumor microenvironment (Cluntun, A.A., et al. Trends Cancer, 2017. 3(3):169-180). Serine also has an important role in T_{EFF} cells supplying glycine for de novo nucleotide synthesis in proliferating T cells. Serine is neo-synthesized from the glycolytic intermediate 3-phosphoglycerate (Ma, E.H., et al. Cell Metab, 2017. 25(2):345-357), and it was posit that increased serine in Sirt2KO T cells is due to increased glycolysis. It is evident that many metabolic pathways are upregulated with Sirt2 deficiency, and together are contributing to the metabolic fitness of T cells in the metabolically challenging microenvironment.

Recent studies revealed that many well-known metabolic playmakers, such as PEP and GAPDH, also serve as critical checkpoints in immune homeostasis and anti-tumor response (Shevchenko, I., et al. Front Immunol, 2018. 9:1816; Chang, C.H., et al. Cell, 2013. 153(6):1239-51; Ho, P.C., et al. Cell, 2015. 162(6):1217-28). The transcription factors c-Myc and HIF-1α are also crucial for T cell metabolic reprogramming to initiate glycolysis upon T cells activation (Wang, R., et al. Immunity, 2011. 35(6):871-82; Doedens, A.L., et al. Nat Immunol, 2013. 14(11):1173-82). Therefore, manipulating those metabolic checkpoints to enhance T cells metabolism could be an attractive strategy. However, such metabolic interventions remain rare to impossible due to difficulties in generating selective drugs, or unintended toxicities arising from targeting ubiquitous pathways with critical cellular functions.

In this regard, Sirt2 blockade offers a unique opportunity to manipulate T cell metabolism to augment immunotherapy. The phenotypic and mechanistic studies in mice were further confirmed in human T cells using 2 selective Sirt2 inhibitors: AGK2 and TM (Jing, H., et al. Cancer Cell, 2016. 29(5):767-768), emphasizing that Sirt2 is indeed an actionable therapeutic target for cancer immunotherapy.

The therapeutic effects of Sirt2 blockade rely on enhancing the capacity of TILs to optimally engage aerobic glycolysis to maintain effector functions while competing with cancer cells for limited resources within the tumor microenvironment. Indeed, one of the key mechanisms by which tumor alter the functional fate of T cells is alteration of nutrient availability in the microenvironment (Chang, C.H., et al. Cell, 2015. 162(6):1229-41). Sirt2 expression is then induced in response to caloric restriction and primes gluconeogenesis process while blocking glycolysis (Gomes, P., et al. Trends Pharmacol Sci, 2015. 36(11):756-768; Jiang, W., et al. Mol Cell, 2011. 43(1):33-440). For examples, the inhibition of Braf and Kras oncogenes stimulates T cell infiltration and INF-γ production in tumors. These observations result from their suppression of tumor aerobic glycolysis, which restores glucose levels in the microenvironment (Ho, P.C., et al. Cancer Res, 2014. 74(12):3205-17; Ying, H., et al. Cell, 2012. 149(3):656-70). As TILs adapt to the harsh tumor microenvironment, they progressively undergo a functional exhaustion process with upregulation of several inhibitory stimuli. Most prominently, PD-1 signaling in T cells suppresses Akt-mTOR pathway and thus aerobic glycolysis (Bengsch, B., et al. Immunity, 2016. 45(2):358-73), while concomitant PD-L1 signaling in tumor cells promotes glycolysis via activation of the Akt-mTOR pathway (Chang, C.H., et al. Cell, 2015. 162(6):1229-41). In this regard, metabolic interventions on tumor cell metabolism, such as the use of mTOR inhibitors, may have poor long-term efficacy because of their unintended effects on the metabolic fitness of TILs (Chaoul, N., et al. Cancer Res, 2015. 75(16):3279-91).

Therefore, Sirt2 blockade to metabolically reprogram TILs may be an effective strategy to synergize with the existing immune checkpoint inhibitors. Interestingly, augmentation of PD-1 blockade by Sirt2 deficiency highlighting the potential benefit to this combination strategy. Of additional note, targeting Sirt2 may also be efficacious to augment metabolic fitness of cell-based immunotherapy and improve their therapeutic outcome. In this case, manipulation of Sirt2 may be achieved by gene editing during TILs or chimeric antigen receptor (CAR)-T cells generation *ex vivo* in addition to pharmacologic intervention (Rosenberg, S.A., et al. Science, 2015. 348(6230):62-8; Lim, W.A., et al. Cell, 2017. 168(4):724-740).

In summary Sirt2 deletion enhances tumor-specific T cell responses by promoting metabolic reprogramming towards aerobic glycolysis in mice and human. Sirt2 is thus an actionable target with distinct mechanism to further augment existing cancer immunotherapy including immune checkpoint inhibitors or cell-based therapies.

### Example 2. Sirt2 Inhibition Reprograms T Cell Metabolism to Confer Superior Anti-Tumor Immunity

### Methods

### Mice

C57BL/6J mice, Pmel mice with a gp100-reactive transgenic T cell receptor (TCR): B6.Cg-*Thy1^{a}*/Cy Tg(TcraTcrb)8Rest/J, major histocompatibility complex class II-restricted OVA-specific TCR (OT-II) transgenic mice: B6.Cg-Tg(TcraTcrb)425Cbn/J, *Sirt2*^{*-*/*-*} mice: B6.129-*Sirt2*^{tm1.1Fwa}/J and immunodeficient NOD scid gamma (NSG) mice: NOD.Cg-*Prkdc*^{scid} *II2rg*^{tm1Wjl}/SzJ were purchased from The Jackson Laboratory. Pmel TCR transgenic mice and OT-II TCR transgenic mice were crossed with *Sirt2*^{*-*/*-*} mice to generate *Sirt2*^{*-*/*-*} Pmel mice and *Sirt2*^{*-*/*-*} OT-II mice respectively. Mice were used at 7-8 weeks of age with age- and sex-matched controls.

### Mouse T cell culture

Spleens and/or lymph nodes (LN) were collected from WT, *Sirt2*^{*-*/}*⁻,* WT Pmel, and *Sirt2*^{*-*/*-*} Pmel mice and were processed into single-cell suspensions. CD3⁺, CD4⁺ and CD8⁺ T cells were negatively enriched using Mouse Pan T cell, CD4⁺ T cell and CD8⁺ T cell isolation kits^{™}, respectively according to the manufacturer's instructions. The purity of the isolated cells was confirmed by flow cytometry (>95%, not shown). Purified T cells were cultured in complete RPMI 1640 medium (Thermo Fisher Scientific) supplemented with 10% (vol/vol) fetal bovine serum (FBS, Biowest) and 1% (vol/vol) penicillin/streptomycin (P/S, Thomas Scientific Inc.). WT and *Sirt2*^{*-*/*-*}T cells were activated with plate-bound anti-CD3 antibody (5 µg/ml, 145-2C11, BioXCell) for the indicated time periods. WT Pmel and *Sirt2*^{*-*/*-*} Pmel T cells were stimulated with gp100 (1 µg/ml, AnaSpec) for the indicated time periods.

### Memory differentiation

OT-II splenocytes were activated with 10 µg/ml OVA-peptide (AnaSpec) for 3 days and subsequently cultured in the presence of 10 ng/ml IL-15 (R&D) for 4 days to generate T_{M} cells.

### Human PBMC

Human peripheral blood mononuclear cells (PBMC) were obtained from healthy donors by density gradient centrifugation using Ficoll-Paque^{™} PLUS Media (GE Healthcare). CD3⁺ lymphocytes were negatively enriched using human Pan T cell isolation kit^{™} (Miltenyi Biotec). Enriched CD3⁺ T cells were cultured in complete RPMI medium and activated with plate-bound anti-CD3 antibody (5 µg/ml, OKT-3, BioXCell) in the presence of DMSO (vehicle), or one of the Sirt2 inhibitors, AGK2 and Thiomyristoyl (Selleck Chemicals), at the indicated concentrations.

### Human TILs

Human tumor-infiltrating lymphocytes (TILs) isolated from tumor biopsies of patients with non-small cell lung cancer (NSCLC). All human samples provided were and remained de-identified. For Sirt2 staining of human TILs and the corresponding PBMC, the cohort characteristics including 12 patients, are as previously described (Creelan, B., et al. (2018). Journal of Thoracic Oncology 13).

For TIL expansion, TILs were cultured in completed RPMI 1640 medium, supplemented with 10% human AB serum (Valley Biomedical, Inc.), 1 mM Hepes (Sigma-Aldrich), 1% P/S (Thomas Scientific, 50 µg/ml gentamicin (Thermo Fisher Scientific), 50 µM β-mercaptoethanol (Thermo Fisher Scientific) and 6,000U/ml of recombinant human rhIL-2 (Peprotech). For metabolic and functional assays, TILs were cultured in complete RPMI medium, IL-2 free, on plate-bound anti-CD3 antibody (5 µg/ml, OKT-3, BioXCell) in the presence of vehicle or the indicated concentrations of the AGK2, the Sirt2 inhibitor (Selleck Chemicals).

### Tumor cells

B16F10 melanoma cells were obtained from the American Type Culture Collection. Cells were passaged minimally and maintained in complete Dulb'cco's Modified Eagle Medium DMEM/F12 (Thermo Fisher Scientific) containing 10% FBS (Biowest) and 1% P/S (Thomas Scientific Inc).

### Melanoma lung metastasis model

B16F10 tumor cells (n = 2.5 × 10⁵) in 100 µl of sterile phosphate buffered saline (PBS, Sigma-Aldrich) were injected intravenously (i.v.) into lateral tail veins of mice. For anti-PD-1 administration, mice were injected intraperitoneally (i.p.) with 200 µg of anti-PD-1 antibody (RMP1-14, BioXCell) every 3 days for a total of three injections. Eighteen days post-tumor cell injection, lungs were removed from euthanized mice. The number of visible black metastatic nodules on the lung surface was counted.

### Subcutaneous tumor model

Anesthetized mice were injected subcutaneously (s.c.) into the hind limb with 2.5 × 10⁵ B16F10 tumor cells in 200 µl of sterile PBS. Tumor volumes were monitored twice a week over 3 weeks and were calculated using the equation for ellipsoidal volume = length × width × height × π/6.

### Isolation of mouse TILs

Lymphocytes from subcutaneous tumors were isolated by dicing the tissues followed by enzymatic digestion in PBS containing collagenase type 4 (2 mg/ml, Worthington Biochemical) and DNase I (0.25 mg/ml, Sigma-Aldrich) for 45 min with occasional shaking at 37 °C. Cell suspensions were then twice filtered through 100 µm and 40 µm cell strainers (Thermo Fisher Scientific) to obtain single-cell suspension followed by a PBS wash and red blood cell lysis using RBC Lysis Buffer (Biolegend). TILs were finally isolated by density gradient centrifugation using Percoll (GE Healthcare) and were further purified using CD3ε MicroBeads kit^{™} (Miltenyi Biotec) according to the manufact'rer's instructions. T cell purity was greater than 90%. Fresh TILs were used directly for phenotypic and metabolic analyses or were cultured in complete RPMI medium for functional assays.

### In vivo T cell depletion

*In vivo* depletion of CD4⁺ or CD8⁺ T lymphocyte subsets was performed by i.p. injection of 100 µg of purified monoclonal anti-CD4 (GK1.5, BioXCell) or anti-CD8 (2.43, BioXCell) antibodies twice weekly. Control (non-depleted) mice were treated with i.p. injection of 100 µg of nonimmune IgG isotype control (LTF-2, BioXCell). Depleted and control mice received 2.5 × 10⁵ B16F10 cells (n = 5 per group) i.v.

### Adoptive transfer of Pmel T cells

Naïve CD8⁺ T cells were purified from spleens of WT Pmel and *Sirt2*^{*-*/*-*}Pmel donor mice using CD8⁺ T Cell Isolation kit^{™} (Miltenyi Biotec). NSG mice (n = 8 per group) were injected i.v. with 1 × 10⁵ B16F10 melanoma cells. Concurrently 5 × 10⁵ naïve CD8⁺ WT Pmel or *Sirt2*^{*-*/*-*} Pmel T cells were adoptively transferred into the recipient mice. Control mice received B16F10 tumor cells only. The number of visible black metastatic nodules on the lung surface was manually counted 18 days post-tumor injection. The lungs with excess metastatic nodules that could not be accurately enumerated were assigned the number 200.

### Flow cytometry

For analysis of surface markers, mouse T cells were stained in PBS containing 5% FBS (vol/vol, FACS buffer) with: CD3 (BUV395), CD4 (BUV805), PD-1 (BV605), Gr1 (BUV395), F4/80 (BV711), CD19 (PerCP-Cy5.5), NK1.1 (PE) from BD Biosciences, CD8 (Alexa Fluor 700), CD44 (Alexa Fluor 488), CD62L (PE-Cy7), CD27 (BV 510), CD11b (BV605), CD11c (Alexa488), IA/IE (Alexa647), B220 (APC-H7) from Biolegend, PBS57-loaded CD1d tetramer (Alexa647) from NIH Tetramer Facility, and glucose transporter GLUT1 (Alexa647) from Abcam incubated at 4 °C for 1 hr, then washed twice with FACS buffer, and finally fixed in PBS containing 1% paraformaldehyde. For human T cells staining the following antibodies were used instead: CD8 (BUV395), CD3 (BUV496), CD4 (BUV737), CD45RA (FITC) from BD Biosciences. Dead cells were excluded using the Zombie Violet or Zombie NIR Fixable Viability Kit (Biolegend) following the manufacturer's protocol. For glucose uptake, the fluorescent glucose analog 2-NBDG (Thermo Fisher Scientific) was added to the cells at 10 µM for 1 hr prior cell surface staining. For Sirt2 intracellular staining, cells were first labeled with surface markers, and then fixed and permeabilized with Cytofix/Cytoperm^{™} Buffer (BD Biosciences). Intracellular labeling of Sirt2 was then performed using a specific Sirt2 antibody (Sigma-Aldrich, #9441) combined with a secondary goat anti-rabbit IgG antibody (Alexa647, Thermo Fisher Scientific) according to the manufacturer's instructions. For intracellular cytokine staining, cells were first stimulated with phorbol 12-myristate 13-acetate (PMA, 10 ng/ml, Sigma-Aldrich) and ionomycin (1 µM, Sigma-Aldrich) for 1 hr followed by GolgiPlug^{™} treatment (1‰, BD Biosciences) for additional 6 hr and then stained with anti-TNF-α (FITC, Biolegend), -IFN-γ (BV711, BD Biosciences) and anti-granzyme B (Alexa647, Biolegend) antibodies. Cells were acquired on a BD FACSymphony^{™} A5 and LSR II (Becton Dickinson), and data were analyzed with FlowJo Version 10.0 software.

### CFSE proliferation assay

Naive Pmel T cells were first labelled with 5 µM of carboxyl fluorescein succinimidyl ester (CFSE, CellTrace^{™}, Thermo Fisher Scientific) for 30 min at room temperature and then stimulated with gp100 or remained non-stimulated for 3-4 days. Cell proliferation was determined by flow cytometry through CFSE dilution with cell divisions.

### Enzyme-linked immunospot (ELISPOT) assay

The number of IFN-γ producing cells was evaluated in an ELISPOT assay. Briefly, mouse TILs were plated at 1 x 10⁵ cells and co-cultured with 1 x 10³ irradiated B16F10 tumor cells (50 Gray) in the presence of the indicated concentrations of 2-DG (Sigma-Aldrich), in a 96-well nitrocellulose flat-bottomed plate (ELISPOT plate, Millipore) pre-coated with an anti-mouse IFN-γ antibody (AN-18, Mabtech, Inc.) and incubated for 48 hr at 37°C. For Pmel T cells, splenocytes were first stimulated with gp100 (1 µg/ml, Anaspec) for 48 hr in the presence of the indicated concentrations of 2-DG (Sigma-Aldrich) and then transferred to the ELISPOT plate pre-coated with an anti-mouse IFN-γ antibody (AN-18, Mabtech, Inc.) for an additional 48 hr. For human CD3⁺ T cells and TILs, cells were first stimulated with plate-coated anti-CD3 for 48 hr in the presence of vehicle, AGK2 or TM at the indicated concentration and then transferred to the ELISPOT plate pre-coated with an anti-human IFN-γ antibody (1-D1K, Mabtech, Inc.). PMA (10 ng/ml, Sigma-Aldrich) was used as positive assay control. After washing steps and successive biotinylated anti-IFN-γ antibody (R4-6A2, Mabtech, Inc.) and Streptavidin-HRP (BD Bioscience) labeling, spots were detected using AEC Substrate Kit (BD Biosciences) and counted using an AID ELISPOT Reader System (Autoimmun Diagnostika GmbH).

### Lactate dehydrogenase (LDH) release assay

Evaluation of functional cytotoxic activity was performed using Pierce^{™} LDH Cytotoxicity Assay Kit (Thermo Fisher Scientific) according to the manufacturer's protocol. Briefly, B16F10 cells were used as targets, mouse CD3⁺ TILs isolated from subcutaneous B16F10 tumor nodules or CD8⁺ enriched T cells from gp100-stimulated Pmel splenocytes were used as effector cells. Target B16F10 tumor cells were co-cultured with effector cells in 96-well round-bottom microplates at the indicated effector:target ratios. After 6 hr, the percentage of specific LDH release was calculated according to the following formula: % cell lysis = (experimental value - effector cells spontaneous release - target cells spontaneous release) x 100/(target cells maximum release - target cells spontaneous release), where "experimental" corresponds to the experimental signal value, "effector spontaneous" to spontaneous release of LDH from effector cells alone, "target spontaneous" to spontaneous release of LDH from target cells alone and "maximum release" to the maximum release of LDH from target cells in medium containing 1% Triton X-100.

### Extracellular Flux Analysis

Extracellular acidification rate (ECAR) and oxygen consumption rate (OCR) were measured using the Seahorse XF analyzer (Agilent technologies). Glycolysis Stress Test (GST), glycolytic rate assay (GRA) and mitochondrial stress test (MST) were were purchased from Agilent technologies and performed according to the kit manufacturer's instructions.

The day of the assay, 2 x 10⁵ activated T cells were plated in Cell-Tak (Corning)-coated XF96 plates in XF media (XF RPMI Medium pH 7.4 containing 2 mM L-glutamine only for GST or supplemented with 10 mM glucose, 2 mM L-glutamine and 1 mM sodium pyruvate for GRA and MST, all from Agilent technologies) via centrifugation to ensure cell attachment. Cells were equilibrated for 1 hr in a non-CO₂ incubator before starting the assay.

For GST, ECAR and OCR were measured under basal conditions and in response to 10 mM glucose, 1 µM oligomycin and 50 mM 2-DG successively to calculate basal glycolytic rate, glycolytic capacity (in response to oligomycin), and glycolytic reserve (glycolytic capacity - basal rate).

For GRA, ECAR and OCR were measured under basal conditions and in response to 0.5 µM roteone/antimycin A and 50 mM 2-DG successively to calculate basal glycolysis and compensatory glycolysis (in response to rotenone/antimycin A).

For MST, OCR and ECAR were measured under basal conditions and in response to sequential injections of 1 µM oligomycin, 1 µM FCCP (Carbonyl cyanide 4-trifluoromethoxyphenylhydrazone) and 0.5 µM rotenone/antimycin A to calculate basal respiration rates (baseline OCR - rotenone/antimycin A OCR), maximal respiration (FCCP OCR - rotenone/antimycin A OCR), and oxidative reserve (maximum respiration rate - basal respiration rate).

To measure the contribution of glycolysis, glutaminolysis and FAO as fuels for mitochondrial metabolism, OCR and ECAR were measured under basal conditions and in response to sequential injections of vehicle, UK5099 (2 µM), BPTES (3 µM) or Etomoxir (4 µM) inhibitors and then 1 µM oligomycin, 1 µM FCCP (Carbonyl cyanide 4-trifluoromethoxyphenylhydrazone) and 0.5 µM rotenone/antimycin A.

To measure oxidation of exogenous and endogenous FA, T cells were activated in completed RPMI media for 3 days and transferred to substrate-limited media overnight. Substrate limited media contained 0.5 mM glucose, 1 mM glutamine, 0.5 mM carnitine (all from Sigma) and 1% FBS (Biowest). Palmitate or BSA substrates were added immediately before the assay. OCR was measured under basal conditions and in response to sequential injections of vehicle, or Etomoxir (4 µM) and then 1 µM oligomycin, 1 µM FCCP (Carbonyl cyanide 4-trifluoromethoxyphenylhydrazone) and 0.5 µM rotenone/antimycin A.

The relative contribution of FAO to OCR was calculated as follows: maximal respiration due to exogenous FA oxidation = Maximal Palmitate + vehicle OCR rate - maximal BSA + vehicle OCR r-te - OCR due to uncoupling by free FA. Uncoupling by free FA =, Palmitate + vehicle OCR rate after oligomycin injection - BSA + vehicle OCR rate after oligomycin injection. Maximal OCR due to endogenous FA consumption = Maximal BSA + vehicle OCR rate - maximal BSA + Etomoxir OCR rate.

Results were normalized to total protein quantified using Pierce^{™} BCA Protein Assay Kit (Thermo Fisher Scientific).

### Enzyme activity assay

WT and *Sirt2*^{*-*/*-*} CD3⁺ enriched T cells were activated on anti-CD3 antibody-coated plates for 3 days. Enzyme activities of aldolase, enolase, GAPDH, hexokinase, phosphofructokinase, aconitase, oxoglutarate dehydrogenase, succinate dehydrogenase, succinyl-CoA ligase were measured from activated T cells lysates using an enzymatic colorimetric assay kit (all from Biovision) according to the manufacturer's instructions.

### Lactate, glutamine, and glutamate measurement

Extracellular lactate, glutamine and glutamate concentrations were determined using the YSI 2900 Biochemistry Analyzer (YSI Incorporated). Briefly, 2 × 10⁶ CD3⁺ enriched T cells were cultured in three separate 2 ml fresh media on anti-CD3 antibody bound wells. Three days post-activation, cell culture supernatants were harvested for metabolite quantitation. All assays were performed in triplicate with results normalized to the Calibrator Standard (YSI Incorporated) and compared to fresh media.

### ADP/ATP ratio assay

The intracellular ATP/ADP level was measured using ADP/ATP Ratio Assay Kit (Sigma-Aldrich), based on bioluminescence detection reaction according to the manufact'rer's instructions. CD3⁺ enriched T cells were plated in anti-CD3 bound 96-well microplate. Three days post-activation, cells were lysed in nucleotide-releasing buffer. ATP and ADP levels were determined by measuring bioluminescent intensities in the absence and presence of ADP converting enzyme successively using a multi-mode microplate reader (Synergy HTX, BioTek).

### Immunoprecipitation

For immunoprecipitation (IP) assays, activated CD3⁺ T cells were lysed in IP lysis buffer [20 mM Hepes, pH 7.9, 180 mM KCI, 0.2 mM EDTA, 1.5 mM MgCl₂, 20% (vol/vol) glycerol, 0.1% Nonidet P-40, containing a mixture of protease inhibitors]. Cell lysates were incubated at 4 °C overnight with specific antibodies against Sirt2 (#9441, 1:50) from Sigma-Aldrich, acetyl-lysine (#9441, 1:50), ENO1 (#3810, 1:50), ALDOA (#8060, 1:50), PKM1/2 (#3106, 1:50), OGDH (#26865, 1:50), ACO2 (#6571, 1:50), SDHA (#11998, 1:50) and SUCLG1 (#8071, 1:50) from Cell Signaling, GAPDH (#ab181602, 1:60), LDH (#ab52488, 1:30), PFKP (#ab204131, 1:80) from Abcam HK1 (#NBP2-67503, 1:50) or PGK1 (#NBP2-67534, 1:50) from Novus biologicals. Rabbit mAb IgG (Cell Signaling, #3900) was used as an isotype control mAb. After addition of anti-rabbit Ig agarose-beads (TrueBlot^{®}, Rockland), samples were incubated at 4 °C for 2 hours. Beads were washed five times with IP lysis buffer and proteins were released from the beads by boiling in 3X SDS sample loading buffer and loaded into 10 % SDS-PAGE gel for Western blot analysis.

### Western blot analysis

Whole cell lysates were prepared using lysis buffer (Pierce RIPA buffer, Thermo Fisher Scientific) supplemented with cOmplete^{™} protease inhibitor cocktail and PhosSTOP phosphatase inhibitor cocktail (Roche Applied Science).

Cell lysates (20 µg) or IP samples were loaded onto 10% SDS-PAGE and separated by electrophoresis followed by semi-dry transfer into polyvinylidene fluoride membranes (Immun-Blot^{®} PVDF membrane, Bio-Rad) using Trans-Blot Turbo transfer system (Bio-Rad). After transfer, the membranes were blocked at room temperature with Tris-buffered saline (TBS) containing 0.05% Tween-20 (TBST) and 5% (w/v) non-fat dry milk for 1 hour and then incubated overnight at 4 °C with a specific primary antibody (indicated below). The membranes were washed three times with TBST and then incubated for 1 hour with horseradish peroxidase (HRP)-conjugated goat anti-mouse IgG H&L (Abcam, #ab97051, 1:3,000) for regular western blotting analysis, or HRP-conjugated mouse anti-rabbit IgG light-chain specific (Cell Signaling, #93702, 1:1,000) for IP samples. After washing 3 times with TBST, bound antibodies were detected by chemiluminescence using the Pierce^{™} ECL Western Blotting Substrate and the Super Signal West Femto Maximum Sensitivity Substrate kits (Thermo Fisher Scientific). Image acquisition was performed with the Amersham imager 600 system (GE Healthcare Bio-Sciences).

Immunoblotting was performed using primary antibodies against: acetyl-lysine (#9441, 1:1,000), ENO1 (#3810, 1:1,000), acetylated-tubulin (#5335, 1:1,000), α-tubulin (#2144, 1:1.000) ALDOA (#8060, 1:1,000), PKM1/2 (#3106, 1:1,000), OGDH (#26865, 1:1,000), ACO2 (#6571, 1:1,000), SDHA (#11998, 1:1,000), SUCLG1 (#8071, 1:1,000), ASCT2 (#5345,1:1,000) and c-Myc (#9402, 1:1,000) from Cell Signaling, β-Actin (#ab8227, 1:5,000), Sirt2 (#ab67299, 1:200), GAPDH (#ab181602, 1:10,000), LDH (#ab52488, 1:5,000), PFKP (#ab204131, 1:2,000), HK1 (#ab150423, 1:1,000), CPT1A (#ab128568, 1:1,000), HADHA (#ab203114, 1:2,000), HADHB (#ab240601, 1:2,000) and GLS (#ab202027, 1:1,000) from Abcam and PGK1 (#NBP2-67534, 1:1,000) from Novus biologicals.

### RNA-sequencing analysis

CD4⁺ purified T cells from WT and *Sirt2*^{*-*/*-*} mouse spleens were stimulated with anti-CD3 plate-coated for 24 hr or remained unstimulated (n = 3 per group). RNA was extracted using the RNeasy Mini Kit (Qiagen; cat. #74106) and processed for RNA-sequencing using the NuGen Ovation Mouse RNA-Seq System (NuGen Inc., San Carlos, CA). Briefly, 100 ng of RNA was used to generate cDNA and a strand-specific library following the manufacturer's protocol. Quality control steps including TapeStation library assessment and quantitative RT-PCR for library quantification were performed. The libraries were sequenced on the Illumina NextSeq 500 sequencer with 2 X 75-base paired-end high output runs in order to generate 30 million read pairs per sample.

### Liquid chromatography-tandem mass spectrometry (LC-MS/MS)

For identification of Sirt2 interacting targets, CD3⁺ purified T cells from WT mouse spleens were cultured on anti-CD3 coated-plates. One day post-activation, cells were collected, washed with PBS and lysed in the IP lysis buffer. Immunoprecipitation assay was performed as described previously using Anti-Sirt2 Ab (Sigma-Aldrich) *versus* isotype control Ab (Cell Signaling). The immune-precipitated proteins were loaded onto 10% SDS-PAGE and separated by electrophoresis. The gel bands were excised, the proteins were reduced with Tris (2-carboxy-ethyl) phosphine hydrochloride (TCEP), followed by alkylation with iodoacetamide. In-gel digestion with trypsin was carried out at 37 °C overnight. Ziptip C18 micropipette columns were used to desalt the tryptic peptides.

For identification of acetylated proteins, CD3⁺ purified T cells from WT and *Sirt2*^{*-*/*-*} mouse spleens were cultured on anti-CD3 coated-plates. Three days post-activation, cells were collected, washed with PBS and lysed in denaturing lysis buffer containing 8 M urea, 20 mM HEPES (pH 8), 1 mM sodium orthovanadate, 2.5 mM sodium pyrophosphate and 1 mM β-glycerophosphate. A Bradford assay was carried out to determine the protein concentration. Equal amounts of total protein were analyzed for each sample. The mixed proteins were reduced with 4.5 mM DTT and alkylated with 10 mM iodoacetamide. Trypsin digestion was carried out at room temperature overnight, and tryptic peptides were then acidified with 1% trifluoroacetic acid (TFA) and desalted with C18 Sep-Pak cartridges according to the manufacturer's procedure.

Following lyophilization, the dried peptide pellet was re-dissolved in IAP buffer containing 50 mM MOPS pH 7.2, 10 mM sodium phosphate and 50 mM sodium chloride. Acetyl-lysine-containing peptides were immune-precipitated with immobilized bead-conjugated anti-acetyl-lysine (Cell Signaling, #13416). After 2 hr incubation, the antibody-beads were washed 3 times with IAP buffer, followed by 2 washes with H₂O. The acetyl-lysine peptides were eluted twice with 0.15% TFA, and the volume was reduced to 20 µl via vacuum centrifugation. The eluted peptides were fractionated using Pierce High pH Reversed-Phase Peptide Fractionation Kit (Thermo Fisher Scientific).

A nanoflow ultra high performance liquid chromatograph (RSLC, Dionex) coupled to an electrospray bench top orbitrap mass spectrometer (Q Exactive Plus, Thermo Fisher Scientific) was used for tandem mass spectrometry peptide sequencing experiments. The sample was first loaded onto a pre-column (100 µm ID x 2 cm in length packed with C18 reversed-phase resin, 5 µm particle size, 100Å pore size) and washed for 8 min with aqueous 2% acetonitrile and 0.04% trifluoroacetic acid. The trapped peptides were eluted onto the analytical column, (C18, 75 µm ID x 25 cm in length, 2 µm particle size, 100Å pore size, Dionex). The 90-minute gradient was programmed as: 95% solvent A (2% acetonitrile + 0.1% formic acid) for 8 min, solvent B (90% acetonitrile + 0.1% formic acid) from 5% to 38.5% in 60 min, then solvent B from 50% to 90% B in 7 min and held at 90% for 5 min, followed by solvent B from 90% to 5% in 1 minute and re-equilibration for 10 min. The flow rate on analytical column was 300 nl/min. Sixteen tandem mass spectra were collected in a data-dependent manner following each survey scan using 15 second exclusion for previously sampled peptide peaks.

For data analysis, both MASCOT(Perkins et al., 1999) and SEQUEST(Eng et al., 1994) search algorithms were used to identify proteins from IP samples. The results were summarized in Scaffold. MaxQuant (Cox, J., and Mann, M. (2008). Nat Biotechnol 26:1367-1372) (version 1.2.2.5) was used to identify and quantify relative intensities of acetyl-lysine containing peptides.

### Metabolomic analysis

CD8⁺ purified T cells from WT (n = 5) and *Sirt2*^{*-*/*-*} (n = 4) mouse spleens were stimulated with anti-CD3 plate-coated for 72 hr, followed by extensive washes with PBS. Metabolites were extracted from cells using 80% methanol (pre-cooled at least 1 hr in advance in -80 °C) containing the corresponding internal standards: Glucose (2,3,4,5,6-¹³C₅), D-Glucose-6-phosphate (U-¹³C₆), D-Fructose-1,6-bisphosphate (U-¹³C₆), L-Serine (¹³C₃), Glycine (1,2-¹³C₂), L-Cysteine (3,3-D₂), Phosphoenol Pyruvate (2,3-¹³C₂), Lactate (3,3,3-D₃), Pyruvate (D₃), Acetyl-1,2-¹³C₂ CoA, Citric Acid (2,2,4,4-D₄), Alpha-Ketoglutaric Acid (1,2,3,4-¹³C₄), Succinic Acid (D₄), Fumaric Acid (D₄), DL-Malic Acid (2,3,3-D₃), D-Fructose-6-phosphate (U-¹³C₆), which were all purchased from Cambridge Isotope Labs. After 30 min incubation in - 80 °C, the samples were centrifuged (10 min, 18,800 x g, 4 °C) and the supernatants were collected, dried and re-suspended in 80% methanol. The protein pellets were used for protein quantification to serve as a sample quality control metric.

UHPLC-MS was performed using a Vanquish LC (Thermo Fisher Scientific) interfaced with a Q Exactive HF mass spectrometer (Thermo Fisher Scientific). Chromatographic separation was performed on a SeQuant ZIC-pHILIC LC column (4.6 mm ID × 150 mm in length, 5 µm particle size, Millipore). In order to maintain stable column pressure and further filtering solvents, SeQuant ZIC-pHILIC guard column (4.6 mm × 20 mm in length, 5 µm particle size, Millipore) was connected before the LC column. The mobile phase A was aqueous 10 mM ammonium carbonate (Sigma-Aldrich) and 0.05% ammonium hydroxide (Sigma-Aldrich) using LC-MS grade Water (VWR), and the mobile phase B was 100% acetonitrile (VWR). The total running time was 20 min. The column temperature was set to 30 °C and the injection volume was 2 µl. Separate UHPLC-MS analyses were carried out in the positive and negative ion modes using the mass scan range from m/z 60 to m/z 900. In addition to MS¹, targeted tandem mass spectra using parallel reaction monitoring (PRM) were acquired for the important intermediates involved in Glycolysis and TCA cycle. mzMine 2.33 coupled with internal retention time library was used to identify and quantify the metabolites. Results were normalized to total protein.

### Acyl-Carnitines, Acyl-CoA and Acetyl-CoA analysis by LC-MS/MS

CD8⁺ purified T cells from WT (n = 5) and *Sirt2*^{*-*/*-*} (n = 6) mouse spleens were stimulated with anti-CD3 plate-coated for 72 hr, followed by extensive washes with PBS. Metabolites were extracted from cells using 80% methanol (pre-cooled at least 1 hr in advance in -80 °C) containing the corresponding internal standards: L-Carnitine (trimethyl-D₉), O-Acetyl-L-Carnitine (*N*-methyl-D₃), O-Butyryl-L-Carnitine (*N*-methyl-D₃), O-Hexanoyl-L-Carnitine (*N*-methyl-D₃), O-Octanoyl-L-Carnitine (*N*-methyl-D₃), O-Decanoyl-L-Carnitine (*N*-methyl-D₃), O-Lauroyl-L-Carnitine (*N,N,N*-trimethyl-D₃), O-Myristoyl-L-Carnitine (*N,N,N*-trimethyl-D₃), O-Palmitoyl-L-Carnitine (*N*-methyl-D₃), O-Stearoyl-L-Carnitine (*N*-methyl-D₃), Acetyl-coenzyme A (1,2-¹³C₂), which were all purchased from Cambridge Isotope Labs. After 30 min incubation in -80 °C, the samples were centrifuged (10 min, 18,800 x g, 4 °C) and the supernatants were collected, dried and re-suspended in 80% methanol. The protein pellets were used for protein quantification using Bradford assay to serve as a sample quality control metric.

UHPLC-MS was performed using a Vanquish LC (Thermo Fisher Scientific) interfaced with a Q Exactive FOCUS mass spectrometer (Thermo Fisher Scientific).

For Acyl-Carnitines: chromatographic separation was performed on an ACQUITY UPLC BEH Amide column (2.1 mm x 150 mm, 1.7 µm particle size, Waters, Milford, MA); the mobile phase A was aqueous 10 mM ammonium carbonate (Sigma-Aldrich) and 0.05% ammonium hydroxide (Sigma-Aldrich), and the mobile phase B was 100% acetonitrile; the total running time was 15 minutes; selected Ion Monitoring (SIM) was performed in positive mode.

For Acyl-CoA: an AccuCore Vanquish C18+ (2.1 mm x 100 mm, 1.5 µm particle size, Thermo Fisher Scientific, San Jose, CA) was used for the LC separation; the mobile phase A was 10:90 acetonitrile: water with 15 mM ammonium hydroxide, and the mobile phase B was 100% acetonitrile with 15 mM ammonium hydroxide; the total running time is 15 minutes; Parallel Reaction Monitoring (PRM) was performed in positive mode.

For Acetyl-CoA: an Accucore C18 column (2.1 mm x 100 mm, 2.6µm particle size, Thermo Fisher Scientific, San Jose, CA) was used; the mobile phase A was 5mM dibutyl ammonium acetate (Sigma-Aldrich) and ammonium acetate pH = 9.0 (Sigma-Aldrich), and the mobile phase B was 100% methanol with 0.1% formic acid; the total running time was 9 minutes; full MS1 was performed in negative mode. For all the analysis, the column temperature was set to 30°C, and the injection volume was 2 µL. Peak heights were determined using Skyline (version 19.1). For Acyl Carnitines and Acetyl CoA the amounts (in µg) were calculated using the peak height ratio of each molecule to its respective internal standard. For Acyl CoA, only peak heights obtained from Skyline were reported due to the unavailability of internal standards during the analysis.

### Enrichment analysis

For gene set enrichment analysis, the upregulated gene list in *Sirt2*^{*-*/*-*} T cells *versus* WT T cells was uploaded to MSigDB (Subramanian, A., et al. (2005). Proc Natl Acad Sci U S A 102:15545-15550) and the overlap with the HALLMARK gene sets was calculated (Liberzon, A., et al. (2015). Cell Syst 1:417-425). A false discovery rate q-value of < 0.001 was used as cut-off.

The web-based tool MSEA (metabolite set enrichment analysis) which is incorporated into MetaboAnalyst 4.0 platform (Xia and Wishart, 2010) (https://www.metaboanalyst.ca/) was used to perform metabolite enrichment. Data for increased metabolites in *Sirt2*^{*-*/*-*} T cells were submitted into MSEA. Overrepresentation analysis was the selected enrichment analysis method. The overlap with pathway-associated metabolites set library was implemented using the hypergeometric test. One-tailed *P* values were provided after adjusting for multiple testing. A false discovery rate q-value < 0.15 was used as a cutoff.

### Statistical Analysis

Statistical parameters can be found in the figure legends. *P* values were calculated by two-tailed unpaired or paired Student's t-test, one-way ANOVA or two-way ANOVA as indicated using GraphPad Prism 7.0. In all cases, statistical significance was considered when *P* <0.05. Graphs show mean values and error bars represent the standard error of the mean (SEM). *P* values were represented as follows: *P* < 0.05, ***P* < 0.01, ****P* < 0.001, *****P* < 0.0001.

### Data and Software

The mass spectrometry proteomics data have been deposited to the ProteomeXchange Consortium via the PRIDE (Vizcaino, J.A., et al. (2014). Nat Biotechnol 32:223-226) partner repository with the dataset identifier PXD012811 for Sirt2 interacting proteins and PXD012796 for lysine acetylation. The Metabolomics data are in the process of submission to the Metabolomics Workbench (Sud, M., t al. (2016). Nucleic Acids Res 44:D463-470). The RNA-sequencing data set generated during this study are in the process of submission in the Gene Expression Omnibus.

### Results

### Sirt2 Expression is Induced in TILs

Because Sirt2 expression is known to be stimulated in response to caloric-restriction (Gomes, P., et al. (2015). Trends Pharmacol Sci 36:756-768; Jiang, W., et al. (2011). Mol Cell 43:33-44), metabolic restriction within the TME may induce Sirt2 expression. In this context, matching TILs and peripheral blood mononuclear cells (PBMC) human samples from 11 patients enrolled into a phase I clinical trial of Nivolumab and TIL therapy in advanced NSCLC (Creelan, B., et al. (2018). Journal of Thoracic Oncology 13) were analyzed for Sirt2 expression by flow cytometry (Figure 23A). Most TILs demonstrated a significant increase in Sirt2 expression when compared to the corresponding peripheral T cells (Figures 16A, 16B and 23B). Strikingly, Sirt2 upregulation in CD8⁺ TILs *versus* PBMC was not observed in 3 patients who achieved partial response to the TIL therapy. Conversely, 6 patients who failed the TIL therapy demonstrated a significant increase in Sirt2 levels in TILs. The clinical response was not evaluable in the remaining 3 patients (Figures 16C and 23B).

The next goal was to confirm these findings in a mouse model. Sirt2 expression was upregulated in CD8⁺ TILs isolated from subcutaneous B16F10 nodules *versus* splenic CD8⁺ T cells (Figure 16D). Sirt2 expression was also upregulated upon *ex vivo* stimulation of T cells from spleens and LN, and enriched CD4⁺ T cells or CD8⁺ T cells from C57BL/6 mice (Figures 16E-16G). Thus, Sirt2 expression is dynamically regulated during T cell activation and modulated by the TME.

### Sirt2 Deficient T Cells are Hyper-Reactive

Since Sirt2 expression is induced upon antigenic stimulation in T cells, the next question was whether Sirt2 contributes to T cell functions. To characterize the impact of Sirt2 deficiency on T cell functions *ex vivo,* Sirt2-wild-type (WT) *versus* Sirt2-knockout (*Sirt2*^{*-*/*-*}) Pmel melanocyte-antigen-specific T cell receptor (TCR)-transgenic T cells were stimulated with melanocyte-specific gp100 peptide antigen *ex vivo.* These studies revealed that *Sirt2*^{*-*/*-*} Pmel T cells had increased proliferation and interferon-γ (IFN-γ) release following antigenic challenge compared to WT Pmel T cells (Figures 16H and 16I). Further, intracellular staining showed increases in granzyme B and IFN-γ expression in *Sirt2*^{*-*/*-*} Pmel T cells compared to WT Pmel T cells after gp100 stimulation (Figures 16J-16L). This hyper-reactive phenotype of *Sirt2*^{*-*/*-*} Pmel T cells led to enhanced cytotoxic activity against B16F10 cell challenge *ex vivo* (Figure 16M). More importantly, when TILs isolated from subcutaneous B16F10 nodules from WT and *Sirt2*^{*-*/*-*} mice were re-challenged *ex vivo* with B16F10 cells, *Sirt2*^{*-*/*-*} TILs demonstrated superior effector functions compared to WT TILs as measured by IFN-γ release and cytotoxic activity (Figures 16N and 16O).

### Sirt2 Targets the Glycolytic and TCA Pathways in T Cells

To gain insight into the molecular mechanism underlying the hyper-reactivity of *Sirt2*^{*-*/*-*} T cells, we sought to identify potential Sirt2 interaction partners that regulate T cell functions. To this end, two independent immunoprecipitation (IP) experiments followed by liquid chromatography-tandem mass spectrometry (LC-MS/MS) analysis were conducted.

First, to identify Sirt2-binding partners, LC-MS/MS analysis was performed on Sirt2-immunoprecipitated proteins from activated WT CD3⁺ T cell protein extracts. This analysis revealed 596 proteins to potentially interact with Sirt2. Interestingly, 8 out of the 10 glycolytic enzymes (Figure 17A): hexokinase 1 (HK1), phosphofructokinase P (PFKP), aldolase A (ALDOA), glyceraldehyde-3-phosphate dehydrogenase (GAPDH), phosphoglycerate kinase 1 (PGK1), enolase 1 (ENO1), pyruvate kinase M (PKM) and lactate dehydrogenase (LDH); and 4 out of the 8 TCA-cycle enzymes (Figure 17B): aconitase 2 (ACO2), oxoglutarate dehydrogenase (OGDH), succinate-CoA ligase alpha subunit (SUCLG1) and succinate dehydrogenase subunit alpha (SDHA) were identified as Sirt2 interacting proteins. Collision-induced dissociation (CID) spectra of selected peptides derived from these proteins are shown in Figure 24. These interactions were confirmed by co-IP/immunoblot (co-IP/IB) (Figures 17C and 17D) and reverse co-IP/IB (Figures 17E and 2F). See input in Figure 25.

Second, to identify hyper-acetylated proteins in *Sirt2*^{*-*/*-*} T cells, LC-MS/MS analysis was performed on acetyl-lysine-immunoprecipitated proteins from WT *versus Sirt2*^{*-*/*-*} CD3⁺ T cells. More than 250 hyper-acetylated proteins were identified in *Sirt2*^{*-*/*-*} T cells compared to WT T cells including aldolase, GAPDH, PGK1, ENO1, PKM, LDH from the glycolytic pathway (Figure 26) and ACO2, isocitrate dehydrogenase, SUCLG, SDHA and malate dehydrogenase from the TCA-cycle (Figure 27) based on the extracted ion chromatogram (EIC) of acetylated peptides.

increased acetylation of PFK, GAPDH, ACO2, OGDH, SDHA and SUCLG1 in *Sirt2*^{*-*/*-*} versus WT T cells was further demonstrated by IP/IB and/or reverse IP/IB while their total proteins (input) were unchanged (Figure 17G and 17H). Taken together, these results demonstrate that the key glycolytic pathway and TCA-cycle enzymes are targets of Sirt2 deacetylase activity.

Accordingly, next investigated was whether the altered acetylation status of the glycolytic and TCA-cycle enzymes in Sirt2 deficient T cells influences their activity. Indeed, enzymatic activities of hexokinase, PFK, aldolase, GAPDH, enolase, ACO2, OGDH, SDH and SUCLG were all elevated in activated *Sirt2*^{*-*/*-*} T cells *versus* WT T cells (Figures 17I and 17J), despite no visible differences in the total protein levels of these enzymes (Figures 28), confirming the post-translational control of their activity by Sirt2.

### Sirt2 Targets FAO and Glutaminolysis in T Cells

β-oxidation is a metabolic pathway that breaks down FA molecules to generate energy. Four enzymatic steps are repeated until all carbons in the fatty acyl-CoA are catabolized into acetyl-CoA, which then enters the TCA cycle. The mitochondrial trifunctional protein (TFP) catalyzes the last three reactions of FAO (Figure 18A). TFP has two subunits: Hydroxyacyl-CoA Dehydrogenase Trifunctional Multienzyme Complex Subunit Alpha (HADHA) and Hydroxyacyl-CoA Dehydrogenase Trifunctional Multienzyme Complex Subunit Beta (HADHB). HADHA comprises the 2,3-enoyl-CoA hydratase and 3-hydroxyacyl-CoA dehydrogenase activities, and HADHB mediates the thiolase activity. Interestingly, our LC-MS/MS analyses revealed HADHA and HADHB as Sirt2 interacting partners (Figure 29A) with hyper-acetylation of HADHB in *Sirt2*^{*-*/*-*} T cells (Figure 29B). These interactions were confirmed by co-IP/IB (Figure 18B). IP/IB also demonstrated hyperacetylation of both HADHA and HADHB subunits in *Sirt2*^{*-*/*-*} T cells compared to WT T cells (Figure 18C) while their total proteins were unchanged (Figure 29C).

Glutaminolysis is an important source of energy for T_{EFF} cells. Glutamine enters cells using the alanine, serine, cysteine-preferring transporter 2 (ASCT2) and it is converted to glutamate by glutaminase (GLS) and to α-ketoglutarate (α-KG) by glutamate dehydrogenase (GDH) which enters the TCA cycle (Figure 18D). Moreover, the LC-MS/MS analyses revealed ASCT2 to bind Sirt2 (Figure 29D) and increased acetylation of GLS and GDH in *Sirt2*^{*-*/*-*} T cells (Figures 29E and 29F). Sirt2 interaction was therefore confirmed with ASCT2 and GLS by co-IP/IB (Figure 18E). Hyper-acetylation of ACST2 and GLS in *Sirt2*^{*-*/*-*} T cells compared to WT T cells was also demonstrated by IP/IB while their total proteins (input) were unchanged (Figure 18F). To address if the acetylation level of these enzymes affects their activity, glutamine consumption and glutamate production were measured in activated WT *versus Sirt2*^{*-*/*-*} CD3⁺ T cells *ex vivo.* Indeed *Sirt2*^{*-*/*-*} T cells showed a significant increase in both glutamine uptake and glutamate production (Figure 18F), while no differences in ASCT2 and GLS protein levels were observed between WT and *Sirt2*^{*-*/*-*} T cells (Figure 29G), confirming the post-translational control of their activities.

Taken together, Sirt2 regulates FAO and glutaminolysis in activated T cells, by targeting and deacetylating the key enzymes of these important metabolic pathways.

### Sirt2 Deficient T Cells are Hypermetabolic

Given that Sirt2 interacts with and modifies the acetylation status of multiple metabolic enzymes, whether *Sirt2*^{*-*/*-*} T cells displayed altered metabolic status during activation was assessed by measuring glycolytic and mitochondrial activities using 3 different assays on the Seahorse Analyzer: the glycolysis stress test, the glycolytic rate assay, and the mitochondrial stress test. Indeed, Sirt2 deficiency increased glycolytic flux evidenced by increased basal, maximal and compensatory glycolysis in activated *Sirt2*^{*-*/*-*} CD3⁺, CD8⁺ and Pmel CD8⁺ T cells when compared to WT controls (Figures 19A, 19B and 30A-30D). Likewise, activated *Sirt2*^{*-*/*-*} CD3⁺ and CD8⁺ T cells displayed increased mitochondrial activity evidenced by increased basal respiration, maximal respiration, and spare respiratory capacity (Figures 19C and S8E).

To explore if Sirt2 deficiency had similar effects on the metabolic status of TILs within the metabolically challenging TME, CD3⁺ TILs were isolated from subcutaneous B16F10 nodules from WT and *Sirt2*^{*-*/*-*} mice, and their metabolic profile was assessed. Notably, *Sirt2*^{*-*/*-*} CD3⁺ TILs displayed increased glycolytic flux and OxPhos compared to WT (Figures 19D-19F).

To investigate if *Sirt2*^{*-*/*-*} T cell hyper-reactivity is dependent on their increased rate of glycolysis and OxPhos, *Sirt2*^{*-*/*-*} and WT T cells were treated with the glycolytic inhibitor 2-deoxyglucose (2-DG) or the OxPhos inhibitor, oligomycin (Figure 30F). As predicted, 2-DG treatment profoundly suppressed IFN-γ release (Figure 30G) and cytotoxicity (Figure 30H) of *Sirt2*^{*-*/*-*} Pmel T cells following gp100-antigenic stimulation and B16F10 challenge respectively. Similarly, 2-DG treatment suppressed IFN-γ release by CD3⁺ TILs that were isolated from subcutaneous B16F10 nodules from *Sirt2*^{*-*/}*⁻ versus* WT mice and subsequently re-challenged with irradiated B16F10 cells *ex vivo* (Figure 30I). In addition, inhibition of OxPhos with oligomycin suppressed IFN-γ release by *Sirt2*^{*-*/*-*} Pmel T cells to the levels exhibited by WT Pmel T cells (Figure 19J). These results are consistent with the prior studies demonstrating the crucial role of aerobic glycolysis and OxPhos during T cell activation (Pearce, E.L., et al. (2013). Science 342:1242454), and suggest that the hyper-reactivity *Sirt2*^{*-*/*-*} T cells is associated with their hypermetabolic phenotype.

OxPhos is fueled by pyruvate, a glycolytic intermediate, acetyl-CoA, the end-product of FAO, or α-KG generated from glutaminolysis. To determine the primary mitochondrial fuel mediating enhanced OxPhos in *Sirt2*^{*-*/*-*} T cells, the mitochondrial activity of activated T cells subjected to selective metabolic pathway inhibitors was measured: UK5099, a mitochondrial pyruvate carrier inhibitor, BPTES, an allosteric inhibitor of GLS, and Etomoxir, a specific inhibitor of carnitine palmitoyl transferase 1A (CPT1A), which transports long-chain FA into the mitochondria (Figure 19G). In activated WT CD3⁺ T cells, the maximal respiration (in response of FCCP) was significantly suppressed by all three inhibitors (UK5099 > BPTES > Etomoxir) indicating that proliferating T cells use all three nutrients to fuel OxPhos with a preference for pyruvate. This is consistent with the concept that activated T cells rely on glycolysis as the primary metabolic program (Figure 19H, upper panel). Surprisingly, *Sirt2*^{*-*/*-*} T cells were insensitive to the inhibitors (Figure 19H, lower panel). Indeed, neither UK5099, BPTES nor Etomoxir were sufficient to disrupt maximal respiration in *Sirt2*^{*-*/*-*} T cells. Such resistance to metabolic inhibitors is consistent with enhanced glycolysis, glutaminolysis and FAO in *Sirt2*^{*-*/*-*} T cells.

Within the metabolically challenging TME, TILs rely on fatty acid catabolism to partially preserve their effector functions due to nutrient competition for glucose and glutamine with tumor cells. Accordingly, the contribution of FA in mitochondrial respiration of WT *versus Sitt2*^{*-*/*-*} TILs isolated from B16F10 tumor nodules was investigated. Interestingly, Etomoxir decreases the maximal respiration of WT TILs, while *Sirt2*^{*-*/*-*} TILs remained resistant to Etomoxir inhibition (Figure 19I).

Recently, long lived T_{M} cells have been shown to synthesize endogenous FA substrates to support FAO and OxPho' (O'Sullivan, D., et al. (2014). Immunity 41:75-88). Whether enhanced FAO in *Sirt2*^{*-*/*-*} T cells is dependent on exogenous or endogenous FA was therefore, investigated. Surprisingly, *Sirt2*^{*-*/*-*} T cells challenged with restricted media did not acquire exogenous FA for respiration. Instead, they displayed significant increase in oxidation of endogenous FA compared to WT T cells (Figure 19J). Utilizing endogenously produced FAO substrates may allow *Sirt2*^{*-*/*-*} T cells to meet energetic demands despite scarce resources within the TME.

T_{M} cells rely on FAO for survival. To assess whether enhanced FAO in *Sirt2*^{*-*/*-*} T cells influences the viability of T_{M} cells, memory T cells were generated *ex vivo* by activating OT-II cells with chicken ovalbumin 323-339 peptide (OVA-peptide) for 3 days followed by IL-15 stimulation for 4 additional days (Figure 19J). Consistent with enhanced FAO with Sirt2 deficiency, *Sirt2*^{*-*/*-*} OT-II T_{M} cells displayed better survival when compared to WT OT-II T_{M} cells (Figure 19K).

### The Hypermetabolic Phenotype of Sirt2 Deficient T Cells is Not Dependent on c-Myc

c-Myc is an important driver of T cell metabolism (Wang et al., 2011). To investigate if the hyper-metabolic phenotype of *Sirt2*^{*-*/*-*} T cells is mediated by c-Myc, RNA-sequencing analysis we performed on naïve and activated WT *versus Sift2*^{*-*/*-*} CD4⁺ T cells. This study revealed more than 800 significant differentially expressed genes (Figure 31A). Consistent with the hyper-reactive phenotype of *Sirt2*^{*-*/-} T cells, pathway enrichment analysis using gene set enrichment analysis (GSEA) platform revealed upregulation of pathways related to T cell effector function and proliferation in *Sirt2*^{*-*/*-*} T cells, most prominently INF-γ response and IL-2 signaling (Figure 31B). However, gene expression profiling did not reveal any significant increase in c-Myc-dependent global metabolic transcriptome between WT and *Sirt2*^{*-*/*-*}T cells (Figure 31C). Further, no significant differences in c-Myc protein expression levels were observed between WT and *Sirt2*^{*-*/*-*} T cells (Figure 31D).

### Sirt2 is a Master Organizer of T Cell Metabolism

To further corroborate the hyper-metabolic phenotype of Sirt2^{-/-} T cells observed with extracellular flux analysis, untargeted metabolomic LC/MS analyses of intracellular metabolite levels of activated WT *versus Sirt2*^{*-*/*-*} CD8⁺ T cells was performed (Figures 20A and 20B). Metabolites set enrichment analysis (MSEA) using the Metaboanalyst tool on the differentially expressed metabolites indicated upregulation of multiple metabolic pathways in *Sirt2*^{*-*/*-*} T cells, most notably the Warburg effect, FA and glutamine metabolisms in accord with the prior extracellular flux and molecular data (Figure 20C).

Indeed, metabolomic analysis illustrates a significant drop in glucose and increase in glucose-6-phosphate levels in *Sirt2*^{*-*/*-*} T cells (Figure 20B), which is consistent with increased glucose catabolism by glycolysis, whereas glucose uptake and glucose transporter (GLUT1) expression were not affected by Sirt2 deficiency in naïve and in activated T cells (Figure 20D). Concomitantly, *Sirt2*^{*-*/*-*} T cells displayed increased levels of early glycolytic intermediates, which appeared to fuel the generation of serine. *Sirt2*^{*-*/*-*} T cells also displayed increased levels of extracellular lactate, confirming preferential pyruvate conversion to lactate.

Despite the increased pyruvate conversion to lactate, *Sirt2*^{*-*/*-*} T cells exhibited increased levels of late TCA-cycle intermediates consistent with increased glutaminolysis and FAO fueling the TCA-cycle in *Sirt2*^{*-*/*-*} T cells. Indeed, *Sirt2*^{*-*/*-*} T cells displayed increased levels of acyl-carnitines, glutamate and glutamine (Figures 20A and 5B). No clear patterns of decreased metabolites were observed in *Sirt2*^{*-*/*-*} T cells, with the exception of decrease in cysteine levels, which may reflect its utilization for nucleic acid metabolism in response to increased proliferation.

Next, a targeted LC-MS/MS analysis of FAO intermediates was conducted in activated WT *versus Sirt2*^{*-*/*-*} CD8⁺ T cells to confirm the effects of Sirt2 deficiency on FA metabolism. As predicted, *Sirt2*^{*-*/*-*} T cells displayed higher levels of acyl-carnitines (C14-C4) concomitantly with increased acetyl-CoA, the end product of FAO, consistent with enhanced FA catabolism in *Sirt2*^{*-*/*-*} T cells (Figure 20E).

CPT1A is the rate limiting factor of FAO pathway, which catalyzes the formation of acyl-carnitines and transports them into the mitochondria. Increases in total acyl-carnitine levels (Figure 20E, pie chart) and mitochondrial resistance to Etomoxir (CPT1A inhibitor) in *Sirt2*^{*-*/*-*} T cells suggest increases in CPT1A expression levels. Indeed, both mRNA and protein expression levels of CPT1A were upregulated in *Sirt2*^{*-*/*-*} T cells (Figures 20F and 20G). Thus, in addition to Sirt2 interaction with TFP, CPT1A upregulation in activated *Sirt2*^{*-*/*-*} T may support increased FAO rate.

Finally, *Sirt2*^{*-*/*-*} T cells displayed higher ATP/ADP ratio reflecting their higher energetic status (Figure 20H).

Taken together, Sirt2 appears to be a master regulator of key metabolic pathways in activated T cells and Sirt2 deficiency dramatically alters T cell metabolism.

### Sirt2 Deficiency Delays Tumor Progression In Vivo

As Sirt2 deficiency endows T cells with superior metabolic fitness and enhanced effector functions *ex vivo,* the *in vivo* effects of Sirt2 deficiency on anti-tumor immune responses was investigated. WT and *Sirt2*^{*-*/*-*} mice were inoculated intravenously (i.v.) or subcutaneously (s.c.) with B16F10 melanoma cells. *Sirt2*^{*-*/*-*}recipient mice transplanted i.v. with B16F10 melanoma cells had markedly reduced number of lung metastases compared to WT recipient mice (Figure 21A, Figure 32A). There was also a significant delay in the growth of s.c. transplanted B16F10 tumors in *Sirt2*^{*-*/*-*} mice *versus* WT mice (Figure 21B, Figure 32B).

Phenotypic characterization of the TILs isolated from subcutaneous B16F10 nodules from WT and *Sirt2*^{*-*/*-*} mice revealed that *Sirt2*^{*-*/*-*} TILs had increased levels of CD8⁺ effector memory T cells (T_{EM}) (Figures 21C and 33) and PD-1 (Programmed cell death-1 receptor) expression compared to WT TILs (Figure 21D). Similarly, there was a pronounced increase in T_{EFF}, T_{EM} and iNKT cells in the secondary lymphoid organs of *Sirt2*^{*-*/*-*} mice upon i.v. tumor challenge whereas no qualitative or quantitative phenotypic differences were observed between unchallenged WT and *Sirt2*^{*-*/*-*} control mice (Figures 34, 35). Interestingly, a significant increase in CD27⁻ T_{EFF} and T_{EM} was observed in *Sirt2*^{*-*/*-*} mice upon i.v. B16F10 tumor challenge (Figure 36). CD27 is constitutively expressed on most T cells. Notably, a decrease in tumor growth was reported in CD27 knockout mice and targeting CD27 using anti-CD27 mAb enhanced CD8⁺ T cell responses and antitumor activity (Wasiuk, A., et al. (2017). J Immunol 199:4110-4123).

Because immune phenotypic changes were observed mostly in T cell subsets, the main effectors against tumor, the next goal was to confirm whether enhanced anti-tumor activity in *Sirt2*^{*-*/*-*} mice depended on T cells. Depletion of either CD8⁺ or CD4⁺ T cells abrogated superior tumor control in *Sirt2*^{*-*/*-*} mice suggesting that the protective effect of Sirt2 deficiency relied on both T cell subsets (Figure 21E, Figure 37A). Notably, adoptive transfer of *Sirt2*^{*-*/*-*} Pmel CD8⁺ T cells into mmunemunodeficient NOD scid gamma (NSG) mice, significantly reduced lung metastases after i.v. B16F10 tumor challenge *versus* adoptive transfer of WT Pmel CD8⁺ T cells (Figure 6F, Figure 37B). NSG mouse model was used in order to mimic the lympho-depleting chemotherapy treatment prior adoptive cell therapy (ACT) in human protocols (Dudley, M.E., et al. (2013). J Clin Oncol 31:2152-2159). Thus, Sirt2 deficiency endows T cells with a superior anti-tumor phenotype in a model of ACT.

PD-1/PD-L1 (Programmed cell death ligand-1) blockade represents the most efficacious cancer immunotherapy to date and forms the backbone of the majority of combination immunotherapy strategies. WT and *Sirt2*^{*-*/*-*} mice were challenged with B16F10 cells i.v. and then treated with anti-PD-1 mAb *versus* isotype control mAb to determine if Sirt2 deficiency augments the efficacy of PD-1/PD-L1 blockade. Notably, these studies revealed a superior anti-PD-1 therapeutic response in *Sirt2*^{*-*/*-*} recipient mice (Figure 21G). Thus, Sirt2 deficiency also augments the anti-PD-1 immune checkpoint therapy.

### Sirt2 is an Actionable Target to Augment the Anti-Tumor Activity of Human T Cells

To extend these findings beyond mouse models, the effects of Sirt2 inhibition were examined in human T cells using 2 different Sirt2 inhibitors: AGK2 and Thiomyristl(TM) (Jing, H., et al. (2016). Cancer Cell 29:767-768; Newton, K., et al. (2014). Nature 506:E4-6). Consistent with murine studies, AGK2 and TM treatment of human CD3⁺ T cells (obtained from PBMC from healthy donors) increased aerobic glycolysis, OxPhos and IFN-γ production (Figure 37). The Sirt2 inhibitors were validated by monitoring acetylated α-tubulin levels (Figures 37E and 37J), a well-established Sirt2 substrate (North, B.J., et al. (2003). Mol Cell 11:437-444). To assess the translational potential of Sirt2 blockade, the effects of AGK2 on human TILs isolated from NSCLC patient tumor samples were investigated. Markedly, TILs from 5 NSCLC patients treated with AGK2 exhibited increased aerobic glycolysis, OxPhos and INF-γ production (Figure 22).

### Discussion

Although cancer immunotherapies targeting immune checkpoints have redefined cancer treatment, several cancers remain resistant and new strategies are urgently needed (Gide, T.N., et al. (2018). Clin Cancer Res 24:1260-1270; Pardoll, D.M. (2012). Nature reviews 12: 252-264; Wei, S.C., et al. (2018). Cancer Discov 8:1069-1086). An attractive strategy to augment anti-tumor immune responses comes from the emerging field of immune metabolism (Chang, C.H., et al. (2015). Cell 162:1229-1241; Shevchenko, I., and Bazhin, A.V. (2018). Frontiers in immunology 9:1816). Despite the growing consensus that the metabolic status of the immune cells drives their key functions and maturation, actionable targets to implement metabolic changes have been elusive. The NAD⁺-dependent histone deacetylase, Sirt2, has long been a topic of debate due to conflicting reports on its dual tumor-suppressor and oncogenic roles (Jing, H., et al. (2016). Cancer Cell 29:767-768; McGlynn, L.M., et al. (2014). Eur J Cancer 50:290-301). Herein we show that Sirt2 is a metabolic immune checkpoint amenable to therapeutic targeting. Strikingly, our results reveal Sirt2 as a master regulator of T cell metabolism. Its broad involvement in regulating multiple metabolic pathways that are crucial to T cell effector functions is exemplified by the profound hypermetabolic phenotype in *Sirt2*^{*-*/*-*}T cells.

Mechanistically, Sirt2 regulation of T cell metabolism is independent of c-Myc, a global modulator of T cell metabolism via transcriptional control of diverse metabolic genes (Wang, R., et al. (2011). Immunity 35:871-882). Instead, Sirt2 directly impacts the acetylation status and activity of key metabolic enzymes for glycolysis, TCA-cycle, FAO and glutaminolysis. While Sirt2 regulation of the glycolytic enzymes has not been previously described in the immune system, prior studies in induced human pluripotent stem cells (Cha, Y., et al. (2017). Nat Cell Biol 19:445-456) and HeLa cells (Park, S.H., et al. (2016). Cancer Res 76:3802-3812) also demonstrated the interaction between Sirt2 and the glycolytic enzymes complementing our study. Interestingly, Sirt2 interaction with the TCA-cycle, FAO and glutaminolysis enzymes has not been previously reported.

Among the glycolytic targets identified as Sirt2 deacetylase targets, GAPDH and ENO1 have been directly implicated in T cell functions (Balmer, M.L., et al. (2016). Immunity 44:1312-1324; Chang, C.H., et al. (2013). Cell 153:1239-1251; Gemta, L.F., et al. (2019). Science immunology 4). GAPDH is known to directly bind IFN-γ mRNA and block its translation (Chang, C.H., et al. (2013). Cell 153:1239-1251). Interestingly, a recent study showed that GAPDH acetylation increases its enzymatic activity and decreases its binding to the IFN-γ mRNA, which enhances the glycolytic flux and IFN-γ production (Balmer, M.L., et al. (2016). Immunity 44:1312-1324) consistent with the effects of Sirt2 deficiency. Notably, a prior study demonstrated down-regulation of ENO1 activity in human and murine melanoma TILs leading to metabolically compromised TILs with suppressed effector functions, without concomitant changes in mRNA and protein expression levels. Although the precise mechanism was not identified, authors concluded that the enzymatic activity of ENO1 is likely post-translationally regulated in TILs (Gemta, L.F., et al. (2019). Science immunology 4). Because Sirt2 is profoundly upregulated in TILs, the defective activity of ENO1 in TILs may be attributable to Sirt2 deacetylase activity.

TILs are often subjected to glucose-deprivation and hypoxia while competing for available nutrients with cancer cells within the TME (Chang, C.H., et al. (2015). Cell 162:1229-1241; Ho, P.C., et al. (2014). Cancer Res 74:3205-3217; Ying, H., et al. (2012). Cell 149:656-670). Although the precise mechanism of Sirt2 upregulation in TILs remains elusive, Sirt2 may be induced by caloric restriction (Gomes, P., et al. (2015). Trends Pharmacol Sci 36:756-768; Jiang, W., et al. (2011). Mol Cell 43:33-44), and thus the metabolic constraints of the TME. Strikingly, upregulation of Sirt2 in TILs is associated with a poor clinical response to immunotherapy in a phase I clinical trial in NSCLC. Sirt2 may therefore function as a metabolic immune checkpoint that promotes a dampened metabolic phenotype in T cells to adapt to the metabolically restricted TME.

As a consequence of their hyper-metabolic phenotype, *Sirt2*^{*-*/*-*} T cells display increased proliferation and effector functions and thus efficiently reject transplantable tumors *in vivo.* Consistent with these findings, the gene expression profiling of *Sirt2*^{*-*/*-*} T cells reveals upregulation of key pathways involved in T cell effector function and proliferation, most prominently, INF-γ response and IL-2 signaling. Notably, there are increases in T_{EM} cells among *Sirt2*^{*-*/*-*} TILs as well as in the peripheral lymphoid organs of *Sirt2*^{*-*/*-*} mice upon tumor challenge, which is consistent with the superior anti-tumor immunity observed in *Sirt2*^{*-*/*-*} mice. T_{EM} cells play an essential role in tumor-specific immune responses (Mami-Chouaib, F., et al. (2018). J Immunother Cancer 6:87) and their high frequency in tumors correlates with favorable prognosis in cancer patients (Pages, F., et al. (2005). The New England journal of medicine 353:2654-2666). In addition, PD-1 expression is increased in *Sirt2*^{*-*/*-*} TILs and its blockade further augments the protective effect of Sirt2 deficiency against tumor challenge. PD-1, a major inhibitory immune checkpoint, is paradoxically a marker of activated tumor-reactive CD8⁺ T cells in melanoma, and tumor regression with PD-1 blockade requires pre-existing PD-1⁺ CD8⁺ T cells (Tumeh, P.C., et al. (2014). Nature 515:568-571). Taken together, it is proposed that the hyper-metabolic phenotype of *Sirt2*^{*-*/*-*} T cells is responsible, at least in part, for the superior effector function against tumor challenge within the TME.

Enhanced glycolysis in *Sirt2*^{*-*/*-*} T cells allows optimal effector functions within the glucose-rich environment. Interestingly, increased serine metabolism in activated *Sirt2*^{*-*/*-*} T cells was observed, which likely reflects increased glycolytic flux as serine is neo-synthesized from the glycolytic intermediate 3-phosphoglycerate (Ma et al., 2017). Serine plays an important role in proliferating T cells by supplying glycine for *de novo* nucleotide synthesis.

However, excess glycolytic activity by cancer cells often depletes glucose within the TME thus impairing CD8⁺ TIL functions (Chang, C.H., et al. (2015). Cell 162:1229-1241; Ying, H., et al. (2012). Cell 149:656-670). In this context, TILs require alternate fuel sources to meet their metabolic demands via OxPhos. In this study, Sirt2 acted as a brake not only on glycolysis, but also on other key metabolic pathways, notably glutaminolysis and FAO. Therefore, the superior effector functions of *Sirt2*^{*-*/*-*} TILs may be a consequence of enhanced glutaminolysis and FA catabolism, thus conferring resistance to the nutritional constraints within the TME via OxPhos. Despite relative hypoxia within the TME, heterogeneity in oxygen levels in the tumor allows TILs to employ OxPhos to preserve their effector functions (Zhang, Y., et al. (2017). Cancer Cell 32:77-391 e379).

More specifically, glutamine is critical for T effector functions and supports their metabolism under glucose limiting conditions (Carr, E.L., et al. (2010). J Immunol 185:1037-1044; Cluntun, A.A., et al. (2017). Trends Cancer 3:169-180; Johnson, M.O., et al. (2018). Cell 175:1780-1795 e1719). However, upon progression, cancer cells become addicted to glutamine, thus in addition to glucose deprivation, T cells are subjected to glutamine competition in the TME limiting their anti-tumor functions (Cluntun, A.A., et al. (2017). Trends Cancer 3:169-180). Therefore, increased glutamine uptake and glutaminolysis in *Sirt2*^{*-*/*-*} T cells may confer resistance to decreased glutamine within the TME.

Notably, CD8⁺ TILs have been shown to be critically dependent on FAO within the hypoglycemic TME, and promoting FA catabolism improves CD8⁺ TIL effector functions (Zhang, Y., et al. (2017). Cancer Cell 32:77-391 e379). Interestingly, most TILs adopt a T_{M} phenotype, which use FAO to persist and to mediate protective immunity (van der Windt, G.J., et al. (2013). Proc Natl Acad Sci U S A 110:14336-14341). Therefore, improved survival of *Sirt2*^{*-*/*-*} T_{M} cells with enhanced FAO may contribute to superior anti-tumor immunity observed in *Sirt2*^{*-*/*-*} mice. Interestingly, *Sirt2*^{*-*/*-*} T cells displayed increased endogenous FAO during activation, thus adopting the metabolic phenotype of T_{M} cells which are primarily dependent on the endogenous source 'f FA (O'Sullivan, D., et al. (2014). Immunity 41:75-88). Although the mechanism of this unique phenotype is not well understood, utilization of immediately available endogenous FA by T_{M} cells may be related to their capacity for rapid recall responses as well as their survival within the TME where exogenous FA may not be readily available.

In addition to contributing to energy production, enhanced FA catabolism *Sirt2*^{*-*/*-*} T cells may also interact with and augment other metabolic pathways by supplying acetyl-CoA for acetylation of key glycolytic and TCA-cycle enzymes and thus enhance their activities (Balmer, M.L., et al. (2016). Immunity 44:1312-1324)(Balmer et al., 2016).

Several metabolic regulators, transcription factors, enzymes, and metabolites, such as c-Myc, HIF-1α, LKB1/STK11, GAPDH, ENO1 and phosphoenol pyruvate, were described as critical metabolic checkpoints in immune homeostasis and anti-tumor immunity (Chang, C.H., et al. (2013). Cell 153:1239-1251; Doedens, A.L., et al. (2013). Nature immunology 14:1173-1182; Gemta, L.F., et al. (2019). Science immunology 4; He, N., et al. (2017). Proc Natl Acad Sci U S A 114:12542-12547; Ho, P.C., et al. (2015). Cell 162:1217-1228; Wang, R., et al. (2011). Immunity 35:871-882). Therefore, manipulating those metabolic regulators to augment T cell metabolism is an attractive strategy to improve immune therapies. However, targeting these metabolic checkpoints has proven difficult due to hurdles in generating selective drugs, or unintended toxicities. In this regard, Sirt2 blockade using selective inhibitors offers a unique opportunity to manipulate T cell metabolism and augment cancer immunotherapy.

Notably, Sirt2 blockade to metabolically reprogram TILs appears to be an effective strategy that synergizes with PD-1 blockade. Therefore, pharmacologic inhibition of Sirt2 in combination with PD-1 blockers may be an effective combination immunotherapy strategy. However, Sirt2 inhibitors may have an unintended consequence of promoting tumor metabolism. Interestingly, contrary to primary cells, in c-Myc dependent tumors, Sirt2 inhibition has been shown to have anti-tumor activity by degradation of c-Myc (Jing, H., et al. (2016). Cancer Cell 29:767-768; McGlynn, L.M., et al. (2014). Eur J Cancer 50:290-301). As c-Myc is a positive regulator of tumor metabolism, promotion of tumor metabolism by Sirt2 inhibition may be avoided by careful patient selection.

Alternatively, manipulation of Sirt2 could be achieved by gene editing during TIL expansion or chimeric antigen receptor (CAR) T-cell generation *ex vivo* thus selectively targeting the T cells (Lim, W.A., and June, C.H. (2017). Cell 168:724-740; Rosenberg, S.A., and Restifo, N.P. (2015). Science 348:62-68). The poor clinical response to TIL therapy observed in NSCLC patients with elevated Sirt2 levels in TILs underscores that Sirt2 blockade may augment the potency and persistence of TILs or CAR T-cells by improving their metabolic fitness.

In summary, Sirt2 inhibition enhances tumor-specific T cell responses by promoting metabolic reprogramming towards a profound hyper-metabolic state with enhanced capacity for aerobic glycolysis, glutaminolysis and FAO in murine and human T cells, thus revealing a striking role for Sirt2 as a metabolic immune checkpoint. Importantly, Sirt2 is an actionable target via pharmacologic or genetic strategies, pointing to tractable means to improve a broad spectrum of cancer immunotherapies.

Unless defined otherwise, all technical and scientific terms used herein have the same meanings as commonly understood by one of skill in the art to which the disclosed invention belongs. Reference should be made to the publications cited herein and the materials for which they are cited.

Those skilled in the art will recognize, or be able to ascertain using no more than routine experimentation, many equivalents to the specific embodiments of the invention described herein, encompassed by the following claims.

## Claims

1. A method comprising
treating lymphocytes collected from a subject with cancer with a Sirt2 inhibitor or genetically modifying the lymphocytes to inhibit or ablate Sirt2 expression.

2. The method of claim 1, wherein the Sirt2 inhibitor is AGK2, AK-1, SirReal2, Tenovin-6, Thiomyristoyl, AEM1, or AEM2.

3. The method of claim 1, wherein the Sirt2 inhibitor is an siRNA, antisense, or gRNA oligonucleotide.

4. The method of claim 1, wherein the lymphocytes are genetically modified by inserting a chimeric receptor into the genome of the cell at a location that disrupts expression or activity of an endogenous Sirt2 protein.

5. The method cell of claim 4, wherein the chimeric receptor is a chimeric antigen receptor (CAR) polypeptide.

6. The method of any one of claims 1 to 5, wherein the lymphocytes are tumor infiltrating lymphocytes (TILs).

## Patentansprüche

1. Verfahren, umfassend
Behandeln von einem Patienten mit Krebs entnommenen Lymphozyten mit einem Sirt2-Inhibitor oder gentechnisches Verändern der Lymphozyten zur Hemmung oder Ablation von Sirt2-Expression.

2. Verfahren nach Anspruch 1, wobei es sich bei dem Sirt2-Inhibitor um AGK2, AK-1, SirReal2, Tenovin-6, Thiomyristoyl, AEM1 oder AEM2 handelt.

3. Verfahren nach Anspruch 1, wobei es sich bei dem Sirt2-Inhibitor um ein siRNA-, Antisense- oder gRNA-Oligonukleotid handelt.

4. Verfahren nach Anspruch 1, wobei die Lymphozyten gentechnisch verändert werden, indem ein chimärer Rezeptor in das Genom der Zelle an einem 0rt eingefügt wird, wodurch die Expression oder Aktivität eines endogenen Sirt2-Proteins gestört wird.

5. Verfahrenszelle nach Anspruch 4, wobei es sich bei dem chimären Rezeptor um ein CAR(Chimeric Antigen Receptor)-Polypeptid handelt.

6. Verfahren nach einem der Ansprüche 1 bis 5, wobei es sich bei den Lymphozyten um tumorinfiltrierende Lymphozyten (TIL) handelt.

## Revendications

1. Procédé comprenant
un traitement de lymphocytes collectés d'un sujet atteint d'un cancer avec un inhibiteur de Sirt2 ou d'une modification génétique des lymphocytes pour inhiber ou ablater l'expression de Sirt2.

2. Procédé selon la revendication 1, dans lequel l'inhibiteur de Sirt2 est AGK2, AK-1, SirReal2, Tenovin-6, Thiomyristoyle, AEM1, ou AEM2.

3. Procédé selon la revendication 1, dans lequel l'inhibiteur de Sirt2 est un oligonucléotide ARNsi, antisens ou ARNg.

4. Procédé selon la revendication 1, dans lequel les lymphocytes sont génétiquement modifiés en insérant un récepteur chimérique dans le génome de la cellule à un emplacement qui perturbe l'expression ou l'activité d'une protéine Sirt2 endogène.

5. Cellule de procédé selon la revendication 4, dans laquelle le récepteur chimérique est un polypeptide récepteur antigénique chimérique (CAR).

6. Procédé selon l'une quelconque des revendications 1 à 5, dans lequel les lymphocytes sont des lymphocytes infiltrant la tumeur (TIL).
